# EUROPEAN PATENT SPECIFICATION

(11) **EP 0 975 588 B1**
(45) Date of publication and mention of the grant of the patent: **03.09.2003**
(21) Application number: 98913173.5
(22) Date of filing: 26.03.1998
(51) Int. Cl.: C07C 271/22, C07C 333/04, C07D 213/30, C07D 339/06, C07D 339/08, C07D 261/18, C07D 209/08, A61K 31/325, A61K 31/44, C07C 271/34

(54) **ANTIPICORNAVIRAL COMPOUDS, COMPOSITIONS CONTAINING THEM, AND METHODS FOR THEIR USE**
GEGEN PICORNA-VIREN GERICHTETE VERBINDUNGEN, ZUSAMMENSETZUNGEN DIE DIESE VERBINDUNGEN ENTHALTEN UND METHODEN ZU IHRER ANWENDUNG
COMPOSES ANTI-PICORNAVIRUS, COMPOSITIONS CONTENANT CES COMPOSES ET LEURS PROCEDES D'UTILISATION

(30) Priority: 28.03.1997 US 825331; 12.05.1997 US 46204 P; 16.12.1997 US 991282
(43) Date of publication of application: 02.02.2000
(73) Proprietor: AGOURON PHARMACEUTICALS, INC., La Jolla, CA 92037 (US)
(72) Inventor: DRAGOVICH, Peter, S., Encinitas, CA 92024 (US); PRINS, Thomas, J., Cardiff, CA 92007 (US); ZHOU, Ru, Carlsbad, CA 92008 (US)
(74) Representative: Grünecker, Kinkeldey, Stockmair & Schwanhäusser Anwaltssozietät
(86) International application number: US9806018
(87) International publication number: WO98043950

(56) References cited:
- EP-A- 0 201 844
- US-A- 5 344 957
- P. WIPF ET AL : "Sn2'-Reactions of peptide aziridines. A cuprate-based approach to (E)-alkene isosteres" JOURNAL OF ORGANIC CHEMISTRY, vol. 59, no. 17, 26 August 1994, pages 4875-4886, XP002072014 EASTON US
- N. MOSS ET AL : "Peptidominetic inhibitors of herpes simplex virus ribonucleotide reductase with improved in Vivo antiviral activity" JOURNAL OF MEDICINAL CHEMISTRY, vol. 39, 1996, pages 4173-4180, XP002072015 WASHINGTON US

## Description

The invention pertains to the discovery and use of new compounds that inhibit the enzymatic activity of picornaviral 3C professes, specifically rhinovirus proteases (RVPs), as well as retard viral growth in cell culture.

The picornaviruses are a family of tiny non-enveloped positive stranded RNA containing viruses that infect humans and other animals. These viruses include the human rhinoviruses, human polioviruses, human coxsackieviruses, human echoviruses, human and bovine enteroviruses, encephalomyocarditis viruses, menigovirus, foot and mouth viruses, hepatitis A virus. The human rhinoviruses are a major cause of the common cold. To date, there are no effective therapies to cure the common cold, only treatments that relieve the symptoms.

Picornaviral infections may be treated by inhibiting the proteolytic 3C enzymes. These enzymes are required for the natural maturation of the picornaviruses. They are responsible for the autocatalytic cleavage of the genomic, large polyprotein into the essential viral proteins. Members of the 3C protease family are cysteine proteases, where the sulfhydryl group most often cleaves the glutamine-glycine amide bond. Inhibition of 3C proteases is believed to block proteolytic cleavage of the polyprotein, which in turn can retard the maturation and replication of the viruses by interfering with viral particle production.

Wipf et al., *J. Org. Chem*, **1994,** 59, 4875-4886, discloses S_{N}2'-reactions of peptide aziridines to obtain (*E*)-alkene dipeptide isosteres.
The object of the present invention is to provide selective small molecules that inhibit the processing of the cysteine protease and are specifically recognised, to treat and cure picornaviral infections, and in particular, the common cold.

The present invention is directed to compounds that function as picornaviral 3C protease inhibitors, particularly those that have antiviral activity. It is further directed to the use of such 3C protease inhibitors. The Inventors demonstrate that the compounds of the present invention bind to rhinovirus 3C proteases and preferably have antiviral cell culture activity. The enzymatic inhibition assays used reveal that these compounds can bind irreversibly, and the cell culture assays demonstrate that these compounds can possess antiviral activity.

Said object is achieved by a compound of the formula (I): 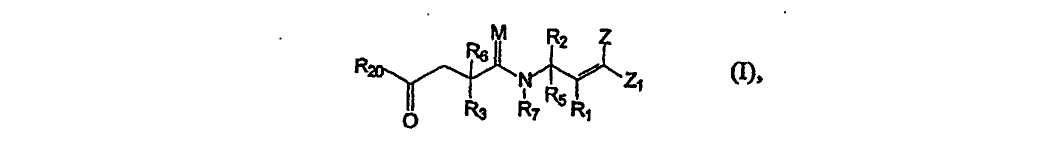 wherein:
M is O or S;
R₁ is H, F, an alkyl group, OH, SH, or an O-alkyl group;
R₂ and R₅ are independently selected from H, 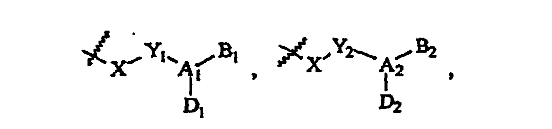 or an alkyl group, wherein said alkyl group is different from 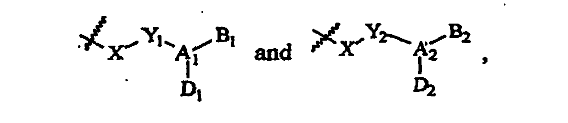 with the proviso that at least one of R₂ or R₅ must be 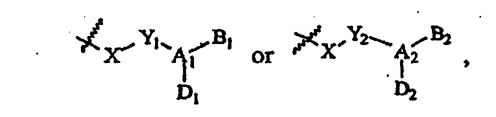
and wherein, when R₂ or R₅ is 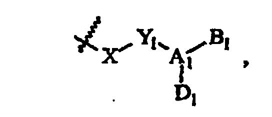
   X is =CH or =CF and Y₁ is =CH or =CF,
   or X and Y₁ together with Q' form a three-membered ring in which Q' is -C(R₁₀)(R₁₁)- or -O-, X is -CH- or -CF-, and Y₁ is -CH-, -CF-, or -C(alkyl)-, where R₁₀ and R₁₁ independently are H, a halogen, or an alkyl group, or, together with the carbon atom to which they are attached, form a cycloalkyl group or a heterocycloalkyl group,
   or X is -CH₂-, -CF₂-, -CHF-, or -S-, and Y₁ is -O-, -S-, -NR₁₂-, -C(R₁₃)(R₁₄)-, -C(O)-, -C(S)-, or -C(CR₁₃R₁₄)-,
      wherein R₁₂ is H or alkyl, and R₁₃ and R₁₄ independently are H, F, or an alkyl group, or, together with the atoms to which they are bonded, form a cycloalkyl group or a heterocycloalkyl group;
   A₁ is C, CH, CF, S, P, Se, N, NR₁₅, S(O), Se(O), P-OR₁₅, or P-NR₁₅R₁₆,
      wherein R₁₅ and R₁₆ independently are an alkyl group, a cycloalkyl group, a heterocycloalkyl group, an aryl group, or a heteroaryl group, or, together with the atom to which they are bonded, form a heterocycloalkyl group;
   D₁ is a moiety with a lone pair of electrons capable of forming a hydrogen bond; and
   B₁ is H, F, an alkyl group, a cycloalkyl group, a heterocycloalkyl group, an aryl group, a heteroaryl group, -OR₁₇, -SR₁₇, -NR₁₇R₁₈, -NR₁₉NR₁₇R₁₈, or -NR₁₇OR₁₈,
      wherein R₁₇, R₁₈, and R₁₉ independently are H, an alkyl group, a cycloalkyl group, a heterocycloalkyl group, an aryl group, a heteroaryl group, or an acyl group;
   and with the provisos that when D₁ is the moiety ≡N with a lone pair of electrons capable of forming a hydrogen bond, B₁ does not exist; and when A₁ is an sp³ carbon, B₁ is not -NR₁₇R₁₈ when D₁ is the moiety -NR₂₅R₂₆ with a lone pair of electrons capable of forming a hydrogen bond, wherein R₂₅ and R₂₆ are independently H, an alkyl group, a cycloalkyl group, a heterocycloalkyl group, an aryl group, or a heteroaryl group;
   and wherein D₁-A₁-B₁ optionally forms a nitro group where A₁ is N; and further wherein, when R₂ or R₅ is 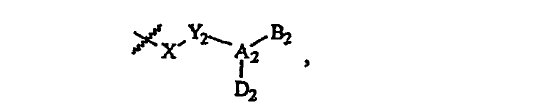
   X is =CH or =CF and Y₂ is =C, =CH, or =CF,
   or X and Y₂ together with Q' form a three-membered ring in which Q' is -C(R₁₀)(R₁₁)- or -O-, X is -CH- or -CF-, and Y₂ is -CH-, -CF-, or -C(alkyl)-, where R₁₀ and R₁₁ independently are H, a halogen, or an allyl group, or, together with the carbon atom to which they are attached, form a cycloalkyl group or a heterocycloalkyl group,
   or X is -CH₂-, -CF₂-, -CHF-, or -S-, and Y₂ is -O-, -S-, -N(R'₁₂)-, -C(O)-, -C(R'₁₃)(R'₁₄)-, -C(S)-, or -C(CR'₁₃R'₁₄)-,
      wherein R'₁₂ is H, an alkyl group, a cycloalkyl group, a heterocycloalkyl group, an aryl group, a heteroaryl group, -OR'₁₃, -NR'₁₃R'₁₄, -C(O)-R'₁₃, -SO₂R'₁₃, or -C(S)R'₁₃, and R'₁₃ and R'₁₄, independently are H, F, or an alkyl group, a cycloalkyl group, a heterocycloalkyl group, an aryl group, or a heteroaryl group, or, together with the atom to which they are attached, form a cycloalkyl group or a heterocycloalkyl group;
   A₂ is C, CH, CF, S, P, Se, N, NR₁₅, S(O), Se(O), P-OR₁₅, or P-NR₁₅R₁₆,
      wherein R₁₅ and R₁₆ independently are an alkyl group, a cycloalkyl group, a heterocycloalkyl group, an aryl group, or a heteroaryl group, or, together with the atom to which they are bonded, form a heterocycloalkyl group;
   D₂ is a moiety with a lone pair of electrons capable of forming a hydrogen bond; and
   B₂ is H, F, an alkyl group, a cycloalkyl group, a heterocycloalkyl group, an aryl group, a heteroaryl group, -OR₁₇, -SR₁₇, NR₁₇R₁₈, -NR₁₉NR₁₇R₁₈, or -NR₁₇OR₁₈,
      wherein R₁₇, R₁₈, and R₁₉ independently are H, an alkyl group, a cycloalkyl group, a heterocycloalkyl group, an aryl group, a heteroaryl group, or an acyl group;
   and further wherein any combination of Y₂, A₂, B₂, and D₂ optionally can form a cycloalkyl group, a heterocycloalkyl group, an aryl group, or a heteroaryl group;
R₃ and R₆ are independently H, F, an alkyl group, a cycloalkyl group, a heterocycloalkyl group, an aryl group, a heteroaryl group, -C(O)R₁₇, -OR₁₇, -SR₁₇, -NR₁₉NR₁₇R₁₈, or -NR₁₇OR₁₈,
   wherein R₁₇, R₁₈, and R₁₉ independently are H, an alkyl group, a cycloalkyl group; a heterocycloalkyl group, an aryl group, a heteroaryl group, or an aryl group;
or R₃ and R₆ together with the carbon atom to which they are attached, form a cycloalkyl group or a heterocycloalkyl group;
R₇ is H, an alkyl group, a cycloalkyl group, a heterocycloalkyl group, an aryl group, a heteroaryl group, -OR₁₇, -SR₁₇, -NR₁₇R₁₈, -NR₁₉NR₁₇R₁₈, or -NR₁₇OR₁₈,
   wherein R₁₇, R₁₈, and R₁₉ independently are H, an alkyl group, a cycloalkyl group, a heterocycloalkyl group, an aryl group, a heteroaryl group, or an acyl group;
or R₇, together with R₃ or R₆ and the atoms to which they are attached, forms a heterocycloalkyl group;
R₂₀ is H, or an hydroxy group, an alkyl group, an oxo group, a cycloalkyl group, an heterocycloalkyl group, an aryl group, a heteroaryl group, an acyl group, a sulfonyl group, a mercapto group, an alkythio group, an alkoxy group, a carboxy group, an amino group, an alkylamino group, a dialkylamino group, a carbamoyl group, an arylthio group, a heteroarylthio group, -OR₁₇, -SR₁₇, NR₁₇R₁₈, -NR₁₉NR₁₇R₁₈ or -NR₁₇OR₁₈, wherein R₁₇, R₁₈ and R₁₉ independently are H, an alkyl group, a cycloalkyl group, a heterocycloalkyl group, and aryl group, a heteroaryl group, or an acyl group; and
Z and Z₁ are independently H, F, an alkyl group, a cycloalkyl group, a heterocycloalkyl group, an aryl group, a heteroaryl group, -C(O)R₂₁, -CO₂R₂₁, -CN, -C(O)NR₂₁,R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), PO(NR₂₁R₂₂)(NR₂₃R₂₄), -C(O)NR₂₁NR₂₂R₂₃, or -C(S)NR₂₁NR₂₂R₂₃,
   wherein R₂₁, R₂₂, R₂₃, and R₂₄ are independently H, an alkyl group, a cycloalkyl group, a heterocycloalkyl group, an aryl group, a heteroaryl group, an acyl group, or a thioacyl group, or wherein any two of R₂₁, R₂₂, R₂₃, and R₂₄, together with the atom(s)
   to which they are bonded, form a heterocycloalkyl group;
or Z₁, as defined above, together with R₁, as defined above, and the atoms to which Z₁
   and R₁ are bonded, form a cycloalkyl or heterocycloalkyl group,
or Z and Z₁, both as defined above, together with the atoms to which they are bonded, form a cycloalkyl or heterocycloalkyl group;
or a pharmaceutically acceptable prodrug, salt, active metabolite, or solvate thereof.

It is preferred that R₁ is H or F, or a pharmaceutically acceptable prodrug, salt, active metabolite, or solvate thereof

It is also preferred that R₂₀ is H, an alkyl group, a cycloalkyl group, a heterocycloalkyl group, an aryl group, a heteroaryl group, -OR₁₇, -SR₁₇, -NR₁₇R₁₈, -NR₁₉NR₁₇R₁₈, or -NR₁₇OR₁₈, wherein R₁₇, R₁₈, and R₁₉ independently are H, an alkyl group, a cycloalkyl group, a heterocycloalkyl group, an aryl group, a heteroaryl group, or an acyl group, or a pharmaceutically acceptable prodrug, salt, active metabolite, or solvate thereof.

It is also preferred that R₂₀ is the alkyl group -C(R₄₁)(R₄₂)NR₄₃R₄₄, wherein:
R₄₁ and R₄₂ independently are H, an alkyl group, a cycloalkyl group, a heterocycloalkyl group, an aryl group, or a heteroaryl group; and
R₄₃ and R₄₄ independently are H, an alkyl group, a cycloalkyl group, a heterocycloalkyl group, an aryl group, a heteroaryl group, -NR₄₅R₄₆, -C(O)R₄₅, -C(S)R₄₅, -C(O)NR₄₅R₄₆, -C(S)NR₄₅R₄₆, -C(O)NR₄₅OR₄₆, -C(S)NR₄₅OR₄₆, -C(O)SR₄₅, -C(O)OR₄₅, -C(S)OR₄₅, -C(S)SR₄₅, -OR₄₅, -SR₄₅, -C(O)NR₄₅NR₄₆R₄₇, -C(S)NR₄₅NR₄₆R₄₇, -SOR₄₅, -SO₂R₄₅, -S(O)NR₄₅R₄₆, -S(O)NR₄₅(OR₄₆), -SO₂NR₄₅R₄₆, -SO₂NR₄₅(OR₄₆), or -SO₃R₄₅,
   wherein R₄₅, R₄₆, and R₄₇ independently are H; an alkyl group, a cycloalkyl group, a heterocycloalkyl group, an aryl group, or a heteroaryl group,
   or wherein any suitable combination of R₄₁, R₄₂, R₄₃, and R₄₄ together form a cycloalkyl group or a heterocycloalkyl group;
or a pharmaceutically acceptable prodrug, salt, active metabolite, or solvate thereof.

It is also preferred that at least one of R₄₃ or R₄₄ is -C(O)SR₄₅ or -C(O)OR₄₅, or a pharmaceutically acceptable prodrug, salt, active metabolite, or solvate thereof.

It is also preferred that R₄₅ is an alkyl group, a cycloalkyl group, an aryl group, a heterocycloalkyl group, or a heteroaryl group, or a pharmaceutically acceptable prodrug, salt, active metabolite, or solvate thereof.

It is also preferred that R₄₅ is a C₁-C₁₀ alkyl group or a cycloalkyl group, or a pharmaceutically acceptable prodrug, salt, active metabolite, or solvate thereof.

It is also preferred that at least one of R₂ or R₅ is 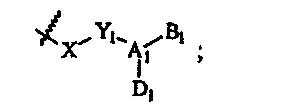 or a pharmaceutically acceptable prodrug, salt, active metabolite, or solvate thereof.

It is also preferred that D₁ is -OR₂₅, =O, =S, ≡N, =NR₂₅, or -NR₂₅R₂₆, wherein R₂₅ and R₂₆ are independently H, an alkyl group, a cycloalkyl group, a heterocycloalkyl group, an aryl group, or a heteroaryl group, or, together with the nitrogen atom to which they are bonded, form a heterocycloalkyl group; or a pharmaceutically acceptable prodrug, salt, active metabolite, or solvate thereof.

It is also preferred that D₁ is =O; or a pharmaceutically acceptable prodrug, salt, active metabolite, or solvate thereof.

It is also preferred that A₁ is C, CH, S, or S(O); or a pharmaceutically acceptable prodrug, salt, active metabolite, or solvate thereof.

It is also preferred that A₁ is C; or a pharmaceutically acceptable prodrug, salt, active metabolite, or solvate thereof.

It is also preferred that B₁ is NR₁₇R₁₈, wherein R₁₇ and R₁₈ are independently H, an alkyl group, a cycloalkyl group, a heterocycloalkyl group, an aryl group, a heteroaryl group, or an acyl group; or a pharmaceutically acceptable prodrug, salt, active metabolite, or solvate thereof.

It is also preferred that at least one of R₂ or R₅ is 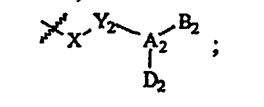 or a pharmaceutically acceptable prodrug, salt, active metabolite, or solvate thereof.

It is also preferred that D₂ is -OR₂₅, =O, =S, ≡N, =NR₂₅, or -NR₂₅R₂₆, wherein R₂₅ and R₂₆ are independently H, an alkyl group, a cycloalkyl group, a heterocycloalkyl group, an aryl group, or a heteroaryl group, or, together with the atom(s) to which they are bonded, form a heterocycloalkyl group; or a pharmaceutically acceptable prodrug, salt, active metabolite, or solvate thereof.

It is also preferred that D₂ is =O; or a pharmaceutically acceptable prodrug, salt, active metabolite, or solvate thereof.

It is also preferred that A₂ is C, CH, S, or S(O); or a pharmaceutically acceptable prodrug, salt, active metabolite, or solvate thereof.

It is also preferred that A₂ is C; or a pharmaceutically acceptable prodrug, salt, active metabolite, or solvate thereof.

It is also preferred that B₂ is -NR₁₇R₁₈, wherein R₁₇ and R₁₈ are independently H, an alkyl group, a cycloalkyl group, a heterocycloalkyl group, an aryl group, a heteroaryl group, or an acyl group; or a pharmaceutically acceptable prodrug, salt, active metabolite, or solvate thereof.

It is also preferred that A₁ is C, CH, S, or S(O) or wherein A₂ is C, CH, S, or S(O); or a pharmaceutically acceptable prodrug, salt, active metabolite, or solvate thereof.

It is further preferred that Z and Z₁ are independently H, an aryl group, or a heteroaryl group, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -C(O)NR₂₁NR₂₂R₂₃, or -C(S)NR₂₁NR₂₂R₂₃;
wherein R₂₁, R₂₂, and R₂₃ are independently H, an alkyl group, a cycloalkyl group, a heterocycloalkyl group, an aryl group, a heteroaryl group, an acyl group, or a thioacyl group, or wherein any two of R₂₁, R₂₂, and R₂₃, together with the atom(s) to which they are bonded, form a heterocycloalkyl group;
or a pharmaceutically acceptable prodrug, salt, active metabolite, or solvate thereof.

It is more preferred that M is O.
Said object is also achieved by a compound having the formula X: 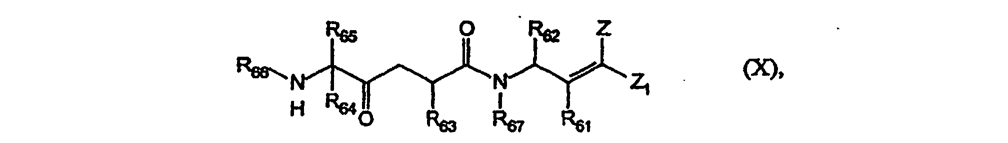 wherein:
R₆₁ is H, F, or an alkyl group;
R₆₂ is selected from one of the following moieties: 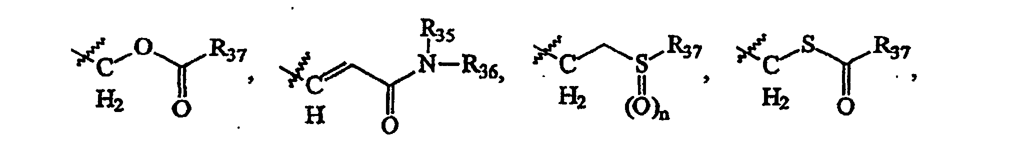 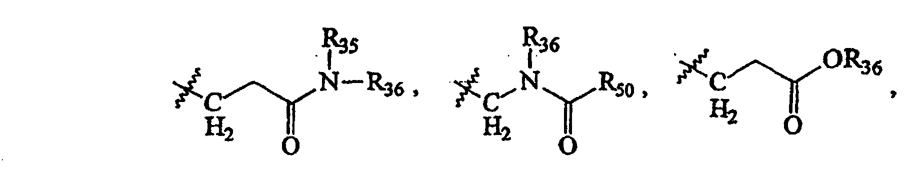 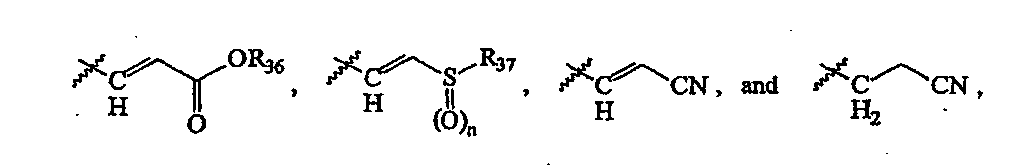 wherein:
   R₃₅ is H, an alkyl group, an aryl group, -OR₃₈, or -NR₃₈R₃₉,
      wherein R₃₈ and R₃₉ independently are H, an alkyl group, a cycloalkyl group, a heterocycloalkyl group, an aryl group, a heteroaryl group, or an acyl group; and
   R₃₆ is H or an alkyl group,
   or R₃₅ and R₃₆, together with the nitrogen atom to which they are attached, form a heterocycloalkyl group or a heteroaryl group;
   R₃₇ is an alkyl group, an aryl group, or -NR₃₈R₃₉, wherein R₃₈ and R₃₉ are as defined above;
   R₅₀ is H, an alkyl group, an aryl group, -OR₃₈, -SR₃₉, -NR₃₈R₃₉, -NR₄₀NR₃₈R₃₉, or -NR₃₈OR₃₉, or R₅₀ and R₃₆, together with the atoms to which they are attached, form a heterocycloalkyl group;
      wherein R₃₈ and R₃₉ are as defined above, and R₄₀ is H, an alkyl group, a cycloalkyl group, a heterocycloalkyl group, an aryl group, a heteroaryl group, or an acyl group; and
n is 0, 1, or 2;
R₆₃ is H or an alkyl group;
R₆₄ is H, an alkyl group, a cycloalkyl group, a heterocycloalkyl group, an aryl group, or a heteroaryl group;
R₆₅ is H or an alkyl group;
R₆₆ is H, an acyl group, an alkyl group, a cycloalkyl group, a heterocycloalkyl group, an aryl group, a sulfonyl group, or a heteroaryl group;
R₆₇ is H or an alkyl group;
   and
Z and Z₁ are independently H, F, an alkyl group, a cycloalkyl group, a heterocycloatkyl group, an aryl group, a heteroaryl group, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, - SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), -PO(NR₂₁R₂₂)(NR₂₃R₂₄), -C(O)NR₂₁NR₂₂R₂₃, or -C(S)NR₂₁NR₂₂R₂₃,
   wherein R₂₁, R₂₂, R₂₃, and R₂₄ are independently H, an alkyl.group, a cycloalkyl group, a heterocycloalkyl group, an aryl group, a heteroaryl group, an acyl group, or a thioacyl group, or wherein any two of R₂₁, R₂₂, R₂₃, and R₂₄, together with the atom(s) to which they are bonded, form a heterocycloalkyl group,
or Z and Z₁, both as defined above, together with the atoms to which they are bonded, form a heterocycloalkyl group;
or a phannaceutically acceptable prodrug, salt, active metabolite, or solvate thereof.

It is preferred that R₆₆ is the acyl group -C(O)OR₆₈ or the acyl group -C(O)SR₆₈, wherein R₆₈ is an alkyl group, a cycloalkyl group, an aryl group, a heterocycloalkyl group, or a heteroaryl group, or a pharmaceutically acceptable prodrug, salt, active metabolite, or solvate thereof.

The compound having the formula II is preferred: 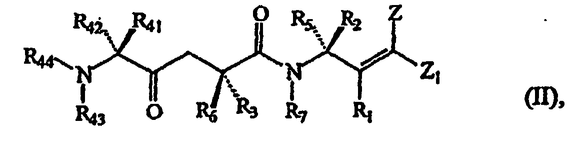 wherein R₁, R₅, R₆, R₇, R₄₂, R₄₃, and Z are H, R₂ is CH₂CH₂C(O)NH₂, and R₃, R₄₁, Z₁, and R₄₄ are selected from one of the following groups:
R₃ is CH₂Ph, R₄₁ is CH₂CH(CH₃)₂, Z₁ is CO₂CH₂CH₃, and R₄₄ is 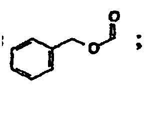
R₃ is CH₂Ph, R₄₁ is CH₂CH(CH₃)₂, Z₁ is CO₂CH₂CH₃, and R₄₄ is 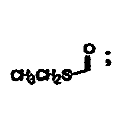
R₃ is CH₂Ph, R₄₁ is CH₂CH(CH₃)₂, Z₁ is 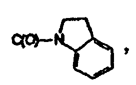 and R₄₄ is 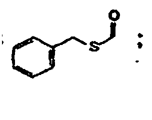
R₃ is CH₂Ph, R₄₁ is CH(CH₃)₂, Z₁ is CO₂CH₂CH₃, and R₄₄ is 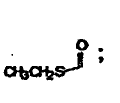
R₃ is CH₂Ph, R₄₁ is CH(CH₃)₂, Z₁ is CO₂CH₂CH₃, and R₄₄ is 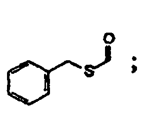
R₃ is CH₂Ph, R₄₁ is CH₂CH(CH₃)₂, Z₁ is CO₂CH₂CH₃, and R₄₄ is 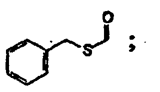
R₃ is CH₂Ph, R₄₁ is CH(CH₃)₂, Z₁ is CO₂CH₂CH₃, and R₄₄ is 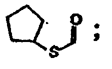
R₃ is CH₂Ph, R₄₁ is CH₂CH(CH₃)₂, Z₁ is CO₂CH₂CH₃, and R₄₄ is 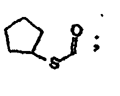
R₃ is CH₂(*p*-CH₃)Ph, R₄₁ is CH(CH₃)₂, Z₁ is CO₂CH₂CH₃, and R₄₄ is 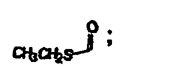 and
R₃ is CH₂(*p*-CH₃)Ph, R₄₁ is CH(CH₃)₂, Z₁ is CO₂CH₂CH₃, and R₄₄ is 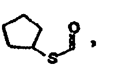
or a pharmaceutically acceptable prodrug, salt, active metabolite, or solvate thereof.

The compound having the formula III is preferred: 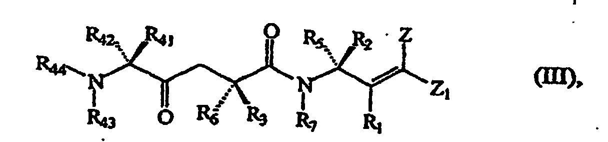 wherein R₁, R₅, R₆, R₇, R₄₂, R₄₃, and Z are H, R₃ is CH₂Ph, R₂ is CH₂CH₂C(O)NH₂, R₄₁ is CH₂CH(CH₃)₂, Z₁ is CO₂CH₂CH₃, and R₄₄ is 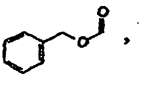 or a pharmaceutically acceptable prodrug, salt, active metabolite, or solvate thereof. Said object is also achieved by a compound of formula (IV): 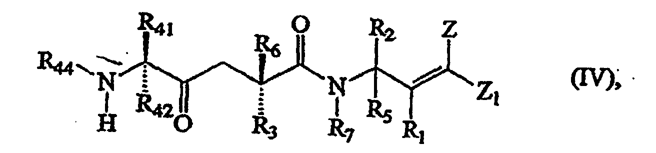 wherein:
R₁, R₅, R₆, R₇, and R₄₂ are H, R₂ is CH₂CH₂C(O)NH₂, and R₃, Z, Z₁, R₄₁, and R₄₄ are selected from one of the following groups:
   R₃ is CH₂(*p*-CH₃)Ph, Z is H, Z, is CO₂CH₂CH₃, R₄₁ is CH₂Ph, and R₄₄ is 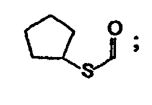
   R₃ is CH₂(*p*-F)Ph, Z is H, Z₁ is CO₂CH₂CH₃, R₄₁ is CH(CH₃)₂, and R₄₄ is 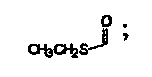
   R₃ is CH₂(*p*-F)Ph, Z is H, Z₁ is CO₂CH₂CH₃, R₄₁ is CH(CH₃)₂, and R₄₄ is 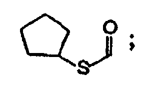
   R₃ is CH₂(*p*-CF₃)Ph, Z is H, Z₁ is CO₂CH₂CH₃, R₄₁ is CH(CH₃)₂, and R₄₄ is 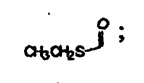
   R₃ is CH₂(*p*-CF₃)Ph, Z is H, Z₁ is CO₂CH₂CH₃, R₄₁ is CH(CH₃)₂, and R₄₄ is 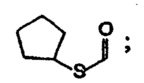
   R₃ is CH₂(*p*-CH₃)Ph, Z and Z₁ together form 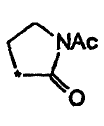 (where * indicates the point of attachment and the carbonyl group is cis to the R₁ group), R₄₁ is CH(CH₃)₂, and R₄₄ is 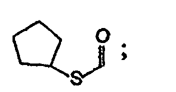
   R₃ is CH₂(*p*-F)Ph, Z is H, Z₁ is CO₂CH₂CH₃, R₄₁ is CH₂Ph, and R₄₄ is 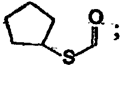
   R₃ is CH₂(*p*-F)Ph, Z is H, Z₁ is CO₂CH₂CH₃, R₄₁ is CH₂CH(CH₃)₂, and R₄₄ is 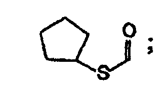
   R₃ is CH₂(*p*-CH₃)Ph, Z is H, Z₁ is CO₂CH₂CH₃, R₄₁ is CH(CH₃)₂, and R₄₄ is 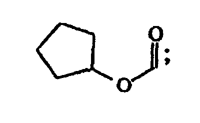
   R₃ is CH₂(*p*-CH₃)Ph, Z is H, Z₁ is CO₂CH₂CH₃, R₄₁ is CH₂CH(CH₃)₂, and R₄₄ is 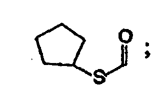
   R₃ is CH₂Ph, Z is H, Z₁ is CO₂CH₂CH₃, R₄₁ is C(CH₃)₃, and R₄₄ is 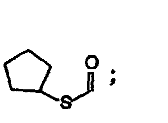
   R₃ is CH₂(*p*-CH₃)Ph, Z is H, Z₁ is CO₂CH₂CH₃, R₄₁ is CH(CH₃)₂, and R₄₄ is 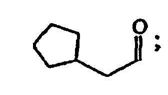
   R₃ is CH₂(*p*-F)Ph, Z is H, Z₁ is CO₂CH₂CH₃, R₄₁ is cyclohexyl, and R₄₄ is 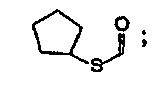
   R₃ is CH₂(*p*-F)Ph, Z is H, Z, is CO₂CH₂CH₃, R₄₁ is CH(CH₃)₂, and R₄₄ is 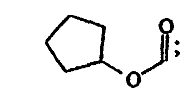
   R₃ is CH₂(*p*-F)Ph, Z is H, Z₁ is CO₂CH₂CH₃, R₄₁ is CH(CH₃)₂, and R₄₄ is 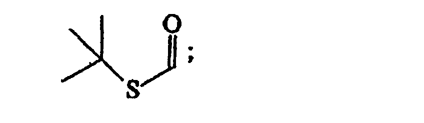
   R₃ is CH₂(*p*-F)Ph, Z is H, Z₁ is CO₂CH₂CH₃, R₄₁ is CH(CH₃)₂, and R₄₄ is 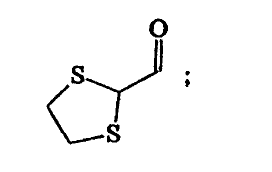
   R₃ is CH₂(*p*-F)Ph, Z is H, Z₁ is 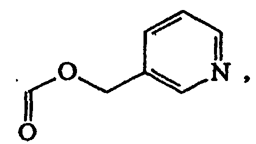 R₄₁ is CH(CH₃)₂, and R₄₄ is 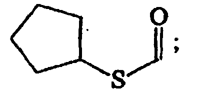
   R₃ is CH₂(*p*-F)Ph, Z is H, Z₁ is 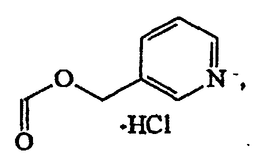 R₄₁ is CH(CH₃)₂, and R₄₄ is 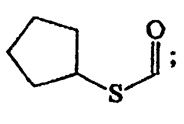
   R₃ is CH₂(*p*-F)Ph, Z is H, Z₁ is 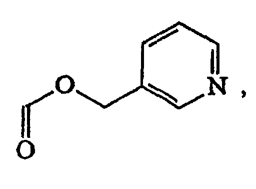 R₄₁ is CH(CH₃)₂, and R₄₄ is 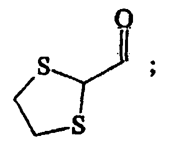
   R₃ is CH₂(*p*-F)Ph, Z is H, Z₁ is CO₂CH₂CH₃, R₄₁ is CH(CH₃)₂, and R₄₄ is 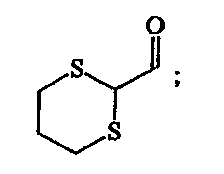
   R₃ is CH₂(*p*-F)Ph, Z is H, Z₁ is 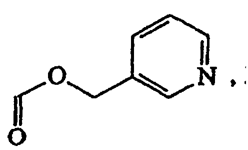 R₄₁ is CH(CH₃)₂, and R₄₄ is 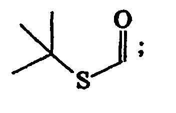
   R₃ is CH₂(*p*-F)Ph, Z is H, Z₁ is CO₂CH₂CH₂OH, R₄₁ is CH(CH₃)₂, and R₄₄ is 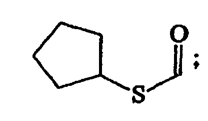 and
   R₃ is CH₂(*p*-F)Ph, Z is H, Z₁ is CO₂CH₂CH₃, R₄₁ is CH(CH₃)₂, and R₄₄ is 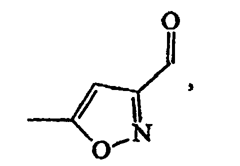
or a pharmaceutically acceptable prodrug, salt, active metabolite, or solvate thereof. Said object is also achieved by a composition comprising at least one compound of formula II: 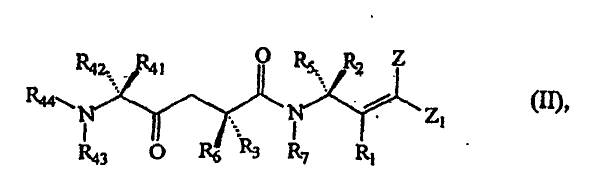 wherein R₁, R₅, R₆, R₇, R₄₂, R₄₃, and Z are H, R₃ is CH₂Ph, R₂ is CH₂CH₂C(O)NH₂,
R₄₁ is CH₂CH(CH₃)₂, Z₁ is CO₂CH₂CH₃, and R₄₄ is 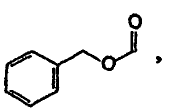 or a pharmaceutically acceptable prodrug, salt, active metabolite, or solvate thereof,
and at least one compound of formula III: 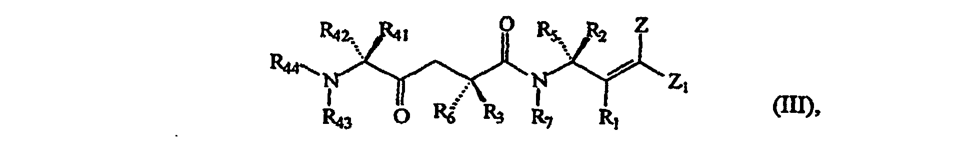 wherein R₁, R₅, R₆, R₇, R₄₂, R₄₃, and Z are H, R₃ is CH₂Ph, R₂ is CH₂CH₂C(O)NH₂,
R₄₁ is CH₂CH(CH₃)₂, Z, is CO₂CH₂CH₃, and R₄₄ is 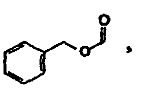 or a pharmaceutically acceptable prodrug, salt, active metabolite, or solvate thereof. Said object is also achieved by a pharmaceutical composition comprising:
(a) a therapeutically effective amount of at least one compound as defined above or a pharmaceutically acceptable prodrug, salt, active metabolite, or solvate thereof; and
(b) a pharmaceutically acceptable carrier, diluent, vehicle, or excipient.
Said object is further achieved by
the use of the compound described above or a pharmaceutically acceptable prodrug, salt, active metabolite, or solvate thereof for the preparation of a medicament for the treatment of mammalian disease condition mediated by picomaviral protease activity.
Said object is also achieved by
the use of the compound described above or a pharmaceutically acceptable prodrug, salt, active metabolite, or solvate thereof for the preparation of a medicament for inhibiting the activity of a picomaviral 3C protease.
Said object is also achieved by
a method of inhibiting in vitro the activity of a picomaviral 3C protease, comprising: contacting the picomaviral 3C protease with an effective amount of at least one compound as described above or a pharmaceutically acceptable prodrug, salt, active metabolite, or solvate thereof.
Said object is also achieved by
the use of the compound described above or a pharmaceutically acceptable prodrug, salt, active metabolite, or solvate thereof for the preparation of a medicament for inhibiting the activity of a rhinoviral protease.
Said object is also achieved by
a method of inhibiting in vitro the activity of a rhinoviral protease, comprising: contacting the rhinoviral protease with an effective amount of at least one compound as described above or a pharmaceutically acceptable prodrug, salt, active metabolite, or solvate thereof.
Said object is further achieved by
a compound described above, or a pharmaceutically acceptable prodrug, salt, active metabolite, or solvate thereof, wherein said antipicomaviral activity is antirhinoviral activity.
Said object is furthermore achieved by
a compound described above, or a pharmaceutically acceptable prodrug, salt, active metabolite, or solvate thereof, wherein said antipicomaviral activity is anticoxsackieviral activity.

The present invention relates to compounds of the formula I: 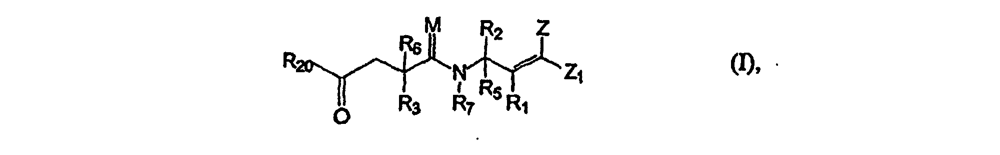 wherein R₁, R₂, R₃, R₅, R₆, R₇, R₂₀, M, Z, and Z₁ are as defined above, and to the pharmaceutically acceptable prodrugs, salts, active metabolites, and solvates thereof, where these compounds, pharmaceutically acceptable prodrugs, salts, active metabolites, and solvates preferably have antipicornaviral activity with an EC₅₀ less than or equal to 10 µM in the HI-HeLa cell culture assay, and more preferably antirhinoviral activity with an EC₅₀ less than or equal to 10 µM in the HI-HeLa cell culture assay and/or anticoxsachieviral activity with an EC₅₀ less than or equal to 10 µM in the HI-HeLa cell culture assay.

The present invention preferably relates to compounds of the formula X: 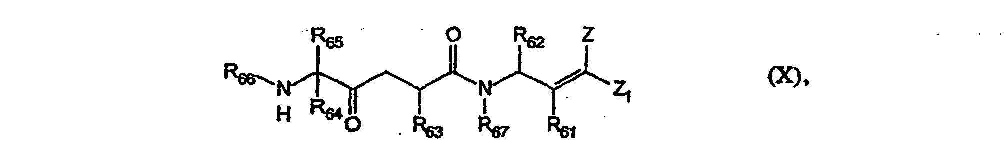 wherein:
R₆₁ is H, F, or an alkyl group;
R₆₂ is selected from one of the following moieties: 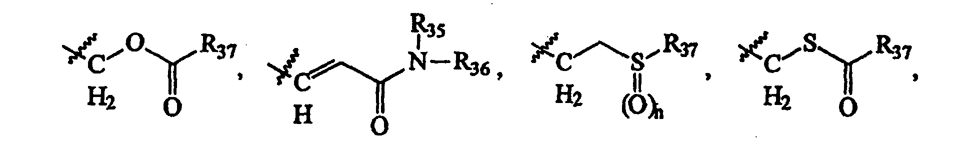 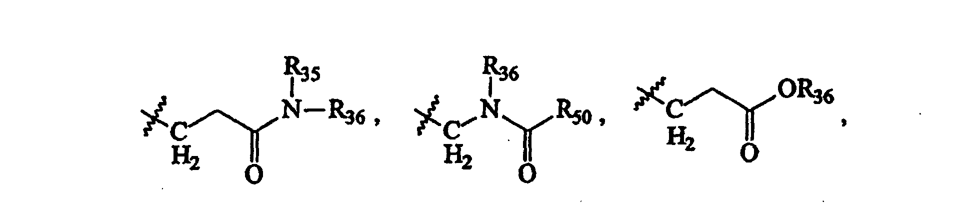 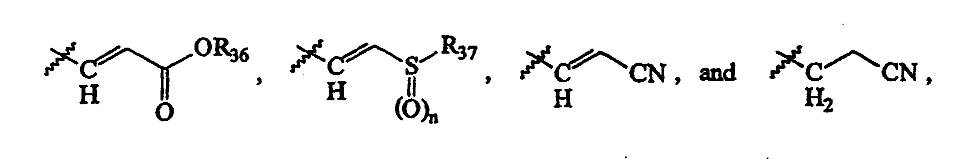 wherein:
   R₃₅ is H, an alkyl group, an aryl group, -OR₃₈, or -NR₃₈R₃₉,
      wherein R₃₈ and R₃₉ independently are H, an alkyl group, a cycloalkyl group, a heterocycloalkyl group, an aryl group, a heteroaryl group, or an acyl group; and
   R₃₆ is H or an alkyl group,
   or R₃₅ and R₃₆, together with the nitrogen atom to which they are attached, form a heterocycloalkyl group or a heteroaryl group;
   R₃₇ is an alkyl group, an aryl group, or -NR₃₈R₃₉, wherein R₃₈ and R₃₉ are as defined above;
   R₅₀ is H, an alkyl group, an aryl group, -OR₃₈, -SR₃₉, -NR₃₈R₃₉, -NR₄₀NR₃₈R₃₉, or -NR₃₈OR₃₉, or R₅₀ and R₃₆, together with the atoms to which they are attached, form a heterocycloalkyl group;
      wherein R₃₈ and R₃₉ are as defined above and R₄₀ is H, an alkyl group, a cycloalkyl group, a heterocycloalkyl group, an aryl group, a heteroaryl group, or an acyl group; and
   n is 0, 1, or 2;
R₆₃ is H or an alkyl group;
R₆₄ is H, an alkyl group, a cycloalkyl group, a heterocycloalkyl group, an aryl group, or a heteroaryl group;
R₆₅ is H or an alkyl group;
R₆₆ is H, an acyl group, an alkyl group, a cycloalkyl group, a heterocycloalkyl group, an aryl group, a sulfonyl group, or a heteroaryl group;
R₆₇ is H or an alkyl group;
   and
Z and Z₁ are independently H, F, an alkyl group, a cycloalkyl group, a heterocycloalkyl group, an aryl group, a heteroaryl group, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), -PO(NR₂₁R₂₂)(NR₂₃R₂₄), -C(O)NR₂₁NR₂₂R₂₃, or -C(S)NR₂₁NR₂₂R₂₃,
   wherein R₂₁, R₂₂, R₂₃, and R₂₄ are independently H, an alkyl group, a cycloalkyl group, a heterocycloalkyl group, an aryl group, a heteroaryl group, an acyl group, or a thioacyl group, or wherein any two of R₂₁, R₂₂, R₂₃, and R₂₄, together with the atom(s) to which they are bonded, form a heterocycloalkyl group,
or Z and Z₁, both as defined above, together with the atoms to which they are attached, form a heterocycloalkyl group;
   and phannaceutically acceptable prodrugs, salts, active metabolites, and solvates thereof.

As used in the present application, the following definitions apply:

An "alkyl group" is intended to mean a straight or branched chain monovalent radical of saturated and/or unsaturated carbon atoms and hydrogen atoms, such as methyl, ethyl, propyl, isopropyl, butyl, isobutyl, t-butyl, ethenyl, pentenyl, butenyl, propenyl, ethynyl, butynyl, propynyl, pentynl, hexynyl, which may be unsubstituted (i.e., containing only carbon and hydrogen) or substituted by one or more suitable substituents as defined below.

A "cycloalkyl group" is intended to mean a non-aromatic, monovalent monocyclic, bicyclic, or tricyclic radical containing 3, 4, 5, 6, 7, 8, 9, 10, 11, 12, 13, or 14 carbon ring atoms, each of which may be saturated or unsaturated, and which may be unsubstituted or substituted by one or more suitable substituents as defined below, and to which may be fused one or more heterocycloalkyl groups, aryl groups, or heteroaryl groups, which themselves may be unsubstituted or substituted by one or more suitable substituents.

Illustrative examples of cycloalkyl groups include, but are not limited to, the following moieties:

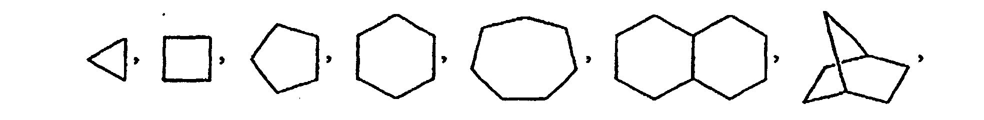

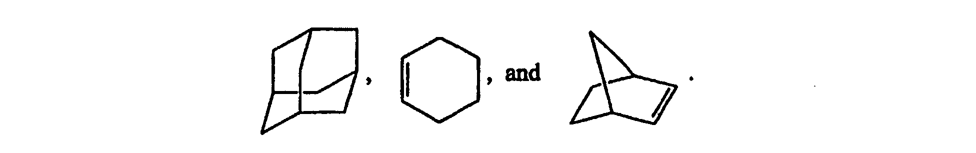

A "heterocycloalkyl group" is intended to mean a non-aromatic, monovalent monocyclic, bicyclic, or tricyclic radical, which is saturated or unsaturated, containing 3, 4, 5, 6, 7, 8, 9, 10, 11, 12, 13, 14, 15, 16, 17, or 18 ring atoms, and which includes 1, 2, 3, 4, or 5 heteroatoms selected from nitrogen, oxygen, and sulfur, wherein the radical is unsubstituted or substituted by one or more suitable substituents as defined below, and to which may be fused one or more cycloalkyl groups, aryl groups, or heteroaryl groups, which themselves may be unsubstituted or substituted by one or more suitable substituents. Illustrative examples of heterocycloalkyl groups include, but are not limited to the following moieties:

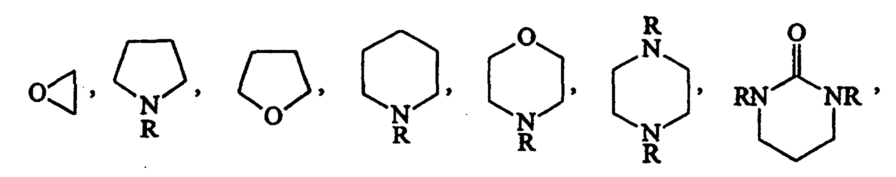

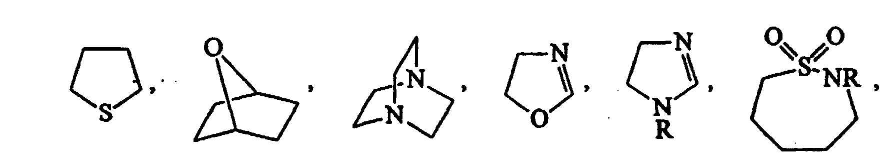

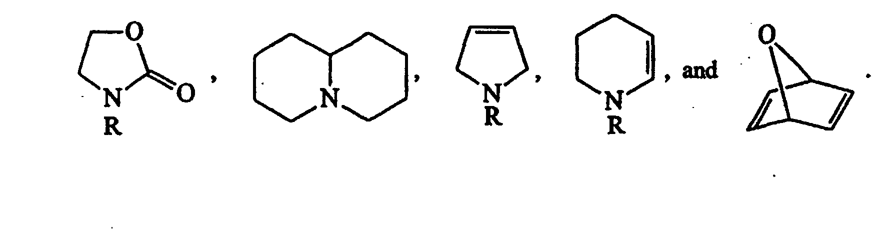

An "aryl group" is intended to mean an aromatic, monovalent monocyclic, bicyclic, or tricyclic radical containing 6, 10, 14, 18 carbon ring atoms, which may be unsubstituted or substituted by one or more suitable substituents as defined below, and to which may be fused one or more cycloalkyl groups, heterocycloalkyl groups, or heteroaryl groups, which themselves may be unsubstituted or substituted by one or more suitable substituents. Illustrative examples of aryl groups include, but are not limited to, the following moieties:

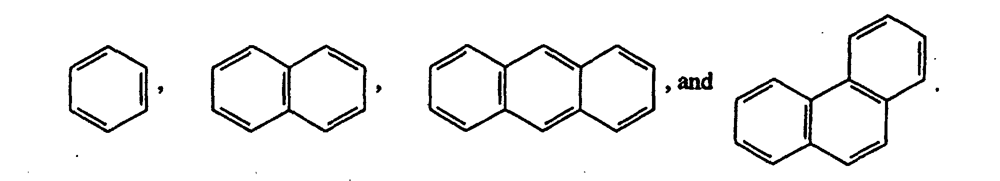

A "heteroaryl group" is intended to mean an aromatic monovalent monocyclic, bicyclic, or tricyclic radical containing 4, 5, 6, 7, 8, 9, 10, 11, 12, 13, 14, 15, 16, 17, or 18 ring atoms, including 1, 2, 3, 4, or 5 heteroatoms selected from nitrogen, oxygen, and sulfur, which may be unsubstituted or substituted by one or more suitable substituents as defined below, and to which may be fused one or more cycloalkyl groups, heterocycloalkyl groups, or aryl groups, which themselves may be unsubstituted or substituted by one or more suitable substituents. Illustrative examples of heteroaryl groups include, but are not limited to, the following moieties: 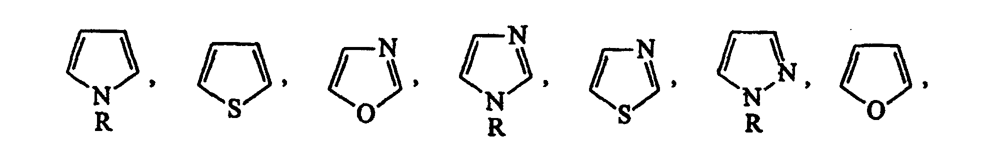 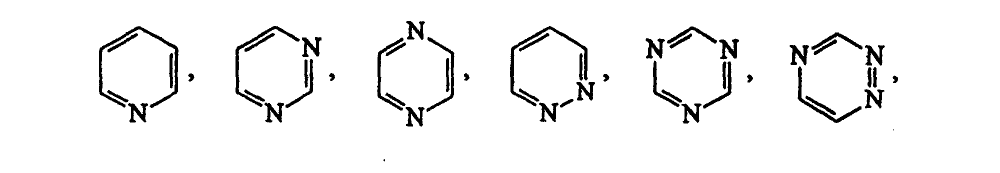 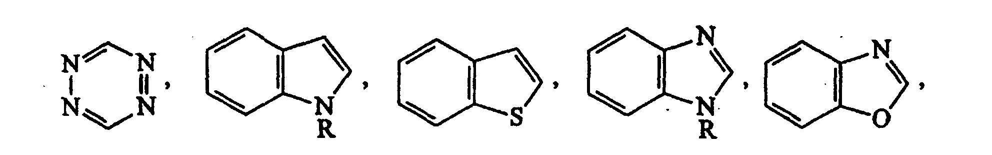 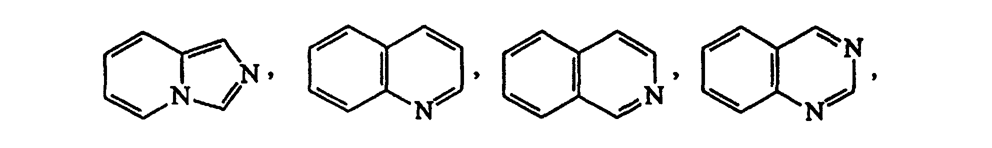 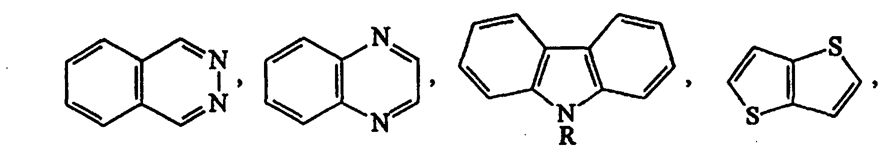 and

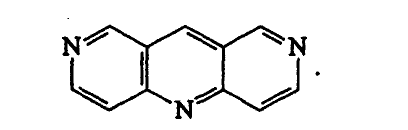

An "acyl group" is intended to mean a -C(O)-R radical, wherein R is any suitable substituent as defined below.

A "thioacyl group" is intended to mean a -C(S)-R radical, wherein R is any suitable substituent as defined below.

A "sulfonyl group" is intended to mean a - SO₂R radical, wherein R is any suitable substituent as defined below.

The term "suitable substituent" is intended to mean any of the substituents recognizable, such as by routine testing, to those skilled in the art as not adversely affecting the inhibitory activity of the inventive compounds. Illustrative examples of suitable substituents include, but are not limited to, hydroxy groups, oxo groups, alkyl groups, acyl groups, sulfonyl groups, mercapto groups, alkylthio groups, alkoxy groups, cycloalkyl groups, heterocycloalkyl groups, aryl groups, heteroaryl groups, carboxy groups, amino groups, alkylamino groups, dialkylamino groups, carbamoyl groups, aryloxy groups, heteroarlyoxy groups, arylthio groups, heteroarylthio groups.

The term "suitable organic moiety" is intended to mean any organic moiety recognizable, such as by routine testing, to those skilled in the art as not adversely affecting the inhibitory activity of the inventive compounds. Illustrative examples of suitable organic moieties include, but are not limited to, hydroxy groups, alkyl groups, oxo groups, cycloalkyl groups, heterocycloalkyl groups, aryl groups, heteroaryl groups, acyl groups, sulfonyl groups, mercapto groups, alkylthio groups, alkoxy groups, carboxy groups, amino groups, alkylamino groups, dialkylamino groups, carbamoyl groups, arylthio groups, heteroarylthio groups.

A "hydroxy group" is intended to mean the radical -OH.

An "amino group" is intended to mean the radical -NH₂.

An "alkylamino group" is intended to mean the radical -NHR where R is an alkyl group as defined above.

A "dialkylamino group" is intended to mean the radical -NRₐR_{b} where Rₐ and R_{b} are each independently an alkyl group as defined above.

An "alkoxy group" is intended to mean the radical -OR where R is an alkyl group as defined above, for example, methoxy, ethoxy, propoxy.

An "alkoxycarbonyl group" is intended to mean the radical -C(O)OR where R is an alkyl group as defined above.

An "alkylsulfonyl group" is intended to mean the radical -SO₂R where R is an alkyl group as defined above.

An "alkylaminocarbonyl group" is intended to mean the radical -C(O)NHR where R is an alkyl group as defined above.

A "dialkylaminocarbonyl group" is intended to mean the radical -C(O)NRₐR_{b} where Rₐ and R_{b} are each independently an alkyl group as defined above.

A "mercapto group" is intended to mean the radical -SH.

An "alkylthio group" is intended to mean the radical -SR where R is an alkyl group as defined above.

A "carboxy group" is intended to mean the radical -C(O)OH.

A "carbamoyl group" is intended to mean the radical -C(O)NH₂.

An "aryloxy group" is intended to mean the radical -OR_{c} where R_{c} is an aryl group as defined above.

A "heteroaryloxy group" is intended to mean the radical -OR_{d} where R_{d} is a heteroaryl group as defined above.

An "arylthio group" is intended to mean the radical -SR_{c} where R_{c} is an aryl group as defined above.

A "heteroarylthio group" is intended to mean the radical -SR_{d} where R_{d} is a heteroaryl group as defined above.

A "pharmaceutically acceptable prodrug" is intended to mean a compound that may be converted under physiological conditions or by solvolysis to a compound of formula I or formula X.

A "pharmaceutically acceptable active metabolite" is intended to mean a pharmacologically active product produced through metabolism in the body of a compound of formula I or formula X.

A "pharmaceutically acceptable solvate" is intended to mean a solvate that retains the biological effectiveness and properties of the biologically active components of compounds of formulas I and X. Examples of pharmaceutically acceptable solvates include, but are not limited to, compounds of formula I or X in combination with water, isopropanol, ethanol, methanol, DMSO, ethyl acetate, acetic acid, or ethanolamine.

A "pharmaceutically acceptable salt" is intended to mean a salt that retains the biological effectiveness and properties of the free acids and bases of compounds of formulas I and X and that is not biologically or otherwise undesirable. Examples of pharmaceutically acceptable salts include, but are not limited to, sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxyenzoates, phthalates, sulfonates, xylenesulfonates, phenylacetates, phenylpropionates, phenylbutyrates, citrates, lactates, γ-hydroxybutyrates, glycollates, tartrates, methane-sulfonates, propanesulfonates, naphthalene-1-sulfonates, naphthalene-2-sulfonates, and mandelates.

If the inventive compound is a base, the desired salt may be prepared by any suitable method known to the art, including treatment of the free base with an inorganic acid, such as hydrochloric acid; hydrobromic acid; sulfuric acid; nitric acid; phosphoric acid; or with an organic acid, such as acetic acid; maleic acid; succinic acid; mandelic acid; fumaric acid; malonic acid; pyruvic acid; oxalic acid; glycolic acid; salicylic acid; pyranosidyl acids such as glucuronic acid and galacturonic acid; alpha-hydroxy acids such as citric acid and tartaric acid; amino acids such as aspartic acid and glutamic acid; aromatic acids such as benzoic acid and cinnamic acid; sulfonic acids such as p-toluenesulfonic acid or ethanesulfonic acid.

If the inventive compound is an acid, the desired salt may be prepared by any suitable method known to the art, including treatment of the free acid with an inorganic or organic base, such as an amine (primary, secondary or tertiary); an alkali metal or alkaline earth metal hydroxide. Illustrative examples of suitable salts include organic salts derived from amino acids such as glycine and arginine; ammonia; primary, secondary and tertiary amines; and cyclic amines such as piperidine, morpholine, and piperazine, and inorganic salts derived from sodium, calcium, potassium, magnesium, manganese, iron, copper, zinc, aluminum, and lithium.

In the case of compounds, salts, or solvates that are solids, it is understood by those skilled in the art that the inventive compounds, salts, and solvates may exist in different crystal forms, all of which are intended to be within the scope of the present invention.

The inventive compounds may exist as single stereoisomers, racemates, and/or mixtures of enantiomers and/or diastereomers. All such single stereoisomers, racemates, and mixtures thereof are intended to be within the scope of the present invention. Preferably, the inventive compounds are used in optically pure form.

As generally understood by those skilled in the art, an optically pure compound is one that is enantiomerically pure. As used herein, the term "optically pure" is intended to mean a compound which comprises at least a sufficient amount of a single enantiomer to yield a compound having the desired pharmacological activity. Preferably, "optically pure" is intended to mean a compound that comprises at least 90% of a single isomer (80% enantiomeric excess), preferably at least 95% (90% e.e.), more preferably at least 97.5% (95% e.e.), and most preferably at least 99% (98% e.e.).

Preferably in the above formulas I and X, R₁ and R₆₁ are H or F. In the compounds of formula I, preferably M is O.

Preferably R₂₀ in formula I is H, an alkyl group, a cycloalkyl group, a heterocycloalkyl group, an aryl group, a heteroaryl group, -OR₁₇, -SR₁₇, -NR₁₇R₁₈, -NR₁₉NR₁₇R₁₈, or -NR₁₇OR₁₈, wherein R₁₇, R₁₈, and R₁₉ independently are H, an alkyl group, a cycloalkyl group, a heterocycloalkyl group, an aryl group, a heteroaryl group, or an acyl group. More preferably R₂₀ is the alkyl group -C(R₄₁)(R₄₂)NR₄₃R₄₄, wherein R₄₁ and R₄₂ independently are H, an alkyl group, a cycloalkyl group, a heterocycloalkyl group, an aryl group, or a heteroaryl group; and R₄₃ and R₄₄ independently are H, an alkyl group, a cycloalkyl group, a heterocycloalkyl group, an aryl group, a heteroaryl group, -NR₄₅R₄₆, -C(O)R₄₅, -C(S)R₄₅, -C(O)NR₄₅R₄₆, -C(S)NR₄₅R₄₆, -C(O)NR₄₅OR₄₆, -C(S)NR₄₅OR₄₆, -C(O)SR₄₅, -C(O)OR₄₅, -C(S)OR₄₅, -C(S)SR₄₅, -OR₄₅, -SR₄₅, -C(O)NR₄₅NR₄₆R₄₇, -C(S)NR₄₅NR₄₆R₄₇, -SOR₄₅, -SO₂R₄₅, -S(O)NR₄₅R₄₆, -S(O)NR₄₅(OR₄₆), -SO₂NR₄₅R₄₆, - SO₂NR₄₅(OR₄₆), or -SO₃R₄₅, wherein R₄₅, R₄₆, and R₄₇ independently are H, an alkyl group, a cycloalkyl group, a heterocycloalkyl group, an aryl group, a heteroaryl group, or wherein any combination of R₄₁, R₄₂, R₄₃, and R₄₄, together with the atoms to which they are attached, form a cycloalkyl group or a heterocycloalkyl group. Preferably at least one of R₄₃ and R₄₄ is -C(O)SR₄₅ or - C(O)OR₄₅, Preferably R₄₅ is an alkyl group, a cycloalkyl group, an aryl group, a heterocycloalkyl group, or a heteroaryl group, and more preferably a C₁-C₁₀ alkyl group.

In the compounds of formula I, preferably D₁ and D₂ are -OR₂₅, =O, =S, ≡N, =NR₂₅, or -NR₂₅R₂₆, wherein R₂₅ and R₂₆ are independently H, an alkyl group, a cycloalkyl group, a heterocycloalkyl group, an aryl group, or a heteroaryl group, or, together with the nitrogen atom to which they are bonded, form a heterocycloalkyl group, and more preferably D₁ and D₂ are =O. Preferably, in the compounds of formula I, A₁ and A₂ are C, CH, S, or S(O), and more preferably A₁ and A₂ are C.

Preferably, in the compounds of formula I, B₁ and B₂ are NR₁₇R₁₈, wherein R₁₇ and R₁₈ are independently H, an alkyl group, a cycloalkyl group, a heterocycloalkyl group, an aryl group, a heteroaryl group, or an acyl group.

In the compounds of formula I and X, preferably Z and Z₁ are independently H, an aryl group, or a heteroaryl group, -C(O)R₂₁, -CO₂R₂₁, -CN, -C(O)NR_{21,}R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -C(O)NR₂₁NR₂₂R₂₃, or -C(S)NR₂₁NR₂₂R₂₃, wherein R₂₁, R₂₂, and R₂₃ are independently H, an alkyl group, a cycloalkyl group, a heterocycloalkyl group, an aryl group, a heteroaryl group, an acyl group, or a thioacyl group, or wherein any two of R₂₁, R₂₂, and R₂₃, together with the atom(s) to which they are bonded, form a heterocycloalkyl group, or Z and Z₁, both as defined above, together with the atoms to which they are attached, form a heterocycloalkyl group.

In the compounds of formula X, preferably R₆₆ is -C(O)OR₆₈ or -C(O)SR₆₈, wherein R₆₈ is an alkyl group, a cycloalkyl group, an aryl group, a heterocycloalkyl group, or a heteroaryl group.

Particularly preferred embodiments of the present invention include the following compounds (**1-8, 10,** and **11**) of formula II: 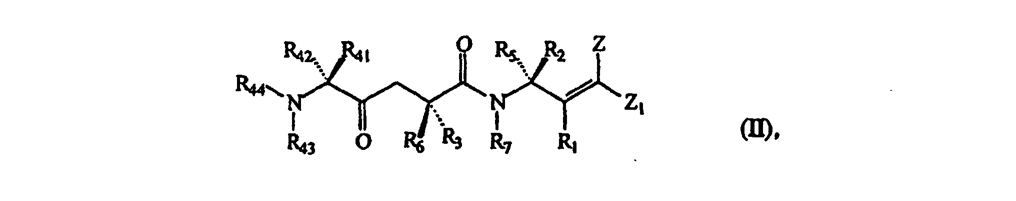 wherein R₁, R₅, R₆, R₇, R₄₂, R₄₃, and Z are H, R₂ is CH₂CH₂C(O)NH₂, and R₃, R₄₁, Z₁, and R₄₄ are selected from one of the following groups:
**(1)** R₃ is CH₂Ph, R₄₁ is CH₂CH(CH₃)₂, Z₁ is CO₂CH₂CH₃, and R₄₄ is 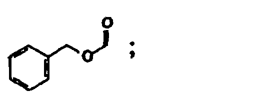
(**2**) R₃ is CH₂Ph, R₄₁ is CH₂CH(CH₃)₂, Z₁ is CO₂CH₂CH₃, and R₄₄ is 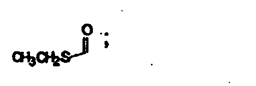
**(3)** R₃ is CH₂Ph, R₄₁ is CH₂CH(CH₃)₂, Z₁ is 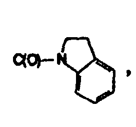 and R₄₄ is 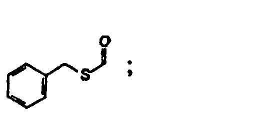
**(4)** R₃ is CH₂Ph, R₄₁ is CH(CH₃)₂, Z₁ is CO₂CH₂CH₃, and R₄₄ is 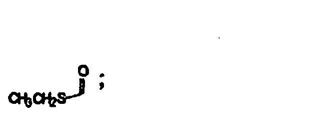
**(5)** R₃ is CH₂Ph, R₄₁ is CH(CH₃)₂, Z₁ is CO₂CH₂CH₃, and R₄₄ is 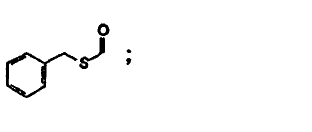
**(6)** R₃ is CH₂Ph, R₄₁ is CH₂CH(CH₃)₂, Z₁ is CO₂CH₂CH₃, and R₄₄ is 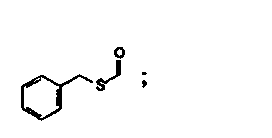
**(7)** R₃ is CH₂Ph, R₄₁ is CH(CH₃)₂, Z₁ is CO₂CH₂CH₃, and R₄₄ is 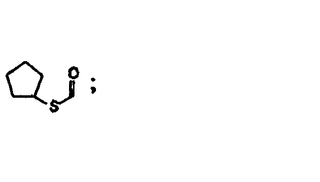
**(8)** R₃ is CH₂Ph, R₄₁ is CH₂CH(CH₃)₂, Z₁ is CO₂CH₂CH₃, and R₄₄ is 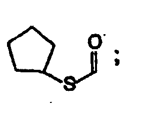
**(10)** R₃ is CH₂(*p*-CH₃)Ph, R₄₁ is CH(CH₃)₂, Z₁ is CO₂CH₂CH₃, and R₄₄ is 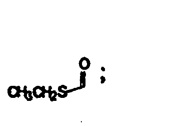 and
**(11)** R₃ is CH₂(*p*-CH₃)Ph, R₄₁ is CH(CH₃)₂, Z₁ is CO₂CH₂CH₃, and R₄₄ is 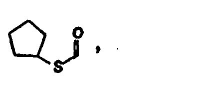
and pharmaceutically acceptable prodrugs, salts, active metabolites, and solvates thereof.

Another preferred embodiment of the invention includes a compound (9) of formula III: 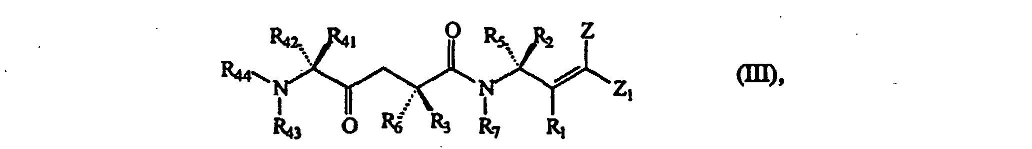 wherein R₁, R₅, R₆, R₇, R₄₂, R₄₃, and Z are H, R₃ is CH₂Ph, R₂ is CH₂CH₂C(O)NH₂,
R₄₁ is CH₂CH(CH₃)₂, Z, is CO₂CH₂CH₃, and R₄₄ is 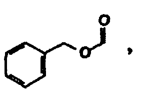 and pharmaceutically acceptable prodrugs, salts, active metabolites, and solvates thereof.

The present invention is still further directed to compositions comprising at least one compound of formula II: 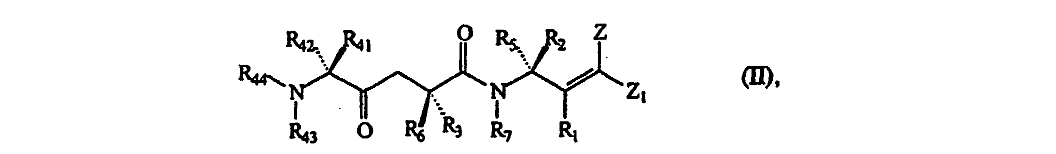 wherein R₁, R₅, R₆, R₇, R₄₂, R₄₃, and Z are H, R₃ is CH₂Ph, R₂ is CH₂CH₂C(O)NH₂,
R₄₁ is CH₂CH(CH₃)₂, Z₁ is CO₂CH₂CH₃, and R₄₄ is 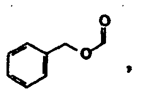 or a pharmaceutically acceptable prodrug, salt, active metabolite, or solvate thereof,
and at least one compound of formula III: 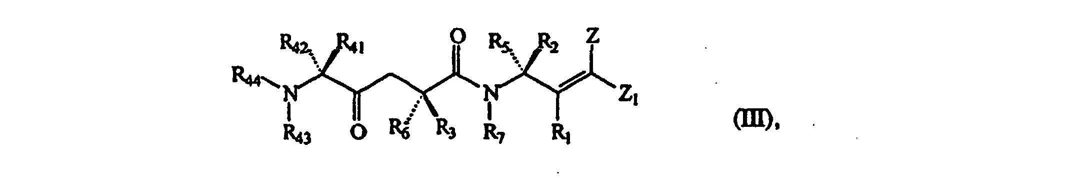 wherein R₁, R₅, R₆, R₇, R₄₂, R₄₃, and Z are H, R₃ is CH₂Ph, R₂ is CH₂CH₂C(O)NH₂,
R₄₁ is CH₂CH(CH₃)₂, Z₁ is CO₂CH₂CH₃, and R₄₄ is 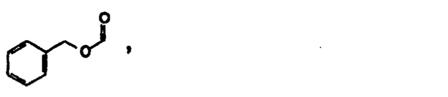 or a pharmaceutically acceptable prodrug, salt, active metabolite, or solvate thereof.

Additional preferred compounds according to the present invention include the following compounds (**12** through **34**) of formula IV: 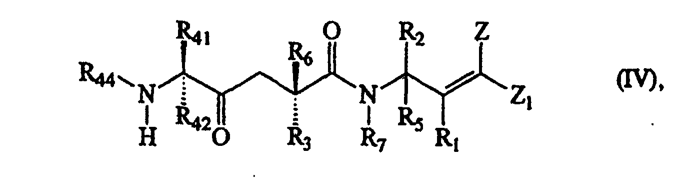 wherein R₁, R₅, R₆, R₇, and R₄₂ are H, R₂ is CH₂CH₂C(O)NH₂, and R₃, Z, Z₁, R₄₁, and R₄₄ are selected from one of the following groups:
**(12)** R₃ is CH₂(*p*-CH₃)Ph, Z is H, Z₁ is CO₂CH₂CH₃, R₄₁ is CH₂Ph, and R₄₄ is 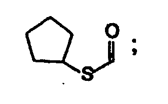
**(13)** R₃ is CH₂(*p*-F)Ph, Z is H, Z₁ is CO₂CH₂CH₃, R₄₁ is CH(CH₃)₂, and R₄₄ is 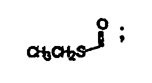
**(14)** R₃ is CH₂(*p*-F)Ph, Z is H, Z₁ is CO₂CH₂CH₃, R₄₁ is CH(CH₃)₂, and R₄₄ is 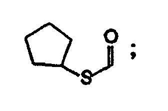
**(15)** R₃ is CH₂(*p*-CF₃)Ph, Z is H, Z₁ is CO₂CH₂CH₃, R₄₁ is CH(CH₃)₂, and R₄₄ is 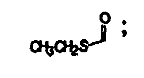
**(16)** R₃ is CH₂(*p*-CF₃)Ph, Z is H, Z₁ is CO₂CH₂CH₃, R₄₁ is CH(CH₃)₂, and R₄₄ is 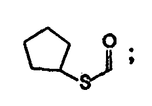
**(17)** R₃ is CH₂(*p*-CH₃)Ph, Z and Z₁ together form 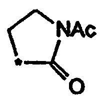 (where * indicates the point of attachment and the carbonyl group is cis to the R₁ group), R₄₁ is CH(CH₃)₂, and R₄₄ is 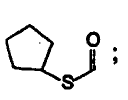
(**18**) R₃ is CH₂(*p*-F)Ph, Z is H, Z₁ is CO₂CH₂CH₃, R₄₁ is CH₂Ph, and R₄₄ is 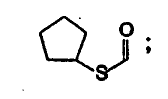
**(19)** R₃ is CH₂(*p*-F)Ph, Z is H, Z₁ is CO₂CH₂CH₃, R₄₁ is CH₂CH(CH₃)₂, and R₄₄ is 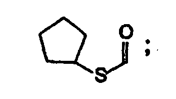
**(20)** R₃ is CH₂(*p*-CH₃)Ph, Z is H, Z₁ is CO₂CH₂CH₃, R₄₁ is CH(CH₃)₂, and R₄₄ is 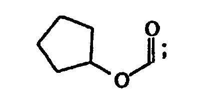
**(21)** R₃ is CH₂(*p*-CH₃)Ph, Z is H, Z₁ is CO₂CH₂CH₃, R₄₁ is CH₂CH(CH₃)₂, and R₄₄ is 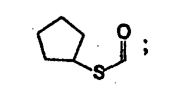
**(22)** R₃ is CH₂Ph, Z is H, Z₁ is CO₂CH₂CH₃, R₄₁ is C(CH₃)₃, and R₄₄ is 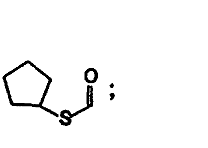
**(23)** R₃ is CH₂(*p*-CH₃)Ph, Z is H, Z₁ is CO₂CH₂CH₃, R₄₁ is CH(CH₃)₂, and R₄₄ is 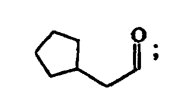
**(24)** R₃ is CH₂(*p*-F)Ph, Z is H, Z₁ is CO₂CH₂CH₃, R₄₁ is cyclohexyl, and R₄₄ is 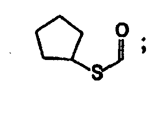
**(25)** R₃ is CH₂(*p*-F)Ph, Z is H, Z₁ is CO₂CH₂CH₃, R₄₁ is CH(CH₃)₂, and R₄₄ is 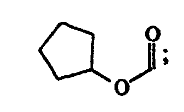
**(26)** R₃ is CH₂(*p*-F)Ph, Z is H, Z₁ is CO₂CH₂CH₃, R₄₁ is CH(CH₃)₂, and R₄₄ is 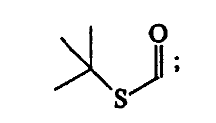
**(27)** R₃ is CH₂(*p*-F)Ph, Z is H, Z₁ is CO₂CH₂CH₃, R₄₁ is CH(CH₃)₂, and R₄₄ is 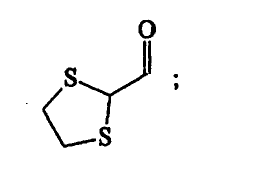
**(28)** R₃ is CH₂(*p*-F)Ph, Z is H, Z₁ is 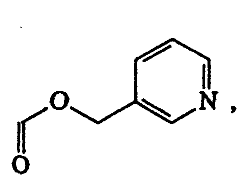 R₄₁ is CH(CH₃)₂, and R₄₄ is 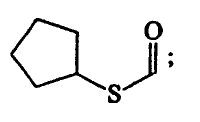
**(29)** R₃ is CH₂(*p*-F)Ph, Z is H, Z₁ is 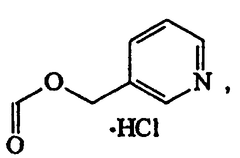 R₄₁ is CH(CH₃)₂, and R₄₄ is 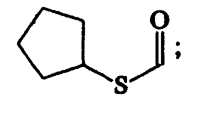
**(30)** R₃ is CH₂(*p*-F)Ph, Z is H, Z₁ is 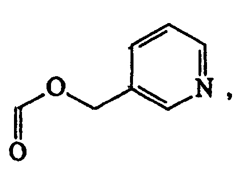 R₄₁ is CH(CH₃)₂, and R₄₄ is 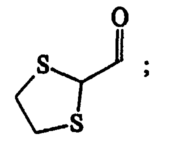
**(31)** R₃ is CH₂(*p*-F)Ph, Z is H, Z₁ is CO₂CH₂CH₃, R₄₁ is CH(CH₃)₂, and R₄₄ is 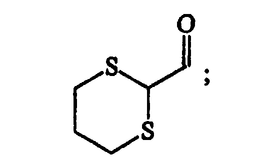
**(32)** R₃ is CH₂(*p*-F)Ph, Z is H, Z₁ is 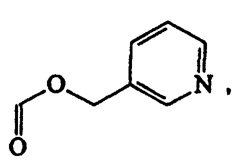 R₄₁ is CH(CH₃)₂, and R₄₄ is 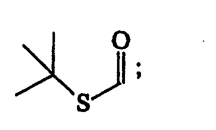
**(33)** R₃ is CH₂(*p*-F)Ph, Z is H, Z₁ is CO₂CH₂CH₂OH, R₄₁ is CH(CH₃)₂, and R₄₄ is 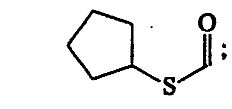 and
**(34)** R₃ is CH₂(*p*-F)Ph, Z is H, Z₁ is CO₂CH₂CH₃, R₄₁ is CH(CH₃)₂, and R₄₄ is 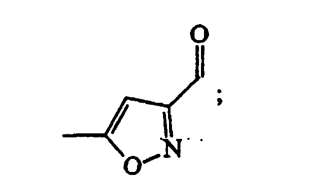 and pharmaceutically acceptable prodrugs, salts, active metabolites, or solvates thereof.

The present invention is even further directed to methods of inhibiting picornaviral 3C protease activity, comprising contacting the protease with an effective amount of a compound of formula I or X or a pharmaceutically acceptable prodrug, salt, active metabolite, or solvate thereof. For example, one can inhibit picornaviral 3C protease activity in mammalian tissue by administering a compound of formula I or X or a pharmaceutically acceptable prodrug, salt, active metabolite, or solvate thereof. More particularly, the present invention is directed to methods of inhibiting rhinoviral protease activity.

The activity of the inventive compounds as inhibitors of picornaviral 3C protease activity may be measured by any of the methods available to those skilled in the art, including in vivo and in vitro assays. An example of a suitable assay for activity measurements is the Antiviral HI-HeLa Cell Culture Assay, described herein.

Administration of the compounds of the formula I or X, or their pharmaceutically acceptable prodrugs, salts, active metabolites, and solvates, may be performed according to any of the accepted modes of administration available to those skilled in the art. Illustrative examples of suitable modes of administration include, but are not limited to, oral, nasal, parenteral, topical, transdermal, and rectal.

The inventive compounds of formulas I and X, and their pharmaceutically acceptable prodrugs, salts, active metabolites, and solvates, may be administered as a pharmaceutical composition in any suitable pharmaceutical form recognizable to the skilled artisan. Suitable pharmaceutical forms include, but are not limited to, solid, semisolid, liquid, or lyopholized formulations, such as tablets, powders, capsules, suppositories, suspensions, and aerosols. The pharmaceutical composition may also include suitable excipients, diluents, vehicles, and carriers, as well as other pharmaceutically active agents, depending upon the intended use.

Acceptable methods of preparing suitable pharmaceutical forms of the pharmaceutical compositions are known to those skilled in the art. For example, pharmaceutical preparations may be prepared following conventional techniques of the pharmaceutical chemist involving steps such as mixing, granulating, and compressing when necessary for tablet forms, or mixing, filling, and dissolving the ingredients as appropriate, to give the desired products for oral, parenteral, topical, intravaginal, intranasal, intrabronchial, intraocular, intraaural, and/or rectal administration.

Solid or liquid pharmaceutically acceptable carriers, diluents, vehicles, or excipients may be employed in the pharmaceutical compositions. Illustrative solid carriers include starch, lactose, calcium sulphate dihydrate, terra alba, sucrose, talc, gelatin, pectin, acacia, magnesium stearate, and stearic acid. Illustrative liquid carriers may include syrup, peanut oil, olive oil, saline solution, and water. The carrier or diluent may include a suitable prolonged-release material, such as glyceryl monostearate or glyceryl distearate, alone or with a wax. When a liquid carrier is used, the preparation may be in the form of a syrup, elixir, emulsion, soft gelatin capsule, sterile injectable liquid (e.g. solution), or a nonaqueous or aqueous liquid suspension.

A dose of the pharmaceutical composition contains at least a therapeutically effective amount of the active compound (i.e., a compound of formula I or X or a pharmaceutically acceptable prodrug, salt, active metabolite, or solvate thereof) and preferably is made up of one or more pharmaceutical dosage units. The selected dose may be administered to a mammal, for example, a human patient, in need of treatment mediated by inhibition of 3C protease activity, by any known method of administering the dose including topical, for example, as an ointment or cream; orally; rectally, for example, as a suppository; parenterally by injection; or continuously by intravaginal, intranasal, intrabronchial, intraaural, or intraocular infusion.

A "therapeutically effective amount" is intended to mean that amount of a compound of formula I or X that, when administered to a mammal in need thereof, is sufficient to effect treatment for disease conditions alleviated by the inhibition of the activity of one or more picornaviral 3C proteases, such as human rhinoviruses, human poliovirus, human coxsackieviruses, encephalomyocarditis viruses, menigovirus, and hepatitis A virus. The amount of a given compound of formula I or X that will correspond to a "therapeutically effective amount" will vary depending upon factors such as the particular compound, the disease condition and the severity thereof, the identity of the mammal in need thereof, but it can nevertheless be readily determined by one of skill in the art.

"Treating" or "treatment" is intended to mean at least the mitigation of a disease condition in a mammal, such as a human, that is alleviated by the inhibition of the activity of one or more picornaviral 3C proteases, such as human rhinoviruses, human poliovirus, human coxsackieviruses, encephalomyocarditis viruses, menigovirus, and hepatitis A virus, and includes:
(a) prophylactic treatment in a mammal, particularly when the mammal is found to be predisposed to having the disease condition but not yet diagnosed as having it;
(b) inhibiting the disease condition; and/or
(c) alleviating, in whole or in part, the disease condition.

The inventive compounds, and their salts, solvates, active metabolites, and prodrugs, may be prepared by employing the techniques available in the art using starting materials that are readily available. Certain novel and exemplary methods of preparing the inventive compounds are described below.

Preferably, the inventive compounds of formula I are prepared by the methods of the present invention, including the general methods shown below. In each of these general methods, R₁, R₂, R₃, R₅, R₆, R₇, R₂₀, R₄₁, R₄₂, Z, and Z₁ are as defined above (for formulae I, II, III, IV, and X).

### General Method I

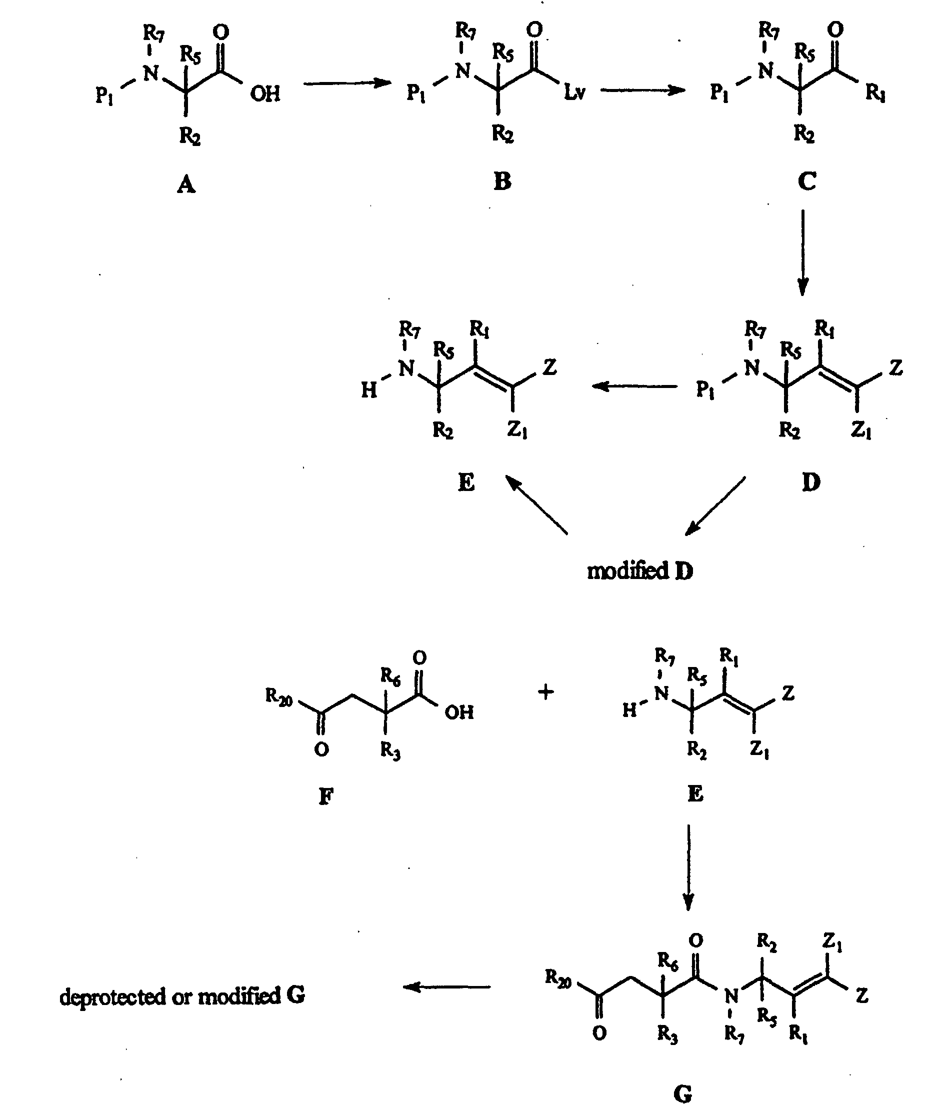

In General Method I, amino acid A, where P₁ is an appropriate protecting group for nitrogen, is converted to carbonyl derivative B, where "Lv" is a leaving group. Compound **B** is subjected to a reaction where "Lv" is replaced by R₁ to give derivative **C**. Derivative **C** is then transformed into unsaturated product **D**. Unsaturated compound **D** is deprotected to give free amine (or salt thereof) **E**, or modified one or more times at R₂, R₅, Z, and/or Z₁ to give one or more modified **D** compounds. Modified **D** is then deprotected to give amine (or salt thereof) **E**.

Amine **E** is subsequently subjected to an amide-forming reaction with carboxylic acid **F** (for which the preparation of representative examples is described below) to give final product **G**. If protecting groups were used on any R groups (R₁, R₂, R₃, R₅, R₆, R₇, and/or R₂₀), on Z and/or on Z₁, product **G** is deprotected and/or further modified to yield "deprotected or modified **G**."

### General Method II

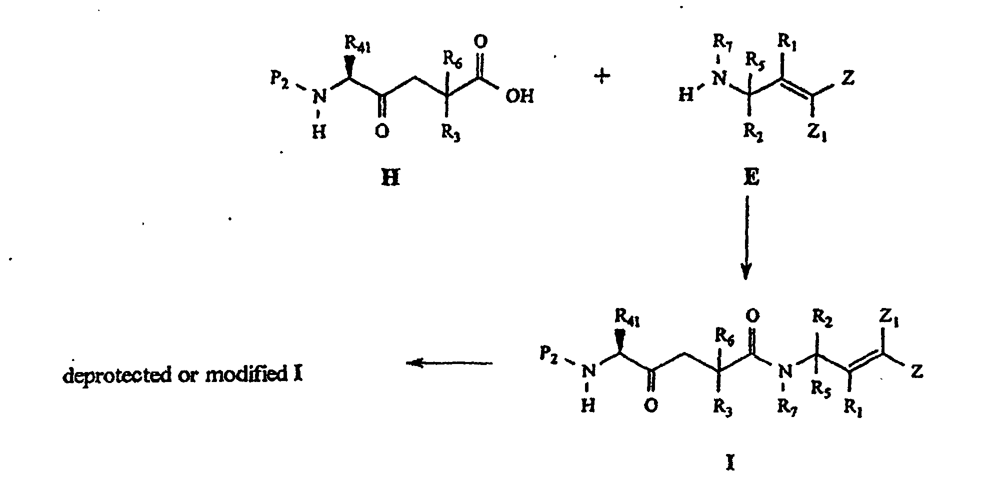

In General Method II, amine **E**, which can be prepared as described in General Method I, is subjected to an amide-forming reaction with carboxylic acid **H**, where P₂ is an appropriate protecting group for nitrogen, and where at least one of R₃ and R₆ is H, to give final product **I**. Carboxylic acid **H** can be prepared as a mixture of diastereomers as described in Harbeson, S.L., Rich, D.H., *J. Med. Chem.* **1989**, *32*, 1378. The P₂ protecting group, along with any additional protecting groups that were used on any R groups (R₁, R₂, R₃, R₅, R₆, R₇, and/or R₄₁), on Z and/or on Z₁, is subsequently deprotected and/or further modified to yield "deprotected or modified **I**."

### General Method III

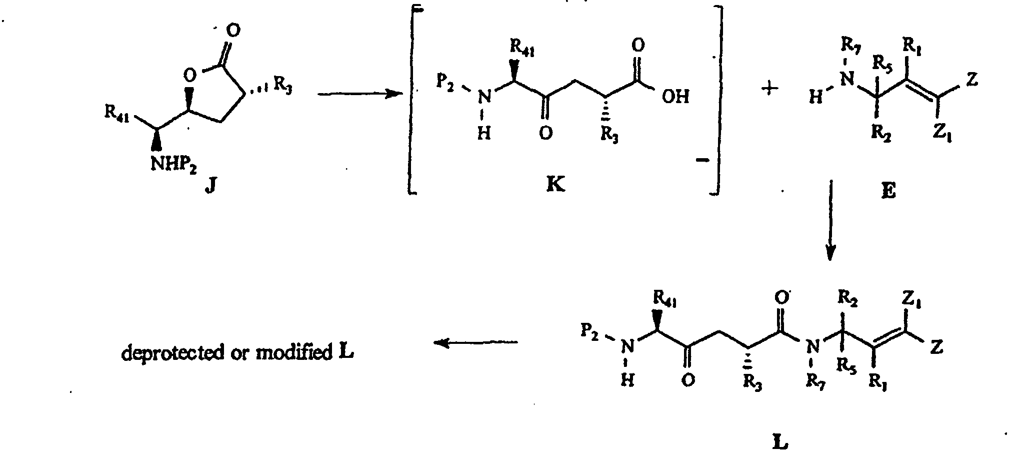

In General Method III, optically active lactone **J**, where P₂ is an appropriate protecting group for nitrogen, is transformed by a two-step procedure (basic hydrolysis and subsequent oxidation) into carboxylic acid **K**. Lactone **J** can be prepared by the method described in General Method IV below and by many literature methods including, but not limited to, those described in the following: (a) Herold, P.; Duthaler, R.; Rihs, G.; Angst, C., *J. Org. Chem*. **1989**, *54*, 1178; (b) Bradbury, R. H.; Revill, J. M.; Rivett, J. E.; Waterson, D., *Tetrahedron Lett.* **1989**, *30*, 3845; (c) Bradbury, R. H.; Major, J. S.; Oldham, A. A.; Rivett, J. E.; Roberts, D. A.; Slater, A. M.; Timms, D.; Waterson, D., *J. Med. Chem.* **1990**, *33*, 2335; (d) Wuts, P. G.; Ritter, A. R.; Pruitt, L. E., *J. Org. Chem*. **1992**, *57*, 6696; (e) Jones, D. M.; Nilsson, B.; Szelke, M., *J. Org. Chem*. **1993**, *58*, 2286; (f) Pégorier, L.; Larchevéque, M., *Tetrahedron Lett*. **1995**, *36*, 2753; (g) Dondoni, A.; Perrone, D.; Semola, M. T., *J. Org. Chem.* **1995**, *60*, 7927. Carboxylic acid **K** is not isolated in pure form, but is subjected to an amide-forming reaction with amine **E**, which can be prepared as described in General Method **I**, to provide final product **L**. The P₂ protecting group, along with any additional protecting groups that were used on any R groups (R₁, R₂, R₃, R₅, R₇, and/or R₄₁), on Z and/or on Z₁, is subsequently deprotected and/or further modified to yield "deprotected or modified **L**."

### General Method IV

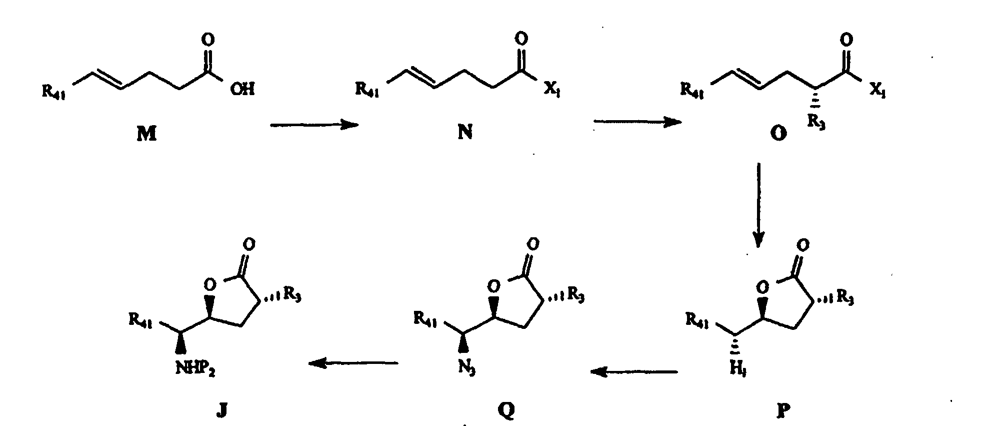

Lactone **J** may be prepared in optically active form by General Method IV (see: (a) Herold, P.; Duthaler, R.; Rihs, G.; Angst, C., *J. Org. Chem.* **1989**, *54*, 1178; (b) Bradbury, R. H.; Revill, J. M.; Rivett, J. E.; Waterson, D., *Tetrahedron Lett*. **1989**, *30*, 3845; (c) Bradbury, R. H.; Major, J. S.; Oldham, A. A.; Rivett, J. E.; Roberts, D. A.; Slater, A. M.; Timms, D.; Waterson, D., *J. Med*. *Chem*. **1990**, *33*, 2335). A γ,δ-unsaturated carboxylic acid **M**, which incorporates R₄₁, is transformed into the corresponding acid chloride (not shown). This acid chloride is subjected to an amide-forming reaction with a chiral amine or a chiral oxazolidone to provide derivative **N** (in which X₁ is a chiral amine or a chiral oxazolidone). Compound **N** is subsequently deprotonated, and the resulting enolate is diastereoselectively alkylated with an electrophile corresponding to R₃ to provide compound **O**. This material is then subjected to a halolactonization reaction to provide halo-lactone **P**, in which H₁ is Br or I. Halo-lactone **P** is subsequently transformed into azide **Q**, and this material is then converted into lactone **J**, where P₂ is an appropriate protecting group for nitrogen.

### General Method V

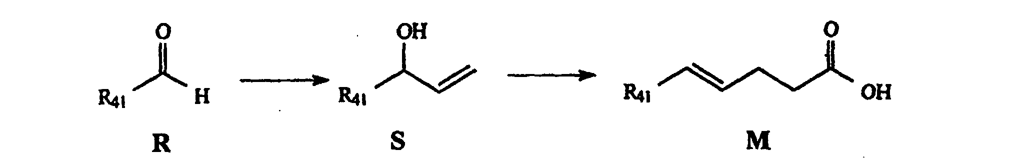

γ,δ-Unsaturated carboxylic acid **M** may be prepared by General Method V (see: Herold, P.; Duthaler, R.; Rihs, G.; Angst, C., *J. Org. Chem*. **1989**, *54*, 1178). An aldehyde R, which incorporates R₄₁, is coupled with vinylmagnesium bromide to give alcohol S. Alcohol S is then transformed into γ,δ-unsaturated carboxylic acid **M** by a three step procedure as follows: (i) treatment with diethyl malonate and catalytic Ti(OEt)₄ at 160 °C for 1 hour, (ii) heating at 190 °C for 4 hours, and (iii) hydrolysis with ethanolic KOH at reflux.

### General Method VI

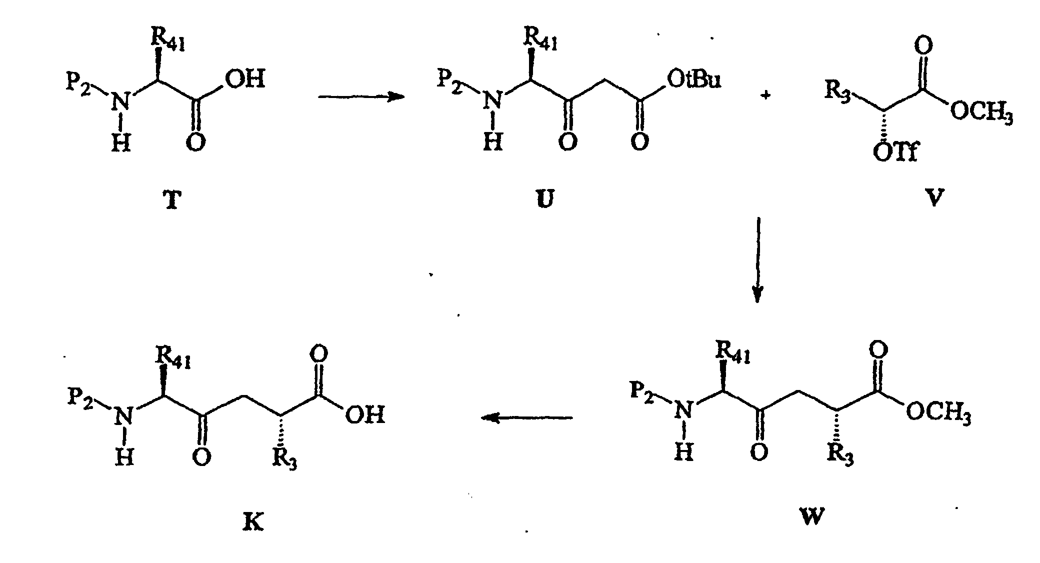

Carboxylic acid **K** also may be prepared by General Method VI (see: Hoffman, R. V., Tao, *J., Tetrahedron*, **1997,** *53*, 7119 ). An amino acid **T**, which incorporates R₄₁ and where P₂ is an appropriate protecting group for nitrogen, is transformed into β-ketoester **U**. Compound **U** is deprotonated, and the resulting anion is condensed with triflate **V**, which incorporates R₃. The coupling product thus obtained is treated with trifluoroacetic acid to provide ketoester W, and this material is subsequently hydrolyzed to afford carboxylic acid **K**. If basic hydrolysis results in epimerization, ketoester **W** can be transesterified [allyl alcohol, Ti(O*i*-Pr)₄] and subsequently deprotected under neutral conditions [Pd(PPh₃)₄, morpholine] to give carboxylic acid **K**. Triflate **V**, in turn, may be prepared from the corresponding alcohol by treatment with trifluoromethanesulfonic anhydride and 2,6-lutidine.

### General Method VII

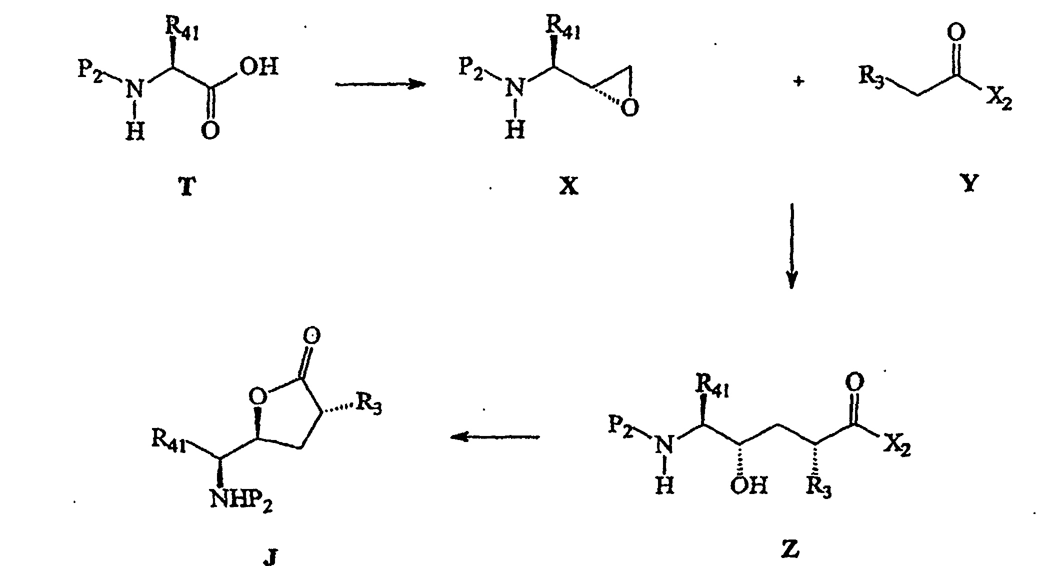

Lactone **J** also may be prepared by General Method VII (see: (a) Askin, D., Wallace, M. A., Vacca, J. P., Reamer, R. A., Volante, R. P., Shinkai, I. *J. Org. Chem*. **1992,** *57*, 2771 (b) McWilliams, J. C., Armstrong, J. D., Zheng, N., Bhupathy, M., Volante, R. P., Reider, P. J., *J. Am. Chem. Soc.* **1996,** *118*, 11970). An amino acid **T**, which incorporates R₄₁ and where P₂ is an appropriate protecting group for nitrogen, is transformed into epoxide **X** (single diastereomer) by the method described in: Luly, J. R., Dellaria, J. F., Plattner, J. J., Soderquist, J. L., Yi, N., *J. Org. Chem*. **1987,** *52*, 1487. Alternatively, **X** may be prepared from **T** as a mixture of diastereomers as described in the "Examples" section of this document. Epoxide **X** is condensed with the anion derived from compound **Y**, which incorporates R₃ and in which X₂ is a chiral auxiliary [including (1*S*,2*R*)-1-aminoindan-2-ol acetonide] to afford coupling product **Z**. If **X** was utilized as a mixture of diastereomers, the diastereomer of **Z** depicted below is purified from other **Z** isomers (if any) produced in the coupling reaction. This material is subsequently cyclized under acidic conditions to provide lactone **J**. Compound **Y** may be prepared from the corresponding carboxylic acid (not shown) by the method outlined in: Askin, D., Wallace, M. A., Vacca, J. P., Reamer, R. A., Volante, R. P., Shinkai, I., *J*. *Org. Chem.* **1992**, *57*, 2771.

Suitable protecting groups for nitrogen are recognizable to those skilled in the art and include, but are not limited to benzyloxycarbonyl, t-butoxycarbonyl, 9-fluorenylmethoxycarbonyl, p-methoxybenxyloxycarbonyl, trifluoroacetamide, and p-toluenesulfonyl. Suitable protecting groups for oxygen are recognizable to those skilled in the art and include, but are not limited to -CH₃, -CH₂CH₃, tBu, -CH₂Ph, -CH₂CH=CH₂, -CH₂OCH₂CH₂Si(CH₃)₃, and -CH₂CCl₃. Other examples of suitable protecting groups for nitrogen or oxygen can be found in T. Green & P. Wuts, Protective Groups in Organic Synthesis (2nd ed. 1991).

Suitable leaving groups also are recognizable to those skilled in the art and include, but are not limited to, Cl, Br, I, sulfonates, O-alkyl groups, 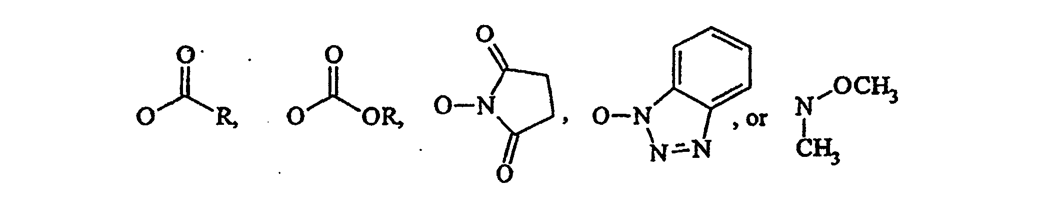 Other examples of suitable leaving groups are described in J. March, Advanced Organic Chemistry, Reactions, Mechanisms, and Structure (4th ed. 1992) at pages 205, 351-56, 642-43, 647, 652-53, 666, 501, 520-21, 569, 579-80, 992-94, 999-1000, 1005, and 1008

Examples of the processes used to make several of the compounds of formula I are set forth below. These Examples are intended to illustrate the present invention without limiting it. The structures of the compounds of the following examples were confirmed by one or more of the following: proton magnetic resonance spectroscopy, infrared spectroscopy, elemental microanalysis, mass spectrometry, thin layer chromatography, melting point, and boiling point.

Proton magnetic resonance (¹H NMR) spectra were determined using a Varian UNITY*plus* 300 spectrometer operating at a field strength of 300 megahertz (MHZ). Chemical shifts are reported in parts per million (ppm, δ) downfield from an internal tetramethylsilane standard. Alternatively, ¹H NMR spectra were referenced to residual protic solvent signals as follows: CHCl₃ = 7.26 ppm; DMSO = 2.49 ppm, C₆HD₅ = 7.15 ppm. Peak multiplicities are designated as follows: s, singlet; d, doublet; dd, doublet of doublets; t, triplet; q, quartet; br, broad resonance; m, multiplet. Coupling constants (*J*) are given in-hertz (Hz). Infrared absorption (IR) spectra were obtained using a. Perkin-Elmer 1600 series FTIR spectrometer. Elemental microanalyses were performed by Atlantic Microlab Inc., Norcross, GA and gave results for the elements stated within ±0.4% of the theoretical values.

Flash column chromatography was performed using Silica gel 60 (Merck Art 9385). Analytical thin layer chromatography (TLC) was performed using precoated sheets of Silica 60 F₂₅₄ (Merck Art 5719). Melting points were determined on a Mel-Temp apparatus and are uncorrected. All reactions were performed in septum-sealed flasks under a slight positive pressure of argon unless otherwise noted. All commercial reagents were used as received from their respective suppliers with the following exceptions. Tetrahydrofuran (THF) was distilled from sodium-benzophenone ketyl prior to use. Dichloromethane (CH₂Cl₂) was distilled from calcium hydride prior to use. Anhydrous lithium chloride was prepared by heating at 110 °C under vacuum overnight. Et₂O refers to diethyl ether. DMF refers to *N,N*-dimethylformamide. DMSO refers to dimethylsulfoxide. Other abbreviations include: CH,OH (methanol), EtOH (ethanol), EtOAc (ethyl acetate), DME (ethylene glycol dimethyl ether), Ac (acetyl), Me (methyl), Et (ethyl), Ph (phenyl), Bn (benzyl), CyPentyl (cyclopentyl), Tr (triphenylmethyl), CBZ (benzyloxycarbonyl), BOC (*tert*-butoxycarbonyl), Gln (glutamine), Leu (leucine), Phe (phenylalanine), Val (valine). Additionally, "L" represents naturally occurring amino acids, "D" represents unnatural amino acids, and "DL" represents a racemic mixture of the two.

A simplified naming system employing amino acid abbreviations is used to identify some intermediates and final products. When naming compounds, italicized amino acid abbreviations represent modifications at the C-terminus of that residue where the following apply:
1. acrylic acid esters are reported as "E" (trans) propenoates;
2. acrylamides are reported as "E" (trans) propenamides; and
3. N-acetyl-pyrrolidin-2-ones are reported as "E" (trans) 1-acetyl-3-methylene-pyrrolidin-2-ones.
In addition, the terminology "AA₁Ψ[COCH₂]-AA₂" indicates that, for any peptide sequence, two amino acids (AA₁ and AA₂) linked by an amide bond are replaced by a ketomethlyene dipeptide isostere moiety.

### Example 1 - Preparation of a Mixture of Compound 1 and Compound 9 (∼1:1): Ethyl-3-(CBZ-L-LeuΨ[COCH₂]-D/L-Phe-L-Gln)-E-Propenoate.

### Preparation of Intermediate [BOC-L-(Tr-Gln)]-N(OMe)Me.

Isobutyl chloroformate (4.77 mL, 36.8 mmol, 1.0 equiv) was added to a solution of [BOC-L-(Tr-Gln)]-OH (18.7 g, 36.7 mmol, 1 equiv) and 4-methylmorpholine (8.08 mL, 73.5 mmol, 2.0 equiv) in CH₂Cl₂ (250 mL) at 0 °C. The reaction mixture was stirred at 0 °C for 20 minutes, then *N,O*-dimethylhydroxylamine hydrochloride (3.60 g, 36.7 mmol, 1.0 equiv) was added. The resulting solution was stirred at 0 °C for 20 minutes and at 23 °C for 2 hours, and then was partitioned between water (150 mL) and CH₂Cl₂ (2 x 150 mL). The combined organic layers were dried over Na₂SO₄, and were concentrated. Purification of the residue by flash column chromatography (gradient elution, 40% - 20% hexanes in EtOAc) provided [BOC-L-(Tr-Gln)]-N(OMe)Me (16.1 g, 82%) as a white foam: R_{*f*} = 0.22 (50% EtOAc in hexanes); IR (cm⁻¹) 3411, 3329, 3062, 1701, 1659; ¹H NMR (CDCl₃) δ 1.42 (s, 9H), 1.63-1.77 (m, 1H), 2.06-2.17 (m, 1H), 2.29-2.43 (m, 2H), 3.17 (s, 3H), 3.64 (s, 3H), 4.73 (s, br, 1H), 5.38-5.41 (m, 1H), 7.20-7.31 (m, 15H); Anal. (C₃₁H₃₇N₃O₅) C, H, N.

### Preparation of Intermediate [BOC-L-(Tr-Gln)]-H.

Diisobutylaluminum hydride (50.5 mL of a 1.5 M solution in toluene, 75.8 mmol, 2.5 equiv) was added to a solution of [BOC-L-(Tr-Gln)]-N(OMe)Me (16.1 g, 30.3 mmol, 1 equiv) in THF at -78 °C, and the reaction mixture was stirred at -78 °C for 4 hours. Methanol (4 mL) and 1.0 M·HCl (10 mL) were added sequentially, and the mixture was warmed to 23 °C. The resulting suspension was diluted with Et₂O (150 mL) and was washed with 1.0 M HCl (3 x 100 mL), half-saturated NaHCO₃ (100 mL), and water (100 mL). The organic layer was dried over MgSO₄, filtered, and concentrated to give crude [BOC-L-(Tr-Gln)]-H (13.8 g, 97%) as a white solid: mp = 114-116 °C; R_{*f*} = 0.42 (50% EtOAc in hexanes); IR (cm⁻¹) 3313, 1697, 1494; ¹H NMR (CDCl₃) δ 1.44 (s, 9H), 1.65-1.75 (m, 1H), 2.17-2.23 (m, 1H), 2.31-2.54 (m, 2H), 4.11 (s, br, 1H), 5.38-5.40 (m, 1H). 7.11 (s, 1H), 7.16-7.36 (m, 15H), 9.45 (s, 1H).

### Preparation of Intermediate Ethyl-3-[BOC-L-(Tr-Gln)]-E-Propenoate.

Sodium bis(trimethylsilyl)amide (22.9 mL of a 1.0 M solution in THF, 22.9 mmol, 1.0 equiv) was added to a solution of triethyl phosphonoacetate (5.59 g, 22.9 mmol, 1.0 equiv) in THF (200 mL) at -78 °C, and the resulting solution was stirred for 20 minutes at that temperature. Crude [BOC-L-(Tr-Gln)]-H (10.8 g, 22.9 mmol, 1 equiv) in THF (50 mL) was added via cannula, and the reaction mixture was stirred for 2 hours at -78 °C, warmed to 0 °C for 10 minutes, and partitioned between 0.5 M HCl (150 mL) and a 1:1 mixture of EtOAc and hexanes (2 x 150 mL). The combined organic layers were dried over Na₂SO₄ and concentrated. Purification of the residue by flash column chromatography (40% EtOAc in hexanes) provided ethyl-3-[BOC-L-(Tr-*Gln*)]-E-propenoate (10.9 g, 88%) as a white foam: R_{*f*} = 0.60 (50% EtOAc in hexanes); IR (cm⁻¹) 3321, 1710; ¹H NMR (CDCl₃) δ 1.27 (t, 3H, *J* = 7.2), 1.42 (s, 9H), 1.70-1.78 (m, 1H), 1.80-1.96 (m, 1H), 2.35 (t, 2H, *J* = 7.0), 4.18 (q, 2H, *J* = 7.2), 4.29 (s, br, 1H), 4.82-4.84 (m, 1H), 5.88 (dd, 1H, *J* = 15.7, 1.6), 6.79 (dd, 1H, *J* = 15.7, 5.3), 6.92 (s, 1H), 7.19-7.34 (m, 15H); Anal. (C₃₃H₃₈N₂O₅) C, H, N.

### Preparation of Intermediate Ethyl-3-[CBZ-L-LeuΨ[COCH₂]-D/L-Phe-L-(Tr-Gln)]-E-Propenoate.

HCl (3 mL of a 4.0 M solution in 1,4-dioxane) was added to a solution of ethyl-3-[BOC-L-(Tr-*Gln*)]-E-propenoate (0.091 g, 0.17 mmol, 1 equiv) in 1,4-dioxane (3 mL) at 23 °C. The reaction mixture was stirred at 23 °C for 1.5 hours, then was concentrated under reduced pressure to afford crude ethyl-3-[H₂N-L-(Tr-*Gln*)]-E-propenoate•HCl as a viscous oil. This material was dissolved in CH₂Cl₂ (6 mL) and CBZ-L-LeuΨ[COCH₂]-D/L-Phe-OH (0.068 g, 0.17 mmol, 1.0 equiv) [prepared as described in: Harbeson, S. L., Rich, D. H., *J. Med. Chem.* **1989**, *32*, 1378], 1-hydroxybenzotriazole hydrate (0.030 g, 0.22 mmol, 1.3 equiv), 4-methylmorpholine (0.055 mL, 0.50 mmol, 3.0 equiv), and 1-(3-dimethylaminopropyl)-3-ethylcarbodiimide hydrochloride (0.042 g, 0.22 mmol, 1.3 equiv) were added sequentially. The reaction mixture was stirred for 21 hours at 23 °C and then partitioned between water (100 mL) and EtOAc (2 x 100 mL). The combined organic layers were dried over Na₂SO₄ and were concentrated. Purification of the residue by flash column chromatography (50% EtOAc in hexanes) provided ethyl-3-[CBZ-L-LeuΨ[COCH₂]-D/L-Phe-L-(Tr-*Gln*)]-E-propenoate (0.040 g, 28%) as a white foam: R_{*f*} = 0.44 (50% EtOAc in hexanes); IR (cm⁻¹) 3317, 1712, 1667; ¹H NMR (CDCl₃, approximately 1:1 mixture of diastereomers) δ 0.84-0.91 (m), 1.20-1.31 (m), 1.51-1.58 (m), 1.73-1.96 (m), 2.29-2.39 (m), 2.51-2.72 (m), 2.94-3.07 (m), 4.11-4.30 (m), 4.47-4.50 (m), 4.84 (d, *J* = 7.8 Hz), 4.94-5.08 (m), 5.09 (s), 5.30 (d, *J* = 7.2 Hz), 5.48 (d, *J* = 14.3 Hz), 5.72-5.95 (m), 6.55-7.01 (m), 7.14-7.61 (m), 8.02-8.05 (m); Anal. (C₅₂H₅₇N₃O₇•0.75H₂O) C, H, N.

### Preparation of Product Ethyl-3-(CBZ-L-LeuΨ[COCH₂]-D/L-Phe-L-Gln)-E-Propenoate.

Triisopropylsilane (0.10 mL) and trifluoroacetic acid (3 mL) were added sequentially to a solution of ethyl-3-[CBZ-L-LeuΨ[COCH₂]-D/L-Phe-L-(Tr-*Gln*)]-E-propenoate (0.035 g, 0.042 mmol) in CH₂Cl₂ (4 mL) producing a bright yellow solution. The reaction mixture was stirred for 30 minutes at 23 °C, then carbon tetrachloride (4 mL) was added, and the mixture was concentrated under reduced pressure. The residue was purified by flash column chromatography (5% CH₃OH in CH₂Cl₂) to afford ethyl-3-(CBZ-L-LeuΨ[COCH₂]-D/L-Phe-L-*Gln*)-E-propenoate (0.014 g, 56%) as a white foam: R_{*f*} = 0.39 (10% CH₃OH in CH₂Cl₂); IR (cm⁻¹) 3306, 1712, 1661; ¹H NMR (CDCl₃, approximately 1:1 mixture of diastereomers) δ 0.87-0.93 (m), 1.24-1.33 (m), 1.39-1.96 (m), 2.17-2.21 (m), 2.58-2.79 (m), 2.87-3.09 (m), 4.10-4.27 (m), 4.44 (s, br), 4.55 (s, br), 5.01-5.10 (m), 5.14-5.69 (m), 5.79 (s), 5.82-5.91 (m), 6.13 (d, *J* = 7.5 Hz), 6.42 (s, br), 6.59 (dd, *J* = 16.0, 7.5 Hz), 6.74 (dd, *J* = 15.6, 4.7 Hz), 7.16-7.39 (m); Anal. (C₃₃H₄₃N₃O₇) C, H, N.

### Example 2 - Preparation of Compound 1; Ethyl-3-(CBZ-L-LeuΨ[COCH₂]-L-Phe-L-Gln)-E-Propenoate.

### Preparation of Intermediate trans-7-Methyl-oct-4-enoic Add.

A solution of isovaleraldehyde (8.61 g, 100 mmol, 1 equiv) in THF (30 mL) was added dropwise via addition funnel to a solution of vinylmagnesium bromide (100 mL of a 1.0 M solution in THE, 100 mmol, 1.0 equiv) in THE (150 mL) at 0 °C. After the addition was completed, the reaction mixture was stirred for 30 minutes at 0 °C, and then was partitioned between saturated NH₄Cl (150 mL) and Et₂O (2 x 150 mL). The combined organic layers were dried over Na₂SO₄ and concentrated to afford a yellow oil. This material was combined (neat) with diethyl malonate (16.7 mL, 110 mmol, 1.1 equiv) and Ti(OEt)₄ (2.10 mL, 10.0 mmol, 0.10 equiv), and the combination was heated to 160 °C for 1 hour (distilling out EtOH as it was formed). The reaction mixture was then maintained at 190 °C for 4 hours and then cooled to 60 °C. EtOH (50 mL) and 6.0 M KOH (50 mL) were added sequentially, and the brown reaction mixture was refluxed for 4 hours. After cooling to 23 °C, the reaction mixture was filtered through a medium frit, and the filtrate was partitioned between water (150 mL) and Et₂O (2 x 150 mL). The aqueous layer was then acidified to pH = 2 (as indicated by pH paper) with concentrated HCl and extracted with a 1:1 mixture of EtOAc and hexanes (2 x 150 mL). The combined organic layers were dried over Na₂SO₄, concentrated, and the residue was distilled at reduced pressure to afford trans-7-methyl-oct-4-enoic acid (4.62 g, 30%) as a colorless liquid: bp: 115-120 °C (1 torr); ¹H NMR (CDCl₃) δ 0.86 (d, 6H, *J* = 5.5), 1.51-1.65 (m, 1H), 1.87 (t, 2H, *J* = 6.5), 2.22-2.38 (m, 2H), 2.40-2.45 (m, 2H), 5.34-5.52 (m, 2H); Anal. (C₉H₁₆O₂) C, H.

### Preparation of Intermediate trans-7-Methyl-oct-4-enoic Acid (2R-Hydroxy-1R-methyl-2-phenyl-ethyl)-methyl Amide.

Oxalyl chloride (2.71 mL, 31.1 mmol, 1.05 equiv) was added to a solution of trans-7-methyl-oct-4-enoic acid (4.62 g, 29.6 mmol, 1 equiv) and *N,N*-dimethylformamide (0.03 mL, 0.39 mmol, 0.012 equiv) in benzene (100 mL) at 23 °C. The reaction mixture was stirred at 23 °C for 2 hours and then concentrated under reduced pressure. The resulting oil was dissolved in THF (20 mL) and added via cannula to a solution of (1*R*,2*R*)-(-)-pseudoephedrine (4.45 g, 26.9 mmol, 0.91 equiv) and triethylamine (4.50 mL, 32.3 mmol, 1.1 equiv) in THF (200 mL) at 0 °C. The reaction mixture was stirred at 0 °C for 30 minutes and then partitioned between half-saturated NH₄Cl (150 mL) and EtOAc (2 x 150 mL). The combined organic layers were dried over Na₂SO₄, concentrated, and the residue purified by flash column chromatography (gradient elution 40 → 50% EtOAc in hexanes) to afford trans-7-methyl-oct-4-enoic acid (2*R*-hydroxy-1*R*-methyl-2-phenyl-ethyl)-methyl amide (7.55 g, 93%) as a viscous oil: R_{*f*} = 0.27 (50% EtOAc in hexanes); IR (cm⁻¹) 3382, 1622; ¹H NMR (CDCl₃, mixture of rotamers) δ 0.87 (d, *J* = 6.5), 0.99 (d, *J* = 6.8), 1.11 (d, *J* = 7.2), 1.53-1.66 (m), 1.86 (t, *J* = 6.1), 2.26-2.54 (m), 2.82 (s), 2.92 (s), 3.99-4.04 (m), 4.29 (s, br), 4.42-4.47 (m), 4.56-4.62 (m), 5.37-5.51 (m), 7.26-7.36 (m); Anal. (C₁₉H₂₉NO₂) C, H, N.

### Preparation of Intermediate trans-2S-Benzyl-7-methyl-oct-4-enoic Acid (2R-Hydroxy-1R-methyl-2-phenyl-ethyl)-methyl Amide.

*n*-Butyllithium (52.6 mL of a 1.6 M solution in hexanes, 84.2 mmol, 2.1 equiv) was added to a suspension of anhydrous lithium chloride (11.9 g, 282 mmol, 7.0 equiv) and diisopropylamine (12.7 mL, 90.3 mmol, 2.25 equiv) in THF (300 mL) at -78 °C. The reaction mixture was stirred for 20 minutes at -78 °C and then maintained at 0 °C for 5 minutes and subsequently cooled again to -78 °C. trans-7-Methyl-oct-4-enoic acid (2*R*-hydroxy-1*R*-methyl-2-phenyl-ethyl)-methyl amide (12.2 g, 40.1 mmol, 1 equiv) in THF (40 mL) was added via cannula, and the resulting solution was stirred at -78 °C for 1 hour, maintained at 0 °C for 15 minutes, stirred at 23 °C for 5 minutes, and then cooled again to 0 °C. Benzyl bromide (7.15 mL, 60.1 mmol, 1.5 equiv) was added, and the reaction mixture was stirred at 0 °C for 30 minutes and then partitioned between half-saturated NH₄Cl (200 mL) and a 1:1 mixture of EtOAc and hexanes (2 x 200 mL). The combined organic layers were dried over Na₂SO₄ and concentrated. Purification of the residue by flash column chromatography (gradient elution 20 → 40% EtOAc in hexanes) provided trans-2*S*-benzyl-7-methyl-oct-4-enoic acid (2*R*-hydroxy-1*R*-methyl-2-phenyl-ethyl)-methyl amide (12.0 g, 76%) as a viscous oil: R_{*f*} = 0.54 (50% EtOAc in hexanes); IR (cm⁻¹) 3382, 1617; ¹H NMR (CDCl₃, mixture of rotamers) δ 0.81-0.90 (m), 1.42-1.61 (m), 1.80-1.95 (m), 2.17-2.25 (m), 2.33-2.54 (m), 2.55 (s), 2.73-2.99 (m), 3.05-3.16 (m), 3.93-4.00 (m), 4.31-4.51 (m), 5.25-5.56 (m), 7.14-7.37 (m); Anal. (C₂₆H₃₅NO₂) C, H, N.

### Preparation of Intermediate 3R-Benzyl-5S-(1R-bromo-3-methyl-butyl)-dihydrofuran-2-one.

*N-*Bromosuccinimide (5.97 g, 33.5 mmol, 1.1 equiv) was added in small portions over 5 minutes to a solution of trans-2*S*-benzyl-7-methyl-oct-4-enoic acid (2*R*-hydroxy-1*R*-methyl-2-phenyl-ethyl)-methyl amide (12.0 g, 30.5 mmol, 1 equiv) and glacial acetic acid (8.73 mL, 152 mmol, 5.0 equiv) in a 4:1 mixture of THE and H₂O (250 mL) at 0 °C. The resulting yellow solution was stirred for 15 minutes at 0 °C, then warmed to 23 °C, and subsequently refluxed for 1 hour. After cooling to 23 °C, the reaction mixture was partitioned between half-saturated NaHCO₃ (300 mL) and a 1:1 mixture of EtOAc and hexanes (2 x 200 mL). The combined organic layers were dried over Na₂SO₄ and concentrated. Flash chromatographic purification of the residue (5% EtOAc in hexanes) gave 3*R*-benzyl-5*S*-(1*R*-bromo-3-methyl-butyl)-dihydrofuran-2-one (7.09 g, 65%) as a pale yellow oil: R_{*f*} = 0.79 (30% EtOAc in hexanes); IR (cm⁻¹) 1777; ¹H NMR (CDCl₃) δ 0.87 (d, 3H, *J =* 6.5), 0.94 (d, 3H, *J* = 6.9), 1.53-1.72 (m, 2H), 1.82-1.93 (m, 1H), 2.10-2.20 (m, 1H), 2.23-2.33 (m, 1H), 2.83 (dd, 1H, *J* = 13.5, 8.9), 3.04-3.12 (m, 1H), 3.14-3.22 (m, 1H), 4.05-4.12 (m, 1H), 4.23-4.29 (m, 1H), 7.20-7.36 (m, 5H); Anal. (C₁₆H₂₁BrO₂) C; H, N.

### Preparation of Intermediate 5S-(1S-Azido-3-methyl-butyl)-3R-benzyldihydrofuran-2-one.

A suspension of sodium azide (2.83 g, 43.5 mmol, 2.0 equiv) and 3*R*-benzyl-5*S*-(1*R*-bromo-3-methyl-butyl)-dihydrofuran-2-one (7.09 g, 21.8 mmol, 1 equiv) in *N,N*-dimethylformamide (50 mL) was heated at 50 °C for 20 hours. The reaction mixture was cooled to 23 °C and partitioned between water (200 mL) and a 1:1 mixture of EtOAc and hexanes (2 x 200 mL). The combined organic layers were dried over Na₂SO₄, concentrated, and the residue purified by flash column chromatography (10% EtOAc in hexanes) to give 5*S*-(1*S*-azido-3-methyl-butyl)-3R-benzyl- dihydrofuran-2-one (3.26 g, 52%) as a colorless oil: R_{*f*} = 0.47 (20% EtOAc in hexanes); IR (cm⁻¹) 2109, 1775; ¹H NMR (CDCl₃) δ 0.93 (d, 3H, *J* = 6.5), 0.94 (d, 3H, *J* = 6.5), 1.32-1.41 (m, 1H), 1.55-1.65 (m, 1H), 1.70-1.85 (m, 1H), 2.03-2.18 (m, 2H), 2.80 (dd, 1H, *J* = 13.5, 8.9), 3.05-3.22 (m, 2H), 3.27-3.33 (m, 1H), 4.22-4.27 (m, 1H), 7.18-7.36 (m, 5H); Anal. (C₁₆H₂₁N₃O₂) C, H, N.

### Preparation of Intermediate [1S-(4R-Benzyl-5-oxo-tetrahydrofuran-2S-yl)-3-methyl-butyl]-carbamic Add tert-Butyl Ester.

A suspension of 5*S*-(1*S*-azido-3-methyl-butyl)-3*R*-benzyl-dihydrofuran-2-one (3.26 g, 11.3 mmol, 1 equiv) and Pd/C (10%, 0.40 g) in CH₃OH (60 mL) was stirred under a hydrogen atmosphere (balloon) for 2 hours. The reaction mixture was filtered through celite, concentrated, and the residue dissolved in 1,4-dioxane (80 mL). *N,N*-diisopropylethylamine (3.94 mL, 22.6 mmol, 2.0 equiv) and di-*tert*-butyl dicarbonate (3.70 g, 17.0 mmol, 1.5 equiv) were added sequentially, and the resulting solution was stirred at 23 °C for 2 hours. The reaction mixture was then partitioned between water (150 mL) and a 1:1 mixture of EtOAc and hexanes (2 x 150 mL). The combined organic layers were dried over Na₂SO₄ and concentrated. Purification of the residue by flash column chromatography (gradient elution, 10 → 15% EtOAc in hexanes) provided [1*S*-(4*R*-benzyl-5-oxo-tetrahydrofuran-2*S*-yl)-3-methyl-butyl]-carbamic acid *tert*-butyl ester (2.53 g, 62%) as a white solid: mp = 84-86 °C; R_{*f*} = 0.66 (30% EtOAc in hexanes); IR (cm⁻¹) 3338, 1767, 1704; ¹H NMR (CDCl₃) δ 0.89 (d, 3H, *J* = 6.5), 0.90 (d, 3H, *J* = 6.5), 1.18-1.32 (m, 1H), 1.40 (s, 9H), 1.43-1.56 (m, 1H), 1.98-2.07 (m, 1H), 2.20-2.29 (m, 1H), 2.78 (dd, 1H, *J* = 13.7, 9.0), 2.91-3.01 (m, 1H), 3.15 (dd, 1H, *J* = 13.7, 4.4), 3.71-3.81 (m, 1H), 4.23-4.28 (m, 1H), 4.34 (d, 1H, *J* = 9.7), 7.16-7.33 (m, 6H); Anal. (C₂₁H₃₁NO₄) C, H, N.

### Preparation of Intermediate Ethyl-3-[BOC-L-LeuΨ[COCH₂]-L-Phe-L-(Tr-Gln)]-E-Propenoate.

Lithium hydroxide (7.2 mL of a 1 M aqueous solution, 7.2 mmol, 5.0 equiv) was added to a solution of [1*S*-(4*R*-benzyl-5-oxo-tetrahydrofuran-2*S*-yl)-3-methyl-butyl]-carbamic acid *tert*-butyl ester (0.521 g, 1.44 mmol, 1 equiv) in DME (7 mL) at 23 °C. The resulting suspension was stirred at 23 °C for 30 minutes and then partitioned between 0.5 M HCl (100 mL) and EtOAc (2 x 100 mL). The combined organic layers were dried over Na₂SO₄, concentrated, and the residue dissolved in CH₂Cl₂ (30 mL). 4-Methylmorpholine *N*-oxide (0.337 g, 2.88 mmol, 2.0 equiv), powdered 4Å molecular sieves (0.55 g), and tetrapropylammonium perruthenate (0.050 g, 0.142 mmol, 0.10 equiv) were added sequentially. The resulting dark reaction mixture was stirred for 2.5 hours at 23 °C, and then it was filtered through celite. The filtrate was concentrated under reduced pressure to provide a brown oil which was dissolved in CH₂Cl₂ (30 mL). Crude ethyl-3-[H₂N-L-(Tr-*Gln*)]-E-propenoate•HCl (2.87 mmol, 2.0 equiv, prepared as described in Example 1 for the preparation of ethyl-3-[CBZ-L-LeuΨ[COCH₂]-D/L-Phe-L-(Tr-*Gln*)]- E-propenoate), 1-hydroxybenzotriazole hydrate (0.409 g, 3.03 mmol, 2.1 equiv), 4-methylmorpholine (0.633 mL, 5.76 mmol, 4.0 equiv), and 1-(3-dimethylaminopropyl)-3-ethylcarbodiimide hydrochloride (0.58 g, 3.03 mmol, 2.1 equiv) were added sequentially, and the reaction mixture was stirred for 15 hours at 23 °C and then partitioned between water (150 mL) and EtOAc (2 x 150 mL). The combined organic layers were dried over Na₂SO₄ and concentrated. Purification of the residue by flash column chromatography (gradient elution 30 → 40% EtOAc in hexanes) provided ethyl-3-[BOC-L-LeuΨ[COCH₂]-L-Phe-L-(Tr-*Gln*)]-E-propenoate (0.502 g, 44%) as a white foam: R_{*f*} = 0.49 (50% EtOAc in hexanes); IR (cm⁻¹) 3314, 1707, 1667; ¹H NMR (CDCl₃) δ 0.84-0.91 (m, 6H), 1.29 (t, 3H, *J* = 7.2); 1.39 (s, 9H), 1.42-1.61 (m, 4H), 1.98-2.05 (m, 1H), 2.35 (t, 2H, *J* = 7.2), 2.54 (d, 1H, *J* = 16.2), 2.70 (dd, 1H, *J* = 11.5, 5.6), 2.78-2.99 (m, 3H), 4.07-4.10 (m, 1H), 4.17 (q, 2H, *J* = 7.2), 4.47 (s, br, 1H), 4.58 (d, 1H, *J* = 7.5), 5.46 (d, 1H, *J* = 15.8), 5.87 (d, 1H, *J* = 8.7), 6.58 (dd, 1H, *J* = 15.8, 5.1), 7.12-7.31 (m, 21H); Anal. (C₄₉H₅₉N₃O₇) C, H, N.

### Preparation of Intermediate Ethyl-3-[CBZ-L-LeuΨ[COCH₂]-L-Phe-L-(Tr-Gln)]-E-Propenoate.

HCl (5 mL of a 4.0 M solution in 1,4-dioxane) was added to a solution of ethyl-3-[BOC-L-LeuΨ[COCH₂]-L-Phe-L-(Tr-*Gln*)]-E-Propenoate (0.096 g, 0.120 mmol, 1 equiv) in 1,4-dioxane (5 mL). The reaction mixture was stirred at 23 °C for 30 minutes and then concentrated. The resulting oil was dissolved in CH₂Cl₂ (10 mL), and 4-methylmorpholine (0.033 mL, 0.300 mmol, 2.5 equiv) and benzyl chloroformate (0.025 mL, 0.175 mmol, 1.4 equiv) were added sequentially. The reaction mixture was stirred for 30 minutes at 23 °C and then partitioned between water (100 mL) and a 1:1 mixture of EtOAc and hexanes (2 x 100 mL). The combined organic layers were dried over Na₂SO₄, concentrated, and the residue was chromatographed on silica gel (40% EtOAc in hexanes) to afford ethyl-3-[CBZ-L-LeuΨ[COCH₂]-L-Phe-L-(Tr-*Gln*)]-E-propenoate (0.050 g, 50%) as a white foam: R_{*f*} = 0.38 (50% EtOAc in hexanes); IR (cm-') 3318, 1712, 1664; ¹H NMR (CDCl₃) δ 0.85-0.87 (m, 6H), 1.05-1.13 (m, 1H), 1.29 (t, 3H, *J* = 7.2); 1.35-1.42 (m, 2H), 1.97 (s, br, 1H), 2.29-2.31 (m, 2H), 2.54 (d, 1H. *J* = 16.5), 2.69-3.03 (m, 5H), 4.17 (q, 2H, *J* = 7.2), 4.47 (s, br, 1H), 4.81 (d, 1H, *J* = 7.5), 4.94-5.06 (m, 3H), 5.48 (d, 1H, *J* = 15.6), 5.87 (d, 1H, *J* = 8.1), 6.58 (dd, 1H, *J* = 15.6, 5.0), 7.12-7.32 (m, 26H); Anal. (C₅₂H₅₇N₃O₇•0.25 H₂O) C, H, N.

### Preparation of Product Ethyl-3-(CBZ-L-LeuΨ[COCH₂]-L-Phe-L-Gln)-E-Propenoate.

Triisopropylsilane (0.10 mL) and trifluoroacetic acid (5 mL) were added sequentially to a solution of ethyl-3-[CBZ-L-LeuΨ[COCH₂]-L-Phe-L-(Tr-*Gln*)]-E-propenoate (0.050 g, 0.060 mmol) in CH₂Cl₂ (6 mL) producing a bright yellow solution. The reaction mixture was stirred for 30 minutes at 23 °C, then carbon tetrachloride (4 mL) was added, and the mixture was concentrated under reduced pressure. The residue was purified by flash column chromatography (5% CH₃OH in CH₂Cl₂) to afford ethyl-3-(CBZ-L-LeuΨ[COCH₂]-L-Phe-L-*Gln*)-E-propenoate (0.026 g, 73%) as a white solid: mp = 162-164 °C; R_{*f*} = 0.66 (10% CH,OH in CH₂Cl₂); IR (cm⁻¹) 3412, 3292, 1718, 1689, 1650; ¹H NMR (CDCl₃) δ 0.92 (d, 6H, *J* = 6.5), 1.30 (t, 3H, *J* = 7.2), 1.45-1.59 (m, 3H), 1.97-2.05 (m, 1H), 2.07 (d, 1H, *J* = 15.9), 2.17 (s, br, 2H), 2.70-2.79 (m, 1H), 2.91-3.09 (m, 3H), 4.18 (q, 2H, *J* = 7.2), 4.23-4.27 (m, 1H), 4.54 (s, br, 1H), 5.03 (d, 1H, *J* = 12.1), 5.08 (d, 1H, *J* = 12.1), 5.23 (d, 1H, *J* = 6.9), 5.38 (s, br, 1H), 5.47 (d, 1H, *J =* 15.6), 5.92 (d, 1H, *J* = 8.7), 6.43 (s, br, 1H), 6.60 (dd, 1H, *J* = 15.6, 4.8), 7.16-7.39 (m, 11H); Anal. (C₃₃H₄₃N₃O₇) C, H, N.

### Example 3 - Preparation of Compound 2: Ethyl-3-(EtSCO-L-LeuΨ[COCH₂]-L-Phe-L-Gln)-E-Propenoate.

### Preparation of Intermediate Ethyl-3-[EtSCO-L-LeuΨ[COCH₂]-L-Phe-L-(Tr-Gln)]-E-Propenoate.

HCl (4 mL of a 4.0 M solution in 1,4-dioxane) was added to a solution of ethyl-3-[BOC-L-LeuΨ[COCH₂]-L-Phe-L-(Tr-*Gln*)]-E-propenoate (0.216 g, 0.269 mmol, 1 equiv) in 1,4-dioxane (6 mL). The reaction mixture was stirred at 23 °C for 1.5 hours and then concentrated. The resulting oil was dissolved in CH₂Cl₂ (6 mL), cooled to 0 °C, and *N,N*-diisopropylethylamine (0.094 mL, 0.540 mmol, 2.0 equiv) and ethyl chlorothiolformate (0.034 mL, 0.326 mmol, 1.2 equiv) were added sequentially. The reaction mixture was stirred for 1 hour at 0 °C, and it then was partitioned between water (100 mL) and EtOAc (2 x 100 mL). The combined organic layers were dried over Na₂SO₄, concentrated, and the residue was purified by flash column chromatography (40% EtOAc in hexanes) to afford ethyl-3-[EtSCO-L-LeuΨ[COCH₂]-L-Phe-L-(Tr-*Gln*)]-E-propenoate (0.130 g, 61%) as a white foam: R_{*f*} = 0.45 (50% EtOAc in hexanes); IR (cm⁻¹) 3307, 1713, 1656; ¹H NMR (CDCl₃) δ 0.86 (d, 6H, *J* = 6.5), 1.05-1.19 (m, 1H), 1.21-1.39 (m, 8H), 1.41-1.58 (m, 2H), 1.96-2.05 (m, 1H), 2.28-2.35 (m, 2H), 2.54 (d, 1H, *J* = 14.6), 2.70 (dd, 1H, *J* = 11.7, 5.8), 2.79-3.00 (m, 4H), 4.17 (q, 2H, *J* = 7.2), 4.41-4.45 (m, 2H), 5.40 (d, 1H, *J* = 7.5), 5.49 (dd, 1H, *J* = 15.8, 1.6), 5.93 (d, 1H, *J* = 8.4), 6.59 (dd, 1H, *J* = 15.8, 5.1), 7.10-7.31 (m, 21H); Anal. (C₄₇H₅₅N₃O₆S) C, H, N.

### Preparation of Product Ethyl-3-(EtSCO-L-LeuΨ[COCH₂]-L-Phe-L-Gln)-E-Propenoate

Ethyl-3-[EtSCO-L-LeuΨ[COCH₂]-L-Phe-L-(Tr-*Gln*)]-E-propenoate (0.120 g, 0.152 mmol) was dissolved in CH₂Cl₂ (5 mL), and triisopropylsilane (0.10 mL) and trifluoroacetic acid (5 mL) were added sequentially producing a bright yellow solution. The reaction mixture was stirred for 30 minutes at 23 °C, then carbon tetrachloride (6 mL) was added, and the mixture was concentrated under reduced pressure. The residue was purified by flash column chromatography (5% CH₃OH in CH₂Cl₂) to afford ethyl-3-(EtSCO-L-LeuΨ[COCH₂]-L-Phe-L-*Gln*)-E-propenoate (0.056 g, 68%) as a beige foam: R_{*f*} = 0.55 (10% CH₃OH in CH₂Cl₂); IR (cm⁻¹) 3385, 2293, 3199, 1717, 1653; ¹H NMR (CDCl₃) δ 0.91 (d, 3H, *J* = 6.2), 0.92 (d, 3H, *J* = 6.5), 1.21-1.38 (m, 8H), 1.46-1.65 (m, 2H), 1.83-2.05 (m, 1H), 2.61 (d, 1H, *J* = 14.9), 2.74-3.09 (m, 7H), 4.18 (q, 2H, *J* = 7.2), 4.49-4.56 (m, 2H), 5.49 (d, 1H, *J* = 15.6), 5.59 (s, br, 1H), 6.05 (d, 1H, *J* = 8.7), 6.20 (d, 1H, *J* = 6.9), 6.49 (s, br, 1H), 6.62 (dd, 1H, *J* = 15.6, 4.8), 7.16-7.32 (m, 6H); Anal. (C₂₈H₄₁N₃O₆S) C, H, N.

### Example 4 - Preparation of Compound 6: Ethyl-3-(BnSCO-L-LeuΨ[COCH₂]-L-Phe-L-Gln)-E-Propenoate.

### Preparation of Intermediate Benzyl Chlorothiolformate

Triethylamine (7.12 mL, 51.1 mmol, 1.0 equiv) was added to a 0 °C solution of benzyl mercaptan (6.0 mL, 51.1 mmol, 1 equiv) and triphosgene (5.76 g, 19.4 mmol, 0.38 equiv) in CH₂Cl₂ (100 mL). The reaction mixture was warmed to 23 °C, stirred for 2 hours, and then concentrated under reduced pressure. The resulting white suspension was slurried with Et₂O (100 mL) and filtered through a medium frit. The filtrate was concentrated under reduced pressure, and the resulting liquid was distilled under vacuum to provide benzyl chlorothiolformate (6.95 g, 73%) as a colorless liquid: bp = 95-100 °C (8 torr); IR (cm⁻¹) 1755; ¹H NMR (CDCl₃) δ 4.19 (s, 2H), 7.30-7.34 (m, 5H).

### Preparation of Intermediate Ethyl-3-[BnSCO-L-LeuΨ[COCH₂]-L-Phe-L-(Tr-Gln)]-E-Propenoate.

HCl (5 mL of a 4.0 M solution in 1,4-dioxane) was added to a solution of ethyl-3-[BOC-L-LeuΨ[COCH₂]-L-Phe-L-(Tr-*Gln*)]-E-propenoate (0.190 g, 0.237 mmol, 1 equiv) in 1,4-dioxane (5 mL). The reaction mixture was stirred at 23 °C for 1.5 hours and then concentrated. The resulting oil was dissolved in CH₂Cl₂ (6 mL), and 4-methylmorpholine (0.078 mL, 0.709 mmol, 3.0 equiv) and benzyl chlorothiolformate (0.050 mL, 0.331 mmol, 1.4 equiv) were added sequentially. The reaction mixture was stirred for 1.5 hours at 23 °C, and it then was partitioned between water (100 mL) and a 1:1 mixture of EtOAc and hexanes (2 x 100 mL). The combined organic layers were dried over Na₂SO₄, concentrated, and the residue was chromatographed on silica gel (40% EtOAc in hexanes) to afford ethyl-3-[BnSCO-L-LeuΨ[COCH₂]-L-Phe-L-(Tr-*Gln*)]-E-propenoate (0.117 g, 58%) as a white foam: R_{*f*} = 0.44 (50% EtOAc in hexanes); IR (cm⁻¹) 3312, 1714, 1656; ¹H NMR (CDCl₃) δ 0.82-0.89 (m, 6H), 1.28 (t, 3H, *J* = 7.2), 1.43-1.57 (m, 2H), 1.95-2.05 (m, 1H), 2.31-2.36 (m, 2H), 2.53 (d, 1H, *J* = 14.6), 2.56-2.70 (m, 1H), 2.72-3.04 (m, 5H), 4.02-4.21 (m, 4H), 4.44 (s, br, 2H), 5.41 (d, 1H, *J* = 7.5), 5.48 (dd, 1H, *J* = 15.8, 1.6), 5.88 (d, 1H, *J* = 8.1), 6.58 (dd, 1H, *J* = 15.8, 5.1), 7.08-7.31 (m, 26H); Anal. (C₅₂H₅₇N₃O₆S) C, H, N.

### Preparation of Product Ethyl-3-(BnSCO-L-LeuΨ[COCH₂]-L-Phe-L-Gln)-E-Propenoate

Ethyl-3-[BnSCO-L-LeuΨ[COCH₂]-L-Phe-L-(Tr-*Gln*)]-E-propenoate (0.117 g, 0.137 mmol) was dissolved in CH₂Cl₂ (5 mL), and triisopropylsilane (0.10 mL) and trifluoroacetic acid (5 mL) were added sequentially producing a bright yellow solution. The reaction mixture was stirred for 20 minutes at 23 °C, then carbon tetrachloride (5 mL) was added, and the mixture was concentrated under reduced pressure. The residue was purified by flash column chromatography (5% CH₃OH in CH₂Cl₂) to afford ethyl-3-(BnSCO-L-LeuΨ[COCH₂]-L-Phe-L-*Gln*)-E-propenoate (0.068 g, 81%) as a white foam: R_{*f*} = 0.52 (10% CH₃OH in CH₂Cl₂); IR (cm⁻¹) 3299, 1717, 1650; ¹H NMR (CDCl₃) δ 0.91 (d, 6H, *J* = 6.2), 1.29 (t, 3H, *J* = 7.2), 1.36-1.63 (m, 3H), 1.80 (s, br, 1H), 1.94-2.05 (m, 1H), 2.16-2.18 (m, 2H), 2.60 (d, 1H, *J =* 16.5), 2.69-2.78 (m, 1H), 2.87-3.13 (m, 3H), 4.05-4.21 (m, 4H), 4.52 (s, br, 2H), 5.47 (d, 1H, *J* = 15.6), 5.57 (s, 1H), 6.06 (d, 1H, *J* = 8.7), 6.32 (d, 1H, *J* = 7.2), 6.40 (s, 1H), 6.60 (dd, 1H, *J* = 15.6, 4.5), 7.15-7.29 (m, 10H); Anal. (C₃₃H₄₃N₃O₆S) C, H, N.

### Example 5 - Preparation of Compound 8: Ethyl-3-(CyPentylSCO-L-LeuΨ[COCH₂]-L-Phe-L-Gln)-E-Propenoate.

### Preparation of Intermediate Cyclopentyl Chlorothiolformate.

Triethylamine (10.4 mL, 74.6 mmol, 1.0 equiv) was added to a 0 °C solution of cyclopentyl mercaptan (8.0 mL, 74.8 mmol, 1 equiv) and triphosgene (8.43 g, 28.4 mmol, 0.38 equiv) in CH₂Cl₂. The reaction mixture was warmed to 23 °C, stirred for 2 hours, and then concentrated under reduced pressure. The resulting white suspension was slurried with Et₂O (100 mL) and filtered through a medium frit. The filtrate was concentrated under reduced pressure, and the resulting liquid was distilled under vacuum to provide cyclopentyl chlorothiolformate (10.4 g, 85%) as a colorless liquid: bp = 70-74 °C (1 torr); IR (cm⁻¹) 1756, 830; ¹H NMR (C₆D₆) δ 1.01-1.23 (m, 6H), 1.49-1.60 (m, 2H), 3.20-3.29 (m, 1H).

### Preparation of Intermediate Ethyl-3-[CyPentylSCO-L-LeuΨ[COCH₂]-L-Phe-L-(Tr-Gln)]-E-Propenoate

HCl (5 mL of a 4.0 M solution in 1,4-dioxane) was added to a solution of ethyl-3-[BOC-L-LeuΨ[COCH₂]-L-Phe-L-(Tr-*Gln*)]-E-propenoate (0.205 g, 0.256 mmol, 1 equiv) in 1,4-dioxane (6 mL). The reaction mixture was stirred at 23 °C for 1.5 hours and then concentrated. The resulting oil was dissolved in CH₂Cl₂ (8 mL), cooled to 0 °C, and 4-methylmorpholine (0.070 mL, 0.637 mmol, 2.5 equiv) and cyclopentyl chlorothiolformate (0.063 mL, 0.383 mmol, 1.5 equiv) were added sequentially. The reaction mixture was stirred for 30 minutes at 0 °C, and it then was partitioned between water (100 mL) and a 1:1 mixture of EtOAc and hexanes (2 x 100 mL). The combined organic layers were dried over Na₂SO₄, concentrated, and the residue was chromatographed on silica gel (40% EtOAc in hexanes) to provide ethyl-3-[CyPentylSCO-L-LeuΨ[COCH₂]- L-Phe-L-(Tr-*Gln*)]-E-propenoate (0.113 g, 53%) as a white foam: R_{*f*} = 0.44 (50% EtOAc in hexanes); IR (cm⁻¹) 3310, 1713, 1654; ¹H NMR (CDCl₃) δ 0.85 (d, 6H, *J=* 6.5), 1.02-1.12 (m, 1H), 1.29 (t, 3H, *J* = 7.2), 1.42-1.68 (m, 7H), 1.98-2.08 (m, 4H), 2.31-2.35 (m, 2H), 2.55 (d, 1H, *J* = 14.3), 2.70 (dd, 1H, *J* = 11.8, 5.9), 2.79-3.09 (m, 4H), 3.55-3.66 (m, 1H), 4.17 (q, 2H, *J* = 7.2), 4.38 (s, br, 1H), 4.48-4.49 (m, 1H), 5.33 (d, 1H, *J* = 7.5), 5.50 (dd, 1H, *J* = 15.9, 1.6), 5.94 (d, 1H, *J* = 8.4), 6.60 (dd, 1H, *J* = 15.9, 5.0), 7.10-7.31 (m, 21H); Anal. (C₅₀H₅₉N₃O₆S) C, H, N.

### Preparation of Product Ethyl-3-(CyPentylSCO-L-LeuΨ[COCH₂]-L-Phe-L-Gln)-E-Propenoate

Ethyl-3-[CyPentylSCO-L-LeuΨ[COCH₂]-L-Phe-L-(Tr-*Gln*)]-E-propenoate (0.090 g, 0.108 mmol) was dissolved in CH₂Cl₂ (6 mL), and triisopropylsilane (0.10 mL) and trifluomacetic acid (5 mL) were added sequentially producing a bright yellow solution. The reaction mixture was stirred for 20 minutes at 23 °C, then carbon tetrachloride (4 mL) was added, and the mixture was concentrated under reduced pressure. The residue was purified by flash column chromatography (5% CH,OH in CH₂Cl₂) to provide ethyl-3-(CyPentylSCO-L-LeuΨ[COCH₂]-L-Phe-L-Gln)-E-propenoate (0.050 g, 79%) as a white foam: R_{*f*} = 0.58 (10% CH,OH in CH₂Cl₂); IR (cm⁻¹) 3385, 3298, 3199, 1717, 1652; ¹H NMR (CDCl₃) δ 0.89-0.93 (m, 6H), 1.31 (t, 3H, *J =* 7.2), 1.40-1.69 (m, 8H). 1.86 (s, br, 1H), 2.04-2.09 (m, 4H), 2.21 (s, br, 2H), 2.58 (d, 1H. *J* = 9.3), 2.64-2.79 (m, 1H), 2.89-3.07 (m, 3H), 3.07-3.69 (m, 1H), 4.18 (q, 2H, *J* = 7.2), 4.47 (s, br, 1H), 4.56 (s, br, 1H), 5.49 (d, 1H, *J =* 15.2), 5.57 (s, br, 1H), 6.07 (d, 1H, *J* = 8.7), 6.15 (d, 1H, *J* = 6.9), 6.54 (s, br, 1H), 6.62 (dd, 1H, *J* = 15.2, 4.8), 7.16-7.33 (m, 5H); Anal. (C₃₁H₄₅N₃O₆S) C, H, N.

### Example 6 - Preparation of Compound 4: Ethyl-3-(EtSCO-L-ValΨ[COCH₂]-L-Phe-L-Gln)-E-Propenoate.

### Preparation of Intermediate trans-6-Methyl-hept-4-enoic Acid

A solution of isobutyraldehyde (9.59 g, 133 mmol, 1 equiv) in THF (50 mL) was added dropwise via addition funnel to a solution of vinylmagnesium bromide (133 mL of a 1.0 M solution in THE, 133 mmol, 1.0 equiv) in THF (300 mL) at 0 °C. Upon completion of the addition, the reaction mixture was stirred for 30 minutes at 0 °C, then ethyl malonyl chloride (17.0 mL, 133 mmol, 1.0 equiv) was added. After stirring for 1 hour at 0°C, the reaction mixture was partitioned between saturated NH₄Cl (150 mL) and a 1:1 mixture of EtOAc and hexanes (2 x 200 mL). The combined organic layers were dried over Na₂SO₄ and concentrated. Purification of the residue by filtration through silica gel (eluting with 5% EtOAc in hexanes) afforded the intermediate malonate ester (11.5 g, 40% yield). This material was not characterized, but was combined (neat) with Ti(OEt)₄ (1.13 mL, 5.39 mmol, 0.10 equiv), heated to 190 °C for 4 hours, and then cooled to 60 °C. EtOH (50 mL) and 6.0 M KOH (50 mL) were added sequentially, and the brown reaction mixture was refluxed for 4 hours. After cooling to 23 °C, the reaction mixture was filtered through a medium frit, and the filtrate was partitioned between water (150 mL) and Et₂O (2 x 150 mL). The aqueous layer was then acidified to pH = 2 (as indicated by pH paper) with concentrated HCl and extracted with a 1:1 mixture of EtOAc and hexanes (2 x 150 mL). The combined organic layers were dried over Na₂SO₄, concentrated, and the residue was distilled at reduced pressure to afford trans-6-methyl-hept-4-enoic acid (3.58 g, 47%) as a colorless liquid: bp: 107-112 °C (1 torr); IR (cm⁻¹) 2960, 1711; ¹H NMR (CDCl₃) δ 0.96 (d, 6H, *J =* 6.5) 2,18-2.45 (m, 5H), 5.31-5.50 (m, 2H); Anal. (C₈H₁₄O₂) C, H.

### Preparation of Intermediate trans-6-Methyl-hept-4-enoic Acid (2R-Hydroxy-1R-methyl-2-phenyl-ethyl)-methyl Amide.

Oxalyl chloride (2.25 mL, 25.8 mmol, 1.05 equiv) was added to a solution of trans-6-methyl-hept-4-enoic acid (3.50 g, 24.6 mmol, 1 equiv) and *N*,*N*-dimethylformamide (0.03 mL, 0.39 mmol, 0.016 equiv) in benzene (60 mL) at 23 °C. The reaction mixture was stirred at 23 °C for 2 hours and then concentrated under reduced pressure. The resulting oil was dissolved in THF (20 mL) and added via cannula to a solution of 1*R*,2*R*-(-)-pseudoephedrine (3.87 g, 23.4 mmol, 1.0 equiv) and triethylamine (3.92 mL, 28.1 mmol, 1.2 equiv) in THF (150 mL) at 0 °C. The reaction mixture was stirred at 0 °C for 30 minutes and then partitioned between half-saturated NH₄Cl (150 mL) and EtOAc (2 x 150 mL). The combined organic layers were dried over Na₂SO₄, concentrated, and the residue purified by flash column chromatography (gradient elution 40 → 50% EtOAc in hexanes) to afford trans-6-methyl-hept-4-enoic acid
(2*R*-hydroxy-1*R*-methyl-2-phenyl-ethyl)-methyl amide (6.31 g, 93%) as a viscous oil: R_{*f*} = 0.35 (50% EtOAc in hexanes); IR (cm⁻¹) 3382, 1622; ¹H NMR (CDCl₃, mixture of rotamers) δ 0.96 (d, *J* = 6.8), 0.97 (d, *J* = 6.5), 1.11 (d, *J* = 6.9), 2.18-2.59 (m), 2.82 (s), 2.92 (s), 3.99-4.04 (m), 4.32-4.42 (m), 4.44-4.49 (m), 4.55-4.62 (m), 5.32-5.49 (m), 7.24-7.42 (m); Anal. (C₁₈H₂₇NO₂) C, H, N.

### Preparation of Intermediate trans-2S-Benzyl-6-methyl-hept-4-enoic Add (2R-Hydroxy-1R-methyl-2-phenyl-ethyl)-methyl Amide.

*n*-Butyllithium (28.6 mL of a 1.6 M solution in hexanes, 45.8 mmol, 2.1 equiv) was added to a suspension of anhydrous lithium chloride (6.47 g, 153 mmol, 7.0 equiv) and diisopropylamine (6.88 mL, 49.1 mmol, 2.25 equiv) in THF (250 mL) at -78 °C. The reaction mixture was stirred for 20 minutes at -78 °C, and it then was maintained at 0 °C for 5 minutes and subsequently cooled again to -78 °C. trans-6-Methyl-hept-4-enoic acid (2*R*-hydroxy-1*R*-methyl-2-phenyl-ethyl)-methyl amide (6.31 g, 21.8 mmol, 1 equiv) in THF (40 mL) was added via cannula, and the resulting solution was stirred at -78 °C for 1 hour, maintained at 0 °C for 15 minutes, stirred at 23 °C for 5 minutes, and then cooled again to 0 °C. Benzyl bromide (3.89 mL, 32.7 mmol, 1.5 equiv) was added, the reaction mixture was stirred at 0 °C for 30 minutes, and it then was partitioned between half-saturated NH₄Cl (200 mL) and a 1:1 mixture of EtOAc and hexanes (2 x 200 mL). The combined organic layers were dried over Na₂SO₄ and concentrated. Purification of the residue by flash column chromatography (gradient elution 20 - 40% EtOAc in hexanes) provided trans-2*S*-benzyl-6-methyl-hept-4-enoic acid (2*R*-hydroxy-1*R*-methyl-2-phenyl-ethyl)-methyl amide (7.91 g, 96%) as a viscous oil: R_{*f*} = 0.52 (50% EtOAc in hexanes); IR (cm⁻¹) 3383, 1616; ¹H NMR (CDCl₃, mixture of rotamers) δ 0.82-0.91 (m), 0.96 (d, *J* = 6.5), 1.24-1.27 (m), 2.14-2.47 (m), 2.56 (s), 2.72-2.99 (m), 3.04-3.15 (m), 3.93-4.00 (m), 4.31-4.51 (m), 5.21-5.39 (m), 5.42-5.55 (m), 7.14-7.37 (m); Anal. (C₂₅H₃₃NO₂) C, H, N.

### Preparation of Intermediate 3R-Benzyl-5S-(1R-bromo-2-methyl-propyl)-dihydrofuran-2-one.

*N*-Bromosuccinimide (3.89 g, 21.9 mmol, 1.05 equiv) was added in small portions over 5 minutes to a solution of trans-2*S*-benzyl-6-methyl-hept-4-enoic acid (2*R*-hydroxy-1*R*-methyl-2-phenyl-ethyl)-methyl amide (7.90 g, 20.8 mmol, 1 equiv) and glacial acetic acid (5.96 mL, 104 mmol, 5.0 equiv) in a 4:1 mixture of THE and H₂O (250 mL) at 0 °C. The resulting yellow solution was stirred for 15 minutes at 0 °C, then was warmed to 23 °C, and subsequently was refluxed for 1 hour. After cooling to 23 °C, the reaction mixture was partitioned between half-saturated NaHCO₃ (300 mL) and a 1:1 mixture of EtOAc and hexanes (2 x 200 mL). The combined organic layers were dried over Na₂SO₄ and concentrated. Flash chromatographic purification of the residue (5% EtOAc in hexanes) gave 3*R*-benzyl-5*S*-(1*R*-bromo-2-methyl-propyl)- dihydromran-2-one (5.09 g, 79%) as a white solid. Minor impurities were removed by recrystallization from hexanes (2.51 g recovery): mp = 75-76 °C; R, = 0.64 (30% EtOAc in hexanes); IR (cm⁻¹) 1774; ¹H NMR (CDCl₃) δ 0.93 (d, 3H, *J* = 6.6), 0.99 (d. 3H, J =6.9). 2.05-2.18 (m, 1H), 2.20-2.33 (m, 2H). 2.83 (dd, 1H, *J* = 13.6, 8.7), 2.95-3.05 (m, 1H), 3.17 (dd, 1H. *J* = 13.6, 4.5), 3.89 (dd, 1H, *J* = 9.0, 3.4), 4.32-4.39 (m, 1H), 7.20-7.36 (m, 5H); Anal. (C₁₅H₁₉BrO₂) C, H.

### Preparation of Intermediate 5S-(1S-Azido-2-methyl-propyl)-3R-benzyldihydrofuran-2-one.

A solution of Aliquat-336 (0.163 g, 0.403 mmol. 0.05 equiv) and 3*R*-benzyl-5*S*-(1*R*-bromo-2-methyl-propyl)-dihydrofuran-2-one (2.51 g, 8.06 mmol, 1 equiv) in toluene (60 mL) was treated with a solution of sodium azide (2.10 g, 32.3 mmol, 4.0 equiv) in H₂O (10 mL). The resulting biphasic mixture was heated to 70 °C and maintained at that temperature for 48 hours. The reaction mixture was cooled to 23 °C and partitioned between water (150 mL) and a 1:1 mixture of EtOAc and hexanes (2 x 150 mL). The combined organic layers were dried over Na₂SO₄, concentrated, and the residue purified by flash column chromatography (gradient elution, 5 → 10% EtOAc in hexanes) to give 5*S*-(1*S*-azido-2-methyl-propyl)- 3*R*-benzyl-dihydrofuran-2-one (1.0 g, 45%) as a viscous oil: R_{*f*} = 0.41 (20% EtOAc in hexanes); IR (cm⁻¹) 2105, 1772; ¹H NMR (CDCl₃) δ 0.86-1.04 (m, 7H), 1.95-2.17 (m, 2H), 2.83 (dd, 1H, *J* = 13.2, 8.3), 2.92 (dd, 1H, *J* = 6.4, 4.5), 3.05-3.21 (m, 2H), 4.33-4.38 (m, 1H), 7.19-7.35 (m, 5H); Anal. (C₁₅H₁₉N₃O₂) C, H, N.

### Preparation of Intermediate [1S-(4R-Benzyl-5-oxo-tetrahydrofuran-2S-yl)-2-methyl-propyl]-carbamic Add tert-Butyl Ester.

A suspension of 5*S*-(1*S*-azido-2-methyl-propyl)-3*R*-benzyl-dihydrofuran-2-one (1.00 g, 3.66 mmol, 1 equiv) and Pd/C (10%, 0.090 g) in CH₃OH (60 mL) was stirred under a hydrogen atmosphere (balloon) for 1 hour. The reaction mixture was filtered through celite, concentrated, and the residue dissolved in 1,4-dioxane (50 mL). *N,N*-diisopropylethylamine (1.28 mL, 7.35 mmol, 2.0 equiv) and di-*tert*-butyl dicarbonate (1.20 g, 5.50 mmol, 1.5 equiv) were added sequentially, and the resulting solution was stirred at 23 °C for 18 hours. The reaction mixture was then partitioned between water (100 mL) and a 1:1 mixture of EtOAc and hexanes (2 x 150 mL). The combined organic layers were dried over Na₂SO₄ and concentrated. Purification of the residue by flash column chromatography (gradient elution, 10 → 15% EtOAc in hexanes) provided [1*S*-(4*R*-benzyl-5-oxo-tetrahydrofuran-2*S*-yl)-2-methyl-propyl]- carbamic acid *tert*-butyl ester (0.496 g, 39%) as a colorless oil: R_{*f*} = 0.44 (20% EtOAc in hexanes); IR (cm⁻¹) 3340, 1768, 1708; ¹H NMR (CDCl₃) δ 0.92 (d, 6H, *J* = 6.9), 1.41 (s, 9H), 1.70-1.80 (m, 1H), 1.98-2.07 (m, 1H), 2.14-2.24 (m, 1H), 2.81 (dd, 1H, *J* = 13.6, 8.9), 2.92-3.01 (m, 1H), 3.13 (dd, 1H, *J* = 13.6, 4.4), 3.31-3.38 (m, 1H), 4.39-4.47 (m, 2H), 7.17-7.33 (m, 5H); Anal. (C₂₀H₂₉NO₄) C, H, N.

### Preparation of Intermediate Ethyl-3-[BOC-L-ValΨ[COCH₂]-L-Phe-L-(Tr-Gln)]-E-Propenoate.

Lithium hydroxide (7.14 mL of a 1 M aqueous solution, 7.14 mmol, 5.0 equiv) was added to a solution of [1*S*-(4*R*-benzyl-5-oxo-tetrahydrofuran-2*S*-yl)-2-methyl-propyl]-carbamic acid *tert*-butyl ester (0.496 g, 1.43 mmol, 1 equiv) in DMB (7 mL) at 23 °C. The resulting suspension was stirred at 23 °C for 30 minutes and then partitioned between 0.5 M HCl (100 mL) and EtOAc (2 x 100 mL). The combined organic layers were dried over Na₂SO₄, concentrated, and the residue dissolved in CH₂Cl₂ (20 mL). 4-Methylmorpholine *N*-oxide (0.334 g, 2.85 mmol, 2.0 equiv), powdered 4Å molecular sieves (0.51 g), and tetrapropylammonium perruthenate (0.050 g, 0.142 mmol, 0.10 equiv) were added sequentially. The resulting dark reaction mixture was stirred for 2.5 hours at 23 °C and then filtered through celite. The filtrate was concentrated under reduced pressure to provide a brown oil which was dissolved in CH₂Cl₂ (30 mL). Crude ethyl-3-[H₂N-L-(Tr-*Gln*)]- E-propenoate•HCl, prepared as described in Example 1 for the preparation of ethyl-3-[CBZ-L-LeuΨ[COCH₂]-D/L-Phe-L-(Tr-*Gln*)]-E-propenoate (1.86 mmol, 1.3 equiv), 1-hydroxybenzotriazole hydrate (0.289 g, 2.14 mmol, 1.5 equiv), 4-methylmorpholine (0.629 mL, 5.72 mmol, 4.0 equiv), and 1-(3-dimethylaminopropyl)-3-ethylcarbodiimide hydrochloride (0.411 g, 2.14 mmol, 1.5 equiv) were added sequentially, and the reaction mixture was stirred for 8 hours at 23 °C then partitioned between water (150 mL) and EtOAc (2 x 150 mL). The combined organic layers were dried over Na₂SO₄ and concentrated. Purification of the residue by flash column chromatography (40% EtOAc in hexanes) provided ethyl-3-[BOC-L-ValΨ[COCH₂]-L-Phe-L-(Tr-*Gln*)]-E-propenoate (0.497 g, 44%) as an off-white foam: R_{*f*} = 0.40 (50% EtOAc in hexanes); IR (cm⁻¹) 3314, 1709, 1663; ¹H NMR (CDCl₃) δ 0.65 (d, 3H, *J* = 6.9), 0.91 (d, 1H, *J* = 6.9), 1.29 (t, 3H, *J* = 7.2), 1.40 (s, 9H), 1.94-2.20 (m, 2H), 2.30-2.35 (m, 2H), 2.51 (d, 1H, *J* = 17.1), 2.68 (dd, 1H, *J* = 11.7, 5.8), 2.74-2.90 (m, 3H), 3.04 (dd, 1H, *J* = 17.3, 9.8), 4.05-4.09 (m, 1H), 4.17 (q, 2H, *J* = 7.2), 4.38-4.45 (m, 1H), 4.83 (d, 1H, *J* = 8.1), 5.45 (d, 1H, *J* = 15.7), 5.87 (d, 1H, *J* = 8.1), 6.57 (dd, 1H, *J* = 15.7, 5.0), 7.10-7.31 (m, 21H); Anal. (C₄₈H₅₇N₃O₇) C, H, N.

### Preparation of Intermediate Ethyl-3-[EtSCO-L-ValΨ[COCH₂]-L-Phe-L-(Tr-Gln)]-E-Propenoate.

HCl (5 mL of a 4.0 M solution in 1,4-dioxane) was added to a solution of ethyl-3-[BOC-L-ValΨ[COCH₂]-L-Phe-L-(Tr-*Gln*)]-E-propenoate (0.225 g, 0.286 mmol, 1 equiv) in 1,4-dioxane (6 mL). The reaction mixture was stirred at 23 °C for 1.5 hours and then concentrated. The resulting oil was dissolved in CH₂Cl₂ (10 mL), and *N*,*N*-diisopropylethylamine (0.125 mL, 0.718 mmol, 2.5 equiv) and ethyl chlorothiolformate (0.040 mL, 0.384 mmol, 1.3 equiv) were added sequentially. The reaction mixture was stirred for 1 hour at 23 °C and then partitioned between water (100 mL) and a 1:1 mixture of EtOAc and hexanes (2 x 100 mL). The combined organic layers were dried over Na₂SO₄, concentrated, and the residue was chromatographed on silica gel (40% EtOAc in hexanes) to afford ethyl-3-[EtSCO-L-ValΨ[COCH₂]-L-Phe-L-(Tr-*Gln*)]-E-propenoate (0.148 g, 66%) as a white foam: R_{*f*} = 0.37 (50% EtOAc in hexanes); IR (cm⁻¹) 3314, 1714, 1653; ¹H NMR (CDCl₃) δ 0.68 (d, 3H, *J* = 6.9), 0.92 (d, 3H, *J* = 6.5), 1.21-1.32 (m, 7H), 1.92-2.03 (m, 2H), 2.32-2.36 (m, 2H), 2.51 (dd, 1H, *J =* 17.4. 2.2), 2.67 (dd, 1H, *J* = 11.8, 5.9), 2.73-2.90 (m, 4H), 3.05 (dd, 1H, *J* = 17.6, 9.8), 4.17 (q, 2H, *J* = 7.2), 4.39-4.44 (m, 2H), 5.46 (dd, 1H, *J* = 15.7, 1.7), 5.62 (d, 1H, *J* = 8.1), 5.96 (d, 1H, *J* = 8.4), 6.57 (dd, 1H, *J* = 15.7, 5.1), 7.10-7.12 (m, 2H), 7.15-7.31 (m, 19H); Anal. (C₄₆H₅₃N₃O₆S) C, H, N.

### Preparation of Product Ethyl-3-(EtSCO-L-ValΨ[COCH₂]-L-Phe-L-Gln)-E-Propenoate.

Ethyl-3-[EtSCO-L-ValΨ[COCH₂]-L-Phe-L-(Tr-*Gln*)]-E-propenoate (0.148 g, 0.191 mmol) was dissolved in CH₂Cl₂ (5 mL), and triisopropylsilane (0.10 mL) and trifluoroacetic acid (5 mL) were added sequentially producing a bright yellow solution. The reaction mixture was stirred for 20 minutes at 23 °C, then carbon tetrachloride (6 mL) was added, and the mixture was concentrated under reduced pressure. The residue was purified by flash column chromatography (5% CH₃OH in CH₂Cl₂) to afford ethyl-3-(EtSCO-L-ValΨ[COCH₂]-L-Phe-L-*Gln*)-E-propenoate (0.078 g, 77%) as a white solid: mp = 205 °C (dec); R_{*f*} = 0.45 (10% CH₃OH in CH₂Cl₂); IR (cm⁻¹) 3424, 3304, 1715, 1658, 1640, 1624; ¹H NMR (DMSO-*d*₆) δ 0.75 (d, 3H, *J* = 6.5), 0.82 (d, 3H, *J* = 6.9), 1.14 (t, 3H, *J* = 7.2), 1.21 (t, 3H, *J* = 7.2), 1.56-1.71 (m, 2H), 1.99-2.11 (m, 2H), 2.44-2.59 (m, 4H), 2.70-2.85 (m, 3H), 2.93-2.95 (m, 1H), 4.06-4.18 (m, 3H), 4.31 (s, br, 1H), 5.53 (d, 1H, *J* = 15.6), 6.63 (dd, 1H, *J* = 15.6, 5.1), 6.75 (s, 1H), 7.14-7.26 (m, 6H), 8.06 (d, 1H, *J* = 8.1), 8.33 (d, 1H, *J* = 8.1); Anal. (C₂₇H₃₉N₃O₆S) C, H, N.

### Example 7 - Preparation of Compound 5: Ethyl-3-(BnSCO-L-ValΨ[COCH₂]-L-Phe-L-Gln)-E-Propenoate

### Preparation of Intermediate Ethyl-3-[BnSCO-L-ValΨ[COCH₂]-L-Phe-L-(Tr-Gln)]-E-Propenoate.

HCl (8 mL of a 4.0 M solution in 1,4-dioxane) was added to a solution of ethyl-3-[BOC-L-ValΨ[COCH₂]-L-Phe-L-(Tr-*Gln*)]-E-propenoate (0.272 g, 0.345 mmol, 1 equiv) in 1,4-dioxane (10 mL). The reaction mixture was stirred at 23 °C for 1.5 hours and then concentrated. The resulting oil was dissolved in CH₂Cl₂ (10 mL), and 4-methylmorpholine (0.095 mL, 0.864 mmol, 2.5 equiv) and benzyl chlorothiolformate (0.068 mL, 0.450 mmol, 1.3 equiv) were added sequentially. The reaction mixture was stirred for 1 hour at 23 °C and then partitioned between water (100 mL) and a 1:1 mixture of EtOAc and hexanes (2 x 100 mL). The combined organic layers were dried over Na₂SO₄, concentrated, and the residue was chromatographed on silica gel (40% EtOAc in hexanes) to afford ethyl-3-[BnSCO-L-ValΨ[COCH₂]-L-Phe-L-(Tr-*Gln*)]-E-propenoate (0.160 g, 55%) as a white foam: R_{*f*} = 0.41 (50% EtOAc in hexanes); IR (cm⁻¹) 3316, 1716, 1658; ¹H NMR (CDCl₃) δ 0.66 (d, 3H, *J* = 6.9), 0.92 (d, 3H, *J* = 6.5), 1.29 (t, 3H, J = 7.2), 1.93-2.03 (m, 2H), 2.32-2.36 (m, 2H), 2.50 (dd, 1H, *J* = 17.7, 2.5), 2.67 (dd, 1H, *J* = 11.5, 6.2), 2.73-2.89 (m, 3H). 3.07 (dd, 1H, *J* = 17.7, 10.0), 4.09 (s, 2H), 4.16 (q. 2H, *J* = 7.2), 4.38-4.42 (m, 2H), 5.46 (dd, 1H, *J* = 15.8, 1.6), 5.65 (d, 1H, *J* = 8.4), 5.95 (d, 1H, *J* = 8.1), 6.57 (dd, 1H, *J* = 15.8, 5.1), 7.03 (s, 1H), 7.09-7.12 (m, 2H), 7.15-7.31 (m, 23H); Anal. (C₅₁H₅₅N₃O₆S) C, H, N.

### Preparation of Product Ethyl-3-(BnSCO-L-ValΨ[COCH₂]-L-Phe-L-Gln)-E-Propenoate.

Ethyl-3-[BnSCO-L-ValΨ[COCH₂]-L-Phe-L-(Tr-*Gln*)]-E-propenoate (0.160 g, 0.191 mmol) was dissolved in CH₂Cl₂ (5 mL), and triisopropylsilane (0.10 mL) and trifluoroacetic acid (5 mL) were added sequentially producing a bright yellow solution. The reaction mixture was stirred for 20 minutes at 23 °C, then carbon tetrachloride (5 mL) was added, and the mixture was concentrated under reduced pressure. The residue was purified by flash column chromatography (5 % CH₃OH in CH₂Cl₂) to provide ethyl-3-(BnSCO-L-ValΨ[COCH₂]-L-Phe-L-*Gln*)-E-propcnoate (0.097 g, 85%) as a white solid: mp = 185-189 °C; R_{*f*} = 0.55 (10% CH₃OH in CH₂Cl₂); IR (cm⁻¹) 3392, 3287, 1710, 1643; ¹H NMR (DMSO-*d*₆) δ 0.74 (d, 3H, J = 6.9), 0.82 (d, 3H, *J* = 6.5), 1.21 (t, 3H, *J* = 7.2), 1.62-1.70 (m, 2H), 1.98-2.12 (m, 3H), 2.44-2.59 (m, 2H), 2.71-2.96 (m, 3H), 4.03 (s, 2H), 4.10 (q, 2H, *J* = 7.2), 4.19-4.23 (m, 1H), 4.32 (s, br, 1H). 5.54 (d, 1H, *J* = 15.6), 6.64 (dd, 1H, *J* = 15.6, 5.3), 6.75 (s, 1H), 7.14-7.28 (m, 11H). 8.06 (d, 1H, *J* = 8.1), 8.42 (d, 1H, J = 7.8); Anal. (C₃₂H₄₁N₃O₆S) C, H, N.

### Example 8 - Preparation of Compound 7: Ethyl-3-(CyPentylSCO-L-ValΨ[COCH₂]-L-Phe-L-Gln)-E-Propenoate.

### Preparation of Intermediate Ethyl-3-[CyPentylSCO-L-ValΨ[COCH₂]-L-Phe-L-(Tr-Gln)]-E-Propenoate

HCl (6 mL of a 4.0 M solution in 1,4-dioxane) was added to a solution of ethyl-3-[BOC-L-ValΨ[COCH₂]-L-Phe-L-(Tr-*Gln*)]-E-propenoate (0.248 g, 0.315 mmol, 1 equiv) in 1.4-dioxane (6 mL). The reaction mixture was stirred at 23 °C for 1.5 hours and then concentrated. The resulting oil was dissolved in CH₂Cl₂ (15 mL), cooled to 0 °C, and 4-methylmorpholine (0.086 mL, 0.782 mmol, 2.5 equiv) and cyclopentyl chlorothiolformate (0.067 mL, 0.407 mmol, 1.3 equiv) were added sequentially. The reaction mixture was stirred for 30 minutes at 0 °C and then partitioned between water (100 mL) and a 1:1 mixture of EtOAc and hexanes (2 x 100 mL). The combined organic layers were dried over Na₂SO₄, concentrated, and the residue was chromatographed on silica gel (40% EtOAc in hexanes) to provide ethyl-3-[CyPentylSCO-L-ValΨ[COCH₂]-L-Phe-L-(Tr-*Gln*)]-E-propenoate (0.126 g, 49%) as a white foam: R_{*f*} = 0.42 (50 % EtOAc in hexanes); IR (cm⁻¹) 3314, 1711, 1654; ¹H NMR (CDCl₃) δ 0.67 (d, 3H, *J* = 6.9), 0.92 (d, 3H, *J* = 6.9), 1.29 (t, 3H, *J* = 7.2), 1.45-1.68 (m, 6H), 1.91-2.05 (m, 4H), 2.31-2.34 (m, 2H), 2.51 (d, 1H, *J* = 17.4), 2.67 (dd, 1H, *J* = 11.5, 6.2), 2.75-2.90 (m, 3H), 3.03 (dd, 1H, *J* = 17.3, 9.8), 3.60-3.64 (m, 1H), 4.17 (q, 2H, *J* = 7.2), 4.36-4.44 (in, 2H), 5.46 (dd, 1H, *J* = 15.8, 1.7), 5.57 (d, 1H, *J* = 8.1), 5.95 (d, 1H, *J* = 8.4), 6.58 (dd, 1H, *J* = 15.8, 5.0), 7.10-7.12 (m, 2H), 7.19-7.31 (m, 19H); Anal. (C₄₉H₅₇N₃O₆S) C, H, N.

### Preparation of Product Ethyl-3-(CyPentylSCO-L-ValΨ[COCH₂]-L-Phe-L-Gln)- E-Propenoate

Ethyl-3-[CyPentylSCO-L-ValΨ[COCH₂]-L-Phe-L-(Tr-*Gln*)]-E-propenoate (0.126 g, 0.154 mmol) was dissolved in CH₂Cl₂ (6 mL), and triisopropylsilane (0.10 mL) and trifluoroacetic acid (6 mL) were added sequentially producing a bright yellow solution. The reaction mixture was stirred for 20 minutes at 23 °C, then carbon tetrachloride (6 mL) was added, and the mixture was concentrated under reduced pressure. The residue was purified by flash column chromatography (5 % CH₃OH in CH₂Cl₂) to afford ethyl-3-(CyPentylSCO-L-ValΨ[COCH₂]-L-Phe-L-*Gln*)-E-propenoate (0.046 g, 52%) as a white solid: mp = 200-204 °C; R_{*f*} = 0.38 (10% CH₃OH in CH₂Cl₂); IR (cm⁻¹) 3431, 3261, 1717, 1642; ¹H NMR (DMSO-*d*₆) δ 0.75 (d, 3H, *J* = 6.5), 0.82 (d, 3H, *J* = 6.5), 1.22 (t, 3H, *J* = 7.2), 1.40-1.74 (m, 7H), 1.95-2.10 (m, 6H), 2.43-2.59 (m, 2H), 2.71-2.84 (m, 2H), 2.93-2.95 (m, 1H), 3.47-3.56 (m, 1H), 4.06-4.16 (m, 3H), 4.32 (s, br, 1H), 5.54 (d, 1H, *J* = 15.9), 6.64 (dd, 1H, *J* = 15.9, 5.3), 6.75 (s, 1H), 7.15-7.27 (m, 6H), 8.06 (d, 1H, *J* = 7.8), 8.27 (d, 1H, *J* = 8.1); Anal. (C₃₀H₄₃N₃O₆S) C, H, N.

### Example 9 - Preparation of Compound 3: (2,3-Dihydroindole)-3-(BnSCO-L-LeuΨ[COCH₂]-L-Phe-L-Gln)-E-Propenamide.

### Preparation of Intermediate [2-(2,3-Dihydro-indol-1-yl)-2-oxo-ethyl]-phosphonic Acid Diethyl Ester.

Oxalyl chloride (5.96 mL, 68.3 mmol, 1.05 equiv) was added to a solution of diethylphosphonoacetic acid (12.8 g, 65.0 mmol, 1 equiv) and *N,N*-dimethylformamide (0.03 mL, 0.39 mmol, 0.006 equiv) in benzene (150 mL) at 23 °C. The reaction mixture was stirred at 23 °C for 1 hour and then concentrated under reduced pressure. The resulting oil was dissolved in THF (30 mL) and added via cannula to a solution of indoline (7.38 g, 61.9 mmol, 0.95 equiv) and triethylamine (10.9 mL, 78.0 mmol, 1.2 equiv) in THF (200 mL) at 0 °C. The reaction mixture was stirred at 0 °C for 15 minutes, and it then was partitioned between 0.5 M HCl (150 mL) and EtOAc (2 x 150 mL). The combined organic layers were dried over Na₂SO₄ and concentrated to afford a tan solid. Recrystallization from Et₂O provided [2-(2,3-dihydro-indol-1-yl)-2-oxo-ethyl]- phosphonic acid diethyl ester (12.2 g, 63%) as a light brown solid: mp: 97-99 °C; R_{*f*} = 0.06 (50% EtOAc in hexanes); IR (cm⁻¹) 3460, 1657, 1597, 1482; ¹H NMR (CDCl₃) δ 1.35 (t, 6H, *J* = 7.2), 3.14 (d, 2H, *J* = 22.4), 3.22 (d, 2H, J = 8.4), 4.15-4.30 (m, 6H), 7.04 (t, 1H, *J* = 7.0), 7.17-7.28 (m, 2H), 8.21 (d, 1H, *J* = 9.0); Anal. (C₁₄H₂₀NO₄P) C, H, N.

### Preparation of Intermediate (2,3-Dihydroindole)-3-[BOC-L-(Tr-Gln)]-E-Propenamide.

Sodium bis(trimethylsilyl)amide (11.9 mL of a 1.0 M solution in THF, 11.9 mmol, 1.0 equiv) was added to a solution of [2-(2,3-dihydro-indol-1-yl)-2-oxo-ethyl]-phosphonic acid diethyl ester (3.54 g, 11.9 mmol, 1.0 equiv) in THF (150 mL) at -78°C, and the resulting solution was stirred for 20 minutes at that temperature. Crude [BOC-L-(Tr-Gln)]-H (5.63 g, 11.9 mmol, 1 equiv), prepared as described in Example 1, in THF (40 mL) was added via cannula, and the reaction mixture was stirred for 1 hour at -78°C, warmed to 0 °C for 10 minutes, and partitioned between 0.5 M HCl (150 mL) and EtOAc (2 x 150 mL). The organic layers were dried over Na₂SO₄ and concentrated. Purification of the residue by flash column chromatography (50% EtOAc in hexanes) provided (2,3-dihydroindole)- 3-[BOC-L-(Tr-*Gln*)]-E-propenamide (6.35 g, 87%) as an off-white foam: R_{*f*} = 0.28 (50% EtOAc in hexanes); IR (cm⁻¹) 3401, 3307, 1690, 1665; ¹H NMR (CDCl₃) δ 1.44 (s, 9H), 1.76-2.05 (m, 2H), 2.37-4.06 (m, 2H), 3.11-3.22 (m, 2H), 4.02-4.16 (m, 2H), 4.27-4.40 (m, 1H), 4.91-4.97 (m, 1H), 6.29 (d, 1H, *J* = 14.9), 6.77-6.96 (m, 2H), 6.98-7.05 (m, 1H), 7.14-7.37 (m, 17H), 8.25 (d, 1H, *J* = 7.5); Anal. (C₃₉H₄₁N₃O₄) C, H, N.

### Preparation of Intermediate (2,3-Dihydroindole)-3-[BOC-L-LeuΨ[COCH₂]-L-Phe-L-(Tr-Gln)]-E-Propenamide.

Lithium hydroxide (8.0 mL of a 1 M aqueous solution, 8.0 mmol, 5.0 equiv) was added to a solution of [1*S*-(4*R*-benzyl-5-oxo-tetrahydrofuran-2*S*-yl)-3-methyl-butyl]-carbamic acid *tert*-butyl ester (0.576 g, 1.59 mmol, 1 equiv), prepared as described in Example 1, in DME (8 mL) at 23 °C. The resulting suspension was stirred at 23 °C for 45 minutes, and it then was partitioned between 0.5 M HCl (100 mL) and EtOAc (2 x 100 mL). The combined organic layers were dried over Na₂SO₄, concentrated, and the residue dissolved in CH₂Cl₂ (30 mL). 4-Methylmorpholine *N*-oxide (0.373 g, 3.18 mmol, 2.0 equiv), powdered 4Å molecular sieves (0.60 g), and tetrapropylammonium perruthenate (0.056 g, 0.159 mmol, 0.10 equiv) were added sequentially. The resulting dark reaction mixture was stirred for 1 hour at 23°C and then filtered through celite. The filtrate was concentrated under reduced pressure to provide a brown oil which was dissolved in CH₂Cl₂ (20 mL). Crude (2,3-dihydroindole)-3-(H₂N-L-Tr-*Gln*)-E-propenamide•HCl (prepared from (2,3-dihydroindole)-3-[BOC-L-(Tr-*Gln*)]-E-propenamide in a manner analogous to the method described in Example 1 for the preparation of ethyl-3-[H₂N-L-(Tr-*Gln*)]-E-propenoate•HCl (2.39 mmol, 1.5 equiv), 1-hydroxybenzotriazole hydrate (0.322 g, 2.38 mmol, 1.5 equiv), 4-methylmorpholine (0.699 mL, 6.36 mmol, 4.0 equiv), and 1-(3-dimethylaminopropyl)-3-ethylcarbodiimide hydrochloride (0.322 g, 2.38 mmol, 1.5 equiv) were added sequentially, and the reaction mixture was stirred for 16 hours at 23°C and then partitioned between water (100 mL) and EtOAc (2 x 100 mL). The combined organic layers were dried over Na₂SO₄ and concentrated. Purification of the residue by flash column chromatography (40% hexanes in EtOAc) provided (2,3-dihydroindole)-3 -[BOC-L-LeuΨ[COCH₂]-L-Phe-L-(Tr-*Gln*)]- E-propenamide (0.568 g, 40%) as tan foam: R_{*f*} = 0.64 (10% CH₃OH in CH₂Cl₂); IR (cm⁻¹) 3304, 1700, 1665; ¹H NMR (CDCl₃) δ 0.86 (d, 6H, *J* = 6.5), 0.97-1.12 (m, 1H), 1.39 (s, 9H), 1.45-1.64 (m, 2H), 2.39-2.43 (m, 2H), 2.51 (d, 1H, *J* = 15.6), 2.67 (dd, 1H, *J* = 12.3, 7.0), 2.79-2.96 (m, 3H), 3.16 (s, br, 2H), 4.01-4.20 (m, 5H), 4.52 (s, br, 1H), 4.63 (d, 1H, *J* = 7.8), 6.09 (d, 1H, *J* = 14.7), 6.17 (d, 1H, *J* = 8.1), 6.65 (dd, 1H, *J* = 14.7, 5.8), 6.99-7.04 (m, 1H), 7.11-7.39 (m, 23H), 8.26 (d, 1H, *J* = 7.5).

### Preparation of Product (2,3-Dihydroindole)-3-[BnSCO-L-LeuΨ[COCH₂]-L-Phe-L-(Tr-Gln)]-E-Propenamide

HCI (6 mL of a 4.0 M solution in 1,4-dioxane) was added to a solution of (2,3-dihydroindole)-3-[BOC-L-LeuΨ[COCH₂]-L-Phe-L-(Tr-*Gln*)]-E-propenamide (0.204 g, 0.233 mmol, 1 equiv) in 1,4-dioxane (8 mL). The reaction mixture was stirred at 23 °C for 2.5 hours and then concentrated. The resulting oil was dissolved in CH₂Cl₂ (8 mL), and *N*,*N*-diisopropylethylamine (0.081 mL, 0.465 mmol, 2.0 equiv) and benzyl chlorothiolformate (0.042 mL, 0.279 mmol, 1.2 equiv) were added sequentially. The reaction mixture was stirred for 2 hours at 23°C and then partitioned between water (100 mL) and a 1:1 mixture of EtOAc and hexanes (2 x 100 mL). The combined organic layers were dried over Na₂SO₄ and concentrated to provide crude (2,3-dihydroindole)-3- [BnSCO-L-LeuΨ[COCH₂]-L-Phe-L-(Tr-*Gln*)]-E-propenamide as an off-white foam. This material was dissolved in CH₂Cl₂ (5 mL), and triisopropylsilane (0.075 mL), and trifluoroacetic acid (5 mL) were added sequentially producing a bright yellow solution. The reaction mixture was stirred for 30 minutes at 23 °C, then carbon tetrachloride (4 mL) was added, and the mixture was concentrated under reduced pressure. The residue was purified by flash column chromatography (5 % CH₃OH in CH₂Cl₂) to afford (2,3-dihydroindole)-3-(BnSCO- L-LeuΨ[COCH₂]-L-Phe-L-*Gln*)-E-propenamide (0.016 g, 10%) as a white foam *R*_{*f*} = 0.51 (10% CH₃OH in CH₂Cl₂); ¹H NMR (CDCl₃) δ 0.92 (d, 6H, *J* = 6.2), 1.11-1.25 (m, 1H), 1.33-1.36 (m, 1H), 1.49-2.17 (m, 4H), 2.25 (s, br, 2H), 2.58 (d, 1H, *J* = 17.1), 2.70-2.72 (m, 1H), 2.94-3.17 (m, 4H), 4.01-4.04 (m, 2H), 4.11 (s, 2H), 4.52-4.58 (m, 2H), 5.47 (s, br, 1H), 6.13 (d, 1H, *J* = 13.4), 6.29 (s, br, 1H), 6.41 (s, br, 1H), 6.66 (s, br, 1H), 7.00-7.05 (m, 1H), 7.17-7.29 (m, 13H), 8.25 (d, 1H, *J* = 7.2).

### Example 10 - Preparation of Compound 10: Ethyl-3-(EtSCO-L-ValΨ[COCH₂]-L-(p-CH₃)Phe-L-Gln)-E-Propenoate.

### Preparation of Intermediate trans-6-Methyl-2S-(4-methyl-benzyl)-hept-4-enoic Acid (2R-Hydroxy-1R-methyl-2-phenyl-ethyl)-methyl Amide.

*n*-Butyllithium (33.7 mL of a 1.6 M solution in hexanes, 53.9 mmol, 2.15 equiv) was added to a suspension of anhydrous lithium chloride (7.47 g, 176 mmol, 7.0 equiv) and diisopropylamine (8.09 mL, 57.7 mmol, 2.3 equiv) in THF (260 mL) at -78 °C. The reaction mixture was stirred for 30 minutes at -78°C, maintained at 0 °C for 5 minutes, and subsequently cooled again to -78 °C. trans-6-Methyl-hept-4-enoic acid (2*R*-hydroxy-1*R*-methyl-2-phenyl-ethyl)-methyl amide (7.26 g, 25.1 mmol, 1 equiv) in THF (50 mL) was added via cannula, and the resulting solution was stirred at -78 °C for 1.75 hours, maintained at 0 °C for 20 minutes, stirred at 23 °C for 5 minutes, and then cooled again to 0 °C. A solution of 4-methylbenzyl bromide (6.96 g, 37.6 mmol, 1.5 equiv) in THF (15 mL) was added, and the reaction mixture was stirred at 0 °C for 30 minutes and then partitioned between half-saturated NH₄Cl (230 mL) and a 1:1 mixture of EtOAc and hexanes (200 mL, 2 x 150 mL). The combined organic layers were dried over Na₂SO₄ and concentrated. Purification of the residue by flash column chromatography (gradient elution 20 → 40% EtOAc in hexanes) provided trans-6-methyl-25-(4-methyl-benzyl)- hept-4-enoic acid (2*R*-hydroxy-1*R*-methyl-2- phenyl-ethyl)-methyl amide (933 g, 95%) as a viscous oil: R_{*f*} = 0.44 (40% EtOAc in hexanes); IR (cm⁻¹) 3378, 1619; ¹H NMR (CDCl₃, mixture of rotamers) δ 0.84-0.97 (m), 2.12-2.27 (m), 2.29 (s), 2.35-2.46 (m), 2.58 (s), 2.66-2.84 (m), 2.86-3.05 (m), 3.91-4.13 (m), 4.30-4.44 (m), 4.45-4.53 (m), 5.17-5.54 (m), 7.04 (s), 7.12-733 (m); Anal. (C₂₆H₃₅NO₂) C, H, N.

### Preparation of Intermediate 5S-(1R-Bromo-2-methyl-propyl)-3R-(4-methyl-benzyl)-dihydrofuran-2-one

*N*-Bromosuccinimide (4.37 g, 24.6 mmol, 1.05 equiv) was added in small portions over 10 minutes to a solution of trans-6-methyl-2*S*-(4-methyl-benzyl)-hept-4-enoic acid (2*R*-hydroxy-1*R*-methyl-2-phenyl-ethyl)-methyl amide (9.21 g, 23.4 mmol, 1 equiv) and glacial acetic acid (6.70 mL, 117 mmol, 5.0 equiv) in a 4:1 mixture of THE and H₂O (250 mL) at 0 °C. The resulting yellow solution was stirred for 15 minutes at 0 °C, then warmed to 23°C, and subsequently refluxed for 45 minutes. After cooling to 23 °C, the reaction mixture was partitioned between half-saturated NaHCO₃ (200 mL) and a 1:1 mixture of EtOAc and hexanes (2 x 200 mL, 100 mL). The combined organic layers were dried over Na₂SO₄ and concentrated. Flash chromatographic purification of the residue (gradient elution 4 → 6% EtOAc in hexanes) gave 5*S*-(1*R*-bromo-2-methyl-propyl)-3*R*-(4-methyl-benzyl)-dihydrofuran-2-one (6.45 g, 85%) as a white solid (containing approximately 5-10% unidentified impurities by ¹H NMR): mp = 94-100 °C; R_{*f*} = 0.64 (25% EtOAc in hexanes); IR (cm⁻¹) 1772; ¹H NMR (CDCl₃, major isomer) δ 0.93 (d, 3H, *J =* 6.5), 0.99 (d, 3H, *J* = 6.5), 2.05-2.18 (m, 1H), 2.21-2.28 (m, 2H), 2.33 (s, 3H), 2.79 (dd, 1H, *J* = 13.5, 9.0), 2.92-3.03 (m, 1H), 3.12 (dd, 1H, *J* = 13.5, 4.5), 3.89 (dd, 1H, *J* = 9.0, 3.4), 4.32-4.41 (m, 1H), 7.06-7.16 (m, 4H); Anal. (C₁₆H₂₁BrO₂) C, H.

### Preparation of Intermediate 5S-(1S-Azido-2-methyl-propyl)-3R-(4-methyl-benzyl)-dihydrofuran-2-one

A suspension of sodium azide (2.55 g, 39.2 mmol, 2.0 equiv) and 5*S*-(1*R*-bromo-2-methyl-propyl)-3*R*-(4-methyl-benzyl)-dihydrofuran-2-one (6.37 g, 19.6 mmol, 1 equiv) in *N,N*-dimethylformamide (63 mL) was heated at 50 °C for 48 hours. The reaction mixture was cooled to 23 °C and partitioned between water (370 mL) and a 1:1 mixture of EtOAc and hexanes (2 x 370 mL, 200 mL). The combined organic layers were dried over Na₂SO₄, concentrated, and the residue purified by flash column chromatography (gradient elution 8 → 12% EtOAc in hexanes) to give 5*S*-(1*S*-azido-2-methyl-propyl)-3*R*-(4-methyl-benzyl)-dihydrofuran-2-one (3.33 g, 59%) as a colorless oil (containing approximately 5-10% unidentified impurities by 'H NMR): R_{*f*} = 0.52 (25% EtOAc in hexanes); IR (cm⁻¹) 2097, 1772; ¹H NMR (CDCl₃, major isomer) δ 0.97 (d, 3H, *J* = 6.5), 1.01 (d, 3H, *J* = 6.8), 1.94-2.06 (m, 1H), 2.07-2.13 (m, 2H), 2.33 (s, 3H), 2.79 (dd, 1H, *J* = 13.2, 8.2), 2.92 (dd, 1H. *J* = 6.8, 4.4), 3.02-3.17 (m, 2H), 4.32-4.40 (m, 1H), 7.07-7.15 (m, 4H); Anal. (C₁₆H₂₁N₃O₂) C, H, N.

### Preparation of Intermediate {2-Methyl-1S-[4R-(4-methyl-benzyl)-5-oxo-tetrahydrofuran-2S-yl]-propyl}-carbamic Acid tert-Butyl Ester

A suspension of 5*S*-(1*S*-azido-2-methyl-propyl)-3*R*-(4-methyl-benzyl)-dihydrofuran-2-one (3.24 g, 11.3 mmol, 1 equiv) and Pd/C (10%, 0.25 g) in CH₃OH (190 mL) was stirred under a hydrogen atmosphere (balloon) for 1 hour. The reaction mixture was vacuum filtered through Whatman #3 paper, concentrated, and the residue dissolved in 1,4-dioxane (100 mL). *N,N*-diisopropylethylamine (3.93 mL, 22.6 mmol, 2.0 equiv) and di-*tert*-butyl dicarbonate (3.69 g, 16.9 mmol, 1.5 equiv) were added sequentially, and the resulting solution was stirred at 23 °C for 16 hours. The reaction mixture was then partitioned between water (200 mL) and a 1:1 mixture of EtOAc and hexanes (2 x 300 mL, 200 mL). The combined organic layers were dried over Na₂SO₄ and concentrated. Purification of the residue by flash column chromatography (gradient elution, 11 → 17% EtOAc in hexanes) provided {2-methyl-1*S*-[4*R*-(4-methyl-benzyl)-5-oxo-tetrahydrofuran-2*S*-yl]-propyl}-carbamic acid *tert*-butyl ester (1.71 g, 42%) as a viscous oil: R_{*f*}= 0.54 (25% EtOAc in hexanes); IR (cm⁻¹) 3331, 1766, 1696; ¹H NMR (CDCl₃) δ 0.92 (d, 3H, *J =* 6.5), 0.93 (d, 3H, *J* = 6.5), 1.41 (s, 9H), 1.72-1.83 (m, 1H), 1.96-2.06 (m, 1H), 2.11-2.22 (m, 1H), 2.31 (s, 3H), 2.77 (dd, 1H, *J* = 13.5, 8.7), 2.88-2.99 (m, 1H), 3.08 (dd, 1H, *J* = 13.5, 4.5), 3.30-3.38 (m, 1H), 4.38-4.44 (m, 1H), 4.49 (d, 1H, *J=* 10.0), 7.04-7.13 (m, 4H); Anal. (C₂₁H₃₁NO₄) C, H, N.

### Preparation of Intermediate Ethyl-3-[BOC-L-ValΨ[COCH₂]-L-(p-CH₃)Phe-L-(Tr-Gln)]-E-Propenoate

Lithium hydroxide (7.9 mL of a 1 M aqueous solution, 7.9 mmol, 5.0 equiv) was added to a solution of {2-methyl-1*S*-[4*R*-(4-methyl-benzyl)-5-oxo-tetrahydrofuran-2*S*-yl]-propyl}-carbamic acid *tert*-butyl ester (0.570 g, 1.58 mmol, 1 equiv) in DME (15 mL) at 23 °C. The resulting suspension was stirred at 23 °C for 30 minutes and then partitioned between 10% KHSO₄ (35 mL) and CH₂Cl₂ (100 mL, 2 x 70 mL). The combined organic layers were dried over Na₂SO₄, concentrated, and the residue dissolved in CH₂Cl₂ (25 mL) Powdered 4Å molecular sieves (0.56 g), 4-methylmorpholine *N*-oxide (0.369 g, 3.15 mmol, 2.0 equiv), and tetrapropylammonium perruthenate (0.055 g, 0.16 mmol, 0.10 equiv) were added sequentially. The resulting dark reaction mixture was stirred for 1.33 hours at 23 °C, and the mixture then was vacuum filtered through Whatman #3 paper and then through Whatman #5 paper. The filtrate was concentrated under reduced pressure to provide a dark residue which was dissolved in CH₂Cl₂ (25 mL). Crude ethyl-3-[H₂N-L-(Tr*-Gln*)]-E-propenoate•HCl (see preparation of ethyl-3-[CBZ-L-LeuΨ[COCH₂]-D/L-Phe-L-(Tr-*Gln*)]-E-propenoate, 1.90 mmol, 1.2 equiv), 4-methylmorpholine (0.693 mL, 6.30 mmol, 4.0 equiv), 1-hydroxybenzotriazole hydrate (0.320 g, 2.37 mmol, 1.5 equiv), and 1-(3-dimethylaminopropyl)-3-ethylcarbodiimide hydrochloride (0.453 g, 2.36 mmol, 1.5 equiv) were added sequentially, and the reaction mixture was stirred for 20 hours at 23°C and then partitioned between water (70 mL) and EtOAc (2 x 100 mL, 50 mL). The combined organic layers were dried over Na₂SO₄ and were concentrated. Purification of the residue by flash column chromatography (38% EtOAc in hexanes) provided ethyl-3-[BOC-L-ValΨ[COCH₂]-L-(*p*-CH₃)Phe-L-(Tr-*Gln*)]-E-propenoate (0.677 g, 54%) as an off-white foam: R_{*f*} = 0.44 (50% EtOAc in hexanes); IR (cm⁻¹) 3307, 1708, 1666; ¹H NMR (CDCl₃) δ 0.64 (d, 3H, *J* = 6.8), 0.91 (d, 3H, *J* = 6.5), 1.28 (t, 3H, *J* = 7.2), 1.40 (s, 9H), 1.55-1.67 (m, 1H), 1.92-2.04 (m, 2H), 2.31 (s, 3H), 2.32-2.40 (m, 2H), 2.45-2.54 (m, 1H), 2.63 (dd, 1H, *J* = 11.5, 5.9), 2.68-2.87 (m, 2H), 3.03 (dd, 1H, *J* = 17.4, 10.0), 4.04-4.51 (m, 1H), 4.17 (q, 2H, *J* = 7.2), 4.41-4.52 (m, 1H), 4.83 (d, 1H, *J* = 8.4), 5.49 (d, 1H, *J* = 15.7), 5.86 (d, 1H, *J* = 8.4), 6.60 (dd, 1H, *J* = 15.7, 5.0), 7.00 (d, 2H, *J* = 8.0), 7.09 (d, 2H, *J* = 8.0); Anal. (C₄₉H₅₉N₃O₇) C, H, N.

### Preparation of Intermediate Ethyl-3-[EtSCO-L-ValΨ[COCH₂]-L(p-CH₂)Phe-L-(Tr-Gln)]-E-Propenoate

HCl (3 mL of a 4.0 M solution in 1,4-dioxane) was added to a solution of ethyl-3-[BOC-L-ValΨ[COCH₂]-L-(*p*-CH₃)Phe-L-(Tr-*Gln*)]-E-propenoate (0.315 g, 0.393 mmol, 1 equiv) in 1,4-dioxane (3 mL). The reaction mixture was stirred at 23 °C for 2 hours and then concentrated. The resulting oil was dissolved in CH₂Cl₂ (5 mL), and *N,N*-diisopropylethylamine (0.205 mL, 1.18 mmol, 3.0 equiv) and ethyl chlorothiolformate (0.061 mL, 0.59 mmol, 1.5 equiv) were added sequentially. The reaction mixture was stirred for 2 hours at 23 °C and then partitioned between brine (30 mL) and CH₂Cl₂ (3 x 30 mL). The combined organic layers were dried over Na₂SO₄, concentrated, and the residue was chromatographed on silica gel (40% EtOAc in hexanes) to afford ethyl-3-[EtSCO-L-ValΨ[COCH₂]-L-(*p*-CH₃)Phe-L-(Tr-*Gln*)]-E-propenoate (0.220 g, 71%) as a white foam: R_{*f*} = 0.29 (40% EtOAc in hexanes); IR (cm⁻¹) 3307, 1713, 1655; ¹H NMR (CDCl₃) δ 0.67 (d, 3H, *J* = 6.8), 0.92 (d, 3H, *J* = 6.8), 1.25 (t, 3H, *J* = 7.4), 1.28 (t, 3H, *J* = 7.4), 1.55-1.67 (m, 1H), 1.91-2.03 (m, 2H), 2.31 (s, 3H), 2.32-2.37 (m, 2H), 2.49 (dd, 1H, *J* = 17.7, 2.2), 2.62 (dd, 1H, *J* = 11.7, 6.1), 2.69-2.83 (m, 2H), 2.84 (q, 2H, *J* = 7.4), 3.04 (dd, 1H, *J* = 17.7, 10.0), 4.17 (q, 2H, *J* = 7.4), 4.35-4.51 (m, 2H), 5.50 (dd, 1H, *J* = 15.6, 1.6), 5.62 (d, 1H, *J* = 8.1), 5.95 (d, 1H, *J* = 8.1), 6.60 (dd, 1H, *J =* 15.6, 5.1), 6.99 (d, 2H, *J* = 8.1), 7.09 (d, 2H, *J* = 8.1), 7.18-7.31 (m, 16H); Anal. (C₄₇H₅₅N₃O₆S) C, H, N.

### Preparation of Product Ethyl-3-(EtSCO-L-ValΨ[COCH₂]-L-(p-CH₃)Phe-L-Gln)-E-Propenoate

Ethyl-3-[EtSCO-L-ValΨ[COCH₂]-L-(*p*-CH₂)Phe-L-(Tr-*Gln*)]-E-propenoate (0.169 g, 0.214 mmol) was dissolved in CH₂Cl₂ (6 mL), and triisopropylsilane (0.13 mL) and trifluoroacetic acid (3 mL) were added sequentially producing a bright yellow solution. The reaction mixture was stirred for 30 minutes at 23 °C and then concentrated under reduced pressure. The residue was purified by flash column chromatography (6% CH₃OH in CH₂Cl₂) to afford ethyl-3-(EtSCO-L-ValΨ[COCH₂]-L-(*p*-CH₃)Phe-L-*Gln*)-E-propenoate (0.072 g, 63%) as a white solid: mp = 220 °C (dec); R_{*f*} = 0.11 (5% CH₃OH in CH₂Cl₂); IR (cm⁻¹) 3425, 3307, 1713, 1655; ¹H NMR (DMSO-*d*₆) δ 0.74 (d, 3H, *J* = 6.8), 0.82 (d, 3H, *J* = 6.8), 1.14 (t, 3H, *J* = 7.3), 1.20 (t, 3H, *J* = 7.2), 1.54-1.76 (m, 2H), 1.98-2.13 (m, 3H), 2.23 (s, 3H), 2.40-2.54 (m, 2H), 2.65-2.84 (m, 2H), 2.73 (q, 2H, *J* = 7.3), 2.86-2.96 (m, 1H), 4.04-4.19 (m, 3H), 4.26-4.37 (m, 1H), 5.55 (dd, 1H, *J* = 15.7, 1.6), 6.65 (dd, 1H, *J* = 15.7, 5.3), 6.73 (s, 1H), 7.03 (s, 4H), 7.14 (s, 1H), 8.03 (d, 1H, *J* = 8.1), 8.31 (d, 1H, *J* = 7.8); Anal. (C₂₈H₄₁N₃O₆S) C, H, N.

### Example 11 - Preparation of Compound 11: Ethyl-3-(CyPentylSCO-L-ValΨ[COCH₂] -L-(p-CH₃)Phe-L-Gln)-E-Propenoate

### Preparation of Intermediate Ethyl-3-[CyPentylSCO-L-ValY[COCH₂]-L-(p-CH₃)Phe-L-(Tr-Gln)]-E-Propenoate

HCl (3 mL of a 4.0 M solution in 1,4-dioxane) was added to a solution of ethyl-3-[BOC-L-ValΨ[COCH₂]-L-(*p*-CH₃)Phe-L-(Tr-*Gln*)]-E-propenoate (0.304 g, 0.379 mmol, 1 equiv) in 1,4-dioxane (3 mL). The reaction mixture was stirred at 23 °C for 2.3 hours and then concentrated. The resulting oil was dissolved in CH₂Cl₂ (5 mL), and *N,N*-diisopropylethylamine (0.198 mL, 1.14 mmol, 3.0 equiv) and cyclopentyl chlorothiolformate (0.094 mL. 0.571 mmol, 1.5 equiv) were added sequentially. The reaction mixture was stirred for 3 hours at 23 °C and then partitioned between brine (30 mL) and CH₂Cl₂ (3 x 30 mL). The combined organic layers were dried over Na₂SO₄, concentrated, and the residue was chromatographed on silica gel (40% EtOAc in hexanes) to provide ethyl-3-[CyPentylSCO-L-ValΨ[COCH₂]-L-(*p*-CH₃)Phe-L-(Tr-*Gln*)]-E-propenoate (0.198 g, 63%) as a white foam: R_{*f*} = 0.29 (40% EtOAc in hexanes); IR (cm') 3307, 1713, 1655; ¹H NMR (CDCl₃) δ 0.67 (d, 3H, *J* = 6.8), 0.92 (d, 3H, *J* = 6.8), 1.28 (t, 3H, *J* = 7.2), 1.45-1.74 (m, 7H), 1.90-2.11 (m, 4H), 2.31 (s, 3H), 2.32-2.38 (m, 2H), 2.50 (dd, 1H, *J* = 17.7, 2.5), 2.62 (dd, 1H, *J* = 11.7, 5.8), 2.70-2.87 (m, 2H), 3.02 (dd, 1H, *J* = 17.7, 9.8), 3.57-3.67 (m, 1H), 4.17 (q, 2H, *J* = 7.2), 4.22-4.31 (m, 2H). 5.50 (dd, 1H. *J* = 15.8, 1.6), 5.58 (d, 1H. *J* = 8.4), 5.93 (d, 1H, *J* = 8.1), 6.60 (dd, 1H, *J* = 15.8, 5.1), 6.99 (d, 2H, *J* = 7.9), 7.09 (d, 2H, *J* = 7.9), 7.18-7.31 (m, 16H); Anal. (C₅₀H₅₉N₃O₆S) C, H, N.

### Preparation of Product Ethyl-3-(CyPentylSCO-L-ValY[COCH₂]-L-(p-CH₃)Phe-L-Gln)-E-Propenoate

Ethyl-3-[CyPentylSCO-L-ValΨ[COCH₂]-L-(*p*-CH₃)Phe-L-(Tr-*Gln*)]-E-propenoate (0.156 g, 0.188 mmol) was dissolved in CH₂Cl₂ (6 mL), and triisopropylsilane (0.12 mL) and trifluoroacetic acid (3 mL) were added sequentially producing a bright yellow solution. The reaction mixture was stirred for 30 minutes at 23 °C and then concentrated under reduced pressure. Et₂O (5 mL) was added to the residue producing a white precipitate. The precipitate was filtered, washed with Et₂O (3 x 3mL), and dried to provide ethyl-3-(CyPentylSCO-L-ValΨ[COCH₂]-L-(*p*-CH₃)Phe-L-*Gln*)-E-propenoate (0.096 g, 87%) as a white solid: mp = 208-210 °C (dec); R_{*f*} = 0.43 (10% CH₃OH in CH₂Cl₂); IR (cm⁻¹) 3425, 3295, 1713, 1649; ¹H NMR (DMSO-*d*₆) δ 0.74 (d, 3H, *J* = 6.5), 0.81 (d, 3H, *J* = 6.5), 1.20 (t, 3H, *J* = 7.2), 1.37-1.76 (m, 8H), 1.91-2.12 (m, 5H), 2.23 (s, 3H), 2.39-2.54 (m, 2H), 2.65-2.83 (m, 2H), 2.86-2.96 (m, 1H), 3.46-3.57 (m, 1H), 4.04-4.17 (m, 3H), 4.26-4.37 (m, 1H), 5.55 (dd, 1H, *J* = 15.7, 1.4), 6.65 (dd, 1H, *J* = 15.7, 5.4), 6.74 (s, 1H), 7.03 (s, 4H), 7.15 (s, 1H), 8.03 (d, 1H, *J* = 8.4), 8.25 (d, 1H, *J* = 8.1); Anal. (C₃₁H₄₅N₃O₆S) C, H, N.

### Example 12 - Preparation of Compound 12: Ethyl-3-(CyPentylSCO-L-PheΨ[COCH₂]-L-(p-CH₃)Phe-L-Gln)-E-Propenoate.

### Preparation of Intermediate (1R/S-Oxiranyl-2S-phenyl-ethyl)-carbamic Acid tert-Butyl Ester (1:1 mixture of isomers).

DMSO (30 mL) was added to sodium hydride (0.900 g of a 60% dispersion in mineral oil, 22.5 mmol, 2.1 equiv), and the resulting suspension was heated to 70 °C for 20 min. The clear solution thus obtained was cooled to 23 °C, and THF (40 mL) was added. The reaction mixture was then cooled to 0 °C, and a solution of trimethylsulfonium iodide (4.59 g, 22.5 mmol. 2.1 equiv) in DMSO (30 mL) was added via cannula over 2 min. After stirring for 1 min at 0 °C, a solution of BOC-L-Phe-H (prepared according to Luly, J. R; Dellaria, J. F.; Plattner, J. J.; Soderquist, J, L.; Yi, N. *J. Org*. *Chem.* **1987**, *52*, 1487) (2.5 g, 10.7 mmol, 1 equiv) in THF (20 mL) was added via cannula over 5 min. The reaction mixture was stirred at 0 °C for 2 h and then partitioned between 0.5 M HCl (150 mL) and a 1:1 mixture of EtOAc and hexanes (2 x 150 mL). The organic layers were dried over Na₂SO₄ and concentrated. Purification of the residue by flash column chromatography (gradient elution, 15→20% EtOAc in hexanes) afforded (1*R*/*S*-oxiranyl-2*S*-phenyl-ethyl)-carbamic acid *tert*-butyl ester (1:1 mixture of isomers, 1.71 g, 60%) as a colorless oil: R_{*f*}= 0.29 (isomer #1), 0.35 (isomer #2) (20% EtOAc in hexanes); IR (cm⁻¹) 3347, 2977, 1700; ¹H NMR (CDCl₃) δ_1.38 (s), 1.43 (s), 2.57-3.03 (m), 3.76 (s, br), 3.98 (s, br), 4.11 (s, br), 4.48 (s, br), 4.88 (s, br), 7.18-7.38 (m); Anal. (C₁₅H₂₁NO₃) C, H, N.

### Preparation of Intermediate 3-p-Tolyl-propionic Acid cis-1S-Amino-2R-indanol-acetonide Amide.

Oxalyl chloride (3.07 mL, 35.2 mmol, 1.05 equiv) was added to a solution of 3-*p*-tolyl-propionic acid (5.50 g, 33.5 mmol, 1 equiv) and *N,N*-dimethylformamide (0.03 mL, 0.39 mmol, 0.012 equiv) in benzene (150 mL) at 23 °C. The reaction mixture was stirred at 23 °C for 3 h and then concentrated. The resulting oil was dissolved in THF (30 mL) and added to a 0 °C solution of (1*S*,2*R*)-cis-1-amino-2-indanol (5.0 g, 33.5 mmol, 1.0 equiv) and Et₃N (5.14 mL, 36.9 mmol, 1.1 equiv) in THF (250 mL). After stirring for 30 min at 0 °C, the reaction mixture was partitioned between half-saturated NH₄Cl (150 mL) and EtOAc (2 x 150 mL). The organic layers were dried over Na₂SO₄ and concentrated to afford a white solid. This material was dissolved in a 2:1 mixture of CH₂Cl₂ and 2-methoxypropene (150 mL), and the resulting solution was treated with methanesulfonic acid (1.0 mL). After stirring 1 h at 23 °C, the reaction mixture was partitioned between half-saturated NaHCO₃ (150 mL), and CH₂Cl₂ (2 x 100 mL). The combined organic layers were washed with H₂O (100 mL), dried over MgSO₄, and gravity filtered. The filtrate was concentrated, and the residue was purified by flash column chromatography (20% EtOAc in hexanes) to provide 3 *p*-tolyl-propionic acid cis-1*S*-amino-2*R*-indanol-acetonide amide (5.67 g, 51%) as a pale orange oil: R_{*f*}= 0.63 (50% EtOAc in hexanes); IR (cm⁻¹) 2931, 1646; ¹H NMR (CDCl₃) δ_1.35 (s, 3H), 1.61 (s, 3H), 2.33 (s, 3H), 2.90-2.95 (m, 2H), 3.05-3.09 (m, 4H), 4.66-4.69 (m, 1H), 5.05 (d, 1H, J= 4.7), 7.10-7.27 (m, 8H); Anal. (C₂₂H₂₅NO₂) C, H, N.

### Preparation of Intermediate {1S-[4R-(4-Methyl-benzyl)-5-oxo-tetrahydro-furan-2S-yl]-2-phenyl-ethyl}-carbamic Acid tert-Butyl Ester.

*n*-Butyllithium (8.12 mL of a 1.6 M solution in hexanes, 13.0 mmol, 2.0 equiv) was added to a solution of (1*R*/*S*-oxiranyl-2*S*-phenyl-ethyl)-carbamic acid *tert*-butyl ester (1:1 mixture of isomers, 1.71 g, 6.49 mmol, 1 equiv) and 3-*p*-tolyl-propionic acid cis-1*S*-amino-2*R*-indanol-acetonide amide (2.18 g, 6.50 mmol, 1 equiv) in THF (100 mL) at -78 °C. The reaction mixture was stirred for 10 min at -78 °C, maintained at 0 °C for 1.5 h, and then partitioned between 0.5 M HCl (150 mL) and a 1:1 mixture of EtOAc and hexanes (2 x 150 mL). The organic layers were dried over Na₂SO₄ and concentrated. Flash chromatographic purification of the residue (gradient elution, 20→30% EtOAc in hexanes) gave the coupling product (1.02 g, single isomer, 26%) as an orange oil contaminated with several minor impurities. This material was dissolved in a 5:1 mixture of toluene and CH₂Cl₂ (60 mL) and was treated with*p*-toluenesulfonic acid monohydrate (0.324 g, 1.70 mmol, 1.0 equiv) at 23 °C. After stirring 12 h at 23 °C, the reaction mixture was filtered through a medium frit, and the filtrate was partitioned between half-saturated NaHCO₃ (150 mL) and a 1:1 mixture of EtOAc and hexanes (2 x 100 mL). The organic layers were dried over Na₂SO₄ and concentrated. The residue was purified by flash column chromatography (15% EtOAc in hexanes) to provide {1*S*-[4R-(4-methyl-benzyl)-5-oxo-tetrahydro-furan-2S-yl]-2-phenylethyl}-carbamic acid *tert*-butyl ester (0.26 g, 37%) as a white foam: R_{*f*}= 0.60 (30% EtOAc in hexanes); IR (cm⁻¹) 3336, 1768, 1703; ¹H NMR (CDCl₃) δ_1.36 (s, 9H), 1.90-1.99 (m, 1H), 2.15-2.27 (m, 1H), 2.30 (s, 3H), 2.74 (dd, 1H, *J* = 13.5, 8.6), 2.82-2.88 (m, 2H), 2.91-3.00 (m, 1H), 3.06 (dd, 1H, *J* = 13.5, 4.5), 3.88-3.97 (m, 1H), 4.18-4.23 (m, 1H), 4.51 (d, 1H, *J=* 9.7), 7.01-7.08 (m, 4H), 7.17-7.31 (m, 5H); Anal. (C₂₅H₃₁NO₄) C, H, N.

### Preparation of Intermediate Ethyl-3-[BOC-L-PheΨ[COCH₂]-L-(p-CH₃)Phe-L-(Tr-Gln)]-E-Propenoate.

Lithium hydroxide (3.17 mL of a 1 M aqueous solution, 3.17 mmol, 5 equiv) was added to a solution of {1*S*-[4R-(4-methyl-benzyl)-5-oxo-tetrahydro-furan-2S-yl]-2-phenylethyl}-carbamic acid *tert*-butyl ester (0.260 g, 0.635 mmol, 1 equiv) in DME (4 mL) at 23 °C. The resulting suspension was stirred at 23 °C for 30 min and then partitioned between 0.5 M HCl (100 mL) and EtOAc (2 x 100 mL). The combined organic layers were dried over Na₂SO₄, concentrated, and the residue dissolved in a 1:1 mixture of CH₃CN and CH₂Cl₂ (60 mL). 4-Methylmorpholine *N*-oxide (0.149 g, 1.27 mmol, 2 equiv), powdered 4A molecular sieves (0.50 g), and tetrapropylammonium perruthenate (0.022 g, 0.063 mmol, 0.1 equiv) were added sequentially. The resulting dark reaction mixture was stirred for 1 h at 23 °C and then filtered through celite. The filtrate was concentrated under reduced pressure to provide a brown oil which was dissolved in CH₂Cl₂ (20 mL). Crude ethyl-3-[H₂N-L-(Tr-*Gln*)]-E-propenoate•HCl (see preparation of ethyl-3-[CBZ-L-LeuΨ[COCH₂]-D/L-Phe-L-(Tr-*Gln*)]-E-propenoate, 0.762 mmol, 1.2 equiv), 1-hydroxybenzotriazole hydrate (0.112 g, 0.829 mmol, 1.3 equiv), 4-methylmorpholine (0.280 mL, 2.55 mmol, 4 equiv), and 1-(3-dimethylaminopropyl)-3-ethylcarbodiimide hydrochloride (0.158 g, 0.824 mmol, 1.3 equiv) were added sequentially, and the reaction mixture was stirred for 18 h at 23 °C and then partitioned between water (100 mL) and a 1:1 mixture of EtOAc and hexanes (2 x 100 mL). The combined organic layers were dried over Na₂SO₄ and concentrated. Purification of the residue by flash column chromatography (40% EtOAc in hexanes) provided ethyl-3-[BOC-L-PheΨ[COCH₂]-L-(*p*-CH₃)Phe-L-(Tr-*Gln*)]-E-propenoate (0.190 g, 35%) as a white foam: R_{*f*} = 0.43 (50% EtOAc in hexanes); IR (cm⁻¹) 3314, 1710, 1665; ¹H NMR (CDCl₃) δ 1.28 (t, 3H, *J* = 7.2), 1.35 (s, 9H), 1.63-1.65 (m, 1H), 1.97-2.05 (m, 1H), 2.33 (s, 3H), 2.36-2.38 (m, 2H), 2.48 (d, 1H. *J* = 15.9), 2.57-2.64 (m, 2H), 2.75-3.00 (m, 4H), 4.17 (q, 2H, *J* = 7.2), 4.25-4.32 (m, 1H), 4.52 (s, br, 1H), 4.77 (d, 1H, *J=* 6.9), 5.53 (dd, 1H, *J* = 15.9, 1.6), 5.94 (d, 1H, *J=* 8.4), 6.63 (dd, 1H, *J* = 15.9, 5.0), 6.99-7.09 (m, 5H), 7.11-7.32 (m, 20H); Anal. (C₅₃H₅₉N₃O₇) C, H, N.

### Preparation of Intermediate Ethyl-3-[CyPentylSCO-L-PheΨ[COCH₂]-L-(p-CH₃)Phe-L-(Tr-Gln)]-E-Propenoate.

HCl (5 mL of a 4.0 M solution in 1,4-dioxane) was added to a solution of ethyl-3-[BOC-L-PheΨ[COCH₂]-L-(*p*-CH₃)Phe-L-(Tr-*Gln*)]-E-propenoate (0.190 g, 0.224 mmol, 1 equiv) in 1,4-dioxane (6 mL). The reaction mixture was stirred at 23 °C for 1.5 h and then concentrated. The resulting oil was dissolved in CH₂Cl₂ (10 mL), cooled to 0 °C, and 4-methylmorpholine (0.075 mL, 0.682 mmol, 3.0 equiv) and cyclopentyl chlorothiolformate (0.055 mL, 0.334 mmol, 1.5 equiv) were added sequentially. The reaction mixture was stirred for 1 h at 0 °C and then partitioned between water (100 mL) and a 1:1 mixture of EtOAc and hexanes (2 x 100 mL). The combined organic layers were dried over Na₂SO₄, concentrated, and the residue was chromatographed on silica gel (40% EtOAc in hexanes) to afford ethyl-3-[CyPentylSCO-L-PheΨ[COCH₂]-L-(*p*-CH₃)Phe-L-(Tr-*Gln*)]-E-propenoate (0.102 g, 50%) as an off-white foam: R_{*f*}= 0.49 (50% EtOAc in hexanes); IR (cm⁻¹) 3316, 1718, 1655; ¹H NMR (CDCl₃) δ 1.29 (t, 3H, *J*= 7.2), 1.45-1.67 (m, 7H), 1.98-2.05 (m, 2H), 2.33 (s, 3H), 2.42 (d, 1H, *J* = 15.9), 2.55-2.98 (m, 6H), 3.52-3.63 (m, 1H), 4.17 (q, 2H, *J*= 7.2), 4.38-4.54 9m, 2H), 5.52-5.58 (m, 2H), 6.03 (d, 1H, *J*= 8.4), 6.64 (dd, 1H, *J*= 15.9, 5.0), 6.95-7.08 (m, 5H), 7.11-7.32 (m, 21H); Anal. (C₅₄H₅₉N₃O₆S) C, H, N.

### Preparation of Product Ethyl-3-(CyPentylSCO-L-PheΨ[COCH₂]-L-(p-CH₃)Phe-L-Gln)-E-Propenoate.

Triisopropylsilane (0.10 mL) and trifluoroacetic acid (5 mL) were added sequentially to a solution of ethyl-3-[CyPentylSCO-L-PheΨ[COCH₂]-L-(*p*-CH₃)Phe-L-(Tr-*Gln*)]-E-propenoate (0.075 g, 0.085 mmol) in CH₂Cl₂ (6 mL) producing a bright yellow solution. The reaction mixture was stirred for 15 min at 23 °C, then carbon tetrachloride (5 mL) was added, and the mixture was concentrated under reduced pressure. The residue was purified by flash column chromatography (5% CH₃OH in CH₂Cl₂) to afford ethyl-3-(CyPentylSCO-L-PheΨ[COCH₂]-L-(*p*-CH₃)Phe-L-*Gln*)-E-propenoate (0.047 g, 87%) as a white foam: R_{*f*}= 0.62 (10% CH₃OH in CH₂Cl₂); IR (cm⁻¹) 3286, 1718, 1637; ¹H NMR (DMSO-*d*₆) δ 1.20 (t, 3H, *J*= 6.8), 1.33-1.70 (m, 10H), 1.91-2.07 (m, 4H), 2.24 (s, 3H), 2.38-3.05 (m, 5H), 3.42-3.46 (m, 1H), 4.07-4.11 (m, 2H), 4.34 (s, br, 2H), 5.58 (d, 1H, *J* = 15.7), 6.67 (dd, 1H, *J*= 15.7, 4.4), 6.76 (s, br, 1H), 7.02-7.25 (m, 10H), 8.07 (d, 1H, *J* = 7.8), 8.44 (d, 1H, *J*= 7.5); Anal. (C₃₅H₄₅N₃O₆S) C, H, N.

### Example 13 - Preparation of Compound 13: Ethyl-3-(EtSCO-L-ValΨ[COCH₂]-L-(p-F)Phe-L-Gln)-E-Propenoate.

### Preparation of Intermediate trans-6-Methyl-2S-(4-fluoro benzyl)-hept-4-enoic Acid (2R-Hydroxy-1R-methyl-2-phenyl-ethyl)-methyl Amide.

*n*-Butyllithium (32.5 mL of a 1.6 M solution in hexanes, 52.0 mmol, 3.1 equiv) was added to a suspension of anhydrous lithium chloride (7.18 g, 169 mmol, 10 equiv) and diisopropylamine (7.80 mL, 55.7 mmol, 3.3 equiv) in THF (250 mL) at -78 °C. The reaction mixture was stirred for 30 min at -78 °C, then was maintained at 0 °C for 5 min, and subsequently was cooled again to -78 °C. trans-6-Methyl-hept-4-enoic acid (2*R*-hydroxy-1*R*-methyl-2-phenyl-ethyl)-methyl amide (4.91 g, 17.0 mmol, 1 equiv) in THF (50 mL) was added via cannula, and the resulting solution was stirred at -78 °C for 1.75 h, maintained at 0 °C for 20 min, stirred at 23 °C for 5 min, and then cooled again to 0 °C. A solution of 4-fluorobenzyl bromide (6.34 mL, 50.9 mmol, 3 equiv) in THE (15 mL) was added, and the reaction mixture was stirred at 0 °C for 30 min, and it then was partitioned between half-saturated NH₄Cl (230 mL) and a 1:1 mixture of EtOAc and hexanes (200 mL, 2 x 150 mL). The combined organic layers were dried over Na₂SO₄ and concentrated. Purification of the residue by flash column chromatography (gradient elution 20→40% EtOAc in hexanes) provided trans-6-methyl-2*S*-(4-fluoro-benzyl)-hept-4-enoic acid (2*R*-hydroxy-1*R*-methyl-2-phenyl-ethyl)-methyl amide (6.33 g, 94%) as a viscous oil: R_{*f*}=0.38 (40% EtOAc in hexanes); IR (cm⁻¹) 3378, 1614; ¹H NMR (CDCl₃, mixture of rotamers) δ 0.85-0.95 (m), 0.96 (d, *J=* 6.8), 2.10-2.32 (m), 2.34-2.46 (m), 2.58 (s), 2.67-2.79 (m), 2.82-2.94 (m), 3.00-3.18 (m), 3.94 (br), 4.37-4.52 (m), 5.24-5.42 (m), 5.44-5.56 (m), 6.89-7.01 (m), 7.08-7.14 (m), 7.19-7.38 (m); Anal. (C₂₅H₃₂FNO₂) C, H, N.

### Preparation of Intermediate 5S-(1R-Bromo-2-methyl-propyl)-3R-(4-fluoro-benzyl)-dihydrofuran-2-one.

*N*-Bromosuccinimide (2.93 g, 16.5 mmol, 1.05 equiv) was added in small portions over 10 min to a solution of trans-6-methyl-2*S*-(4-fluoro-benzyl)-hept-4-enoic acid (2*R*-hydroxy-1*R*-methyl-2-phenyl-ethyl)-methyl amide (6.24 g, 15.7 mmol, 1 equiv) and glacial acetic acid (4.49 mL, 78.4 mmol, 5 equiv) in a 4:1 mixture of THF and H₂O (165 mL) at 0°C. The resulting yellow solution was stirred for 15 min at 0 °C, then was warmed to 23 °C, and subsequently was refluxed for 45 min. After cooling to 23 °C, the reaction mixture was partitioned between half-saturated NaHCO₃ (200 mL) and a 1:1 mixture of EtOAc and hexanes (2 x 200 mL, 100 mL). The combined organic layers were dried over Na₂SO₄ and concentrated. Flash chromatographic purification of the residue (gradient elution 4→6→10% EtOAc in hexanes) gave 5*S*-(1*R*-bromo-2-methyl-propyl)-3*R*-(4-fluoro-benzyl)-dihydrofuran-2-one (4.14 g, 80%) as a pale yellow oil (containing approximately 5-10% unidentified impurities by ¹H NMR): R_{*f*}= 0.56 (25% EtOAc in hexanes); IR (cm⁻¹) 1772; ¹H NMR (CDCl₃, major isomer) δ 0.94 (d, 3H, *J*= 6.5), 1.00 (d, 3H, *J*= 6.8), 2.05-2.35 (m, 3H), 2.83 (dd, 1H, *J* = 13.6, 8.4), 2.92-3.03 (m, 1H), 3.11 (dd, 1H, *J* = 13.6, 4.7), 3.90 (dd, 1H, *J* = 9.0, 3.7), 4.33-4.40 (m, 1H), 6.98-7.06 (m, 2H), 7.14-7.20 (m, 2H); Anal. (C₁₅H₁₈BrFO₂) C, H.

### Preparation of Intermediate 5S-(1S-Azido-2-methyl-propyl)-3R-(4-fluoro-benzyl)-dihydrofuran-2-one.

A suspension of sodium azide (1.90 g, 29.2 mmol, 2.5 equiv) and 5*S*-(1*R*-bromo-2-methyl-propyl)-3*R*-(4-fluoro-benzyl)-dihydrofuran-2-one (3.85 g, 11.7 mmol, 1 equiv) in *N*,*N*-dimethylformamide (40 mL) was heated at 50 °C for 67 hours. The reaction mixture was cooled to 23 °C and partitioned between half-saturated NaCI (200 mL) and a 1:1:1 mixture of EtOAc, hexanes, and acetone (2 x 200 mL, 100 mL). The combined organic layers were dried over Na₂SO₄, concentrated, and the residue purified by flash column chromatography (gradient elution 9→12→17% EtOAc in hexanes) to give 5*S*-(1*S*-azido-2-methyl-propyl)-3*R*-(4-fluoro-benzyl)-dihydrofuran-2-one (2.10 g, 62%) as a white solid (containing approximately 5-10% unidentified impurities by ¹H NMR): mp 91-96 °C; R_{*f*}= 0.44 (25% EtOAc in hexanes); IR (cm⁻¹) 2097, 1772; ¹H NMR (CDCl₃, major isomer) δ 0.99 (d, 3H, *J=* 6.5), 1.02 (d, 3H, *J=* 6.8), 1.95-2.20 (m, 3H), 2.78-2.88 (m, 1H), 2.94 (dd, 1H, *J=* 7.0, 4.2), 3.03-3.17 (m, 2H), 4.37-4.43 (m, 1H), 6.97-7.09 (m, 2H), 7.14-7.21 (m, 2H).

### Preparation of Intermediate {2-Methyl-1S-[4R-(4-fluoro-benzyl)-5-oxo-tetrahydrofuran-2S-yl]-propyl}-carbamic Acid tert-Butyl Ester.

A suspension of 5*S*-(1*S*-azido-2-methyl-propyl)-3*R*-(4-fluoro-benzyl)-dihydrofuran-2-one (2.02 g, 6.93 mmol, 1 equiv), di-*tert*-butyl dicarbonate (2.12 g, 9.71 mmol, 1.4 equiv) and Pd/C (10%, 0.20 g) in CH₃OH (100 mL) was stirred under a hydrogen atmosphere (balloon) for 16 hours. The reaction mixture was vacuum filtered through Whatman #3 paper and concentrated. Purification of the residue by flash column chromatography (15% EtOAc in hexanes) provided {2-methyl-1*S*-[4*R*-(4-fluoro-benzyl)-5-oxo-tetrahydrofuran-2*S*-yl]-propyl}-carbamic acid *tert*-butyl ester (1.58 g, 62%) as a white foam: R_{*f*}= 0.80 (5% MeOH in CH₂Cl₂); IR (cm⁻¹) 3331, 1766, 1702; ¹H NMR (CDCl₃) δ 0.93 (d, 3H, *J* = 6.8), 0.95 (d, 3H, *J* = 6.5), 1.41 (s, 9H), 1.71-1.83 (m, 1H), 1.95-2.06 (m, 1H), 2.16-2.27 (m, 1H), 2.80 (dd, 1H, *J* = 13.5, 8.6), 2.88-2.99 (m, 1H), 3.09 (dd, 1H, *J* = 13.5, 4.4), 3.32-3.40 (m, 1H), 4.42-4.48 (m, 2H), 6.95-7.03 (m, 2H), 7.11-7.18 (m, 2H); Anal. (C₂₀H₂₈FNO₄) C, H, N.

### Preparation of Intermediate Ethyl-3-[BOC-L-ValΨ[COCH₂]-L-(p-F)Phe-L-(Tr-Gln)]-E-Propenoate.

Lithium hydroxide (9.62 mL of a 1 M aqueous solution, 9.62 mmol, 5 equiv) was added to a solution of {2-methyl-1*S*-[4*R*-(4-fluoro-benzyl)-5-oxo-tetrahydrofuran-2*S*-yl]-propyl}-carbamic acid *tert-*butyl ester (0.703 g, 1.92 mmol, I equiv) in DME (25 mL) at 23°C. The resulting suspension was stirred at 23 °C for 30 min, and it then was partitioned between 10% KHSO₄ (50 mL) and CH₂Cl₂ (3 x 100 mL). The combined organic layers were dried over Na₂SO₄, concentrated, and the residue dissolved in CH₂Cl₂ (30 mL). Powdered 4A molecular sieves (0.70 g), 4-methylmorpholine *N*-oxide (0.451 g, 3.85 mmol, 2 equiv), and tetrapropylammonium perruthenate (0.068 g, 0.19 mmol, 0.10 equiv) were added sequentially. The resulting dark reaction mixture was stirred for 1.33 hours at 23 °C, and it then was vacuum filtered through Whatman #3 paper and then through Whatman #5 paper. The filtrate was concentrated under reduced pressure to provide a dark residue which was dissolved in CH₂Cl₂ (30 mL). Crude ethyl-3-[H₂N-L-(Tr-*Gln*)]-E-propenoate•HCl (2.30 mmol, 1.2 equiv, prepared as described in Example 1 for the preparation of ethyl-3-[CBZ-L-LeuΨ[COCH₂]-D/L-Phe-L-(Tr-*Gln*)]-E-propenoate), 4-methylmorpholine (0.846 mL, 7.69 mmol, 4 equiv), 1-hydroxybenzotriazole hydrate (0.390 g, 2.89 mmol, 1.5 equiv), and 1-(3-dimethylaminopropyl)-3-ethylcarbodiimide hydrochloride (0.553 g, 2.88 mmol, 1.5 equiv) were added sequentially, and the reaction mixture was stirred for 19 hours at 23 °C and then partitioned between brine (100 mL) and CH₂Cl₂ (3 x 100 mL). The combined organic layers were dried over Na₂SO₄ and concentrated. Purification of the residue by flash column chromatography (gradient elution 35→40% EtOAc in hexanes) provided ethyl-3-[BOC-L-ValΨ[COCH₂]-L-(*p*-F)Phe-L-(Tr-*Gln*)]-E-propenoate (0.820 g, 53%) as a tan foam: R_{*f*}= 0.50 (50% EtOAc in hexanes); IR (cm⁻¹) 3307, 1708, 1666; ¹H NMR (CDCl₃) δ 0.67 (d, 3H, *J*= 6.8), 0.92 (d, 3H, *J* = 6.8), 1.28 (t, 3H, *J =* 7.2), 1.40 (s, 9H), 1.53-1.67 (m, 1H), 1.91-2.04 (m, 2H), 2.32-2.41 (m, 2H), 2.46-2.55 (m, 1H), 2.63 (dd, 1H, *J* = 12.1, 5.9), 2.69-2.80 (m, 1H), 2.83 (dd, 1H, *J* = 12.1, 8.2), 3.03 (dd, 1H, *J* = 17.7, 10.0), 4.05-4.11 (m, 1H), 4.17 (q, 2H, *J*= 7.2), 4.40-4.50 (m, 1H), 4.84 (d, 1H, *J*= 8.4), 538 (d, 1H, *J* = 15.7), 6.01 (d, 1H, *J*= 8.4), 6.60 (dd, 1H. *J*= 15.7, 5.0), 6.92-6.99 (m, 2H), 7.03-7.12 (m, 3H), 7.17-7.30 (m, 15H); Anal. (C₄₈H₅₆FN₃O₇) C, H, N.

### Preparation of Intermediate Ethyl-3-[EtSCO-L-ValΨ[COCH₂]-L-(p-F)Phe-L-(Tr-Gln)]-E-Propenoate.

HCl (3 mL of a 4.0 M solution in 1,4-dioxane) was added to a solution of ethyl-3-[BOC-L-ValΨ[COCH₂]-L-(*p*-F)Phe-L-(Tr-*Gln*)]-E-propenoate (0.271 g, 0.336 mmol) in 1,4-dioxane (3 mL). The reaction solution was stirred at 23 °C for 2 hours and then was concentrated. The residue was dissolved in dry CH₂Cl₂ (5 mL). *N,N*-diisopropylethylamine (0.176 mL, 1.01 mmol, 3 equiv) and ethyl chlorothiolformate (0.060 mL, 0.58 mmol, 1.7 equiv) were added sequentially. The reaction solution was stirred 2 hours at 23 °C and then was partitioned between brine (30 mL) and CH₂Cl₂ (3 x 30 mL). The combined organic phases were dried over Na₂SO₄, concentrated, and the residue was chromatographed on silica gel (gradient elution 40-50% EtOAc in hexanes) to afford ethyl-3-[EtSCO-L-ValΨ[COCH₂]-L-(*p*-F)Phe-L-(Tr-*Gln*)]-E-propenoate (0.183 g, 69%) as a white foam: R_{f}= 0.43 (50% EtOAc in hexanes); IR (cm⁻¹) 3307, 1708, 1655; ¹H NMR (CDCl₃) δ 0.70 (d, 3H, *J*= 6.8), 0.93 (d, 3H, *J* = 6.5), 1.25 (t, 3H, *J* = 7.2), 1.29 (t, 3H, *J* = 7.2), 1.55-1.66 (m. 1H), 1.91-2.05 (m, 2H), 2.30-2.39 (m, 2H), 2.51 (dd, 1H, *J* = 17.4, 2.5), 2.63 (dd, 1H, *J* = 12.1, 5.9), 2.69-2.90 (m, 4H), 3.02 (dd, 1H, *J*= 17.4, 10.0), 4.17 (q, 2H, *J* = 7.2), 4.35-4.49 (m, 2H), 5.39 (dd, 1H, *J* = 15.6, 1.7), 5.66 (d, 1H, *J* = 8.1), 6.12 (d, 1H, *J* = 8.1), 6.60 (dd, 1H, *J* = 15.6, 5.0), 6.91-6.99 (m, 2H), 7.02-7.10 (m, 2H), 7.17-7.32 (m, 16H); Anal. (C₄₆H₅₂N₃O₆S) C, H, N.

### Preparation of Product Ethyl-3-(EtSCO-L-ValΨ[COCH₂]-L-(p-F)Phe-L-Gln)-E-Propenoate.

Ethyl-3-[EtSCO-L-ValΨ[COCH₂]-L-(*p*-F)Phe-L-(Tr-*Gln*)]-E-propenoate (0.149 g, 0.188 inmol, 1 equiv) was dissolved in dry CH₂Cl₂ (6 mL). Triisopropylsilane (0.115 mL, 0.561 mmol, 3 equiv) and trifluoroacetic acid (3 mL) were added sequentially producing a bright yellow solution. This solution was stirred for 30 minutes and thenconcentrated. The residue was stirred in Et₂O (6 mL), and the solid was collected by filtration, washed with Et₂O (3 mL then 2 mL), and then dried under vacuum to give ethyl-3-(EtSCO-L-ValΨ[COCH₂]-L-(*p*-F)Phe-L*-Gln*)-E-propenoate (0.090 g, 87%) as a white solid: mp = 214 °C (dec); R_{*f*}= 0.49 (10% CH₃OH in CH₂Cl₂); IR (cm⁻¹) 3425, 3284, 1713, 1643; ¹H NMR (DMSO-*d*₆) δ 0.76 (d, 3H, *J*= 6.5), 0.82 (d, 3H, *J* = 6.8), 1.15 (t, 3H, *J* = 7.2), 1.21 (t, 3H, *J* = 7.2), 1.53-1.75 (m, 2H), 1.99-2.14 (m, 3H), 2.52-2.85 (in, 6H), 2.88-2.99 (m, 1H), 4.09 (q, 2H, *J* = 7.2), 4.13-4.20 (m, 1H), 4.25-4.36 (m, 1H), 5.40 (dd, 1H, *J* = 15.6, 1.4), 6.61 (dd, 1H, *J* = 15.6, 5.3), 6.74 (s, 1H), 6.99-7.24 (m, 5H), 8.01 (d, 1H, *J* = 8.1), 8.34 (d, 1H, *J* = 8.1); Anal. (C₂₇H₃₈FN₃O₆S) C, H, N.

### Example 14 - Preparation of Compound 14: Ethyl-3-(CyPentylSCO-L-ValΨ[COCH₂]-L-(p-F)Phe-L-Gln)-E-Propenoate.

### Preparation of Intermediate Ethyl-3-[CyPentylSCO-L-ValΨ[COCH₂]-L-(p-F)Phe-L-(Tr-Gln)]-E-Propenoate.

HCl (3 mL of a 4.0 M solution in 1,4-dioxane) was added to a solution of ethyl-3-[BOC-L-ValΨ[COCH₂]-L-(*p*-F)Phe-L-(Tr-*Gln*)]-E-propenoate (0.273 g, 0.339 mmol) in 1,4-dioxane (3 mL). The reaction solution was stirred at 23 °C for 2 hours and then concentrated. The residue was dissolved in dry CH₂Cl₂ (5 mL). *N,N*-diisopropylethylamine (0.177 mL, 1.02 mmol, 3 equiv) and cyclopentyl chlorothiolformate (0.095 mL, 1.7 equiv) were added sequentially. The reaction solution was stirred 2 hours at 23 °C and then partitioned between brine (30 mL) and CH₂Cl₂ (3 x 30 mL). The combined organic phases were dried over Na₂SO₄, concentrated, and the residue was chromatographed on silica gel (40% EtOAc in hexanes) to afford ethyl-3-[CyPentylSCO-L-ValΨ[COCH₂]-L-(*p*-F)Phe-L-(Tr-*Gln*)]-E-propenoate (0.166 g, 59%) as a white foam: R_{*f*}= 0.24 (40% EtOAc in hexanes); IR (cm⁻¹) 3307, 1713, 1654; ¹H NMR (CDCl₃) δ 0.69 (d, 3H, *J* = 6.8), 0.93 (d, 3H, *J* = 6.8), 1.29 (t, 3H, *J*= 7.2), 1.47-1.75 (m, 7H), 1.91-2.12 (m, 4H), 2.30-2.41 (m, 2H), 2.51 (dd, 1H, *J* = 17.2, 2.3), 2.63 (dd, 1H, *J* = 12.3, 5.9), 2.69-2.80 (m, 1H), 2.84 (dd, 1H, *J* = 12.3, 8.4), 3.01 (dd, 1H, *J*= 17.2, 10.0), 3.57-3.67 (m, 1H), 4.17 (q, 2H, *J*= 7.2), 4.33-4.50 (m, 2H), 5.39 (dd, 1H, *J*= 15.7, 1.7), 5.61 (d, 1H, *J* = 7.8), 6.11 (d, 1H, *J* = 8.1), 6.60 (dd, 1H, *J* = 15.7, 4.8), 6.92-7.00 (m, 2H), 7.03-7.12 (m, 3H), 7.18-7.32 (m, 15H); Anal. (C₄₉H₅₆FN₃O₆S•0.5 H₂O) C, H, N.

### Preparation of Product Ethyl-3-(CyPentylSCO-L-ValΨ[COCH₂]-L-(p-F)Phe-L-Gln)-E-Propenoate.

Ethyl-3-[CyPentylSCO-L-ValΨ[COCH₂]-L-(*p*-F)Phe-L-(Tr-*Gln*)]-E-propenoate (0.132 g, 0.158 mmol, 1 equiv) was dissolved in dry CH₂Cl₂ (6 mL). Triisopropylsilane (0.097 mL, 0.47 mmol, 3 equiv) and trifluoroacetic acid (3 mL) were added sequentially producing a bright yellow solution. This solution was stirred for 30 minutes and then concentrated. The residue was stirred in Et₂O (6 mL), and the solid was collected by filtration, washed with Et₂O (3 mL then 2 mL), and then dried under vacuum to give ethyl-3-(CyPentylSCO-L-ValΨ[COCH₂]-L-(*p*-F)Phe-L-*Gln*)-E-propenoate (0.077 g, 82%) as a white solid: mp = 215 °C (dec); R_{*f*}= 0.45 (10% CH₃OH in CH₂Cl₂); IR (cm⁻¹) 3413, 3296, 1715, 1649; ¹H NMR (DMSO-*d*₆) δ 0.75 (d, 3H, *J* = 6.5), 0.82 (d, 3H, *J* = 6.5), 1.21 (t, 3H, *J=* 6.9), 1.36-1.75 (m, 8H), 1.92-2.14 (m, 5H), 2.52-2.85 (m, 4H), 2.87-2.99 (m, 1H), 3.47-3.58 (m, 1H), 4.06-4.18 (m, 1H), 4.09 (q, 2H, *J* = 6.9), 4.25-4.36 (m, 1H), 5.41 (d, 1H, *J* = 15.6), 6.61 (dd, 1H, *J*= 15.6, 5.1), 6.74 (s,1H), 6.98-7.23 (m, 5H), 8.01 (d, 1H, *J =* 8.4), 8.28 (d, 1H, *J*= 8.1); Anal. (C₃₀H₄₂FN₃O₆S•0.25 H₂O) C, H, N.

### Example 15 - Preparation of Compound 15: Ethyl-3-(EtSCO-L-ValΨ[COCH₂]-L-(p-CF₃)Phe-L-Gln)-E-Propenoate.

### Preparation of Intermediate trans-6-Methyl-2S-(4-trifluoromethyl-benzyl)-hept-4-enoic Acid (2R-Hydroxy-1R-methyl-2-phenyl-ethyl)-methyl Amide.

*n*-Butyllithium (25.5 mL of a 1.6 M solution in hexanes, 40.8 mmol, 2.15 equiv) was added to a suspension of anhydrous lithium chloride (5.64 g, 133 mmol, 7 equiv) and diisopropylamine (6.13 mL, 43.7 mmol, 2.3 equiv) in THF (200 mL) at -78 °C. The reaction mixture was stirred for 30 min at -78 °C, then maintained at 0 °C for 5 min, and subsequently cooled again to -78 °C. trans-6-Methyl-hept-4-enoic acid (2*R*-hydroxy-1*R*-methyl-2-phenyl-ethyl)-methyl amide (5.5 g, 19.0 mmol, 1 equiv) in THF (40 mL) was added via cannula, and the resulting solution was stirred at -78 °C for 1.75 h, maintained at 0 °C for 20 min, stirred at 23 °C for 5 min, and then cooled again to 0 °C. A solution of 4-trifluoromethylbenzyl bromide (6.81 g, 28.5 mmol, 1.5 equiv) in THF (10 mL) was added, and the reaction mixture was stirred at 0 °C for 30 min and then partitioned between half-saturated NH₄Cl (230 mL) and a 1:1 mixture of EtOAc and hexanes (200 mL, 2 x 150 mL). The combined organic layers were dried over Na₂SO₄ and concentrated. Purification of the residue by flash column chromatography (gradient elution 20→40% EtOAc in hexanes) provided trans-6-methyl-2*S*-(4-trifluoromethyl-benzyl)-hept-4-enoic acid (2*R*-hydroxy-1*R*-methyl-2-phenyl-ethyl)-methyl amide (7.64 g, 90%) as a viscous oil: R_{*f*}= 0.47 (40% EtOAc in hexanes); IR (cm⁻¹) 3378, 1619; ¹H NMR (CDCl₃, mixture of rotamers) δ 0.85 (d, *J* = 6.5), 0.89 (d, *J*= 6.5), 0.91 (d, *J*= 6.5), 0.96 (d, *J*= 6.8), 2.07-2.34 (m), 2.36-2.47 (m), 2.59 (s), 2.76-2.86 (m), 2.88-3.01 (m), 3.07-3.22 (m), 3.96 (br), 3.99-4.09 (m), 4.37-4.52 (m), 5.19-5.57 (m), 7.19-7.40 (m), 7.47-7.57 (m)..

### Preparation of Intermediate 5S-(1R-Bromo-2-methyl-propyl)-3R-(4-trifluoromethyl-benzyl)-dihydrofuran-2-one.

*N*-Bromosuccinimide (3.17 g, 17.8 mmol, 1.05 equiv) was added in small portions over 10 min to a solution of trans-6-methyl-2*S*-(4-trifluoromethyl-benzyl)-hept-4-enoic acid (2*R*-hydroxy-1*R*-methyl-2-phenyl-ethyl)-methyl amide (7.58 g, 16.9 mmol, I equiv) and glacial acetic acid (4.85 mL, 84.7 mmol, 5 equiv) in a 4:1 mixture of THF and H₂O (180 mL) at 0 °C. The resulting yellow solution was stirred for 15 min at 0 °C, then warmed to 23 °C, and subsequently refluxed for 45 min. After cooling to 23 °C, the reaction mixture was partitioned between half-saturated NaHCO₃ (200 mL) and a 1:1 mixture of EtOAc and hexanes (2 x 200 mL, 100 mL). The combined organic layers were dried over Na₂SO₄ and concentrated. Flash chromatographic purification of the residue (gradient elution 5→9→12% EtOAc in hexanes) gave 5*S*-(1*R*-bromo-2-methyl-propyl)-3*R*-(4-trifluoromethyl-benzyl)-dihydrofuran-2-one (5.28 g, 82%) as a white solid (containing approximately 5-10% unidentified impurities by ¹H NMR): mp = 83-85 °C; R_{*f*}= 0.60 (25% EtOAc in hexanes); IR (cm⁻¹) 1778; ¹H NMR (CDCl₃, major isomer) δ 0.95 (d, 3H, *J=* 6.5), 1.01 (d, 3H, *J=* 6.8), 2.05-2.25 (m, 2H), 2.28-2.38 (m, 1H), 2.89 (dd, 1H, *J* = 13.5, 8.9), 2.98-3.09 (m, 1H), 3.22 (dd, 1H, *J* = 13.5, 4.5), 3.91 (dd, 1H, *J* = 8.9, 3.6), 4.40-4.49 (m, 1H), 7.34 (d, 2H, *J* = 8.1), 7.59 (d, 2H, *J* = 8.1); Anal. (C₁₆H₁₈BrF₃O₂) C, H.

### Preparation of Intermediate 5S-(1S-Azido-2-methyl-propyl)-3R-(4-trifluoromethyl-benzyl)-dihydrofuran-2-one.

A suspension of sodium azide (2.22 g, 34.1 mmol, 2.5 equiv) and 5*S*-(1*R*-bromo-2-methyl-propyl)-3*R*-(4-trifluoromethyl-benzyl)-dihydrofuran-2-one(5.18g, 13.7 mmol, 1 equiv) in *N,N*-dimethylformamide (50 mL) was heated at 50 °C for 66 hours. The reaction mixture was cooled to 23 °C and partitioned between half-saturated NaCl (200 mL) and a 1:1 mixture of EtOAc and hexanes (3 x 200 mL). The combined organic layers were dried over Na₂SO₄, concentrated, and the residue purified by flash column chromatography (gradient elution 9→12→20% EtOAc in hexanes) to give 5*S*-(1*S*-azido-2-methyl-propyl)-3*R*-(4-trifluoromethyl-benzyl)-dihydrofuran-2-one (2.77 g, 59%) as a viscous oil (containing approximately 5-10% unidentified impurities by ¹H NMR): R_{*f*}= 0.42 (25% EtOAc in hexanes); IR (cm⁻¹) 2097, 1772; ¹H NMR (CDCl₃, major isomer) δ 1.01 (d, 3H, *J*= 6.5), 1.02 (d, 3H, *J=* 6.8), 1.96-2.23 (m, 3H), 2.84-2.98 (m, 2H), 3.09-3.20 (m, 1H), 3.25 (dd, 1H, *J*=13.7, 4.7), 4.44-4.52 (m, 1H), 7.34 (d, 2H, *J*=8.1), 7.59 (d, 2H, *J=* 8.1); Anal. (C₁₆H₁₈F₃N₃O₂) C, H, N.

### Preparation of Intermediate {2-Methyl-1S-[4R-(4-trifluoromethyl-benzyl)-5-oxo-tetrahydrofuran-2S-yl]-propyl}-carbamic Acid tert-Butyl Ester.

A suspension of 5*S*-(1*S*-azido-2-methyl-propyl)-3*R*-(4-trifluoromethyl-benzyl)-dihydrofuran-2-one (2.71 g, 7.94 mmol, 1 equiv), di-*tert*-butyl dicarbonate (2.43 g, 11.1 mmol, 1.4 equiv) and Pd/C (10%, 0.225 g) in CH₃OH (110 mL) was stirred under a hydrogen atmosphere (balloon) for 5 hours. The reaction mixture was vacuum filtered through Whatman #5 paper and concentrated. Purification of the residue by flash column chromatography (gradient elution 12→20% EtOAc in hexanes) provided {2-methyl-1*S*-[4*R*-(4-trifluoromethyl-benzyl)-5-oxo-tetrahydrofuran-2*S*-yl]-propyl}-carbamic acid *tert*-butyl ester (1.87 g, 57%) as a white foam: R_{*f*}= 0.84 (5% MeOH in CH₂Cl₂); IR (cm⁻¹) 3331, 1766, 1708, 1690; ¹H NMR (CDCl₃) δ 0.94 (d, 3H, *J* = 6.8), 0.96 (d, 3H, *J* = 6.5), 1.40 (s, 9H), 1.71-1.86 (m, 1H), 1.95-2.06 (m, 1H), 2.20-2.31 (m, 1H), 2.86 (dd, 1H, *J* = 13.5, 8.9), 2.93-3.04 (m, 1H), 3.20 (dd, 1H, *J* = 13.5, 4.2), 3.33-3.42 (m, 1H), 4.41-4.56 (m, 2H), 7.31 (d, 2H, *J* = 8.1), 7.56 (d, 2H, *J*= 8.1); Anal. (C₂₁H₂₈F₃NO₄) C, H, N.

### Preparation of Intermediate Ethyl-3-[BOC-L-ValΨ[COCH₂]-L-(p-CF₃)Phe-L-(Tr-Gln)]-E-Propenoate.

Lithium hydroxide (8.8 mL of a 1 M aqueous solution, 8.8 mmol, 5 equiv) was added to a solution of {2-methyl-1*S*-[4*R*-(4-trifluoromethyl-benzyl)-5-oxo-tetrahydrofuran-2*S*-yl]-propyl}-carbamic acid *tert*-butyl ester (0.731 g, 1.76 mmol, 1 equiv) in DME (25 mL) at 23 °C. The resulting suspension was stirred at 23 °C for 30 min and then partitioned between 10% KHSO₄ (35 mL) and CH₂Cl₂ (2 x 100 mL, 70 mL). The combined organic layers were dried over Na₂SO₄, concentrated, and the residue dissolved in CH₂Cl₂/CH₃CN 10:1 (33 mL). Powdered 4A molecular sieves (0.65 g), 4-methylmorpholine *N*-oxide (0.412 g, 3.52 mmol, 2 equiv), and tetrapropylammonium perruthenate (0.062 g, 0.18 mmol, 0.10 equiv) were added sequentially. The resulting dark reaction mixture was stirred for 2 hours at 23 °C, and then vacuum filtered through Whatman #5 paper. The filtrate was then transferred to a flask containing crude ethyl-3-[H₂N-L-(Tr-*Gln*)]-E-propenoate•HCl (2.12 mmol, 1.2 equiv, prepared as described in Example 1 for the preparation of ethyl-3-[CBZ-L-LeuΨ[COCH₂]-D/L-Phe-L-(Tr-*Gln*)]-E-propenoate), 4-methylmorpholine (0.774 mL, 7.04 mmol, 4 equiv), 1-hydroxybenzotriazole hydrate (0.357 g, 2.64 mmol, 1.5 equiv), and 1-(3-dimethylaminopropyl)-3-ethylcarbodiimide hydrochloride (0.506 g, 2.64 mmol, 1.5 equiv) were added sequentially, and the reaction mixture was stirred for 17 hours at 23 °C. This mixture then was partitioned between brine (100 mL) and EtOAc (3 x 100 mL). The combined organic layers were dried over Na₂SO₄ and concentrated. Purification of the residue by flash column chromatography (40% EtOAc in hexanes) provided ethyl-3-[BOC-L-ValΨ[COCH₂]-L-(*p*-CF₃)Phe-L-(Tr-*Gln*)]-E-propenoate (0.839 g, 56%) as a tan foam: R_{*f*}= 0.27 (40% EtOAc in hexanes); IR (cm⁻¹) 3308, 1711, 1666; ¹H NMR (CDCl₃) δ 0.67 (d, 3H, *J=* 6.5), 0.92 (d, 3H, *J* = 6.5), 1.27 (t, 3H, *J* = 7.2), 1.39 (s, 9H), 1.58-1.71 (m, 1H), 1.90-2.06 (m, 2H), 2.35-2.50 (m, 3H), 2.65-2.84 (m, 2H), 2.89-3.07 (m, 2H), 4.03-4.22 (m, 3H), 4.38-4.45 (m, 1H), 4.86 (d, 1H, *J* = 8.1), 5.51 (d, 1H, *J* = 15.6), 6.32 (d, 1H, *J* = 8.1), 6.62 (dd, 1H, *J* = 15.6, 5.0), 7.10 (s, 1H), 7.17-7.31 (m, 17H), 7.53 (d, 2H, *J =* 7.8); Anal. (C₄₉H₅₆F₃N₃O₇•0.5 H₂O) C, H, N.

### Preparation of Intermediate Ethyl-3-[EtSCO-L-ValΨ[COCH₂]-L-(p-CF₃)Phe-L-(Tr-Gln)]-E-Propenoate.

HCl (3 mL of a 4.0 M solution in 1,4-dioxane) was added to a solution of ethyl-3-[BOC-L-ValΨ[COCH₂]-L-(*p*-CF3)Phe-L-(Tr-*Gln*)]-E-propenoate (0.271 g, 0.317 mmol) in 1,4-dioxane (3 mL). The reaction solution was stirred at 23 °C for 2 hours and then concentrated. The residue was dissolved in dry CH₂Cl₂ (5 mL). *N,N*-diisopropylethylamine (0.165 mL, 0.947 mmol, 3 equiv) and ethyl chlorothiolformate (0.056 mL, 0.54 mmol, 1.7 equiv) were added sequentially. The reaction solution was stirred 2 hours at 23 °C and then partitioned between brine (30 mL) and CH₂Cl₂ (3 x 30 mL). The combined organic phases were dried over Na₂SO₄, concentrated, and the residue was chromatographed on silica gel (40% EtOAc in hexanes) to afford ethyl-3-[EtSCO-L-ValΨ[COCH₂]-L-(*p*-CF₃)Phe-L-(Tr-*Gln*)]-E-propenoate (0.142 g, 53%) as a white foam: R_{*f*}=0.25 (40% EtOAc in hexanes); IR (cm⁻¹) 3304, 1713, 1653; ¹H NMR (CDCl₃) δ 0.70 (d, 3H, *J=* 6.8), 0.94 (d, 3H, *J=* 6.5), 1.24 (t, 3H, *J*=7.5), 1.27 (t, 3H, *J*=7.2), 1.59-1.72 (m, 1H), 1.91-2.04 (m, 2H), 2.33-2.51 (m, 3H), 2.65-3.08 (m, 6H), 4.08-4.22 (m, 2H), 4.34-4.48 (m, 2H), 5.52 (dd, 1H, *J*= 15.7, 1.7), 5.66 (d, 1H, *J* = 8.1), 6.39 (d, 1H, *J* = 7.8), 6.62 (dd, 1H, *J* = 15.7, 5.1), 7.04 (s, 1H), 7.17-7.30 (m, 17H), 7.53 (d, 2H, *J* = 7.8).

### Preparation of Product Ethyl-3-(EtSCO-L-ValΨ[COCH₂]-L-(p-CF₃)Phe-L-Gln)-E-Propenoate.

Ethyl-3-[EtSCO-L-ValΨ[COCH₂]-L-(*p*-CF₃)Phe-L-(Tr-*Gln*)]-E-propenoate (0.112 g, 0.133 mmol, 1 equiv) was dissolved in dry CH₂Cl₂ (6 mL). Triisopropylsilane (0.082 mL, 0.40 mmol, 3 equiv) and trifluoroacetic acid (3 mL) were added sequentially producing a bright yellow solution. This solution was stirred for 40 minutes and then concentrated. The residue was stirred in Et₂O (6 mL), and the solid was collected by filtration, washed with Et₂O (2 x 3 mL), and then dried under vacuum to give ethyl-3-(EtSCO-L-ValΨ[COCH₂]-L-(*p*-CF₃)Phe-L-*Gln*)-E-propenoate (0.068 g, 85%) as a white solid: mp = 216 °C (dec); R_{*f*}= 0.43 (10% CH₃OH in CH₂Cl₂); IR (cm⁻¹) 3425, 3308, 1717, 1655; ¹H NMR (DMSO-*d*₆) δ 0.76 (d, 3H, *J* = 6.5), 0.82 (d, 3H, *J* = 6.5), 1.12-1.23 (m, 6H), 1.53-1.75 (m, 2H), 1.99-2.14 (m, 3H), 2.50-2.60 (m, 1H), 2.64-2.87 (m, 5H), 2.93-3.04 (m, 1H), 3.98-4.21 (m, 3H), 4.26-4.36 (m, 1H), 5.50 (d, 1H, *J* = 15.5), 6.62 (dd, 1H, *J* = 15.5, 5.4), 6.74 (s, 1H), 7.15 (s, 1H), 7.37 (d, 2H, *J* = 8.1), 7.59 (d, 2H, *J* = 8.1), 8.06 (d, 1H, *J* = 8.1), 8.35 (d, 1H, *J* = 8.1); Anal. (C₂₈H₃₈F₃N₃O₆S) C, H, N.

### Example 16 - Preparation of Compound 16: Ethyl-3-(CyPentylSCO-L-ValΨ[COCH₂]-L-(p-CF₃)Phe-L-Gln)-E-Propenoate.

### Preparation of Intermediate Ethyl-3-[CyPentylSCO-L-ValΨ[COCH₂]-L-(p-CF₃)Phe-L-(Tr-Gln)]-E-Propenoate.

HCI (3 mL of a 4.0 M solution in 1,4-dioxane) was added to a solution of ethyl-3-[BOC-L-ValΨ[COCH₂]-L-(*p*-CF₃)Phe-L-(Tr-*Gln*)]-E-propenoate (0.268 g, 0.313 mmol) in 1,4-dioxane (3 mL). The reaction solution was stirred at 23 °C for 2 hours and then concentrated. The residue was dissolved in dry CH₂Cl₂ (5 mL). *N,N*-diisopropylethylamine (0.164 mL, 0.941 mmol, 3 equiv) and cyclopentyl chlorothiolformate (0.088 mL, 1.7 equiv) were added sequentially. The reaction solution was stirred 3 hours at 23 °C and then was partitioned between brine (30 mL) and CH₂Cl₂ (3 x 30 mL). The combined organic phases were dried over Na₂SO₄, concentrated, and the residue was chromatographed on silica gel (40% EtOAc in hexanes) to afford ethyl-3-[CyPentylSCO-L-ValΨ[COCH₂]-L-(*p*-CF₃)Phe-L-(Tr-*Gln*)]-E-propenoate (0.169 g, 61%) as a white foam: R_{*f*}= 0.25 (40% EtOAc in hexanes); IR (cm⁻¹) 3324, 1718, 1657; ¹H NMR (CDCl₃) δ 0.70 (d, 3H, *J=* 6.8), 0.93 (d, 3H, *J=* 6.5), 1.27 (t, 3H, *J* = 7.2), 1.46-1.73 (m, 7H), 1.91-2.12 (m, 4H), 2.30-2.52 (m, 3H), 2.65-2.83 (m, 2H), 2.90-3.06 (m, 2H), 3.56-3.66 (m, 1H), 4.08-4.22 (m, 2H), 4.31-4.49 (m, 2H), 5.51 (dd, 1H, *J* = 15.8, 1.6), 5.65 (d, 1H, *J* = 7.8), 6.40 (d, 1H, *J* = 7.8), 6.62 (dd, 1H, *J* = 15.8, 5.1), 7.08 (s, 1H), 7.17-7.30 (m, 17H), 7.53 (d, 2H, *J* = 8.1); Anal. (C₅₀H₅₆F₃N₃O₆S•0.5 H₂O) C, H, N.

### Preparation of Product Ethyl-3-(CyPentylSCO-L-ValΨ[COCH₂]-L-(p-CF₃)Phe-L-Gln)-E-Propenoate.

Ethyl-3-[CyPentylSCO-L-ValΨ[COCH₂]-L-(*p*-CF₃)Phe-L-(Tr-*Gln*)]-E-propenoate (0.148 g, 0.167 mmol, 1 equiv) was dissolved in dry CH₂Cl₂ (6 mL). Triisopropylsilane (0.103 mL, 0.503 mmol, 3 equiv) and trifluoroacetic acid (3 mL) were added sequentially producing a bright yellow solution. This solution was stirred for 40 minutes and then concentrated. The residue was stirred in Et₂O (6 mL), and the solid was collected by filtration, washed with Et₂O (2 x 3 mL), and then dried under vacuum to give ethyl-3-(CyPenty1SCO-L-ValΨ[COCH₂]-L-(*p*-CF₃)Phe-L-*Gln*)-E-propenoate (0.089 g, 83%) as a white solid: mp = 225 °C (dec); R_{*f*}= 0.44 (10% CH₃OH in CH₂Cl₂); IR (cm⁻¹) 3425, 3303, 1717, 1654; ¹H NMR (DMSO-*d*₆) δ 0.76 (d, 3H, *J* = 6.5), 0.82 (d, 3H, *J* = 6.8), 1.21 (t, 3H, *J*= 7.2), 1.37-1.74 (m, 8H), 1.92-2.13 (m, 5H), 2.50-2.59 (m, 1H), 2.64-2.87 (m, 3H), 2.93-3.04 (m, 1H), 3.46-3.57 (m, 1H), 4.02-4.18 (m, 3H), 4.25-4.36 (m, 1H), 5.51 (dd, 1H, *J*= 15.8, 1.2), 6.62 (dd, 1H, *J* = 15.8, 5.4), 6.74 (s, 1H), 7.16 (s, 1H), 7.37 (d, 2H, *J* = 8.1), 7.59 (d, 2H, *J*= 8.1), 8.06 (d, 1H, *J*= 8.4), 8.28 (d, 1H, *J*= 7.8); Anal. (C₃₁H₄₂F₃N₃O₆S) C, H, N.

### Example 17 - Preparation of Compound 17: [CyPentylSCO-L-ValΨ[COCH₂]-L-(p-CH₃)Phe-L-Gln]-E-1-Acetyl-3-methylene-pyrrolidin-2-one.

### Preparation of Intermediate [1-(1-Acetyl-2-oxo-pyrrolidin-3-ylidenemethyl)-3-(S)-(trityl-carbamoyl)-propyl]-carbamic Acid tert-Butyl Ester.

Triphenylphosphine (0.646 g, 2.46 mmol, 1.40 equiv) was added to a solution of 1-acetyl-3-bromo-pyrrolidin-2-one (prepared as described in: Ikuta, H., Shirota, H., Kobayashi, S., Yamagishi, Y., Yamada, K., Yamatsu, I., Katayama, K., *J. Med. Chem*. **1987**, *30*, 1995) (0.378 g, 1.76 mmol, 1.0 equiv) in THF (10 mL). The reaction mixture was refluxed for 5 h, and then the solvent was evaporated to give the crude salt. This material was dissolved in EtOH (10 mL), and Et₃N (0.613 mL, 4.4 mmol, 2.5 equiv) and [BOC-L-(Tr-Gln)]-H (0.832 g, 1.76 mmol, 1 equiv) were added sequentially. The reaction mixture was stirred at 60 °C for 24 h, then the volatiles were evaporated, and the residue was partitioned between CH₂Cl₂ (50 mL) and brine (50 mL). The organic layer was dried over Na₂SO₄ and concentrated. The residue was purified by flash chromatography on silica gel (35% EtOAc in hexanes) to afford [1-(1-acetyl-2-oxo-pyrrolidin-3-ylidenemethyl)-3-(*S*)-(trityl-carbamoyl)-propyl]-carbamic acid *tert*-butyl ester as a pale yellow foam (0.381 g, 38%): R_{*f*}=0.30 (33% EtOAc in hexanes); IR (cm⁻¹) 1687, 1510, 1366, 1274; ¹H NMR (CDCl₃) δ 1.42 (s, 9H), 1.80-1.89 (m, 2H), 2.35-2.44 (m, 2H), *2.55* (s, 3H), 2.57-2.64 (m, 1H), 2.77-2.82 (m, 1H), 3.72-3.77 (m, 2H), 4.25 (m, 1H). 4.80 (d, 1H, *J* = 8.1), 6.40-6.44 (m, 1H), 6.82 (s, br, 1H), 7.20-7.33 (m, 15H); Anal. (C₃₅H₃₉N₃O₅•1.0 H₂O) C, H, N.

### Preparation of Intermediate [BOC-L-ValΨ[COCH₂]-L-(p-CH₃)Phe-L-(Tr-Gln)]-E-1-Acetyl-3-methylene-pyrrolidin-2-one.

HCl (5 mL of a 4.0 M solution in 1,4-dioxane) was added to a solution of[1-(1-acetyl-2-oxo-pyrrolidin-3-ylidenemethyl)-3-*S*-(trityl-carbamoyl)-propyl]-carbamic acid *tert*-butyl ester (0.442 g, 0.760 mmol) in 1,4-dioxane (5 mL). The reaction solution was stirred at 23 °C for 2 hours. It then was concentrated and set aside.

Lithium hydroxide (3.46 mL of a 1 M aqueous solution, 3.46 mmol, 5 equiv) was added to a solution of {2-methyl-1*S*-[4*R*-(4-methyl-benzyl)-5-oxo-tetrahydrofuran-2*S*-yl]-propyl}-carbamic acid *tert*-butyl ester (0.250 g, 0.692 mmol, 1 equiv) in DME (10 mL) at 23°C. The resulting suspension was stirred at 23 °C for 30 min and then partitioned between 10% KHSO₄ (20 mL) and CH₂Cl₂ (3 x 70 mL). The combined organic layers were dried over Na₂SO₄, concentrated, and the residue dissolved in CH₂Cl₂ (12 mL). Powdered 4Å molecular sieves (0.25 g), 4-methylmorpholine *N*-oxide (0.162 g, 1.38 mmol, 2 equiv), and tetrapropylammonium perruthenate (0.024 g, 0.068 mmol, 0.10 equiv) were added sequentially. The resulting dark reaction mixture was stirred for 1.5 hours at 23 °C and then vacuum filtered through Whatman #5 paper. The filtrate was added directly to the crude amine salt prepared above. 4-Methylmorpholine (0.304 mL, 2.77 mmol, 4 equiv), 1-hydroxybenzotriazole hydrate (0.140 g, 1.04 mmol, 1.5 equiv), and 1-(3-dimethylaminopropyl)-3-ethylcarbodiimide hydrochloride (0.199 g, 1.04 mmol, 1.5 equiv) were added sequentially, and the reaction mixture was stirred for 19 hours at 23 °C and then loaded directly onto a flash column for chromatographic purification (50% EtOAc in hexanes) providing [BOC-L-ValΨ[COCH₂]-L-(*p*-CH₃)Phe-L-(Tr-*Gln*)]-E-1-acetyl-3-methylene-pyrrolidin-2-one (0.233 g, 40%) as a white foam: R_{*f*}= 0.25 (50% EtOAc in hexanes); IR (cm⁻¹) 3336, 1687; ¹H NMR (CDCl₃) δ 0.60 (d, 3H, *J*= 6.8), 0.91 (d, 3H, *J*= 6.8), 1.42 (s, 9H), 1.55-1.68 (m, 2H), 1.82-1.96 (m, 2H), 2.30 (s, 3H), 2.34-2.83 (m, 7H), 2.55 (s, 3H), 2.87-2.98 (m, 1H), 3.64-3.72 (m, 2H), 4.03-4.08 (m, 1H), 4.26-4.38 (m, 1H), 4.78 (d, 1H, *J*= 8.1), 5.79 (d, 1H, *J* = 8.1), 6.10-6.16 (m, 1H), 6.96 (d, 2H, *J* = 7.9), 7.06 (d, 2H, *J* = 7.9), 7.17-7.33 (m, 16H).

### Preparation of Intermediate [CyPentylSCO-L-ValΨ[COCH₂]-L-(p-CH₃)Phe-L-(Tr-Gln)]-E-1-Acetyl-3-methylene-pyrrolidin-2-one.

HCl (3 mL of a 4.0 M solution in 1,4-dioxane) was added to a solution of [BOC-L-ValΨ[COCH₂]-L-(*p*-CH₃)Phe-L-(Tr-*Gln*)]-E-1-acetyl-3-methylene-pyrrolidin-2-one (0.217 g, 0.258 mmol) in 1,4-dioxane (3 mL). The reaction solution was stirred at 23 °C for 2 hours and then concentrated. The residue was dissolved in dry CH₂Cl₂ (5 mL). *N,N*-diisopropylethylamine (0.135 mL, 0.775 mmol, 3 equiv) and cyclopentyl chlorothiolformate (0.072 mL, 1.7 equiv) were added sequentially. The reaction solution was stirred 3 hours at 23 °C and then partitioned between brine (30 mL) and CH₂Cl₂ (3 x 30 mL). The combined organic phases were dried over Na₂SO₄, concentrated, and the residue was chmmatographed on silica gel (50% EtOAc in hexanes) to afford [CyPentylSCO-L-ValΨ[COCH₂]-L-(*p*-CH₃)Phe-L-(Tr-*Gln*)]-E-1-acetyl-3-methylene-pyrrolidin-2-one (0.114 g, 51%) as a colorless glass: R_{*f*}= 0.26 (50% EtOAc in hexanes); IR (cm⁻¹) 3328, 1719, 1670; ¹H NMR (CDCl₃) δ 0.64 (d, 3H, *J*= 6.8), 0.92 (d, 3H, *J =* 6.5), 1.47-1.78 (m, 8H), 1.80-1.95 (m, 2H), 1.99-2.17 (m, 2H), 2.29-2.84 (m, 7H), 2.30 (s, 3H), 2.55 (s, 3H), 2.94 (dd, 1H, *J* = 17.4, 10.0), 3.59-3.73 (m, 3H), 4.25-4.38 (m, 2H), 5.57 (d, 1H, *J*= 8.1), 5.89 (d, 1H, *J* = 8.1), 6.11-6.17 (m, 1H), 6.96 (d, 2H, *J=* 7.9), 7.06 (d, 2H, *J*= 7.9), 7.18-7.33 (m, 16H); Anal. (C₅₂H₆₀N₄O₆S) C,H,N.

### Preparation of Product [CyPentylSCO-L-ValΨ[COCH₂]-L-(p-CH₃)Phe-L-Gln)-E-1-Acetyl-3-methylene-pyrrolidin-2-one.

[CyPentylSCO-L-ValΨ[COCH₂]-L-(*p*-CH₃)Phe-L-(Tr-*Gln*)]-E-1-acetyl-3-methylene-pyrrolidin-2-one (0.093 g, 0.107 mmol, 1 equiv) was dissolved in dry CH₂Cl₂ (6 mL). Triisopropylsilane (0.066 mL, 0.32 mmol, 3 equiv) and trifluoroacetic acid (3 mL) were added sequentially producing a bright yellow solution. This solution was stirred for 40 minutes and then concentrated. The residue was stined in Et₂O (6 mL), and the solid was collected by filtration, washed with Et₂O (2 x 3 mL), and then dried under vacuum to give [CyPentylSCO-L-ValΨ[COCH₂]-L-(*p*-CH₃)Phe-L-*Gln*]-E-1-acetyl-3-methylene-pyrrolidin-2-one (0.049 g, 73%) as a white solid: mp =200 °C (dec); R_{*f*}= 0.47 (10% CH₃OH in CH₂Cl₂); IR (cm⁻¹) 3425, 3337, 1719, 1670; ¹H NMR (DMSO-*d*₆) δ 0.74 (d, 3H, *J=* 6.5), 0.81 (d, 3H, *J=* 6.5), 1.36-1.79 (m, 8H), 1.89-2.13 (m, 5H), 2.20 (s, 3H), 2.39-2.90 (m, 7H), 2.43 (s, 3H), 3.46-3.64 (m, 3H), 4.10-4.16 (m, 1H), 4.24-4.36 (m, 1H), 6.12-6.18 (m, 1H), 6.74 (s, 1H), 6.99 (s, 4H), 7.15 (s, 1H), 8.05 (d, 1H, *J=* 7.8), 8.25 (d, 1H, *J* = 7.8); Anal. (C₃₃H₄₆N₄O₆S•0.5 H₂O) C, H, N.

### Example 18 - Preparation of Compound 18: Ethyl-3-(CyPentylSCO-L-PheΨ[COCH₂]-L-(p-F)Phe-L-Gln)-E-Propenoate.

### Preparation of Intermediate 3-(4-Fluoro-phenyl)-propionic Acid cis-1S-Amino-2R-indanol-acetonide Amide.

Oxalyl chloride (6.14 mL, 70.4 mmol, 1.05 equiv) was added to a solution of 3-(4-fluoro-phenyl)-propionic acid (11.3 g, 67.2 mmol, 1 equiv) and *N,N*-dimethylformamide (0.03 mL, 0.39 mmol, 0.006 equiv) in benzene (150 mL) at 23 °C. The reaction mixture was stirred at 23 °C for 1.5 h and then concentrated. The resulting oil was dissolved in THF (30 mL) and was added to a 0 °C solution of (1*S*,2*R*)-cis-1-amino-2-indanol (10.0 g, 67.0 mmol, 1.0 equiv) and Et₃N (10.3 mL, 73.9 mmol, 1.1 equiv) in THF (250 mL). After stirring for 20 min at 0 °C, the reaction mixture was partitioned between half-saturated NH₄Cl (150 mL) and EtOAc (2 x 150 mL). The organic layers were dried over Na₂SO₄ and concentrated to afford a white solid. This material was dissolved in a mixture of CH₂Cl₂ (400 mL) and 2-methoxypropene (30 mL), and the resulting solution was treated with methanesulfonic acid (0.20 mL). After stirring 15 min at 23 °C, the reaction mixture was partitioned between half-saturated NaHCO₃ (150 mL) and CH₂Cl₂ (2 x 150 mL). The combined organic layers were dried over MgSO₄ and gravity filtered. The filtrate was concentrated, and the residue was purified by flash column chromatography (gradient elution, 10→20% EtOAc in hexanes) to provide 3-(4-fluoro-phenyl)-propionic acid cis-1*S*-amino-2R-indanol-acetonide amide (18.2 g, 83%) as a pale yellow oil: R_{*f*}= 0.52 (50% EtOAc in hexanes); IR (cm⁻¹) 2934, 1645; ¹H NMR (CDCl₃) δ 1.34 (s, 3H), 1.60 (s, 3H), 2.91-2.95 (m, 2H), 3.04-3.13 (m, 4H), 4.68-4.71 (m, 1H), 5.06 (d, 1H, *J* = 4.7), 6.94-7.00 (m, 2H), 7.02-7.30 (m, 6H); Anal. (C₂₁H₂₂FNO₂) C, H, N.

### Preparation of Intermediate {1S-[4R-(4-Fluoro-benzyl)-5-oxo-tetrahydro-furan-2S-yl]-2-phenyl-ethyl}-carbamic Acid tert-Butyl Ester.

*n*-Butyllithium (13.5 mL of a 1.6 M solution in hexanes, 21.6 mmol, 2.0 equiv) was added to a solution of (1*R*-oxiranyl-2*S*-phenyl-ethyl)-carbamic acid *tert*-butyl ester (prepared according to Luly, J. R, Dellaria, J. F., Plattner, J. J., Soderquist, J, L., Yi, N., *J. Org. Chem.* **1987**, *52*, 1487) (2.85 g, 10.8 mmol, 1 equiv) and 3-(4-fluoro-phenyl)-propionic acid cis-1*S*-amino-2*R*-indanol-acetonide amide (3.67 g, 10.8 mmol, 1.0 equiv) in THF (150 mL) at -78°C. The reaction mixture was stirred for 5 min at -78 °C, maintained at 0 °C for 1 h, and then partitioned between 0.5 M HCl (150 mL) and a 1:1 mixture of EtOAc and hexanes (2 x 150 mL). The organic layers were dried over Na₂SO₄ and concentrated. Flash chromatographic purification of the residue (gradient elution, 25→40% EtOAc in hexanes) gave the coupling product (3.19 g, 49%) as a yellow oil contaminated with several minor impurities. This material was dissolved in a 5:1 mixture of toluene and CH₂Cl₂ (180 mL) and treated with *p*-toluenesulfonic acid monohydrate (1.01 g, 5.31 mmol, 1.0 equiv) at 23°C. After stirring for 13 h at 23 °C, the reaction mixture was filtered through a medium frit, and the filtrate was partitioned between half- saturated NaHCO₃ (150 mL) and a 1:1 mixture of EtOAc and hexanes (2 x 150 mL). The organic layers were dried over Na₂SO₄ and concentrated. The residue was purified by flash column chromatography (20% EtOAc in hexanes) to provide {1*S*-[4*R*-(4-fluoro-benzyl)-5-oxo-tetrahydro-furan-2*S*-yl]-2-phenyl-ethyl}-carbamic acid *tert*-butyl ester (1.28 g, 59%) as a white foam: R_{*f*}= 0.46 (30% EtOAc in hexanes); IR (cm⁻¹) 3332, 2976, 1767, 1702; ¹H NMR (CDCl₃) δ 1.36 (s, 9H), 1.88-1.97 (m, 1H), 2.19-2.29 (m, 1H), 2.75-2.99 (m, 4H), 3.05 (dd, 1H, *J* = 13.5, 4.5), 3.93 (q, 2H, *J* = 8.5), 4.13-4.18 (m, 1H), 4.54 (d, 1H, *J*= 9.7), 6.91-6.98 (m, 2H), 7.08-7.32 (m, 7H); Anal. (C₂₄H₂₈FNO₄) C, H, N

### Preparation of Intermediate Ethyl-3-[BOC-L-PheΨ[COCH₂]-L-(p-F)Phe-L-(Tr-Gln)]-E-Propenoate.

Lithium hydroxide (7.6 mL of a 1 M aqueous solution, 7.6 mmol, 5 equiv) was added to a solution of {1*S*-[4*R*-(4-fluoro-benzyl)-5-oxo-tetrahydro-furan-2*S*-yl]-2-phenyl-ethyl}-carbamic acid *tert*-butyl ester (0.630 g, 1.52 mmol, 1 equiv) in DME (8 mL) at 23 °C. The resulting suspension was stirred at 23 °C for 20 min and then partitioned between 0.5 M HCl (100 mL) and EtOAc (2 x 100 mL). The combined organic layers were dried over Na₂SO₄, concentrated, and the residue dissolved in a 1:1 mixture of CH₂Cl₂ and CH₃CN (100 mL). 4-Methylmorpholine *N*-oxide (0.357 g, 3.05 mmol, 2 equiv), powdered 4Å molecular sieves (0.70 g), and tetrapropylammonium perruthenate (0.054 g, 0.153 mmol, 0.1 equiv) were added sequentially. The resulting dark reaction mixture was stirred for 3 h at 23 °C and then filtered through celite. The filtrate was concentrated under reduced pressure to provide a brown oil which was dissolved in CH₂Cl₂ (40 mL). Crude ethyl-3-[H₂N-L-(Tr-*Gln*)]-E-propenoate•HCl (see preparation of ethyl-3-[CBZ-L-LeuΨ[COCH₂]-D/L-Phe-L-(Tr-*Gln*)]-E-propenoate, 1.27 mmol, 1.2 equiv), 1-hydroxybenzotriazole hydrate (0.268 g, 1.98 mmol, 1.3 equiv), 4-methylmorpholine (0.670 mL, 6.09 mmol, 4 equiv), and 1-(3-dimethylaminopropyl)-3-ethylcarbodiimide hydrochloride (0.380 g, 1.98 mmol, 1.3 equiv) were added sequentially, and the reaction mixture was stirred for 22 h at 23 °C and then partitioned between water (150 mL) and a 1:1 mixture of EtOAc and hexanes (2 x 150 mL). The combined organic layers were dried over Na₂SO₄ and concentrated. Purification of the residue by flash column chromatography (40% EtOAc in hexanes) provided ethyl-3-[BOC-L-PheΨ[COCH₂]-L-(*p*-F)Phe-L-(Tr-*Gln*)]-E-propenoate (0.558 g, 43%) as a white solid: mp = 89-100 °C; R_{*f*}=0.44 (50% EtOAc in hexanes); IR (cm⁻¹) 3316, 2972, 1708, 1665; ¹H NMR (CDCl₃) δ 1.29 (t, 3H, *J=* 7.2), 1.35 (s, 9H), 1.95-2.05 (m, 1H), 2.34-2.39 (m, 2H), 2.46 (d. 1H, *J* = 16.8), 2.57-2.99 (m, 7H), 4.17 (q, 2H, *J* = 72), 4.27-4.33 (m, 1H), 4.48 (s, br, 1H), 4.58(d, 1H, *J* = 6.9), 5.42 (d, 1H, *J* = 15.3), 6.08 (d, 1H, *J* = 8.4), 6.62 (dd, 1H, *J* = 15.3, 4.8), 6.93-7.19 (m, 6H), 7.21-7.29 (m, 19H); Anal. (C₅₂H₅₆FN₃O₇) C, H, N.

### Preparation of Intermediate Ethyl-3-[CyPentylSCO-L-PheΨ[COCH₂]-L-(p-F)Phe-L-(Tr-Gln)]-E-Propenoate.

HCl (8 mL of a 4.0 M solution in 1,4-dioxane) was added to a solution of ethyl-3-[BOC-L-PheΨ[COCH₂]-L-(*p*-F)Phe-L-(Tr-*Gln*)]-E-propenoate (0.302 g, 0.354 mmol, 1 equiv) in 1,4-dioxane (10 mL). The reaction mixture was stirred at 23 °C for 1.5 h and then concentrated. The resulting oil was dissolved in CH₂Cl₂ (15 mL), cooled to 0 °C, and 4-methylmorpholine (0.117 mL, 1.06 mmol, 3.0 equiv) and cyclopentyl chlorothiolformate (0.087 mL, 0.528 mmol, 1.5 equiv) were added sequentially. The reaction mixture was stirred for 30 min at 0 °C and then partitioned between water (100 mL) and a 1:1 mixture of EtOAc and hexanes (2 x 100 mL). The combined organic layers were dried over Na₂SO₄, concentrated, and the residue was chromatographed on silica gel (gradient elution, 30-40% EtOAc in hexanes) to afford ethyl-3-[CyPentylSCO-L-PheΨ[COCH₂]-L-(*p*-F)Phe-L-(Tr-*Gln*)]-E-propenoate (0.163 g, 52%) as a white solid: mp = 75-85 °C; R_{*f*}= 0.48 (50% EtOAc in hexanes); IR (cm⁻¹) 3314, 1710, 1655; ¹H NMR (CDCl₃) δ 1.29 (t, 3H, *J* = 7.2), 1.48-1.67 (m, 6H), 1.97-2.03 (m, 2H), 2.29-2.42 (m, 2H), 2.54-2.97 (m, 8H), 3.54-3.63 (m, 1H), 4.18 (q, 2H, *J* = 7.2), 4.51-4.58 (m, 2H), 5.44 (dd, 1H, *J* = 15.6, 1.7), 5.59 (d, 1H, *J* = 6.9), 5.90 (d, 1H, *J* = 7.2), 6.16 (d, 1H, *J* = 8.4), 6.64 (dd, 1H, *J* = 15.6, 5.0), 6.91-7.08 (m, 5H), 7.14-7.29 (m, 20H); Anal. (C₅₃H₅₆FN₃O₆S) C, H, N.

### Preparation of Product Ethyl-3-(CyPentylSCO-L-PheΨ[COCH₂]-L-(p-F)Phe-L-Gln)-E-Propenoate.

Triisopropylsilane (0.10 mL) and trifluoroacetic acid (6 mL) were added sequentially to a solution of ethyl-3-[CyPentylSCO-L-PheΨ[COCH₂]-L-(*p*-F)Phe-L-(Tr-*Gln*)]-E-propenoate (0.160 g, 0.181 mmol) in CH₂Cl₂ (10 mL) producing a bright yellow solution. The reaction mixture was stirred for 30 min at 23 °C, then carbon tetrachloride (6 mL) was added, and the mixture was concentrated under reduced pressure. The residue was purified by flash column chromatography (5% CH₃OH in CH₂Cl₂) to afford ethyl-3-(CyPentylSCO-L-PheΨ[COCH₂]-L-(*p*-F)Phe-L-*Gln*)-E-propenoate (0.082 g, 71%) as a white solid: mp = 210-212 °C; R_{*f*}=0.10 (10% CH₃OH in CH₂Cl₂); IR (cm⁻¹) 3284, 1717, 1637; ¹H NMR (DMSO-*d*₆) δ 1.21 (t, 3H, *J=* 6.8), 1.33-1.72 (m, 8H), 1.90-2.07 (m, 4H), 2.49-3.07 (m, 7H), 3.43-3.47 (m, 1H), 4.09 (q, 2H, *J=* 6.8), 4.33-4.35 (m, 2H), 5.37-5.46 (m, 1H), 6.59-6.67 (m, 1H), 6.77 (s, br, 1H), 7.00-7.28 (m, 9H), 8.04 (d, 1H, *J* = 7.8), 8.46 (d, 1H, *J* = 7.5), 8.53 (d, 1H, *J=* 7.5); Anal. (C₃₂H₄₂FN₃O₆S) C, H, N.

### Example 19 - Preparation of Compound 19: Ethyl-3-(CyPentyl-L-LeuΨ[COCH₂]-L-(p-F)Phe-L-Gln)-E-Propenoate.

### Preparation of Intermediate BOC-L-Leucinol.

To a solution of BOC-Leu-OH (15.09 g, 65 mmol, 1 equiv) in THF (150 mL) at 0 °C was added BH₃•THF (163 mL of a 1.0 M solution in THF, 163 mmol, 2.51 equiv). The reaction mixture was stirred at 23 °C for 3 h and then cooled to 0 °C. Brine (100 mL) was added carefully, and the aqueous layer was extracted with EtOAc (2 x 100 mL). The combined organic layers weredried over Na₂SO₄ and concentrated. The residue was purified by flash column chromatography (gradient elution, 25→35% EtOAc in hexanes) to afford BOC-L-Leucinol as a colorless oil (12.59 g, 89%): IR (cm⁻¹): 3434, 1699, 1256, 739 ; ¹H NMR (CDCl₃) δ 0.93 (dd, 6H, *J* = 6.6, 1.5), 1.29-1.31 (m, 2H), 1.45 (s, 9H), 1.60-2.05 (m, 2H), 3.47-3.53 (m, 1H), 3.65-3.72 (m, 2H), 4.56 (s, br, 1H); Anal. (C₁₁H₂₃NO₃) C, H, N.

### Preparation of Intermediate BOC-L-Leucinal.

A solution of sulfur trioxide pyridine complex (7.8 g, 49.1 mmol, 3.0 equiv) in a 1:1 mixture of DMSO and CH₂Cl₂ (100 mL) was added to a solution of BOC-L-Leucinol (3.56 g, 16.4 mmol. 1 equiv) and Et₃N (8 mL) in a 1:1 mixture of DMSO and CH₂Cl₂ (100 mL) at 0 °C. The reaction mixture was stirred at 23 °C for 30 min, then poured into ice water (300 mL), and extracted with Et₂O (2 x 200 mL). The combined organic layers were washed with 0.5 M HCl (150 mL), half saturated NaHCO₃ (150 mL) and H₂O (2 x 150 mL). then dried over Na₂SO₄, and concentrated to afford crude BOC-L-Leucinal as pale yellow oil (3.48 g, 99%). This material was used without further purification. ¹H NMR (CDCl₃) δ 0.92-0.94 (m, 6H), 1.44 (s, 9H), 1.59-1.64 (m, 1H), 1.67-1.85 (m, 2H), (4.21 (m, 1H), 5.12 (s, br, 1H), 9.57 (s, 1H).

### Preparation of Intermediate [1S-Isobutyl-allyl]-carbamic Acid tert-Butyl Ester.

KN(TMS)₂ (67.9 mL of a 0.5 M solution in toluene, 33.9 mmol, 2.1 equiv) was added dropwise to a solution of methyltriphenylphosphonium bromide (12.1 g, 33.9 mmol, 2.1 equiv) in a 5:1 mixture of THF and DMSO (600 mL) at 0 °C. The reaction mixture was stirred at 0 °C for 1 h, then cooled to -78 °C, and a solution of BOC-L-Leucinal (3.48 g, 16.2 mmol, 1 equiv) in THF (60 mL) was added. The reaction mixture was stirred at -78 °C for 10 min and then warmed to 23 °C slowly. The mixture was partitioned between 0.5 M HCl (200 mL) and a 1:1 mixture of EtOAc in hexanes (2 x 150 mL). The combined organic layers were washed with H₂O (150 mL), dried over Na₂SO₄, and concentrated. The residue was purified by flash column chromatography (10% EtOAc in hexanes) to afford (1*S*-isobutyl-allyl)-carbamic acid *tert*-butyl ester as a pale yellow solid (2.44 g, 70%): R_{*f*}= 0.78 (20% EtOAC in hexanes); IR (cm⁻¹): 3343, 2958, 1594, 1520; ¹H NMR (CDCl₃) δ 0.92 (dd, 6H, *J* = 6.6, 2.1), 1.30-1.38 (m, 2H), 1.45 (s, 9H), 1.61-1.74 (m, 1H), 4.13 (m, 1H), 4.38 (s, br, 1H), 5.08 (dd, 1H, *J* = 10.2, 1.5), 5.15 (dd, 1H, *J* = 17.4, 1.5), 5.73 (dd. 1H, *J* = 16.8, 10.2); Anal. (C₁₁H₂₃NO₂•H₂O) C, H, N.

### Preparation of Intermediate [3S-methyl-1R-oxiranyl-butyl]-carbamic Acid tert-Butyl Ester

3-Chloroperoxybenzoic acid (∼60%, 8.40 g, ∼29.2 mmol, 1.8 equiv) was added to a solution of(1*S*-isobutyl-allyl)-carbamic acid *tert*-butyl ester (3.48 g, 16.2 mmol, 1 equiv) in CH₂Cl₂ (100 mL). The reaction mixture was stirred at 23 °C for 6 h, then poured into a 1:1 mixture of EtOAc and hexanes (150 mL), and washed with 10% Na₂S₂O₅ (150 mL) and half-saturated NaHCO₃ (150 mL). The organic layer was dried over Na₂SO₄ and concentrated. The residue was purified by flash column chromatography (10% EtOAc in hexanes) to afford [3*S*-methyl-1*R*-oxiranyl-butyl]-carbamic acid *tert*-butyl ester as a pale yellow oil (3.15 g, 85%): R_{*f*}= 0.55 (20% EtOAc in hexanes); IR (cm⁻¹) 1702, 1501, 1366, 1168; ¹H NMR (CDCl₃) δ 0.96 (d, 6H, J= 6.9),1.43 (s, 9H), 1.64-1.81 (m, 1H), 2.60 (s, br, 1H), 2.72-2.76 (m, 2H), 2.84 (m, 1H), 2.99 (s, br, 1H), 3.60 (s, br, 1H), 3.97-3.99 (m, 1H), 4.29 (m, 1H).

### Preparation of Intermediate {1S-[4R'-(4-Fluoro-benzyl)-5-oxo-tetrahydrofuran-2S'-yl]-3-methylbutyl}-carbamic Acid tert-Butyl Ester.

*n*-Butyllithium (8.79 mL of a 1.6 M solution in hexanes, 14.06 mmol, 2.0 equiv) was added to a solution of [3*S*-methyl-1*R*-oxiranyl-butyl]-carbamic acid *tert*-butyl ester (1.61 g, 7.03 mmol, 1.0 equiv) and 3-(4-fluoro-phenyl)-propionic acid cis-1*S*-amino-2*R*-indanol-acetonide amide (2.39 g, 7.03 mmol, 1 equiv) in THF (100 mL) at -78 °C. The resulting yellow solution was stirred at -78 °C for 5 min and then warmed to 0 °C for 1 h. The reaction was quenched with 0.5 M HCl (100 mL) and extracted with a 1:1 mixture of EtOAc in hexanes (2 x 100 mL). The combined organic layers were washed with brine (150 mL), dried over Na₂SO₄, and concentrated. The residue was purified by flash chromatography on silica gel (20% EtOAc in hexanes) to afford the coupling product as a white foam (2.28 g, 57%): R_{*f*}= 0.44 (25% EtOAc in hexanes); IR (cm⁻¹) 3431, 1637, 1510, 1223; ¹H NMR (CDCl₃) δ (mixture of diastereomers) 0.92 (dd, *J* = 6.6, 2.1), 1.26-1.46 (m), 1.60 (s), 1.65 (s), 1.68-1.89 (m), 2.65-2.69 (m), 2.75-2.81 (m), 2.91-2.96 (m), 3.06-3.13 (m), 3.24 (m), 3.34-3.48 (m), 3.66 (m), 4.60 (d, *J=* 7.5), 4.68-4.71 (m), 4.76-4.78 (m), 4.83 (s, br), 5.06 (d, *J* = 4.8), 6.22 (d, *J* = 7.2), 6.91-7.31 (m).

To a solution of this material (2.21 g, 3.89 mmol, 1 equiv) in a 5:1 mixture of toluene and CH₂Cl₂ (120 mL) was added *p*-toluenesulfonic acid monohydrate (0.739 g, 3.89 mmol, 1.0 equiv) at 23 °C. The reaction mixture was stirred at 23 °C for 14 h and then quenched with half-saturated NaHCO₃ (100 mL). The resulting mixture was extracted with a 1:1 mixture of EtOAc in hexanes (2 x 100 mL), and the organic layers were dried over Na₂SO₄ and concentrated. The residue was purified by chromatography on silica gel (15% EtOAc in hexanes) to afford {1*S*-[4*R*'-(4-fluoro-benzyl)-5-oxo-tetrahydrofuran-2*S*'-yl]-3-methylbutyl}-carbamic acid *tert*-butyl ester as a white foam (0.426 g, 31%): R_{*f*}= 0.75 (25% EtOAc in hexanes); IR (cm⁻¹) 1765, 1702, 1510, 1186; ¹H NMR (CDCl₃) δ 0.89 (d, 3H, *J* = 6.6), 0.91 (d, 3H, *J* = 6.6), 1.23-1.33 (m, 2H), 1.40 (s, 9H), 1.45-1.52 (m, 1H), 1.96-2.05 (m, 1H), 2.22-2.32 (m, 1H), 2.77 (dd, 1H, *J* = 13.5, 8.7), 2.87-2.97 (m, 1H), 3.10 (dd, 1H, *J* = 13.5, 4.2), 3.74-3.82 (m, 1H),4.25(t, 1H, *J* = 6.9), 4.34 (d, 1H, *J* = 6.9), 6.95-7.01 (m, 2H), 7.11-7.16 (m, 2H); Anal. (C₂₁H₃₀FNO₄) C, H, N.

### Preparation of Intermediate Ethyl-3-[BOC-L-LeuΨ[COCH₂]-L-(p-F)-Phe-L-(Tr-Gln)]-E-Propenoate.

To a solution of {1*S*-[4*R*'-(4-fluoro-benzyl)-5-oxo-tetrahydrofuran-2*S*"-yl]-3-methylbutyl}-carbamic acid *tert*-butyl ester (0.40 g, 1.06 mmol, 1 equiv) in 1,2-dimethoxyethane (8 mL) was added LiOH (1.0 M solution in H₂O, 5.28 mL, 5.28 mmol, 5.0 equiv). The reaction mixture was stirred at 23 °C for 20 min, then quenched with 0.5 M HCl (100 mL), and extracted with a 1:1 mixture of EtOAc in hexanes (2 x 100 mL). The combined organic layers were dried over Na₂SO₄ and concentrated. The residue was dissolved in a 1:1 mixture of CH₂Cl₂ and CH₃CN (100 mL), and 4A molecular sieves (0.70 g), 4-methylmorpholine *N*-oxide (0.248 g, 2.12 mmol, 2.0 equiv), and tetrapropylammonium perruthenate (0.037 g, 0.106 mmol, 0.1 equiv) were added sequentially. The reaction mixture was stirred at 23 °C for 1 h and filtered through celite. The filtrate was concentrated to give a brown oil which was dissolved in CH₂Cl₂ (15 mL). Crude ethyl-3-[H₂N-L-(Tr-*Gln*)]-E-propenoate•HCl (see preparation of ethyl-3-[CBZ-L-LeuΨ[COCH₂]-D/L-Phe-L-(Tr-*Gln*)]-E-propenoate, 1.27 mmol, 1.2 equiv), hydroxybenzotriazole hydrate (0.186g, 1.38 mmol, 1.3 equiv), 4-methylmorpholine (0.466 mL, 4.24 mmol, 4.0 equiv), and 1-(3-dimethylaminopropyl)-3-ethylcarbodiimide hydrochloride (0.265 g, 1.38 mmol, 1.3 equiv) were added sequentially. The reaction mixture was stirred overnight, then poured into H₂O (50 mL), and extracted with CH₂Cl₂ (2 x 50 mL). The combined organic layers were dried over Na₂SO₄ and concentrated. The residue was purified by flash chromatography (40% EtOAc in hexanes) to provide ethyl-3-[BOC-L-LeuΨ[COCH₂]-L-(*p*-F)-Phe-L-(Tr-*Gln*)]-E-propenoate as a white foam: mp: 174-176 °C; R_{*f*}= 0.56 (50% EtOAc in hexanes); IR (cm⁻¹) 1706, 1662, 1509; ¹H NMR (CDCl₃) (mixture of rotamers) δ 0.87 (dd, *J* = 6.9, 2.4), 1.02-1.12 (m), 1.27 (t, *J* = 7.2), 1.39 (s), 1.96-2.02 (m), 2.37 (t, *J* = 7.2), 2.53 (d, *J* = 12.3), 2.65 (dd, *J* = 12.3, 5.7), 2.76-2.99 (m), 3.62-3.68 (m), 4.17 (q, *J*= 7.2), 4.47 (m), 4.60 (d, *J*= 7.5), 5.39 (dd, *J* = 15.9, 1.5), 5.97 (d, *J*= 8.7), 6.61 (dd, *J* = 15.3, 5.1), 6.96 (t, *J* = 8.4), 7.06-7.16 (m), 7.19-7.30 (m); Anal. (C₄₉H₅₈FN₅O₇) C, H, N.

### Preparation of Intermediate Ethyl-3-[CyPentylSCO-L-LeuΨ[COCH₂]-L-(p-F)Phe-L-(Tr-Gln)]-E-Propenoate.

Ethyl-3-[BOC-L-LeuΨ[COCH₂]-L-(*p*-F-Phe)-L-(Tr-*Gln*)]-E-propenoate (0.415 g, 0.50 mmol, 1 equiv) was dissolved in 1,4-dioxane (6 mL). A solution of HCl in 1,4-dioxane (4.0 M, 6 mL) was added dropwise. The reaction mixture was stirred for 2 h at 23 °C, and then the solvent was evaporated to provide the amine salt as a white foam. The crude amine salt was dissolved in dry CH₂Cl; (10 mL) and cooled to 0 °C. 4-Methylmorpholine (0.166 mL, 1.51 mmol, 3.0 equiv) and cyclopentyl chlorothiolformate (0.123 mL, 0.75 mmol, 1.5 equiv) were added sequentially. The reaction mixture was stirred at 0 °C for 30 min, poured into H₂O (50 mL), and extracted with a 1:1 mixture of EtOAc and hexanes (3 x 50 mL). The combined organic layers were dried over Na₂SO₄ and concentrated. The residue was purified by flash chromatography (50% EtOAc in hexanes) to provide ethyl-3-[CyPentylSCO-L-LeuΨ[COCH₂]-L-(*p*-F)Phe-L-(Tr-*Gln*)]-E-propenoate as a white foam (0.347 g, 83%): R_{*f*}= 0.43 (50% EtOAc in hexanes); IR (cm⁻¹) 1716, 1651, 1510; ¹H NMR (CDCl₃) (mixture of rotamer) δ 0.87 (d, *J* = 6.6), 1.06-1.16 (m), 1.29 (t, *J* = 7.2), 1.53-1.60 (m), 1.67-1.69 (m), 2.02-2.06 (m), 2.35 (t, *J*= 7.2), 2.54 (d, *J* = 15.3), 2.63-2.69 (m), 2.78-2.97 (m), 3.57-3.68 (m), 4.17 (q, *J* = 7.2), 4.38-4.49 (m), 4.42 (dd, *J* = 18.0, 14.1), 6.05 (d, *J* = 8.1), 6.62 (dd, *J*= 15.6, 4.8), 6.94-7.00 (m), 7.05-7.12 (m), 7.23-7.31 (m); Anal. (C₅₀H₅₈FN₃O₆S) C, H, N.

### Preparation of Product Ethyl-3-[CyPentylSCO-L-LeuΨ[COCH₂]-L-(p-F)Phe-L-Gln]-E-Propenoate.

Ethyl-3-[CyPentylSCO-L-LeuΨ[COCH₂]-L-(*p*-F)Phe-L-(Tr-*Gln*)]-E-propenoate (0.285 g) was dissolved in CH₂Cl₂ (4 mL). Trifluoroacetic acid (4 mL) and triisopropylsilane (0.077 mL) were added sequentially to give a bright yellow solution. After stirring for 30 min, no yellow color remained. The solvents were evaporated to provide a white solid which was triturated with Et₂O (8 mL) and filtered to give ethyl-3-[CyPentylSCO-L-LeuΨ[COCH₂]-L-(*p*-F)Phe-L-*Gln*]-E-propenoate as white solid (0.134 g, 65%): mp 179-180 °C; IR (cm⁻¹): 1718, 1656, 1511; ¹H NMR (DMSO-*d*₆) δ 0.83 (d, 3H, *J* = 6.0), 0.85 (d, 3H, *J* = 6.0), 1.21 (t, 3H, *J* = 7.2), 1.30-1.44 (m, 4H), 1.53-1.70 (m, 7H), 1.97-2.05 (m, 4H), 2.43-2.60 (m, 2H), 2.67-2.80 (m, 2H), 2.92-2.96 (m, 1H), 3.02-3.56 (m, 2H), 4.09 (q, 2H, *J* = 7.2), 4.17-4.19 (m, 1H), 4.31 (m, 1H), 5.42 (d, 1H, *J* = 15.3), 6.62 (dd, 1H, *J* = 15.3, 5.1), 6.75 (s, br, 1H), 7.00-7.06 (m, 2H), 7.14-7.19 (m, 2H), 8.01 (d, 1H, *J* = 8.1), 8.37 (d, 1H, *J* = 7.8); Anal. (C₃₁H₄₄FN₃O₆S) C, H, N.

### Example 20 - Preparation of Compound 20: Ethyl-3-(CyPentylOCO-L-ValΨ[COCH₂]-L-(p-CH₃)Phe-L-Gln)-E-Propenoate.

### Preparation of Intermediate Ethyl-3-[CyPentylOCO-L-ValΨ[COCH₂]-L-(p-CH₃)Phe-L-(Tr-Gln)]-E-Propenoate.

HCl (3 mL of a 4.0 M solution in 1,4-dioxane) was added to a solution of ethyl-3-[BOC-L-ValΨ[COCH₂]-L-(*p*-CH₃)Phe-L-(Tr-*Gln*)]-E-propenoate (0.311 g, 0.388 mmol) in 1,4-dioxane (3 mL). The reaction solution was stirred at 23 °C for 2 hours and then concentrated. The residue was dissolved in dry CH₂Cl₂ (6 mL). *N,N*-diisopropylethylamine (0.203 mL, 1.17 mmol, 3 equiv) and cyclopentyl chloroformate (0.098 mL, 1.7 equiv) were added sequentially. The reaction solution was stirred 3 hours at 23 °C and then partitioned between brine (15 mL) and CH₂Cl₂ (3 x 30 mL). The combined organic phases were dried over Na₂SO₄, concentrated, and the residue was chromatographed on silica gel (40% EtOAc in hexanes) to afford ethyl-3-[CyPentylOCO-L-ValΨ[COCH₂]-L-(*p*-CH₃)Phe-L-(Tr-*Gln*)]-E-propenoate (0.189 g, 60%) as a white foam: R_{*f*}= 0.22 (40% EtOAc in hexanes); IR (cm⁻¹) 3316, 1712, 1667; ¹H NMR (CDCl₃) δ 0.65 (d, 3H, *J* = 6.8), 0.92 (d, 3H, *J*= 6.8), 1.28 (t, 3H, *J* = 7.2), 1.50-1.87 (m, 10H), 1.91-2.05 (m, 2H), 2.29-2.38 (m, 1H), 2.31 (s, 3H), 2.51 (d, 1H, *J*= 16.8), 2.60-2.69 (m, 1H), 2.72-2.89 (m, 2H). 3.00 (dd, 1H, *J* = 17.3, 9.8), 4.08-4.22 (m, 3H), 4.42-4.53 (m, 1H), 4.88 (d, 1H, *J* = 8.1), 4.95-5.02 (m, 1H), 5.49 (dd, 1H, *J* = 15.8, 1.6), 5.88 (d, 1H, *J =* 8.4), 6.60 (dd, 1H, *J* = 15.8, 5.1), 7.00 (d, 2H, *J* = 7.9), 7.09 (d, 2H, *J* = 7.9), 7.17-7.31 (m, 16H); Anal. (C₅₀H₅₉N₃O₇) C, H, N.

### Example 21 - Preparation of Compound 21: Ethyl-3-(CyPentylSCO-L-LeuΨ[COCH₂]-L-(p-CH₃)Phe-L-Gln)-E-Propenoate.

### Preparation of Intermediate {1S-[4R'-(4-Methyl-benzyl)-5-oxo-tetrahydrofaran-2S'-yl]-3-methylbutyl}-carbamic Acid tert-Butyl Ester.

*n*-Butyllithium (8.43 mL of a 1.6 M solution in hexanes, 13.48 mmol, 2.0 equiv) was added to a solution of (3*S*-methyl-1*R*-oxiranyl-butyl)-carbamic acid *tert*-butyl ester (1.55 g, 6.74 mmol, 1.0 equiv) and 3-*p*-tolyl-propionic acid cis-1*S*-amino-2*R*-indanol-acetonide amide (2.39 g, 7.03 mmol, 1 equiv) in THF (100 mL) at -78 °C. The resulting yellow solution was stirred at -78 °C for 5 min and then warmed to 0 °C for 1 h. The reaction was quenched with 0.5 M HCl (100 mL) and extracted with a 1:1 mixture of EtOAc and hexanes (2 x 100 mL). The combined organic layers were washed with brine (150 mL), dried over Na₂SO₄, and concentrated. The residue was purified by chromatography on silica gel (20% EtOAc in hexanes) to afford the coupling product as a white foam (2.07 g, 54%): R_{*f*}= 0.30 (25% EtOAc in hexanes); IR (cm⁻¹) 3415, 2955, 1687, 1612, 1355, 1166; ¹H NMR (CDCl₃) (mixture of diastereomers) δ 0.91 (d, *J* = 6.3), 0.92 (d, *J* = 6.9), 1.34 (s), 1.40 (s), 1.59 (s), 1.65-1.73 (m), 1.82-1.84 (m), 2.31 (s), 2.36 (s), 2.76 (dd, *J* = 12.9, 6.0), 3.06 (s), 3.20 (m), 3.34-3.47 (m), 3.68 (m), 4.58 (d, *J*= 9.3), 4.83 (s, br), 5.66 (m), 6.26 (d, *J*= 7.5), 6.85-6.90 (m), 7.09-7.24 (m).

This material was dissolved in a 5:1 mixture of toluene and CH₂Cl₂ (120 mL) and was treated with *p*-toluenesulfonic acid monohydrate (0.697 g, 3.67 mmol, 1.0 equiv). The reaction mixture was stirred at 23 °C for 14 h and then filtered through a medium frit The clear filtrate was poured into half-saturated NaHCO₃ (100 mL) and extracted with a 1:1 mixture of EtOAc in hexanes (2 x 100 mL). The organic layers were dried over Na₂SO₄ and concentrated. The residue was purified by flash chromatography on silica gel (10% EtOAc in hexanes) to afford {1*S*-[4*R*'-(4-methyl-benzyl)-5-oxo-tetrahydrofuran-2*S*"-yl]-3-methylbutyl}-carbamic acid *tert*-butyl ester as a white foam (0.572 g, 32%): IR (cm⁻¹) 1765, 1707, 1167; ¹H NMR (CDCl₃) δ 0.89 (d, 3H, *J=* 6.3), 0.90 (d, 3H, *J=* 6.6) 1.19-1.33 (m, 2H), 1.42 (s, 9H), 1.43-1.54 (m, 1H), 1.96-2.06 (m, 1H), 2.18-227 (m, 1H), 2.32 (s, 3H), 2.75 (dd, 1H, *J* = 13.5, 9.0), 2.88-2.98 (m, 1H), 3.10 (dd, 1H, *J* = 13.5, 4.2), 3.72-3.80 (m, 1H), 4.24 (t, 1H, *J* = 6.3), 4.34 (d, 1H, *J* = 9.9), 7.04-7.12 (m, 4H); Anal. (C₂₂H₃₃NO₄) C, H, N.

### Preparation of Intermediate Ethyl-3-[BOC-L-LeuΨ[COCH₂]-L-(p-CH₃)Phe-L-(Tr-Gln)-E-Propenoate.

This material was prepared from {1*S*-[4*R*'-(4-methyl-benzyl)-5-oxo-tetrahydrofuran-2*S*'-yl]-3-methylbutyl}-carbamic acid *tert*-butyl ester (0.572 g, 1.52 mmol) as described previously for the formation of ethyl-3-[BOC-L-LeuΨ[COCH₂]-L-(*p*-F)-Phe-L-(Tr-*Gln*)]-E-propenoate (Example 19) to give ethyl-3-[BOC-L-LeuΨ[COCH₂]-L-(*p*-CH₃)Phe-L-(Tr-*Gln*)-E-propenoate as a white foam (0.785 g, 63%): R_{*f*}= 0.70 (50% EtOAc in hexanes); IR (cm⁻¹) 1709, 1664, 1170; ¹H NMR (CDCl₃) (mixture of rotamer) δ 0.85 (d, *J* = 6.3), 0.98-1.08 (m), 1.28 (t, *J* = 72), 1.39 (s), 1.52-1.62 (m), 1.97-2.05 (m), 2.31 (s), 2.36 (t, *J* = 6.9), 2.52 (d, *J* = 16.5), 2.64-2.67 (m), 2.83-1.97 (m), 4.17 (q, *J*= 72), 4.48 (m), 4.58 (d, *J*= 7.2), 5.35 (dd, *J*= 15.9, 1.5), 5.87 (d, J= 8.4), 6.61 (dd, *J* = 15.9, 5.1), 7.02-7.11 (m), 7.20-7.30 (m); Anal. (C₅₀H₆₁N₃O₇•0.1H₂O) C, H, N.

### Preparation of Intermediate Ethyl-3-[CyPentylSCO-L-LeuΨ[COCH₂]-L-(p-CH₃)Phe-L-(Tr-Gln)-E-Propenoate.

Ethyl-3-[BOC-L-LeuΨ[COCH₂]-L-(*p*-CH₃)Phe-L-(Tr-*Gln*)-E-propenoate (0.523 g, 0.64 mmol) was deprotected and coupled with cyclopentyl chlorothiolformate (0.158 mL, 0.96 mmol) as described previously for the formation of ethyl-3-[CyPentylSCO-L-LeuΨ[COCH₂]-L-(*p*-F)Phe-L-(Tr-*Gln*)-E-propenoate (Example 19) to give ethyl-3-[CyPentylSCO-L-LeuΨ[COCH₂]-L-(*p*-CH₃)Phe-L-(Tr-*Gln*)-E-propenoate as a white foam (0.301 g, 56%): IR (cm⁻¹) 1716, 1651, 1518; ¹H NMR (CDCl₃) (mixture of rotamers) δ 0.85 (d, *J* = 6.3), 1.02-1.12 (m,), 1.29 (t, *J=* 7.2), 1.52-1.62 (m), 1.67-1.68 (m), 1.98-2.10 (m), 2.31-2.36 (m), 2.53 (d, *J* = 15.0), 2.63-2.68 (m), 2.76-2.95 (m), 3.57-3.66 (m), 4.16 (q, *J*= 7.2), 4.38 (m); 4.48-4.52 (m), 5.35 (d, *J* = 7.5), 5.53 (dd, *J* = 15.9, 1.5), 5.94 (d, *J*= 8.2), 6.62 (dd, *J* = 15.9, 4.8), 6.99-7.12 (m), 7.21-7.31 (m); Anal. (C₅₁H₆₁N₃O₆S•0.25H₂O) C, H, N.

### Preparation of Product Ethyl-3-[CyPentylSCO-L-LeuΨ[COCH₂]-L-(p-CH₃)Phe-L-Gln]-E-Propenoate.

Ethyl-3-[CyPentylSCO-L-LeuΨ[COCH₂]-L-(*p*-CH₃)Phe-L-(Tr-*Gln*)-E-propenoate (0.272 g, 0.32 mmol) was deprotected using the procedure described for the formation of ethyl-3-[CyPentylSCO-L-LeuΨ[COCH₂]-L-(*p*-F)Phe-L-*Gln*]-E-propenoate (Example 19) to give ethyl-3-[CyPentylSCO-L-LeuΨ[COCH₂]-L-(*p*-CH₃)Phe-L-*Gln*]-E-propenoate as white solid (0.106 g, 55%): mp 173-174 °C; IR (cm⁻¹) 1719, 1655, 1522, 1198; ¹H NMR (DMSO-*d*₆) δ 0.83 (d, 3H, *J=* 6.0), 0.85 (d, 3H, *J* = 6.0), 1.21 (t, 3H, *J* = 7.2), 1.30-1.44 (m, 4H), 1.53-1.70 (m, 7H), 1.97-2.05 (m, 4H), 2.43-2.60 (m, 2H), 2.67-2.80 (m, 2H), 2.92-2.96 (m, 1H), 3.02-3.56 (m, 2H), 4.09 (q, 2H, *J* = 7.2), 4.17-4.19 (m, 1H), 4.31 (m, 1H), 5.42 (d, 1H, *J* = 15.3), 6.62 (dd, 1H, *J* = 15.3, 5.1), 6.75 (s, br, 1H), 7.00-7.06 (m, 2H), 7.14-7.19 (m, 2H), 8.01 (d, 1H, *J* = 8.1), 8.37 (d, 1H, *J* = 7.8); Anal. (C₃₁H₄₄FN₃O₆S) C, H, N.

### Example 22 - Preparation of Compound 22: Ethyl-3-(CyPentylSCO-L-tert-LeuΨ[COCH₂]-L-Phe-L-Gln)-E-Propenoate.

### Preparation of Intermediate CBZ-L-tert-Leucine.

To a solution of L-*tert*-leucine (5.12 g, 39 mmol, 1 equiv) in 1,4-dioxane (80 mL) and 1 M NaOH (39 mL, 39 mmol, 1.0 equiv) at 0 °C was added benzyl chlorofomate (6.24 mL, 43.68 mmol, 1.12 equiv). The reaction mixture was stirred at 23 °C overnight, then concentrated, poured into 1 M HCl (80 mL), and extracted with CH₂Cl₂ (2 x 100 mL). The organic layers were dried over Na₂SO₄ and concentrated to give crude CBZ-L-*tert*-leucine (20.7 g) as a clear oil: IR (cm⁻¹) 3336, 1715, 1521, 1232; ¹H NMR (CDCl₃) δ 1.02 (s, 9H), 4.21 (d, 1H, *J* = 9.9), 5.11 (s, 2H), 5.35 (d, 1H, J= 9.0), 7.34-7.37 (m, 5H).

### Preparation of Intermediate 4-Benzyloxycarbonylamino-5,5-dimethyl-3-oxo-hexanoic Acid tert-Butyl Ester.

To a solution of CBZ-L-*tert*-leucine (20.53 g, 77.4 mmol, 1 equiv) in THF (150 mL) was added 1,1'-carbonyldiimidazole (13.81 g, 85.14 mmol, 1.1 equiv) at 23 °C. The resulting solution was stirred at 23 °C for 1 h. In a separate flask, *n*-butyllithium (101.59 ml of a 1.6 M solution in hexanes, 162.54 mmol, 2.1 equiv) was added to a solution of diisopropylamine (22.78 mL, 162.54 mmol, 2.1 equiv) in THE (100 mL) at -78 °C. The reaction mixture was stirred for 15 min at -78 °C, wanned to 0 °C for 5 min, then cooled back to -78 °C. A solution of *tert*-butyl acetate (21.9 mL, 162.54 mmol, 2.1 equiv) in THE (10 mL) was added via cannula, and the resulting mixture was stirred at -78 °C for 10 min. The above imidazole solution was then added dropwise to the lithium enolate at -78 °C. The resulting mixture was stirred at -78 °C for 1 h, quenched with 1 M HCl (100 mL), and extracted with EtOAc (2 x 100 mL). The combined organic layers were washed with brine (150 mL), dried over Na₂SO₄, and concentrated. The residue was purified by flash chromatography on silica gel (10% EtOAc in hexanes) to afford 4-benzyloxycarbonylamino-5,5-dimethyl-3-oxo-hexanoic acid *tert*-butyl ester as a pale yellow oil (12.06 g, 44%): IR (cm⁻¹) 1717, 1508, 1265, 739; ¹H NMR (CDCl₃) δ 1.09 (s, 9H), 1.44 (s, 9H), 3.50 (s, 2H), 4.29 (d, 1H, *J*= 9.3), 5.03 (s, 2H), 5.35-5.42 (m, 1H). 7.34 (s, 5H).

### Preparation of Intermediate CBZ-L-tert-LeuΨ[COCH₂]-L-Phe-OMe.

To a stirred solution of (*R*)-2-hydroxy-3-phenyl-propionic acid methyl ester (2.53 g, 14.06 mmol, 3.1 equiv) in CH₂Cl₂ (30 mL) at 0 °C was added trifluoromethanesulfonic anhydride (2.50 mL, 14.8 mmol, 3.3 equiv) and 2,6-lutidine (1.72 mL, 14.8 mmol, 3.3 equiv) slowly. The resulting pink solution was stirred at 0 °C for 30 min, then poured into 0.5 M HCl (100 mL), and extracted with a 1:1 mixture of EtOAc in hexanes (2 x 100 mL). The combined organic layers were dried over Na₂SO₄, concentrated, and utilized in the next step below.

A solution of 4-benzyloxycarbonylamino-5,5-dimethyl-3-oxo-hexanoic acid *tert*-butyl ester (1.64 g, 4.51 mmol, 1 equiv) in THF (100 mL) was added dropwise to a stirred suspension of NaH (0.190 g of a 60% dispersion in mineral oil, 4.74 mmol, 1.05 equiv) in THF (100 mL) at 0 °C. After stirring for 10 min, a solution of crude (*R*)-2-triflyoxy-3-phenyl-propionic acid methyl ester (prepared above) in CH₂Cl₂ (10 mL) was added dropwise. The resulting mixture was stirred at 23 °C for 24 h, then quenched with 1 M HCI (50 mL), and extracted with EtOAc (3 x 50 mL). The combined organic layers were washed with brine (100 mL), dried over Na₂SO₄, and concentrated to provide a pale yellow oil. Without further purification, the above oil was dissolved in CH₂Cl₂ (10 mL), treated with trifluoroacetic acid (2 mL), and then maintained at 23 °C for 24 h. After dilution with CH₂Cl₂ (50 mL), the resulting solution was washed with saturated NaHCO₃ (50 mL) and brine (50 mL). The organic layer was dried over Na₂SO₄ and concentrated. The residue was purified by flash chromatography on silica gel (10% EtOAc in hexanes) to afford CBZ-L-*tert*-LeuΨ[COCH₂]-L-Phe-OMe as a pale yellow oil (1.01 g, 54%): R_{*f*}= 0.41 (25% EtOAc in hexanes); IR (cm⁻¹) 1711, 1514, 1233; ¹H NMR (CDCl₃) δ 0.97 (s, 9H), 2.58-2.76 (m, 2H), 2.96-3.17 (m, 3H), 3.62 (s, 3H), 4.17 (d, 1H, *J* = 8.1), 5.06-5.10 (s, 2H), 5.32 (d, 1H, *J* = 8.6), 7.12-7.36 (m, 10 H); Anal. (C₂₅H₃₁NO₅•0.25 H₂O) C, H, N. BOC-L-*tert*-LeuΨ[COCH₂]-L-Phe-OMe.

10% Pd on C (0.110 g) was added to a solution of CBZ-L-*tert*-LeuΨ[COCH₂]-L-Phe-OMe (0.513 g, 1.33 mmol, 1 equiv) and di-*tert*-butyl dicarbonate (0.378 g, 1.73 mmol, 1.3 equiv) in CH₃OH at 23 °C. The reaction mixture was stirred at 23 °C under an H₂ atmosphere (balloon) overnight. The mixture was filtered through celite, and the filtrate was concentrated. The residue was purified by flash chromatography on silica gel (10% EtOAc in hexanes) to afford BOC-L-*tert*-LeuΨ[COCH₂]-L-Phe-OMe as white solid (0.366 g, 70%): mp = 98-99 °C; R_{*f*}= 0.54 (25% EtOAc in hexanes); IR (cm⁻¹) 1707, 1497, 1367, 1236, 1168; ¹H NMR (CDCl₃) δ 0.97 (s, 9H), 1.40 (s, 9H), 2.60-2.78 (m, 2H), 2.95-3.19 (m, 3H), 3.63 (s, 3H), 4.07-4.11 (m, 2H), 5.07 (d, 1H, *J* = 9.3), 7.13-7.32 (m 5H); Anal. (C₂₅H₃₁NO₅) C, H, N.

### Preparation of Intermediate CBZ-L-tert-LeuΨ[COCH₂]-L-Phe-OH.

2 M NaOH (3.35 mL, 6.7 mmol, 8.0 equiv) was added to a solution of BOC-L-*tert*-LeuΨ[COCH₂]-L-Phe-OMe (0.328 g, 0.84 mmol, 1 equiv) in CH₃OH (6 mL) at 0 °C over 10 min. The reaction mixture was stirred at 0 °C for 2 h, then poured into 10% KHSO₄ (80 mL), and extracted with CH₂Cl₂ (2 x 100 mL). The organic layers were dried over Na₂SO₄ and concentrated to give BOC-L-*tert*-LeuΨ[COCH₂]-L-Phe-OH as a white solid (0.315 g, 99%) which was used without further purification: IR (cm⁻¹) 2960, 1710, 1498, 1368, 1167; ¹H NMR (CDCl₃) δ 0.94 (s, 9H), 1.39 (s, 9H), 2.60-2.80 (m, 2H), 2.95-3.16 (m, 3H), 4.08 (d, 2H, *J*= 9.3), 5.09 (d, 1H. *J* = 9.6), 7.12-7.31 (m 5H).

### Preparation of Intermediate Ethyl-3-[BOC-L-tert-LeuΨ[COCH₂]-L-Phe-L-(Tr-Gln)]-E-Propenoate.

Ethyl-3-[BOC-L-(Tr-*Gln*)]-E-propenoate (0.523 g, 0.64 mmol) was deprotected and coupled with BOC-L-*tert*-LeuΨ[COCH₂]-L-Phe-OH (0.315 g, 0.84 mmol) as described for the formation of ethyl-3-[BOC-L-LeuΨ[COCH₂]-L-(*p*-F)Phe-L-(Tr-*Gln*)]-E-propenoate (Example 19) to give ethyl-3-[BOC-L-*tert*-LeuΨ[COCH₂]-L-Phe-L-(Tr-*Gln*)]-E-propenoate as a white foam (0.474 g, 70%): R_{*f*}= 0.58 (50% EtOAc in hexanes); IR (cm⁻¹) 1702, 1669, 1494, 1169; ¹H NMR (CDCl₃) (mixture of rotamers) δ 0.85 (s), 1.30 (t, *J* = 7.2), 1.41 (s), 1.56-1.65 (m), 1.95-2.02 (m), 2.22-2.42 (m), 2.62-2.88 (m), 3.09-3.18 (m), 4.00 (d, *J* = 8.7), 4.17 (t, *J* = 7.2), 4.46-4.51 (m), 4.93 (d, *J* = 8.7), 5.37 (d, *J* = 15.9), 5.69 (d, *J* = 93), 6.54 (dd, *J* = 15.9, 4.8), 7.19-7.15 (m), 7.18-7.31 (m); Anal. (C₄₉H₅₉N₃O₇) C, H, N.

### Preparation of Intermediate Ethyl-3-[CyPentylSCO-L-tert-LeuΨ[COCH₂]-L-Phe-L-(Tr-Gln)]-E-Propenoate.

Ethyl-3-[BOC-L-*tert*-LeuΨ[COCH₂]-L-Phe-L-(Tr-*Gln*)]-E-propenoate (0.441 g, 0.55 mmol) was deprotected and coupled with cyclopentyl chlorothiolformate (0.135 mL, 0.82 mmol) as described previously for the formation of ethyl-3-[CyPentylSCO-L-LeuΨ[COCH₂]-L-(*p*-F)Phe-L-(Tr-*Gln*)]-E-propenoate (Example 19) to give ethyl-3-[CyPentylSCO-L-*tert*-LeuΨ[COCH₂]-L-Phe-L-(Tr-*Gln*)]-E-propenoate as a white foam (0.347 g, 76%): IR (cm⁻¹) 1718, 1656, 1493, 1186; ¹H NMR (CDCl₃) (mixture of rotamers) δ 0.86 (s), 1.27 (t, *J* = 7.2), 1.56-1.68 (m), 1.95-2.12 (m), 2.22-2.39 (m), 2.60-2.74 (m), 2.84-2.90 (m), 3.05-3.14 (m), 3.59-3.64 (m), 4.16 (q, *J*= 7.2), 4.31 (d, *J* = 8.4), 4.48 (m), 5.41 (dd, *J* = 15.9, 1.8), 5.67 (d, *J* = 8.7), 5.82 (d, *J* = 9.0), 6.56 (dd, *J* = 15.6, 5.1), 7.19-7.31 (m); Anal. (C₅₀H₅₉N₃O₆) C, H, N.

### Preparation of Product Ethyl-3-(CyPentylSCO-L-tert-LeuΨ[COCH₂]-L-Phe-L-Gln)-E-Propenoate.

Ethyl-3-[CyPentylSCO-L-*tert*-LeuΨ[COCH₂]-L-Phe-L-(Tr-*Gln*)]-E-propenoate (0.318 g, 0.38 mmol) was deprotected using the procedure described for the formation of ethyl-3-(CyPentylSCO-L-LeuΨ[COCH₂]-L-(*p*-F)Phe-L-*Gln*)-E-propenoate (Example 19) to give ethyl-3-[CyPentylSCO-L-*tert*-LeuΨ[COCH₂]-L-Phe-L-*Gln*]-E-propenoate as white solid (0.204 g, 91%): mp 65-68 °C; IR (cm⁻¹) 1715, 1652, 1520, 1193; ¹H NMR (CDCl₃) (mixture of rotamers) δ 0.96 (s), 1.31 (t, *J*= 7.2), 1.50-1.73(m), 1.96-2.13 (m), 2.23 (t, *J* = 7.5). 2.68-2.79 (m), 2.84-2.95 (m), 3.11-3.21 (m), 3.59-3.69 (m), 4.18 (q, *J*= 7.2), 4.36 (d, *J* = 8.1), 4.52-4.59 (m), 5.37 (s, br), 5.42 (dd, 1H, *J* = 15.9, 1.5), 5.80 (d. *J*= 9.0), 5.90 (d, J= 8.4), 6.46 (s, br), 6.61 (dd, 1H, *J* = 15.9, 5.1), 7.18-7.30 (m); Anal. (C₃₁H₄₅N₃O₆S) C, H, N.

### Preparation of Product Ethyl-3-(CyPentylSCO-L-ValΨ[COCH₂]-L-(p-CH₃)Phe-L-Gln-E-Propenoate.

Ethyl-3-[CyPentylOCO-L-ValΨ[COCH₂]-L-(*p*-CH₃)Phe-L-(Tr-Gln)]-E-propenoate (0.160 g, 0.197 mmol, 1 equiv) was dissolved in dry CH₂Cl₂ (8 mL). Triisopropylsilane (0.121 mL, 0.591 mmol, 3 equiv) and trifluoroacetic acid (4 mL) were added sequentially producing a bright yellow solution. This solution was stirred for 40 minutes and then concentrated. The residue was stirred in Et₂O (8 mL), and the solid was collected by filtration, washed with Et₂O (2 x 4 mL), and then dried under vacuum to give ethyl-3-(CyPentylOCO-L-ValΨ[COCH₂]-L-(*p*-CH₃)Phe-L-*Gln*)-E-propenoate (0.094 g, 84%) as a white solid: mp = 206-207 °C (dec); R_{*f*}= 0.38 (10% CH₃OH in CH₂Cl₂); IR (cm⁻¹) 3413, 3307, 3213, 1708, 1660; ¹H NMR (DMSO-*d*₆) δ 0.72 (d, 3H, *J* = 6.8), 0.81 (d, 3H, *J* = 6.5), 1.20 (t, 3H, *J* = 7.0), 1.46-1.82 (m, 10H), 1.97-2.10 (m, 3H), 2.23 (s, 3H), 2.38-2.54 (m, 1H), 2.65-2.84 (m, 2H), 2.86-2.97 (m, 1H), 3.81-3.88 (m, 1H), 4.03-4.18 (m, 2H), 4.26-4.38 (m, 1H), 4.85-4.94 (m, 1H), 5.55 (d, 1H, *J* = 15.9), 6.65 (dd, 1H, *J* = 15.9, 5.3), 6.73 (s, 1H), 7.03 (s, 4H), 7.14 (s, 1H), 7.27 (d, 1H, *J* = 8.1), 8.02 (d, 1H, *J* = 8.4); Anal. (C₃₁H₄₅N₃O₇) C, H, N.

### Example 23 - Preparation of Compound 23: Ethyl-3-(CyPentylCH₂CO-L-ValΨ[COCH₂]-L-(p-CH₃)Phe-L-Gln)-E-Propenoate.

### Preparation of Intermediate Ethyl-3-[CyPentylCH₂CO-L-ValΨ[COCH₂]-L-(p-CH₃)Phe-L-(Tr-Gln)]-E-Propenoate.

HCl (3 mL of a 4.0 M solution in 1,4-dioxane) was added to a solution of ethyl-3-[BOC-L-ValΨ[COCH₂]-L-(*p*-CH₃)Phe-L-(Tr-*Gln*)]-E-propenoate (0.315 g, 0.393 mmol, 1 equiv) in 1,4-dioxane (3 mL). The reaction solution was stirred at 23 °C for 1.67 hours and then was concentrated to provide crude ethyl-3-[H₂N-L-ValΨ[COCH₂]-L-(*p*-CH₃)Phe-L-(Tr-*Gln*)]-E-propenoate•HCl which was set aside.

Dicyclohexylcarbodiimide (0.162 g, 0.785 mmol, 2 equiv) was added to a solution of cyclopentyl acetic acid (0.197 mL, 1.57 mmol, 4 equiv) in Et₂O (10 mL). The reaction mixture was stirred for 1.5 hours, and then the white precipitate was removed by filtration. The filtrate was concentrated, then dissolved in dry CH₂Cl₂ (5 mL), and added to a solution of crude ethyl-3-[H₂N-L-ValΨ[COCH₂]-L-(*p*-CH₃)Phe-L-(Tr-*Gln*)]-E-propenoate•HCl (from above) and 4-methylmorpholine (0.086 mL, 0.782 mmol, 2 equiv) in dry CH₂Cl₂ (3 mL). The reaction mixture was stirred for 2.5 hours and concentrated. The residue was chromatographed on silica gel (gradient elution 40-50% EtOAc in hexanes) to afford ethyl-3-[CyPentylCH₂CO-L-ValΨ[COCH₂]-L-(*p*-CH₃)Phe-L-(Tr-*Gln*)]-E-propenoate (0.202 g, 63%) as a white foam: R_{*f*}= 0.65 (10% CH₃OH in CHCl₃); IR (cm⁻¹) 3296, 1716, 1650; ¹H NMR (CDCl₃) δ 0.71 (d, 3H, *J* = 6.8), 0.92 (d, 3H, J= 6.8), 1.02-1.16 (m, 2H), 1.29 (t, 3H, *J* = 7.2), 1.47-1.82 (m, 8H), 1.89-2.16 (m, 5H), 2.30-2.36 (m, 1H), 2.31 (s, 3H), 2.46-2.69 (m, 2H), 2.74-2.89 (m, 2H), 2.95-3.08 (m, 1H), 4.12-4.22 (m, 2H), 4.41-4.53 (m, 2H). 5.52 (dd, 1H, *J* = 15.6, 1.6), 5.73 (d, 1H, *J* = 8.1), 6.00 (d, 1H, *J* = 8.1), 6.61 (dd, 1H, *J* = 15.6, 5.1), 6.99 (d, 2H, *J* = 7.9), 7.08 (d, 2H, *J*= 7.9), 7.17-730 (m, 16H); Anal. (C₅₁H₆₁N₃O₆•0.5 H₂O) C, H, N.

### Preparation of Product Ethyl-3-(CyPentylCH₂CO-L-ValΨ[COCH₂]-L-(p-CH₃)Phe-L-Gln)-E-Propenoate.

Ethyl-3-[CyPentylCH₂CO-L-ValΨ[COCH₂]-L-(*p*-CH₃)Phe-L-(Tr-*Gln*)]-E-propenoate (0.167 g, 0.206 mmol, 1 equiv) was dissolved in dry CH₂Cl₂ (8 mL). Triisopropylsilane (0.126 mL, 0.615 mmol, 3 equiv) and trifluoroacetic acid (4 mL) were added sequentially producing a bright yellow solution. This solution was stirred for 40 minutes and then concentrated. The residue was stirred in Et₂O (8 mL), and the solid was collected by filtration, washed with Et₂O (2 x 4 mL), and then dried under vacuum to give ethyl-3-(CyPentylCH₂CO-L-ValΨ[COCH₂]-L-(*p*-CH₃)Phe-L-*Gln*)-E-propenoate (0.092 g, 79%) as a white solid: mp = 253-255 °C (dec); R_{*f*}=0.42 (10% CH₃OH in CHCl₃); IR (cm⁻¹) 3401, 3284, 1713, 1649; ¹H NMR (DMSO-*d*₆) δ 0.74 (d, 3H, *J=* 6.8), 0.81 (d, 3H, *J=* 6.5), 1.03-1.19 (m, 2H), 1.20 (t, 3H, *J* = 7.0), 1.40-1.75 (m, 9H), 2.00-2.15 (m, 6H), 2.23 (s, 3H), 2.42 (dd, 1H, *J*=18.4, 4.7), 2.66-2.84 (m, 2H), 2.86-2.96 (m, 1H), 4.06-4.17 (m, 3H), 4.27-4.37 (m, 1H), 5.57 (dd, 1H, *J*=15.7, 1.4), 6.66 (dd, 1H, *J*=15.7, 5.4), 6.73 (s, 1H), 7.03 (s, 4H), 7.16 (s, 1H), 7.92 (d, 1H, *J*= 8.1), 8.01 (d, 1H, *J*= 7.8); Anal. (C₃₂H₄₇N₃O₆) C, H, N.

### Example 24 - Preparation of Compound 24: Ethyl-3-(CyPentylSCO-L-CyhexΨ[COCH₂]-L-(p-F)Phe-L-Gln)-E-Propenoae.

### Preparation of Intermediate trans-5-Cyclohexyl-pent-4-enoic Acid.

A solution of cyclohexane carboxaldehyde (11.22 g, 100 mmol, 1 equiv) in THF (100 mL) was added dropwise via addition funnel to a solution of vinylmagnesium bromide (100 mL of a 1.0 M solution in THF, 100 mmol, 1.0 equiv) in Et₂O (100 mL) at 0 °C. After the addition was completed, the reaction mixture was stirred for 1 h at 0 °C and then partitioned between 0.5 M HCl (150 mL) and a 1:1 mixture of EtOAc and hexanes (2 x 150 mL). The combined organic layers were dried over Na₂SO₄ and concentrated to afford a yellow oil. This material was combined (neat) with diethyl malonate (16.7 mL, 110 mmol, 1.1 equiv) and Ti(OEt)₄ (2.10 mL, 10.0 mmol, 0.10 equiv) and was heated to 160 °C for 1 h (distilling out EtOH as it was formed). The reaction mixture was then maintained at 190 °C for 4 h and then cooled to 60 °C. EtOH (50 mL) and 6.0 M KOH (50 mL) were added sequentially, and the brown reaction mixture was refluxed for 3 h. After cooling to 23 °C, the reaction mixture was filtered through a medium frit, and the filtrate was partitioned between water (150 mL) and Et₂O (2 x 100 mL). The aqueous layer was then acidified to pH = 2 (as indicated by pH paper) with concentrated HCl and extracted with a 1:1 mixture of EtOAc and hexanes (2 x 150 mL). The combined organic layers were dried over Na₂SO₄, concentrated, and the residue was distilled at reduced pressure to afford trans-5-cyclohexyl-pent-4-enoic acid (5.68 g, 31%) as a colorless liquid: bp: 150-156 °C (1 torr); IR (cm⁻¹) 3001 (br), 2923, 1711; ¹H NMR (CDCl₃) δ 0.96-1.32 (m, 5H), 1.60-1.76 (m, 5H), 1.85-1.94 (m, 1H), 2.27-2.44 (m, 4H), 5.31-5.48 (m, 2H); Anal. (C₁₁H₁₈O₂) C, H.

### Preparation of Intermediate trans-5-Cyclohexyl-pent-4-enoic Acid (2R-Hydroxy-1R-methyl-2-phenyl-ethyl)-methyl Amide.

Oxalyl chloride (2.81 mL, 32.2 mmol, 1.05 equiv) was added to a solution of trans-5-cyclohexyl-pent-4-enoic acid (5.60 g, 30.7 mmol, 1 equiv) and *N,N*-dimethylformamide (0.03 mL, 0.39 mmol, 0.013 equiv) in benzene (100 mL) at 23 °C. The reaction mixture was stirred at 23°C for 2 h and then concentrated under reduced pressure. The resulting oil was dissolved in THF (20 mL) and added via cannula to a solution of (1*R*,2*R*)-(-)-pseudoephedrine (4.61 g, 27.9 mmol, 0.91 equiv) and triethylamine (5.06 mL, 36.3 mmol, 1.1 equiv) in THF (300 mL) at 0 °C. The reaction mixture was stirred at 0 °C for 30 min and then partitioned between half-saturated NH₄Cl (150 mL) and a 1:1 mixture of EtOAc and hexanes (2 x 150 mL). The combined organic layers were dried over Na₂SO₄, concentrated, and the residue purified by flash column chromatography (50% EtOAc in hexanes) to afford trans-5-cyclohexyl-pent-4-enoic acid (2*R*-hydroxy-1*R*-methyl-2-phenyl-ethyl)-methyl amide (5.21 g, 57%) as a white solid: mp = 89-91 °C; R_{*f*}= 0.33 (50% EtOAc in hexanes); IR (cm⁻¹) 3380, 1621; ¹H NMR (CDCl₃, mixture of rotamers) δ 0.97-1.32 (m), 1.54-1.74 (m), 1.86-1.93 (m), 2.24-2.58 (m), 2.81 (s), 2.91 (s), 3.98-4.06 (m), 4.35-4.48 (m), 4.55-4.61 (m), 5.32-5.47 (m), 7.24-7.41 (m); Anal. (C₂₁H₃₁NO₂) C, H, N.

### Preparation of Intermediate trans-5-Cyclohexyl-2S-(4-fluoro-benzyl)-pent-4-enoic Acid (2R-Hydroxy-1R-methyl-2-phenyl-ethyl-methyl Amide.

*n*-Butyllithium (20.7 mL of a 1.6 M solution in hexanes, 33.1 mmol, 2.1 equiv) was added to a suspension of anhydrous lithium chloride (4.68 g, 110 mmol, 7 equiv) and diisopropylamine (4.98 mL, 35.5 mmol, 2.25 equiv) in THF (300 mL) at -78°C. The reaction mixture was stirred for 20 min at -78 °C, then maintained at 0 °C for 5 min, and subsequently cooled again to -78 °C. A solution of trans-5-cyclohexyl-pent-4-enoic acid (2R-hydroxy-1*R*-methyl-2-phenyl-ethyl)-methyl amide (5.20 g, 15.8 mmol, 1 equiv) in THF (30 mL) was added via cannula, and the resulting solution was stirred at -78 °C for 1 h, maintained at 0°C for 15 min, stirred at 23 °C for 5 min and then cooled again to 0 °C. 4-Fluorobenzyl bromide (2.95 mL, 23.7 mmol, 1.5 equiv) was added, and the reaction mixture was stirred at 0 °C for 30 min and then partitioned between half-saturated NH₄Cl (150 mL) and a 1:1 mixture of EtOAc and hexanes (2 x 150 mL). The combined organic layers were dried over Na₂SO₄ and concentrated. Purification of the residue by flash column chromatography (gradient elution 30→40% EtOAc in hexanes) provided trans-5-cyclohexyl-2*S*-(4-fluoro-benzyl)-pent-4-enoic acid (2*R*-hydroxy-1*R*-methyl-2-phenyl-ethyl)-methyl amide (6.29 g, 91%) as a viscous oil: R_{*f*}= 0.57 (50% EtOAc in hexanes); IR (cm⁻¹) 3382, 1616; ¹H NMR (CDCl₃, mixture of rotamers) δ 0.83-1.27 (m), 1.51-1.70 (m), 1.86-1.95 (m), 2.12-2.21 (m), 2.28-2.44 (m), 2.58 (s), 2.64-2.77 (m), 2.82 (s), 2.85-2.92 (m), 4.00-4.05 (m), 4.37-4.52 (m), 5.22-5.52 (m), 6.88-7.01 (m), 7.07-7.20 (m), 7.20-7.38 (m); Anal. (C28H36FNO₂•0.25H₂O) C, H, N.

### Preparation of Intermediate 5S-(R-Bromo-cyclohexyl-methyl)-3R-(4-fluoro-benzyl)-dihydrofuran-2-one.

*N*-Bromosuccinimide (2.65 g, 14.9 mmol, 1.1 equiv) was added in small portions over 5 min to a solution of trans-5-cyclohexyl-2*S*-(4-fluoro-benzyl)-pent-4-enoic acid (2*R*-hydroxy-1*R*-methyl-2-phenyl-ethyl)-methyl amide (6.20 g, 14.2 mmol, 1 equiv) and glacial acetic acid (3.87 mL, 70.8 mmol, 5 equiv) in a 4:1 mixture of THF and H₂O (250 mL) at 0 °C. The resulting yellow solution was stirred for 15 min at 0 °C, then warmed to 23 °C, and subsequently refluxed for 1 h. After cooling to 23 °C, the reaction mixture was partitioned between half-saturated NaHCO₃ (150 mL) and a 1:1 mixture of EtOAc and hexanes (2 x 150 mL). The combined organic layers were dried over Na₂SO₄ and conoentrated. Flash chromatographic purification of the residue (5% EtOAc in hexanes) gave 5*S*-(*R*-bromo-cyclohexyl-methyl)-3R-(4-fluoro-benzyl)-dihydrofuran-2-one (3.70 g, 71%) as a white solid: mp = 72-75 °C; R_{*f*}= 0.62 (30% EtOAc in hexanes); IR (cm⁻¹) 1774; ¹H NMR (CDCl₃) δ 1.11-1.38 (m, 6H), 1.52-1.77 (m, 5H), 2.13-2.34 (m, 2H), 2.82 (dd, 1H, *J* = 13.7, 8.4), 2.95-3.05 (m, 1H), 3.12 (dd, 1H, *J* = 13.7, 4.7), 3.89 (dd, 1H, *J* = 8.4, 3.7), 4.43-4.51 (m, 1H), 6.98-7.05 (m, 2H), 7.15-7.26 (m, 2H); Anal. (C₁₈H₂₂BrFO₂) C, H, N.

### Preparation of Intermediate 5S-(S-Azido-cyclohexyl-methyl)-3R-(4-fluoro-benzyl)-dihydro-furan-2-one.

A suspension of sodium azide (1.30 g, 20.0 mmol, 2 equiv) and 5*S*-(*R*-bromo-cyclohexyl-methyl)-3*R*-(4-fluoro-benzyl)-dihydrofuran-2-one (3.70 g, 10.0 mmol, 1 equiv) in *N,N*-dimethylformamide (30 mL) was heated at 50 °C for 18 h. The reaction mixture was cooled to 23 °C and then partitioned between water (150 mL) and a 1:1 mixture of EtOAc and hexanes (2 x 150 mL). The combined organic layers were dried over Na₂SO₄, concentrated, and the residue purified by flash column chromatography (10% EtOAc in hexanes) to give 5*S*-(*S*-azido-cyclohexyl-methyl)-3*R*-(4-fluoro-benzyl)-dihydro-furan-2-one (1.76 g, 53%) as a colorless oil: R_{*f*}= 0.33 (20% EtOAc in hexanes); IR (cm⁻¹) 2109, 1772; ¹H NMR (CDCl₃) δ 1.06-1.29 (m, 6H), 1.67-1.82 (m, 5H), 2.02-2.21 (m, 2H), 2.79-2.86 (m, 1H), 2.92-2.95 (m, 1H), 3.05-3.17 (m, 2H), 4.45-4.50 (m, 1H). 6.97-7.04 (m, 2H), 7.15-7.26 (m, 2H).

### Preparation of Intermediate {S-Cyclohexyl-[4R-(4-fluoro-benzyl)-5-oxo-tetrahydro-furan-2S-yl]-methyl}-carbamic Acid tert-Butyl Ester.

A suspension of 5*S*-(*S*-azido-cyclohexyl-methyl)-3*R*-(4-fluoro-benzyl)-dihydro-furan-2-one (1.76 g, 5.31 mmol, 1 equiv) and Pd/C (10%, 0.15 g) in CH₃OH (30 mL) was stirred under a hydrogen atmosphere (balloon) for 3 h. The reaction mixture was filtered through celite, concentrated, and the residue dissolved in 1,4-dioxane (30 mL). *N,N*-diisopropylethylamine (1.85 mL, 10.6 mmol, 2 equiv) and di-*tert*-butyl dicarbonate (1.74 g, 7.97 mmol, 1.5 equiv) were added sequentially, and the resulting solution was stirred at 23 °C for 1.5 h. The reaction mixture was then partitioned between water (150 mL) and a 1:1 mixture of EtOAc and hexanes (2 x 150 mL). The combined organic layers were dried over Na₂SO₄ and concentrated. Purification of the residue by flash column chromatography (15% EtOAc in hexanes) provided {*S*-cyclohexyl-[4*R*-(4-fluoro-benzyl)-5-oxo-tetrahydro-furan-2*S*-yl]-methyl}-carbamic acid *tert*-butyl ester (1.11 g, 52%) as a white foam: R_{*f*}= 0.55 (30% EtOAc in hexanes); IR (cm⁻¹) 3338, 1766, 1699; ¹H NMR (CDCl₃) δ 0.92-1.26 (m, 5H), 1.40 (s, 9H), 1.62-1.80 (m, 6H), 1.95-2.05 (m, 1H), 2.17-2.27 (m, 1H), 2.79 (dd, 1H, *J* = 13.5, 8.6), 2.88-2.98 (m, 1H), 3.09 (dd, 1H, *J* = 13.5, 4.5), 3.37-3.43 (m, 1H), 4.43 (d, 1H, *J* = 10.0), 4.48-4.52 (m, 1H), 6.96-7.01 (m, 2H), 7.12-7.27 (m, 2H); Anal. (C₂₁H₃₁NO₄) C, H, N.

### Preparation of Intermediate Ethyl-3-[BOC-L-CyhexΨ[COCH₂]-L-(p-F)Phe-L-(Tr-Gln)]-E-Propenoate.

Lithium hydroxide (7.0 mL of a 1 M aqueous solution, 7.0 mmol, 5-equiv) was added to a solution of {*S*-cyclohexyl-[4*R*-(4-fluoro-benzyl)-5-oxo-tetrahydro-furan-2*S*-yl]-methyl}-carbamic acid *tert*-butyl ester (0.567 g, 1.40 mmol, 1 equiv) in DME (10 mL) at 23 °C. The resulting suspension was stirred at 23 °C for 30 min and then partitioned between 0.5 M HCl (100 mL) and EtOAc (2 x 100 mL). The combined organic layers were dried over Na₂SO₄, concentrated, and the residue dissolved in CH₂Cl₂ (30 mL). 4-Methylmorpholine *N*-oxide (0.328 g, 2.80 mmol, 2 equiv), powdered 4Å molecular sieves (0.60 g), and tetrapropylammonium perruthenate (0.049 g, 0.139 mmol, 0.1 equiv) were added sequentially. The resulting dark reaction mixture was stirred for 2 h at 23 °C and then filtered through celite. The filtrate was concentrated under reduced pressure to provide a brown oil which was dissolved in CH₂Cl₂ (40 mL). Crude ethyl-3-[H₂N-L-(Tr-*Gln*)]-E-propenoate•HCl (see preparation of ethyl-3-[CBZ-L-LeuΨ[COCH₂]-D/L-Phe-L-(Tr-*Gln*)]-E-propenoate, 1.68 mmol, 1.2 equiv), 1-hydroxybenzotriazole hydrate (0.284 g, 2.10 mmol, 1.5 equiv), 4-methylmorpholine (0.616 mL, 5.60 mmol, 4 equiv), and 1-(3-dimethylaminopropyl)-3-ethylcarbodiimide hydrochloride (0.403 g, 2.10 mmol, 1.5 equiv) were added sequentially, and the reaction mixture was stirred for 20 h at 23 °C and then partitioned between water (150 mL) and a 1:1 mixture of EtOAc and hexanes (2 x 150 mL). The combined organic layers were dried over Na₂SO₄ and concentrated. Purification of the residue by flash column chromatography (40% EtOAc in hexanes) provided ethyl-3-[BOC-L-CyhexΨ[COCH₂]-L-(*p*-F)Phe-L-(Tr-*Gln*)]-E-propenoate (0.379 g, 32%) as a white solid: mp = 192-195 °C; R_{*f*}=0.50 (50% EtOAc in hexanes); IR (cm⁻¹) 3316, 1709, 1667; ¹H NMR (CDCl₃) δ 0.98-1.12 (m, 5H). 1.29 (t, 3H, *J* = 7.2), 1.40 (s, 9H), 1.43-1.74 (m, 6H), 1.96-2.02 (m, 1H), 2.30-2.39 (m, 2H), 2.53 (d, 1H, *J* = 17.1), 2.61-2.88 (m, 4H), 3.00 (dd, 1H, *J* = 17.6, 10.1), 4.01-4.06 (m, 1H), 4.17 (q, 2H, J= 7.2), 4.45 (s, br, 1H), 4.80 (d, 1H, *J* = 8.1), 5.35 (d, 1H, *J* = 15.7), 5.90 (d, 1H, *J* = 8.4), 6.60(dd, 1H, *J* = 15.7, 5.0), 6.93-7.10 (m,4H), 7.17-7.30 (m, 16H); Anal. (C₅₁H₆₀FN₃O₇) C, H, N.

### preparation of Intermediate Ethyl-3-[CyPentylSCO-L-CyhexΨ[COCH₂]-L-(p-F)Phe-L- (Tr-Gln)]-E-Propenoate.

HCl (10 mL of a 4.0 M solution in 1,4-dioxane) was added to a solution of ethyl-3-[BOC-L-LeuΨ[COCH₂]-L-Phe-L-(Tr-*Gln*)]-E-propenoate (0.379 g, 0.448 mmol, 1 equiv) in 1,4-dioxane (10 mL). The reaction mixture was stirred at 23 °C for 2 h and then concentrated. The resulting oil was dissolved in CH₂Cl₂ (15 mL), cooled to 0 °C, and 4-methylmorpholine (0.123 mL, 1.12 mmol, 2.5 equiv) and cyclopentyl chlorothiolformate (0.096 mL, 0.583 mmol, 1.3 equiv) were added sequentially. The reaction mixture was stirred for 30 min at 0 °C and then partitioned between water (100 mL) and a 1:1 mixture of EtOAc and hexanes (2 x 100 mL). The combined organic layers were dried over Na₂SO₄, concentrated, and the residue was chromatographed on silica gel (30% EtOAc in hexanes) to afford ethyl-3-[CyPentyISCO-L-CyhexΨ[COCH₂]-L-(*p*-F)Phe-L-(Tr-*Gln*)]-E-propenoate (0.161 g, 41%) as a white solid: mp = 90-95 °C; R_{*f*}= 0.51 (50% EtOAc in hexanes); IR (cm⁻¹) 3312, 1714, 1654; ¹H NMR (CDCl₃) δ 0.98-1.22 (m, 6H), 1.29 (t, 3H, *J* = 7.2), 1.56-1.70 (m, 11 H), 1.98-2.08 (m, 3H), 2.29-2.37 (in, 2H), 2.51-3.30 (m, 6H), 3.58-3.65 (m, 1H), 4.17 (q, 2H, *J* = 7.2), 4.33 (s, br, 1H), 4.45 (s, br, 1H), 5.37 (dd, 1H, *J* = 15.7, 1.6), 5.55 (d, 1H, *J* = 8.1), 5.99 (d, 1H, *J*= 8.4), 6.61 (dd, 1H, *J* = 15.7, 4.7), 6.93-7.10 (m, 4H), 7.16-7.31 (m, 16H); Anal. (C₅₂H₆₀FN₃O₆S) C, H, N.

### Preparation of Product Ethyl-3-(CyPentylSCO-L-CyhexΨ[COCH₂]-L-(p-F)Phe-L-Gln)-E-Propenoate.

Triisopropylsilane (0.10 mL) and trifluoroacetic acid (6 mL) were added sequentially to a solution of ethyl-3-[CyPentylSCO-L-CyhexΨ[COCH₂]-L-(*p*-F)Phe-L-(Tr-*Gln*)]-E-propenoate (0.150 g, 0.172 mmol) in CH₂Cl₂ (8 mL) producing a bright yellow solution. The reaction mixture was stirred for 20 min at 23 °C, then carbon tetrachloride (4 mL) was added, and the mixture was concentrated under reduced pressure. The residue was purified by flash column chromatography (3% CH₃OH in CH₂Cl₂) to afford ethyl-3-(CyPentylSCO-L-CyhexΨ[COCH₂]-L-(*p*-F)Phe-L-*Gln*)-E-propenoate (0.069 g, 63%) as a white foam: R_{f}= 0.56 (10% CH₃OH in CH₂Cl₂); IR (cm⁻¹) 3281, 1716, 1637; ¹H NMR (DMSO-*d*₆) δ 0.93-1.14 (m, 6H), 1.21 (t, 3H, *J* = 7.2), 1.41-1.70 (m, 13H), 1.96-2.03 (m, 4H), 2.53-2.93 (m, 5H), 3.46-3.56 (m, 1H), 3.97-4.15 (m, 3H), 4.30 (s, br, 1H), 5.41 (d, 1H, *J* = 15.7), 6.61 (dd 1H, *J* = 15.7, 5.0), 6.74 (s, 1H), 7.00-7.19 (m, 5H), 8.00 (d, 1H, *J* = 8.4), 8.28 (d, 1H, *J* = 7.8); Anal. (C₃₃H₄₆FN₃O₆S) C, H. N.

### Example 25 - Preparation of Compound 25: Ethyl-3-(CyPentylOCO-L-ValΨ[COCH₂]-L-(p-F)Phe-L-Gln)-E-Propenoate.

### Preparation of Intermediate Ethyl-3-[CyPentylOCO-L-ValΨ[COCH₂]-L-(p-F)Phe-L-(Tr-Gln)]-E-Propenoate.

HCl (3 mL of a 4.0 M solution in 1,4-dioxane) was added to a solution of ethyl-3-[BOC-L-ValΨ[COCH₂]-L-(*p*-F)Phe-L-(Tr-*Gln*)]-E-propenoate (0.220 g, 0.273 mmol, 1 equiv) in 1,4-dioxane (3 mL). The reaction solution was stirred at 23 °C for 2.25 hours and then concentrated to provide crude ethyl-3-[H₂N-L-ValΨ[COCH₂]-L-(*p*-F)Phe-L-(Tr*-Gln*)]-E-propenoate•HCl which was set aside.

Cyclopentanol (0.431 mL, 4.75 mmol, 17.4 equiv) was dissolved in dry CH₂Cl₂ (25 mL). Triethylamine (0.662 mL, 4.75 mmol, 17.4 equiv) and triphosgene (0.507 g, 1.71 mmol, 6.26 equiv) were added sequentially. The reaction solution was stirred 2.5 hours to provide a stock solution of cyclopentyl chloroformate (0.19 M). A portion of this solution (2.87 mL, 0.545 mmol, 2 equiv) was added to a solution of crude ethyl-3-[H₂N-L-ValΨ[COCH₂]-L-(*p*-F)Phe-L-(Tr-*Gln*)]-E-propenoate•HCl (from above) and 4-methylmorpholine (0.120 mL, 1.09 mmol, 4 equiv) in dry CH₂Cl₂ (4 mL). The reaction mixture was stirred for 1.75 hours, then poured into water (30 mL), and extracted with CH₂Cl₂ (3 x 30 mL). The combined organic phases were dried over Na₂SO₄, concentrated, and the residue was chromatographed on silica gel (gradient elution 40→50% EtOAc in hexanes) to afford ethyl-3-[CyPentylOCO-L-ValΨ[COCH₂]-L-(*p*-F)Phe-L-(Tr-*Gln*)]-E-propenoate (0.077 g, 35%) as a colorless glass: ¹H NMR (CDCl₃) δ 0.68 (d, 3H, *J=* 6.8), 0.93 (d, 3H, *J* = 6.8), 1.29 (t, 3H, *J* = 7.2), 1.50-1.86 (m, 10H), 1.93-2.04 (m, 2H), 2.29-2.38 (m, 2H), 2.52 (d, 1H, *J* = 15.9). 2.64 (dd, 1H, *J* = 12.1, 5.6), 2.73-2.90 (m, 2H), 2.99 (dd, 1H, *J* = 17.0, 9.8), 4.10-4.22 (m, 3H), 4.40-4.51 (m, 1H), 4.92 (d, 1H, *J* = 8.1), 4.95-5.03 (m, 1H), 5.38 (d, 1H, *J* = 15.9), 6.06 (d, 1H, *J* = 8.4), 6.60 (dd, 1H, *J* = 15.9, 4.8), 6.92-7.00 (m, 2H), 7.04-7.11 (m, 2H), 7.16-7.32 (m, 15H).

### Preparation of Product Ethyl-3-(CyPentylOCO-L-ValΨ[COCH₂]-L-(p-F)Phe-L-Gln)-E-Propenoate.

Ethyl-3-[CyPentylOCO-L-ValΨ[COCH₂]-L-(*p*-F)Phe-L-(Tr-*Gln*)]-E-propenoate (0.076 g, 0.093 mmol, 1 equiv) was dissolved in dry CH₂Cl₂ (6 mL). Triisopropylsilane (0.057 mL, 0.278 mmol, 3 equiv) and trifluoroacetic acid (3 mL) were added sequentially producing a bright yellow solution. This solution was stirred for 40 minutes and then concentrated. The residue was stirred in Et₂O (6 mL), and the solid was collected by filtration, washed with Et₂O (2 x 3 mL), and then dried under vacuum to give ethyl-3-(CyPentylOCO-L-ValΨ[COCH₂]-L-(*p*-F)Phe-L-*Gln*)-E-propenoate (0.040 g, 75%) as a white solid: mp = 220-222 °C (dec); R_{*f*}= 0.16 (5% CH₃OH in CH₂Cl₂); IR (cm⁻¹) 3413, 3317, 1708, 1658; ¹H NMR (DMSO-*d*₆) δ 0.74 (d, 3H, *J=* 6.5), 0.82 (d, 3H, *J* = 6.5), 1.20 (t, 3H, *J=* 7.2), 1.38-1.84 (m, 10H), 1.96-2.12 (m, 3H), 2.46-2.84 (m, 4H), 2.88-2.97 (m, 1H), 3.82-3.89 (m, 1H), 4.09 (q, 2H, *J* = 7.2), 4.25-4.36 (m, 1H), 4.84-4.94 (m, 1H), 5.40 (d, 1H, *J* = 15.6), 6.61 (dd, 1H, *J* = 15.6, 5.3), 6.73 (s, 1H), 6.98-7.24 (m, 5H), 7.30 (d, 1H, *J* = 8.7), 7.99 (d, 1H, *J* = 8.4); Anal. (C₃₀H₄₂FN₃O₇•0.5 H₂O) C, H, N.

Compounds **26** through **34**, illustrated above, can be produced by the skilled artisan, using routine experimentation, in a manner analogous to the various procedures described above for producing compounds **1** through **25**.

### BIOCHEMICAL AND BIOLOGICAL EVALUATION

### Inhibition of Rhinovirus Protease

Stock solutions (50 mM, in DMSO) of various compounds were prepared; dilutions were in the same solvent. Recombinant Rhinovirus 3C proteases from serotypes 14, 16, and 2 were prepared by the following standard chromatographic procedures: (1) ion exchange using Q Sepharose Fast Flow from Pharmacia; (2) affinity chromatography using Affi-Gel Blue from Biorad; and (3) sizing using Sephadex G-100 from Pharmacia. Assays contained 2% DMSO, 50 mM tris pH 7.6, 1 mM EDTA, a compound at the indicated concentrations, approximately 1µM substrate, and 50-100 nM protease. For Kᵢ determinations, the compound and the enzyme were preincubated for 10 minutes at 30 °C prior to addition of the substrate (substrate start). The k_{obs/1} values were obtained from reactions initiated by addition of enzyme rather than substrate. RVP activity is measured in the fluorescence resonance energy transfer assay. The substrate was (N-terminal) DABCYL-(Gly-Arg-Ala-Val-Phe-Gln-Gly-Pro-Val-Gly)-EDANS. In the uncleaved peptide, the EDANS fluorescence was quenched by the proximal DABCYL moiety. When the peptide was cleaved, the quenching was relieved, and activity was measured as an increase in fluorescence signal. Data was analyzed using standard non-linear fitting programs (Enzfit), and are shown in Table 1.

**TABLE 1**

| COMPOUND | RVP | INHIB | k_{obs/I}(M⁻¹sec⁻¹) |
|---|---|---|---|
| **1** | | ND | 17,380 |
| | (2) | ND | 2,242 |
| | (16) | ND | 3,880 |
| **2** | | ND | 47,000 |
| | (2) | ND | 4,600 |
| | (16) | ND | 10,410 |
| **3** | | >7µM(Kᵢ) | 29,200 |
| **4** | | ND | 180,000 |
| | (2) | ND | 17,800 |
| | (16) | ND | 34,600 |
| **5** | | ND | 500,000 |
| | (2) | ND | 26,900 |
| | (16) | ND | 89,700 |
| **6** | | ND | 87,600 |
| | (2) | ND | 13,350 |
| | (16) | ND | 23,230 |
| **7** | | ND | 255,000 |
| | (2) | ND | 25,000 |
| | (16) | ND | 100,000 |
| **8** | | ND | 55,700 |
| | (2) | ND | 7,000 |
| | (16) | ND | 14,200 |
| **1+9** (∼1:1) | | ND | 5,100 |
| **10** | | ND | 440,000 |
| **11** | | ND | 850,000 |
| **12** | | ND | 404,000 |
| **13** | | 1.6 | 196,000 |
| **14** | | 1.7 | 293,000 |
| **15** | | ND | 127,000 |
| **16** | | 3.4 | 150,000 |
| 17 | | ND | 845,000 |
| **18** | | 0.78 | 127,400 |
| **19** | | ND | 67,200 |
| **20** | | ND | 52,140 |
| **21** | | ND | 243,000 |
| **22** | | ND | 124,000 |
| **23** | | ND | 36,500 |
| **24** | | 0.67 | 240,000 |
| **25** | | ND | 16,000 |
| **26** | | ND | 34,800 |
| **27** | | 0.6 | 167,000 |
| **28** | | 0.46 | 450,000 |
| **29** | | ND | ND |
| **30** | | ND | 292,000 |
| **31** | | ND | 85,300 |
| **32** | | ND | 58,000 |
| **33** | | ND | 281,000 |
| **34** | | 0.65 | 240,000 |

In the above table, all data are for RVP serotype- 14 unless otherwise noted in parentheses. All strains of human rhinovirus (HRV) were purchased from American Type Culture Collection (ATCC) except for serotype 14, which was produced from the infectious cDNA clone constructed and supplied to Applicants by Dr. Robert Rueckert at the Institute for Molecular Virology, University of Wisconsin, Madison, Wisconsin. The column designated INHIB represents the percent inhibition at 10 minute preincubation with 50 nM RVP prior to addition of substrate. The data in the column designated k_{obt/I} was measured from progress curves in enzyme start experiments. The designation ND indicates that a value was not determined for that compound.

### Antirhinoviral HI-HeLa Cell Culture Assay

In the Cell Protection Assay, the ability of compounds to protect cells against HRV infection was measured by the XTT dye reduction method. This method is described in Weislow, O. S., Kiser, R., Fine, D. L., Bader, J., Shoemaker, R. H., Boyd, M. R., *J. Natl. Cancer Inst*. **1989**, *81*, 577-586.

HI-HeLa cells were infected with HRV-14 at a multiplicity of infection (m.o.i.) of 0.13 (virus particles/cell) or mock-infected with medium only. Infected or mock-infected cells were resuspended at 8 x 10⁵ cells per mL and incubated with appropriate concentrations of compounds of formula I. Two days later, XTT/PMS was added to test plates, and the amount of formazan produced was quantified spectrophotometrically at 450/650 nm. The EC₅₀ was calculated as the concentration of compound that increased the percentage of formazan production in compound-treated, virus infected cells to 50% of that produced by compound-free mock-infected cells. The 50% cytotoxic dose (CC₅₀) was calculated as the concentration of compound that decreased the percentage of formazan produced in compound-treated, mock-infected cells to 50% of that produced by compound-free mock-infected cells. The therapeutic index (TI) was calculated by dividing the CC₅₀ by the EC₅₀.

All strains of human rhinovirus (HRV) for use in this assay were purchased from American Type Culture Collection (ATCC) except for HRV serotype-14, which was produced from the infectious cDNA clone constructed and supplied to Applicants by Dr. Robert Rueckert at the Institute for Molecular Virology, University of Wisconsin, Madison, Wisconsin. HRV stocks were propagated, and viral assays were performed in HI-HeLa cells (ATCC). Cells were grown in Minimal Essential Medium, available from Life Technologies, with 10% fetal bovine serum.

The compounds were tested against control compounds WIN 51711, WIN 52084, and WIN 54954, all obtained from Sterling-Winthrop Pharmaceuticals, and control compound Pirodavir, obtained from Janssen Pharmaceuticals. Antiviral data obtained for the test compounds are shown in Table 2 where all data are for HRV serotype-14 unless otherwise noted in parentheses.

**TABLE 2**

| # | HRV | EC₅₀ (µM) | CC₅₀ (µM) | TI |
|---|---|---|---|---|
| **1** | | 0.36 | >320 | >889 |
| **2** | | 0.24 | >320 | >1333 |
| | (2) | 1.8 | >320 | >178 |
| **3** | | 1.9 | 50.1 | 26 |
| **4** | | 0.10 | >320 | >3200 |
| | (2) | 0.50 | >320 | >640 |
| **5** | | 0.19 | >320 | >1730 |
| **6** | | 0.68 | >100 | >147 |
| **7** | | 0.022 | >10 | >454 |
| | (2) | 0.10 | >10 | >100 |
| | (10) | 0.035 | >10 | >286 |
| | (89) | 0.004 | >10 | >2500 |
| | (39) | 0.13 | >10 | >75 |
| **8** | | 0.19 | >100 | >526 |
| **1+9 (∼1:1)** | | 1.3 | >320 | >246 |
| | (16) | 2.8 | >320 | >114 |
| | (2) | 2.0 | >320 | >160 |
| | (10) | 4.1 | >320 | >78 |
| | (89) | 5.1 | >320 | >63 |
| **10** | | 0.011 | >1 | >91 |
| | (2) | 0.18 | >1 | >57 |
| **11** | | 0.006 | >3 | >500 |
| | (2) | 0.12 | >3 | >25 |
| | (39) | 0.13 | >3 | >23 |
| | (10) | 0.060 | >3 | >50 |
| | (16) | 0.025 | >3 | >120 |
| | (1A) | 0.16 | >3 | >18 |
| **12** | | 0.14 | >1 | >7 |
| **13** | | 0.060 | >3 | >50 |
| **14** | | 0.020 | >10 | >500 |
| | (2) | 0.13 | >10 | >76 |
| | (89) | 0.080 | >10 | >125 |
| | (16) | 0.10 | >10 | >100 |
| | (10) | 0.16 | >10 | >62 |
| | (1A) | 0.17 | >10 | >58 |
| | (39) | 0.07 | >10 | >143 |
| **15** | | 0.063 | >1 | >15 |
| **16** | | 0.050 | >3 | >60 |
| | (2) | 0.18 | >3 | >16 |
| | (39) | 0.20 | >3 | >15 |
| | (89) | 0.080 | >3 | >37 |
| | (16) | 0.10 | >3 | >30 |
| | (10) | 0.15 | >3 | >20 |
| | (1A) | 0.18 | >3 | >16 |
| **17** | | 0.027 | >10 | >370 |
| | (2) | 0.36 | >10 | >27 |
| | (39) | 0.48 | >10 | >20 |
| **18** | | 0.48 | >3 | >6 |
| **19** | | 0.28 | >3 | >10 |
| | (2) | 0.71 | >3 | >4 |
| | (10) | 1.6 | >3 | >1.8 |
| | (1A) | 0.60 | >3 | >5 |
| **20** | | 0.042 | >3 | >71 |
| | (2) | 0.56 | >3 | >5 |
| | (39) | 1.2 | >3 | >2.5 |
| | (89) | 0.47 | >3 | >6 |
| | (16) | 0.15 | >3 | >20 |
| | (10) | 0.50 | >3 | >6 |
| | (1A) | 0.53 | >3 | >5 |
| **21** | | 0.16 | >10 | >62 |
| | (2) | 1.0 | >10 | >10 |
| | (10) | 1.4 | >10 | >7 |
| | (1A) | 0.56 | >10 | >17 |
| **22** | | 0.050 | >32 | >640 |
| | (2) | 0.36 | >32 | >88 |
| | (39) | 0.40 | >32 | >80 |
| | (89) | 0.24 | >32 | >133 |
| | (16) | 0.40 | >32 | >80 |
| | (10) | 0.50 | >32 | >64 |
| | (1A) | 0.43 | >32 | >74 |
| **23** | | 20.9 | >10 | ND |
| **24** | | 0.032 | >3 | >93 |
| **25** | | 0.14 | >3 | >21 |
| | (2) | 0.68 | >3 | >4 |
| | (10) | 1.7 | >3 | >1.7 |
| | (IA) | 0.90 | >3 | >3 |
| | (16) | 0.71 | >3 | >4 |
| | (39) | 0.56 | >3 | >5 |
| | (89) | 0.32 | >3 | >9 |
| **26** | | 0.050 | >10 | >200 |
| | (1A) | 0.50 | >10 | >20 |
| | (10) | 0.55 | >10 | >18 |
| **27** | | 0.060 | >1 | >16 |
| | (1A) | 1.8 | >1 | >0.5 |
| | (10) | 3.3 | >1 | >0.3 |
| **28** | | 0.025 | >10 | >400 |
| | (1A) | 0.60 | >10 | >16 |
| | (10) | 1.0 | >10 | >10 |
| **29** | | ND | ND | ND |
| **30** | | 1.5 | >100 | >66 |
| **31** | | 0.16 | >3 | >18 |
| | (1A) | 6.3 | >3 | >0.4 |
| | (10) | 4.7 | >3 | >0.6 |
| **32** | | 0.4 | >32 | >80 |
| | (1A) | 2.2 | >32 | >14 |
| | (10) | 1.9 | >32 | >16 |
| **33** | | 0.51 | >32 | >62 |
| | (1A) | 1.7 | >32 | >18 |
| | (10) | 1.6 | >32 | >20 |
| **34** | | 0.10 | >3 | >30 |
| | (1A) | 0.30 | >3 | >10 |
| | (10) | 0.40 | >3 | >7 |
| | | | | |
| | | | | |
| **WIN 51711** | | 0.78 | >60 | >77 |
| **WIN 52084** | | 0.07 | >10 | >143 |
| **WIN 54954** | | 2.13 | >63 | >30 |
| **Piro- davir** | | 0.03 | >10 | >300 |

### Anticoxsackieviral HI-HeLa Cell Culture Assay

The Coxsackie strain A-21 (CVA-21) was purchased from American Type Culture Collection (ATCC). Virus stocks were propagated, and antiviral assays were performed in HI-HeLa cells (ATCC). Cells were grown in Minimal Essential Medium with 10% fetal bovine serum.

The ability of compounds to protect cells against CVÁ-21 infection was measured by the XTT dye reduction method. This method is described in Weislow, O. S., Kiser, R., Fine, D. L., Bader, J., Shoemaker, R. H., Boyd, M. R., *J. Natl. Cancer Inst.* **1989**, *81*, 577-586. HI-HeLa cells were infected with CVA-21 at a multiplicity of infection (m.o.i.) of 0.05 (CVA-21) or mock-infected with medium only. Infected or uninfected cells were resuspended at 4 x 10⁴ cells per mL and incubated with appropriate concentrations of drug. One day later, XTT/PMS was added to test plates, and the amount of formazan produced was quantified spectrophotometrically at 450/650 nm. The EC₅₀ was calculated as the concentration of drug that increased the percentage of formazan production in drug-treated, virus-infected cells to 50% of that produced by drug-free, uninfected cells. The 50% cytotoxic dose (CC₅₀) was calculated as the concentration of drug that decreased the percentage of formazan produced in drug-treated, uninfected cells to 50% of that produced in drug-free, uninfected cells. The therapeutic index (TI) was calculated by dividing the CC₅₀ by the EC₅₀.

The compounds were tested against control compound WIN 54954, obtained from Sterling-Winthrop Pharmaceuticals, and control compound Pirodavir, obtained from Janssen Pharmaceuticals. Antiviral data obtained for the test compounds against Coxsackie strain A-21 (CVA-21) are shown in Table 3.

**Table 3**

| COMPOUND # | EC₅₀ (µM) | CC₅₀ (µM) | TI |
|---|---|---|---|
| **7** | 0.16 | >10 | >63 |
| | | | |
| **WIN 54954** | >100 | >100 | |
| **Pirodavir** | >100 | >100 | |

In describing the invention, the inventors have set forth certain theories and mechanisms in an effort to disclose how or why the invention works in the manner in which it works. These theories and mechanisms are set forth for informational purposes only. Applicants are not to be bound by any specific chemical or physical mechanisms or theories of operation.

## Claims

1. A compound of the formula (I): 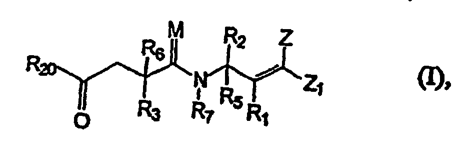 wherein:
M is O or S;
R₁ is H, F, an alkyl group, OH, SH, or an O-alkyl group;
R₂ and R₅ are independently selected from H, 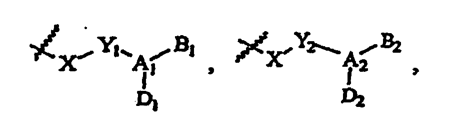 or an alkyl group, wherein said alkyl group is different from 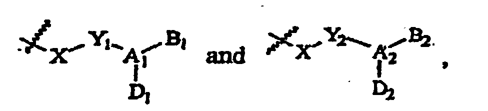 with the proviso that at least one of R₂ or R₅ must be 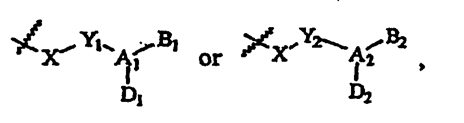
and wherein, when R₂ or R₅ is 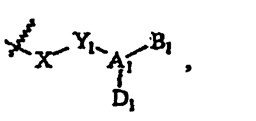
X is =CH or =CF and Y₁ is =CH or =CF,
or X and Y₁ together with Q' form a three-membered ring in which Q' is -C(R₁₀)(R₁₁)- or -O-, X is -CH- or -CF-, and Y₁ is -CH-, -CF-, or -C(alkyl)-, where R₁₀ and R₁₁ independently are H, a halogen, or an alkyl group, or, together with the carbon atom to which they are attached, form a cycloalkyl group or a heterocycloalkyl group,
or X is -CH₂-, -CF₂-, -CHF-, or -S-, and Y₁ is -O-, -S-, -NR₁₂-, -C(R₁₃)(R₁₄)-, -C(O)-, -C(S)-, or -C(CR₁₃R₁₄)-,
wherein R₁₂ is H or alkyl, and R₁₃ and R₁₄ independently are H, F, or an alkyl group, or, together with the atoms to which they are bonded, form a cycloalkyl group or a heterocycloalkyl group;
A₁ is C, CH, CF, S, P, Se, N, NR₁₅, S(O), Se(O), P-OR₁₅, or P-NR₁₅R₁₆,
wherein R₁₅ and R₁₆ independently are an alkyl group, a cycloalkyl group, a heterocycloalkyl group, an aryl group, or a heteroaryl group, or, together with the atom to which they are bonded, form a heterocycloalkyl group;
D₁ is a moiety with a lone pair of electrons capable of forming a hydrogen bond; and B₁ is H, F, an alkyl group, a cycloalkyl group, a heterocycloalkyl group, an aryl group, a heteroaryl group, -OR₁₇, -SR₁₇, -NR₁₇R₁₈, -NR₁₉NR₁₇R₁₈, or -NR₁₇OR₁₈,
wherein R₁₇, R₁₈, and R₁₉ independently are H, an alkyl group, a cycloalkyl group, a heterocycloalkyl group, an aryl group, a heteroaryl group, or an acyl group;
and with the provisos that when D₁ is the moiety ≡N with a lone pair of electrons capable of forming a hydrogen bond, B₁ does not exist; and when A, is an sp³ carbon, B, is not -NR₁₇R₁₈ when D₁ is the moiety -NR₂₅R₂₆ with a lone pair of electrons capable of forming a hydrogen bond, wherein R₂₅ and R₂₆ are independently H, an alkyl group, a cycloalkyl group, a heterocycloalkyl group, an aryl group, or a heteroaryl group;
and wherein D₁-A₁-B₁ optionally forms a nitro group where A₁ is N; and further wherein, when R₂ or R₅ is 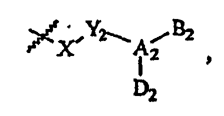
X is =CH or =CF and Y₂ is =C, =CH, or =CF,
or X and Y₂ together with Q' form a three-membered ring in which Q' is -C(R₁₀)(R₁₁)- or -O-, X is -CH- or -CF-, and Y₂ is -CH-, -CF-, or -C(alkyl)-, where R₁₀ and R₁₁ independently are H, a halogen, or an alkyl group, or, together with the carbon atom to which they are attached, form a cycloalkyl group or a heterocycloalkyl group,
or X is -CH₂-, -CF₂-, -CHF-, or -S-, and Y₂ is -O-, -S-, -N(R'₁₂)-, -C(O)-, -C(R'₁₃)(R'₁₄)-, -C(S)-, or -C(CR'₁₃R'₁₄)-,
wherein R'₁₂ is H, an alkyl group, a cycloalkyl group, a heterocycloalkyl group, an aryl group, a heteroaryl group, -OR'₁₃, -NR'₁₃R'₁₄, -C(O)-R'₁₃, -SO₂R'₁₃, or -C(S)R'₁₃, and R'₁₃ and R'₁₄, independently are H, F, or an alkyl group, a cycloalkyl group, a heterocycloalkyl group, an aryl group, or a heteroaryl group, or, together with the atom to which they are attached, form a cycloalkyl group or a hetearocycloalkyl group;
A₂ is C, CH, CF, S, P, Se, N, NR₁₅, S(O), Se(O), P-OR₁₅, or P-NR₁₅R₁₆,
wherein R₁₅ and R₁₆ independently are an alkyl group, a cycloalkyl group, a heterocycloalkyl group, an aryl group, or a heteroaryl group, or, together with the atom to which they are bonded, form a heterocycloalkyl group;
D₂ is a moiety with a lone pair of electrons capable of forming a hydrogen bond; and
B₂ is H, F, an alkyl group, a cycloalkyl group, a heterocycloalkyl group, an aryl group, a heteroaryl group, -OR₁₇, -SR₁₇, -NR₁₇R₁₈, -NR₁₉NR₁₇R₁₈, or -NR₁₇OR₁₈,
wherein R₁₇, R₁₈, and R₁₉ independently are H, an alkyl group, a cycloalkyl group, a heterocycloalkyl group, an aryl group, a heteroaryl group, or an acyl group;
and further wherein any combination of Y₂, A₂, B₂, and D₂ optionally can form a cycloalkyl group, a heterocycloalkyl group, an aryl group, or a heteroaryl group;
R₃ and R₆ are independently H, F, an alkyl group, a cycloalkyl group, a heterocycloalkyl group, an aryl group, a heteroaryl group, -C(O)R₁₇, -OR₁₇, -SR₁₇, -NR₁₉NR₁₇R₁₈, or -NR₁₇OR₁₈,
wherein R₁₇, R₁₈, and R₁₉ independently are H) an alkyl group, a cycloalkyl group, a heterocycloalkyl group, an aryl group, a heteroaryl group, or an acyl group;
or, R₃ and R₆, together with the carbon atom to which they are attached, form a cycloalkyl group or a heterocycloalkyl group;
R₇ is H) an alkyl group, a cycloalkyl group, a heterocycloalkyl group, an aryl group, a heteroaryl group, -OR₁₇, -SR₁₇, -NR₁₇R₁₈, -NR₁₉NR₁₇R₁₈, or -NR₁₇OR₁₈,
wherein R₁₇, R₁₈, and R₁₉ independently are H, an alkyl group, a cycloalkyl group, a heterocycloalkyl group, an aryl group, a heteroaryl group, or an acyl group;
or R₇, together with R₃ or R₆ and the atoms to which they are attached, forms a heterocycloalkyl group;
R₂₀ is H, or an hydroxy group, an alkyl group, an oxo group, a cycloalkyl group, an heterocycloalkyl group, an aryl group, a heteroaryl group, an acyl group, a sulfonyl group, a mercapto group, an alkythio group, an alkoxy group, a carboxy group, an amino group, an alkylamino group, a dialkylamino group, a carbamoyl group, an arylthio group, a heteroarylthio group, -OR₁₇, -SR₁₇, NR₁₇R₁₈, -NR₁₉NR₁₇R₁₈ or -NR₁₇OR₁₈, wherein R₁₇, R₁₈ and R₁₉ independently are H, an alkyl group, a cycloalkyl group, a heterocycloalkyl group, and aryl group, a heteroaryl group, or an acyl group; and
Z and Z₁ are independently H, F, an alkyl group, a cycloalkyl group, a heterocycloalkyl group, an aryl group, a heteroaryl group, -C(O)R₂₁, -CO₂R₂₁, -CN, -C(O)NR₂₁,R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), PO(NR₂₁R₂₂)(NR₂₃R₂₄), -C(O)NR₂₁NR₂₂R₂₃, or -C(S)NR₂₁NR₂₂R₂₃,
wherein R₂₁, R₂₂, R₂₃, and R₂₄ are independently H, an alkyl group, a cycloalkyl group, a heterocycloalkyl group, an aryl group, a heteroaryl group, an acyl group, or a thioacyl group, or wherein any two of R₂₁, R₂₂, R₂₃, and R₂₄, together with the atom(s) to which they are bonded, form a heterocycloalkyl group;
or Z₁, as defined above, together with R₁, as defined above, and the atoms to which Z, and R₁ are bonded, form a cycloalkyl or heterocycloalkyl group,
or Z and Z₁, both as defined above, together with the atoms to which they are bonded, form a cycloalkyl or heterocycloalkyl group;
or a pharmaceutically acceptable prodrug, salt, active metabolite, or solvate thereof.

2. A compound of claim 1, wherein R₁ is H or F, or a pharmaceutically acceptable prodrug, salt, active metabolite, or solvate thereof.

3. A compound of claim 1, wherein R₂₀ is H, an alkyl group, a cycloalkyl group, a heterocycloalkyl group, an aryl group, a heteroaryl group, -OR₁₇, -SR₁₇, -NR₁₇R₁₈, -NR₁₉NR₁₇R₁₈, or -NR₁₇OR₁₈, wherein R₁₇, R₁₈, and R₁₉ independently are H, an alkyl group, a cycloalkyl group, a heterocycloalkyl group, an aryl group, a heteroaryl group, or an acyl group, or a pharmaceutically acceptable prodrug, salt, active metabolite, or solvate thereof.

4. A compound of claim 3, wherein R₂₀ is the alkyl group -C(R₄₁)(R₄₂)NR₄₃R₄₄, wherein:
R₄₁ and R₄₂ independently are H, an alkyl group, a cycloalkyl group, a heterocycloalkyl group, an aryl group, or a heteroaryl group; and
R₄₃ and R₄₄ independently are H, an alkyl group, a cycloalkyl group, a heterocycloalkyl group, an aryl group, a heteroaryl group, -NR₄₅R₄₆, -C(O)R₄₅, -C(S)R₄₅, -C(O)NR₄₅R₄₆, -C(S)NR₄₅R₄₆, -C(O)NR₄₅OR₄₆, -C(S)NR₄₅OR₄₆, -C(O)SR₄₅, -C(O)OR₄₅, -C(S)OR₄₅, -C(S)SR₄₅, -OR₄₅, -SR₄₅, -C(O)NR₄₅NR₄₆R₄₇, -C(S)NR₄₅NR₄₆R₄₇, -SOR₄₅, -SO₂R₄₅, -S(O)NR₄₅R₄₆, -S(O)NR₄₅(OR₄₆), -SO₂NR₄₅R₄₆, -SO₂NR₄₅(OR₄₆), or -SO₃R₄₅,
wherein R₄₅, R₄₆, and R₄₇ independently are H, an alkyl group, a cycloalkyl group, a heterocycloalkyl group, an aryl group, or a heteroaryl group,
or wherein any suitable combination of R₄₁, R₄₂, R₄₃, and R₄₄ together form a cycloalkyl group or a heterocycloalkyl group;
or a pharmaceutically acceptable prodrug, salt, active metabolite, or solvate thereof.

5. A compound of claim 4, wherein at least one of R₄₃ or R₄₄ is -C(O)SR₄₅ or -C(O)OR₄₅, or a pharmaceutically acceptable prodrug, salt, active metabolite, or solvate thereof.

6. A compound of claim 5, wherein R₄₅ is an alkyl group, a cycloalkyl group, an aryl group, a heterocycloalkyl group, or a heteroaryl group, or a pharmaceutically acceptable prodrug, salt, active metabolite, or solvate thereof.

7. A compound of claim 6, wherein R₄₅ is a C₁-C₁₀ alkyl group or a cycloalkyl group, or a pharmaceutically acceptable prodrug, salt, active metabolite, or solvate thereof.

8. A compound of claim I, wherein at least one of R₂ or R₅ is 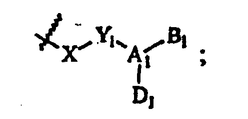 or a pharmaceutically acceptable prodrug, salt, active metabolite, or solvate thereof.

9. A compound according to claim 8, wherein D₁ is -OR₂₅, =O, =S, ≡N, =NR₂₅, or -NR₂₅R₂₆, wherein R₂₅ and R₂₆ are independently H, an alkyl group, a cycloalkyl group, a heterocycloalkyl group, an aryl group, or a heteroaryl group, or, together with the nitrogen atom to which they are bonded, form a heterocycloalkyl group; or a pharmaceutically acceptable prodrug, salt, active metabolite, or solvate thereof

10. A compound according to claim 9, wherein D₁ is =O; or a pharmaceutically acceptable prodrug, salt, active metabolite, or solvate thereof.

11. A compound according to claim 8, wherein A₁ is C, CH, S, or S(O); or a pharmaceutically acceptable prodrug, salt, active metabolite, or solvate thereof.

12. A compound according to claim 11, wherein A₁ is C; or a pharmaceutically acceptable prodrug, salt, active metabolite, or solvate thereof.

13. A compound according to claim 8, wherein B₁ is NR₁₇R₁₈, wherein R₁₇ and R₁₈ are independently H, an alkyl group, a cycloalkyl group, a heterocycloalkyl group, an aryl group, a heteroaryl group, or an acyl group; or a pharmaceutically acceptable prodrug, salt, active metabolite, or solvate thereof.

14. A compound according to claim 1, wherein at least one of R₂ or R₅ is 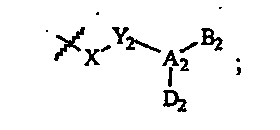 or a phannaceutically acceptable prodrug, salt, active metabolite, or solvate thereof.

15. A compound according to claim 14, wherein D₂ is -OR₂₅, =O, =S, ≡N, =NR₂ or -NR₂₅R₂₆, wherein R₂₅ and R₂₆ are independently H, an alkyl group, a cycloalkyl group, a heterocycloalkyl group, an aryl group, or a heteroaryl group, or, together with the atom(s) to which they are bonded, form a heterocycloalkyl group; or a pharmaceutically acceptable prodrug, salt, active metabolite, or solvate thereof.

16. A compound according to claim 15, wherein D₂ is =O; or a pharmaceutically acceptable prodrug, salt, active metabolite, or solvate thereof.

17. A compound according to claim 14, wherein A₂ is C, CH, S, or S(O); or a pharmaceutically acceptable prodrug, salt, active metabolite, or solvate thereof.

18. A compound according to claim 17, wherein A₂ is C; or a pharmaceutically acceptable prodrug, salt, active metabolite, or solvate thereof.

19. A compound according to claim 14, wherein B₂ is -NR₁₇R₁₈, wherein R₁₇ and R₁₈ are independently H, an alkyl group, a cycloalkyl group, a heterocycloalkyl group, an aryl group, a heteroaryl group, or an acyl group; or a phannaceutically acceptable prodrug, salt, active metabolite, or solvate thereof.

20. A compound according to claim 1, wherein A, is C, CH, S, or S(O) or wherein A₂ is C, CH, S, or S(O); or a pharmaceutically acceptable prodrug, salt, active metabolite, or solvate thereof.

21. A compound according to claim 1, wherein Z and Z₁ are independently H, an aryl group, or a heteroaryl group, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -C(O)NR₂₁NR₂₂R₂₃, or -C(S)NR₂₁NR₂₂R₂₃;
wherein R₂₁, R₂₂, and R₂₃ are independently H, an alkyl group, a cycloalkyl group, a heterocycloalkyl group, an aryl group, a heteroaryl group, an acyl group, or a thioacyl group, or wherein any two of R₂₁, R₂₂, and R₂₃, together with the atom(s) to which they are bonded, form a heterocycloalkyl group;
or a pharmaceutically acceptable prodrug, salt, active metabolite, or solvate thereof.

22. A compound according to claim 1, wherein M is O.

23. A compound having the formula X: 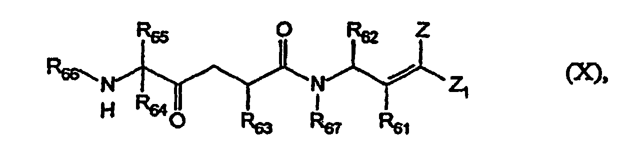 wherein:
R₆₁ is H, F, or an alkyl group;
R₆₂ is selected from one of the following moieties: 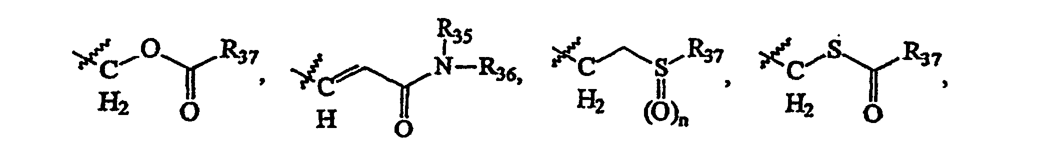 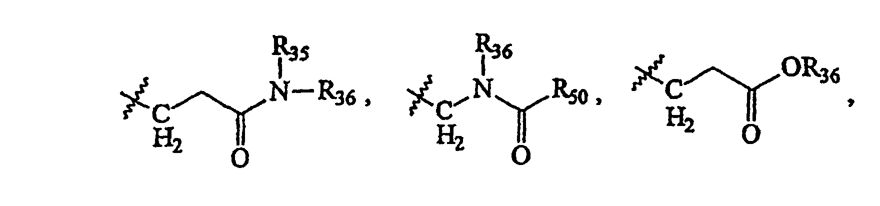 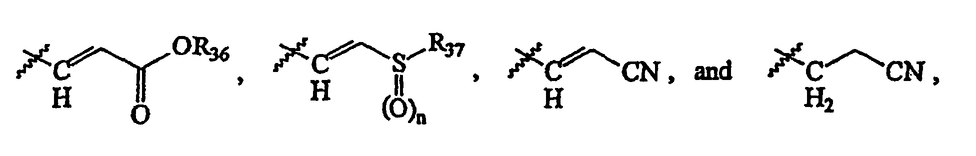
wherein:
R₃₅ is H, an alkyl group, an aryl group, -OR₃₈, or -NR₃₈R₃₉,
wherein R₃₈ and R₃₉ independently are H, an alkyl group, a cycloalkyl group, a heterocycloalkyl group, an aryl group, a heteroaryl group, or an acyl group; and
R₃₆ is H or an alkyl group,
or R₃₅ and R₃₆, together with the nitrogen atom to which they are attached, form a heterocycloalkyl group or a heteroaryl group;
R₃₇ is an alkyl group, an aryl group, or -NR₃₈R₃₉, wherein R₃₈ and R₃₉ are as defined above;
R₅₀ is H, an alkyl group, an aryl group, -OR₃₈, -SR₃₉, -NR₃₈R₃₉, -NR₄₀NR₃₈R₃₉, or -NR₃₈OR₃₉, or R₅₀ and R₃₆, together with the atoms to which they are attached, form a heterocycloalkyl group;
wherein R₃₈ and R₃₉ are as defined above, and R₄₀ is H, an alkyl group, a cycloalkyl group, a heterocycloalkyl group, an aryl group, a heteroaryl group, or an acyl group; and
n is 0, 1, or 2;
R₆₃ is H or an alkyl group;
R₆₄ is H, an alkyl group, a cycloalkyl group, a heterocycloalkyl group, an aryl group, or a heteroaryl group;
R₆₅ is H or an alkyl group;
R₆₆ is H, an acyl group, an alkyl group, a cycloalkyl group, a heterocycloalkyl group, an aryl group, a sulfonyl group, or a heteroaryl group;
R₆₇ is H or an alkyl group;
and
Z and Z₁ are independently H, F, an alkyl group, a cycloalkyl group, a heterocycloalkyl group, an aryl group, a heteroaryl group, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, - SO₂NR₂₁R₂₂, - SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), -PO(NR₂₁R₂₂)(NR₂₃R₂₄), - C(O)NR₂₁NR₂₂R₂₃, or -C(S)NR₂₁NR₂₂R₂₃,
wherein R₂₁, R₂₂, R₂₃, and R₂₄ are independently H, an alkyl group, a cycloalkyl group, a heterocycloalkyl group, an aryl group, a heteroaryl group, an acyl group, or a thioacyl group, or wherein any two of R₂₁, R₂₂, R₂₃, and R₂₄, together with the atom(s) to which they are bonded, form a heterocycloalkyl group,
or Z and Z₁, both as defined above, together with the atoms to which they are bonded, form a heterocycloalkyl group;
or a phannaceutically acceptable prodrug, salt, active metabolite, or solvate thereof.

24. A compound according to claim 23, wherein R₆₆ is the acyl group -C(O)OR₆₈ or the acyl group -C(O)SR₆₈, wherein R₆₈ is an alkyl group, a cycloalkyl group, an aryl group, a heterocycloalkyl group, or a heteroaryl group, or a pharmaceutically acceptable prodrug, salt, active metabolite, or solvate thereof.

25. A compound according to claim 4, having the formula II: 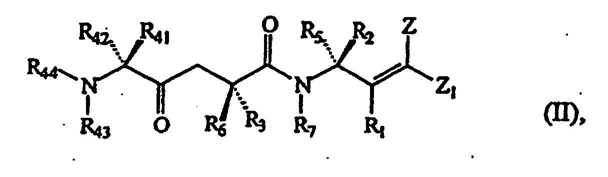 wherein R₁, R₅, R₆, R₇, R₄₂, R₄₃, and Z are H, R₂ is CH₂CH₂C(O)NH₂, and R₃, R₄₁, Z₁, and R₄₄ are selected from one of the following groups:
R₃ is CH₂Ph, R₄₁ is CH₂CH(CH₃)₂, Z₁ is CO₂CH₂CH₃, and R₄₄ is 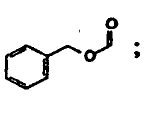
R₃ is CH₂Ph, R₄₁ is CH₂CH(CH₃)₂, Z, is CO₂CH₂CH₃, and R₄₄ is 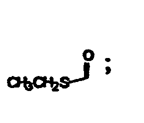
R₃ is CH₂Ph, R₄₁ is CH₂CH(CH₃)₂, Z₁ is 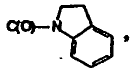 and R₄₄ is 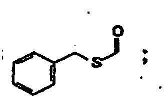
R₃ is CH₂Ph, R₄₁ is CH(CH₃)₂, Z₁ is CO₂CH₂CH₃, and R₄₄ is 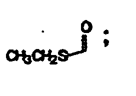
R₃ is CH₂Ph, R₄₁ is CH(CH₃)₂, Z₁ is CO₂CH₂CH₃, and R₄₄ is 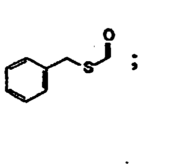
R₃ is CH₂Ph, R₄₁ is CH₂CH(CH₃)₂, Z₁ is CO₂CH₂CH₃, and R₄₄ is 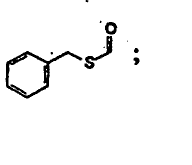
R₃ is CH₂Ph, R₄₁ is CH(CH₃)₂, Z₁ is CO₂CH₂CH₃, and R₄₄ is 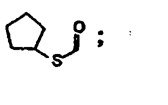
R₃ is CH₂Ph, R₄₁ is CH₂CH(CH₃)₂, Z₁ is CO₂CH₂CH₃, and R₄₄ is 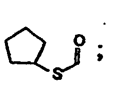
R₃ is CH₂(*p*-CH₃)Ph, R₄₁ is CH(CH₃)₂, Z₁ is CO₂CH₂CH₃, and R₄₄ is 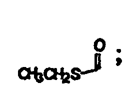 and
R₃ is CH₂(*p*-CH₃)Ph, R₄₁ is CH(CH₃)₂, Z₁ is CO₂CH₂CH₃, and R₄₄ is 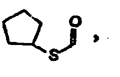 or a pharmaceutically acceptable prodrug, salt, active metabolite, or solvate thereof.

26. A compound according to claim 4, having the formula III: 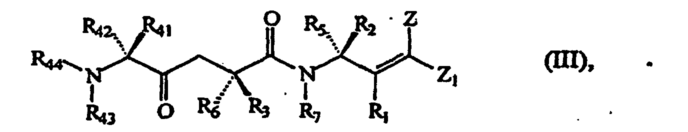 wherein R₁, R₅, R₆, R₇, R₄₂, R₄₃, and Z are H, R₃ is CH₂Ph, R₂ is CH₂CH₂C(O)NH₂,
R₄₁ is CH₂CH(CH₃)₂, Z₁ is CO₂CH₂CH₃, and R₄₄ is 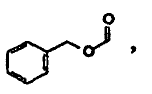 or a pharmaceutically acceptable prodrug, salt, active metabolite, or solvate thereof.

27. A compound of formula (TV): 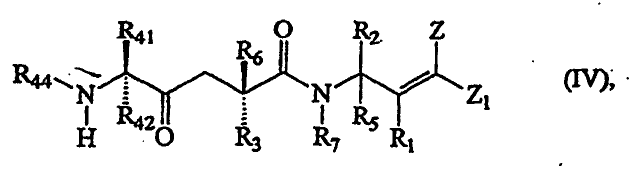 wherein:
R₁, R₅, R₆, R₇, and R₄₂ are H, R₂ is CH₂CH₂C(O)NH₂, and R₃, Z, Z₁, R₄₁, and R₄₄ are selected from one of the following groups:
R₃ is CH₂(*p*-CH₃)Ph, Z is H, Z₁ is CO₂CH₂CH₃, R₄₁ is CH₂Ph, and R₄₄ is 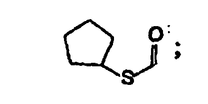
R₃ is CH₂(*p*-F)Ph, Z is H, Z₁ is CO₂CH₂CH₃, R₄₁ is CH(CH₃)₂, and R₄₄ is 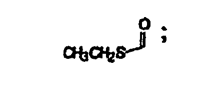
R₃ is CH₂(*p*-F)Ph, Z is H, Z₁ is CO₂CH₂CH₃, R₄₁ is CH(CH₃)₂, and R₄₄ is 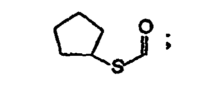
R₃ is CH₂(*p*-CF₃)Ph, Z is H, Z₁ is CO₂CH₂CH₃, R₄₁ is CH(CH₃)₂, and R₄₄ is 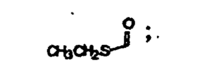
R₃ is CH₂(*p*-CF₃)Ph, Z is H, Z, is CO₂CH₂CH₃, R₄₁ is CH(CH₃)₂, and R₄₄ is 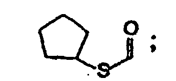
R₃ is CH₂(*p*-CH₃)Ph, Z and Z₁ together form 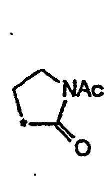 (where * indicates the point of attachment and the carbonyl group is cis to the R₁ group), R₄₁ is CH(CH₃)₂, and R₄₄ is 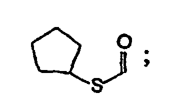
R₃ is CH₂(*p*-F)Ph, Z is H, Z₁ is CO₂CH₂CH₃, R₄₁ is CH₂Ph, and R₄₄ is 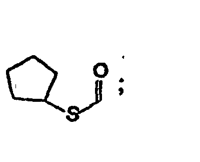
R₃ is CH₂(*p*-F)Ph, Z is H, Z₁ is CO₂CH₂CH₃, R₄₁ is CH₂CH(CH₃)₂, and R₄₄ is 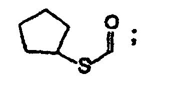
R₃ is CH₂(*p*-CH₃)Ph, Z is H, Z₁ is CO₂CH₂CH₃, R₄₁ is CH(CH₃)₂, and R₄₄ is 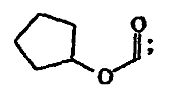
R₃ is CH₂(*p*-CH₃)Ph, Z is H, Z₁ is CO₂CH₂CH₃, R₄₁ is CH₂CH(CH₃)₂, and R₄₄ is 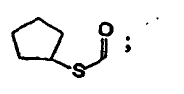
R₃ is CH₂Ph, Z is H, Z₁ is CO₂CH₂CH₃, R₄₁ is C(CH₃)₃, and R₄₄ is 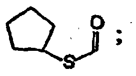
R₃ is CH₂(*p*-CH₃)Ph, Z is H, Z₁ is CO₂CH₂CH₃, R₄₁ is CH(CH₃)₂, and R₄₄ is 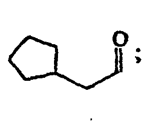
R₃ is CH₂(*p*-F)Ph, Z is H, Z₁ is CO₂CH₂CH₃, R₄₁ is cyclohexyl, and R₄₄ is 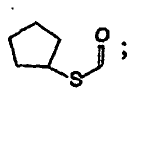
R₃ is CH₂(*p*-F)Ph, Z is H, Z₁ is CO₂CH₂CH₃, R₄₁ is CH(CH₃)₂, and R₄₄ is 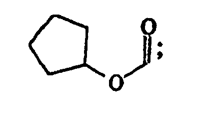
R₃ is CH₂(*p*-F)Ph, Z is H, Z₁ is CO₂CH₂CH₃, R₄₁ is CH(CH₃)₂, and R₄₄ is 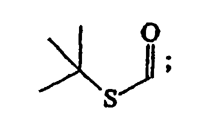
R₃ is CH₂(*p*-F)Ph, Z is H, Z₁ is CO₂CH₂CH₃, R₄₁ is CR(CH₃)₂, and R₄₄ is 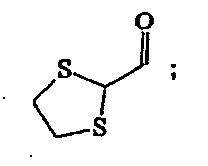
R₃ is CH₂(*p*-F)Ph, Z is H, Z₁ is 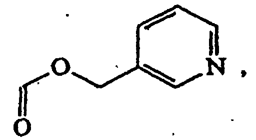 R₄₁ is CH(CH₃)₂, and R₄₄ is 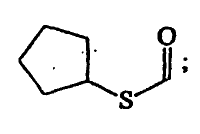
R₃ is CH₂(*p*-F)Ph, Z is H, Z₁ is 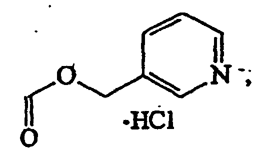 R₄₁ is CH(CH₃)₂, and R₄₄ is 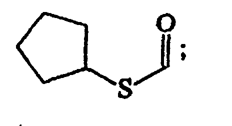
R₃ is CH₂(*p*-F)Ph, Z is H, Z₁ is 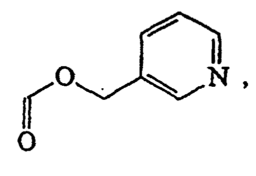 R₄₁ is CH(CH₃)₂, and R₄₄ is 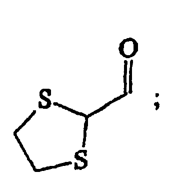
R₃ is CH₂(*p*-F)Ph, Z is H, Z₁ is CO₂CH₂CH₃, R₄₁ is CH(CH₃)₂, and R₄₄ is 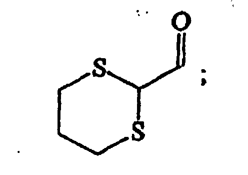
R₃ is CH₂(*p*-F)Ph, Z is H, Z₁ is 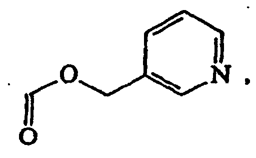 R₄₁ is CH(CH₃)₂, and R₄₄ is 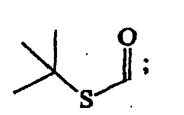
R₃ is CH₂(*p*-F)Ph, Z is H, Z₁ is CO₂CH₂CH₂OH, R₄₁ is CH(CH₃)₂, and R₄₄ is 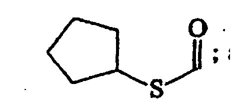 and
R₃ is CH₂(*p*-F)Ph, Z is H, Z₁ is CO₂CH₂CH₃, R₄₁ is CH(CH₃)₂, and R₄₄ is 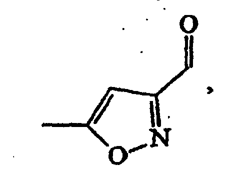
or a pharmaceutically acceptable prodrug, salt, active metabolite, or solvate thereof.

28. A composition comprising at least one compound of formula II: 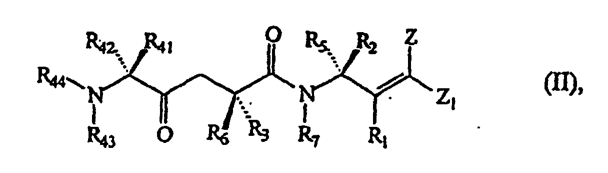 wherein R₁, R₅, R₆, R₇, R₄₂, R₄₃, and Z are H, R₃ is CH₂Ph, R₂ is CH₂CH₂C(O)NH₂,
R₄₁ is CH₂CH(CH₃)₂, Z, is CO₂CH₂CH₃, and R₄₄ is 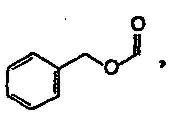 or a pharmaceutically acceptable prodrug, salt, active metabolite, or solvate thereof,
and at least one compound of formula III: 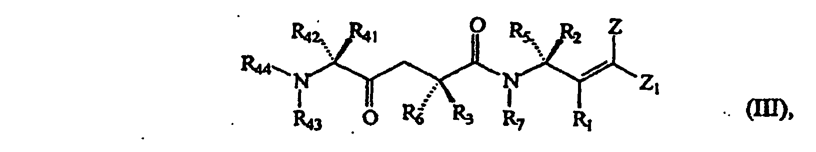 wherein R₁, R₅, R₆, R₇, R₄₂, R₄₃, and Z are H, R₃ is CH₂Ph, R₂ is CH₂CH₂C(O)NH₂,
R₄₁ is CH₂CH(CH₃)₂, Z₁ is CO₂CH₂CH₃, and R₄₄ is 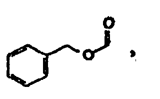 or a pharmaceutically acceptable prodrug, salt, active metabolite, or solvate thereof.

29. A pharmaceutical composition comprising:
(a) a therapeutically effective amount of at least one compound as defined in claim 1 or a pharmaceutically acceptable prodrug, salt, active metabolite, or solvate thereof; and
(b) a pharmaceutically acceptable carrier, diluent, vehicle, or excipient

30. Use of the compound according to claim 1 or a pharmaceutically acceptable prodrug, salt, active metabolite, or solvate thereof for the preparation of a medicament for the treatment of mammalian disease condition mediated by picomaviral protease activity.

31. Use of the compound according to claim 1 or a pharmaceutically acceptable prodrug, salt, active metabolite, or solvate thereof for the preparation of a medicament for inhibiting the activity of a picornaviral 3C protease.

32. A method of inhibiting in vitro the activity of a picornaviral 3C protease, comprising: contacting the picomaviral 3C protease with an effective amount of at least one compound as defined in claim 1 or a pharmaceutically acceptable prodrug, salt, active metabolite, or solvate thereof.

33. Use of the compound according to claim 1 or a pharmaceutically acceptable prodrug, salt, active metabolite, or solvate thereof for the preparation of a medicament for inhibiting the activity of a rhinoviral protease.

34. A method of inhibiting in vitro the activity of a rhinoviral protease, comprising: contacting the rhinoviral protease with an effective amount of at least one compound as defined in claim 1 or a pharmaceutically acceptable prodrug, salt, active metabolite, or solvate thereof.

35. A compound according to claim 1 or a pharmaceutically acceptable prodrug, salt, active metabolite, or solvate thereof, wherein said antipicomaviral activity is antirhinoviral activity.

36. A compound according to claim 1, or a pharmaceutically acceptable prodrug, salt, active metabolite, or solvate thereof, wherein said antipicomaviral activity is anticoxsackieviral activity.

## Patentansprüche

1. Verbindung der Formel (I) 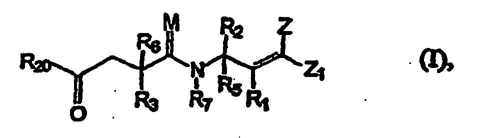 worin:
M O oder S ist;
R₁ H, F, eine Alkylgruppe, OH, SH oder eine O-Alkylgruppe ist;
R₂ und R₅ unabhängig ausgewählt werden aus H, 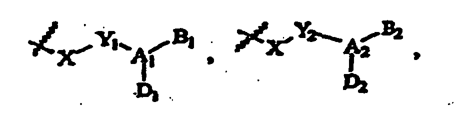 oder einer Alkylgruppe, wobei sich die Alkylgruppe von 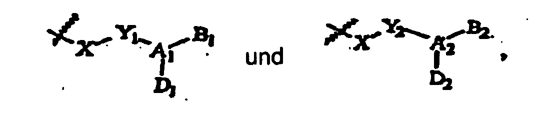 unterscheidet, mit der Maßgabe, dass wenigstens einer von Resten R₂ oder R₅ 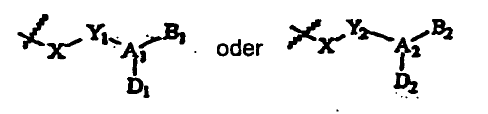 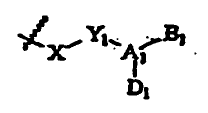 sein muss,
und worin, wenn R₂ oder R₅ 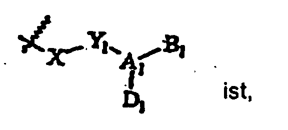
X =CH oder =CF ist und Y₁ =CH oder =CF ist,
oder X und Y₁ zusammen mit Q' einen dreigliedrigen Ring bilden, in welchem Q' -C(R₁₀)(R₁₁)- oder -O- ist, X -CH- oder -CF- ist und Y₁ -CH-, -CF- oder -C(alkyl)- ist, wobei R₁₀ und R₁₁ unabhängig H, ein Halogen oder eine Alkylgruppe sind oder zusammen mit dem Kohlenstoffatom, an das sie gebunden sind, eine Cycloalkylgruppe oder eine Heterocycloalkylgruppe bilden,
oder X -CH₂-, -CF₂-, -CHF- oder -S- ist und Y₁ -O-, -S-, -NR₁₂-, -C(R₁₃)(R₁₄)-, -C(O)-, -C(S)- oder -C(CR₁₃R₁₄)- ist,
worin R₁₂ H oder Alkyl ist und R₁₃ und R₁₄ unabhängig H, F oder eine Alkylgruppe sind oder zusammen mit den Atomen, an die sie gebunden sind, eine Cycloalkylgruppe oder eine Heterocycloalkylgruppe bilden;
A₁ C, CH, CF, S, P, Se, N, NR₁₅, S(O), Se(O), P-OR₁₅ oder P-NR₁₅R₁₆ ist,
worin R₁₅ und R₁₆ unabhängig eine Alkylgruppe, eine Cycloalkylgruppe, eine Heterocycloalkylgruppe, eine Arylgruppe oder eine Heteroarylgruppe sind oder zusammen mit dem Atom, an das sie gebunden sind, eine Heterocycloalkylgruppe bilden;
D₁ eine Komponente mit einem freien Elektronenpaar ist, welches eine Wasserstoffbindung ausbilden kann; und
B₁ H, F, eine Alkylgruppe, eine Cycloalkylgruppe, eine Heterocycloalkylgruppe, eine Arylgruppe, eine Heteroarylgruppe, -OR₁₇, -SR₁₇, -NR₁₇R₁₈, -NR₁₉NR₁₇R₁₈ oder -NR₁₇OR₁₈ ist
worin R₁₇, R₁₈ und R₁₉ unabhängig H, eine Alkylgruppe, eine Cycloalkylgruppe, eine Heterocycloalkylgruppe, eine Arylgruppe, eine Heteroarylgruppe oder eine Acylgruppe sind;
und mit den Maßgaben, dass, wenn D₁ die Komponente ≡N mit einem freien Elektronenpaar ist, das eine Wasserstoffbindung ausbilden kann, B₁ nicht vorhanden ist; und wenn A₁ ein sp³-Kohlenstoff ist, B₁ nicht -NR₁₇R₁₈ ist, wenn D₁ die Komponente -NR₂₅R₂₆ mit einem freien Elektronenpaar ist, das eine Wasserstoffbindung ausbilden kann, worin R₂₅ und R₂₆ unabhängig H, eine Alkylgruppe, eine Cycloalkylgruppe, eine Heterocycloalkylgruppe, eine Arylgruppe oder eine Heteroarylgruppe sind;
und worin D₁-A₁-B₁ gegebenenfalls eine Nitrogruppe bildet, wobei A₁ N ist;
und worin ferner, wenn R₂ oder R₅ 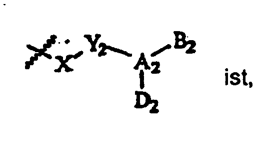
X =CH oder =CF ist und Y₂ =C, =CH oder =CF ist,
oder X und Y₂ zusammen mit Q' einen dreigliedrigen Ring bilden, in welchem Q'-C(R₁₀)(R₁₁)- oder -O- ist, X -CH- oder -CF- ist und Y₂ -CH-, -CF- oder -C(alkyl)- ist, wobei R₁₀ und R₁₁ unabhängig H, ein Halogen oder eine Alkylgruppe sind oder zusammen mit dem Kohlenstoffatom, an das sie gebunden sind, eine Cycloalkylgruppe oder eine Heterocycloalkylgruppe bilden,
oder X -CH₂-, -CF₂-, -CHF- oder -S- ist und Y₂ -O-, -S-, -N(R'₁₂)-, -C(O)-, -C(R'₁₃)(R'₁₄)-, -C(S)- oder -C(CR'₁₃R'₁₄)- ist,
worin R'₁₂ H, eine Alkylgruppe, eine Cydoalkylgruppe, eine Heterocydoalkylgruppe, eine Arylgruppe, eine Heteroarylgruppe, -OR'₁₃, -NR'₁₃R'₁₄, -C(O)-R'₁₃, -SO₂R'₁₃ oder -C(S)R'₁₃ ist und R'₁₃ und R'₁₄ unabhängig H, F oder eine Alkylgruppe, eine Cycloalkylgruppe, eine Heterocydoalkylgruppe, eine Arylgruppe oder eine Heteroarylgruppe sind oder zusammen mit dem Atom, an das sie gebunden sind, eine Cycloalkylgruppe oder eine Heterocycloalkylgruppe bilden;
A₂ C, CH, CF, S, P, Se, N, NR₁₅, S(O), Se(O), P-OR₁₅ oder P-NR₁₅R₁₆ ist,
worin R₁₅ und R₁₆ unabhängig eine Alkylgruppe, eine Cycloalkylgruppe, eine Heterocycloalkylgruppe, eine Arylgruppe oder eine Heteroarylgruppe sind oder zusammen mit dem Atom, an das sie gebunden sind, eine Heterocycloalkylgruppe bilden;
D₂ eine Komponente mit einem freien Elektronenpaar ist, das eine Wasserstoffbindung ausbilden kann; und B₂ H, F, eine Alkylgruppe, eine Cycloalkylgruppe, eine Heterocycloalkylgruppe, eine Arylgruppe, eine Heteroarylgruppe, -OR₁₇-, -SR₁₇-, -NR₁₇R₁₈, -NR₁₉NR₁₇R₁₈ oder -NR₁₇OR₁₈ ist,
worin R₁₇, R₁₈ und R₁₉ unabhängig H, eine Alkylgruppe, eine Cycloalkylgruppe, eine Heterocycloalkylgruppe, eine Arylgruppe, eine Heteroarylgruppe oder eine Acylgruppe sind;
und worin ferner jede Kombination von Y₂, A₂, B₂ und D₂ gegebenenfalls eine Cycloalkylgruppe, eine Heterocycloalkylgruppe, eine Arylgruppe oder eine Heteroarylgruppe bilden kann;
R₃ und R₆ unabhängig H, F, eine Alkylgruppe, eine Cycloalkylgruppe, eine Heterocycloalkylgruppe, eine Arylgruppe, eine Heteroarylgruppe, -C(O)R₁₇, -OR₁₇, -SR₁₇, -NR₁₉NR₁₇R₁₈ oder -NR₁₇OR₁₈ sind,
worin R₁₇, R₁₈ und R₁₉ unabhängig H, eine Alkylgruppe, eine Cycloalkylgruppe, eine Heterocycloalkylgruppe, eine Arylgruppe, eine Heteroarylgruppe oder eine Acylgruppe sind;
oder R₃ und R₆ zusammen mit dem Kohlenstoffatom, an das sie gebunden sind, eine Cycloalkylgruppe oder eine Heterocycloalkylgruppe bilden;
R₇ H, eine Alkylgruppe, eine Cydoalkylgruppe, eine Heterocycloalkylgruppe, eine Arylgruppe, eine Heteroarylgruppe, -OR₁₇, -SR₁₇, -NR₁₇R₁₈, -NR₁₉NR₁₇R₁₈ oder -NR₁₇OR₁₈ ist,
worin R₁₇, R₁₈ und R₁₉ unabhängig H, eine Alkylgruppe, eine Cycloalkylgruppe, eine Heterocycloalkylgruppe, eine Arylgruppe, eine Heteroarylgruppe oder eine Acylgruppe sind;
oder R₇ zusammen mit R₃ oder R₆ und den Atomen, an die sie gebunden sind, eine Heterocycloalkylgruppe bildet;
R₂₀ H oder eine Hydroxygruppe, eine Alkylgruppe, eine Oxogruppe, eine Cydoalkylgruppe, eine Heterocycloalkylgruppe, eine Arylgruppe, eine Heteroarylgruppe, eine Acylgruppe, eine Sulfonylgruppe, eine Mercaptogruppe, eine Alkylthiogruppe, eine Alkoxygruppe, eine Carboxygruppe, eine Aminogruppe, eine Alkylaminogruppe, eine Dialkylaminogruppe, eine Carbamoylgruppe, eine Arylthiogruppe, eine Heteroarylthiogruppe, -OR₁₇, -SR₁₇, NR₁₇R₁₈, -NR₁₉NR₁₇R₁₈ oder -NR₁₇OR₁₈ ist, worin R₁₇, R₁₈ und R₁₉ unabhängig H, eine Alkylgruppe, eine Cycloalkylgruppe, eine Heterocycloalkylgruppe, eine Arylgruppe, eine Heteroarylgruppe oder eine Acylgruppe sind; und
Z und Z₁ unabhängig H, F, eine Alkylgruppe, eine Cycloalkylgruppe, eine Heterocycloalkylgruppe, eine Arylgruppe, eine Heteroarylgruppe, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), PO(NR₂₁R₂₂)(NR₂₃R₂₄), -C(O)NR₂₁NR₂₂R₂₃ oder -C(S)NR₂₁NR₂₂R₂₃ sind,
worin R₂₁, R₂₂, R₂₃ und R₂₄ unabhängig H, eine Alkylgruppe, eine Cycloalkylgruppe, eine Heterocycloalkylgruppe, eine Arylgruppe, eine Heteroarylgruppe, eine Acylgruppe oder eine Thioacylgruppe sind oder worin beliebige zwei von R₂₁, R₂₂, R₂₃ und R₂₄, zusammen mit dem Atom bzw. den Atomen, an das bzw. die sie gebunden sind, eine Heterocycloalkylgruppe bilden;
oder Z₁, wie vorstehend definiert, zusammen mit R₁, wie vorstehend definiert, und den Atomen, an welche Z₁ und R₁ gebunden sind, eine Cycloalkyl- oder Heterocycloalkylgruppe bilden,
oder Z und Z₁, beide wie vorstehend definiert, zusammen den Atomen, an welche sie gebunden sind, eine Cycloalkyl- oder Heterocydoalkylgruppe bilden,
oder ein pharmazeutisch annehmbares Prodrug, Salz, aktiver Metabolit oder Solvat davon.

2. Verbindung nach Anspruch 1, worin R₁ H oder F ist, oder ein pharmazeutisch annehmbares Prodrug, Salz, aktiver Metabolit oder Solvat davon.

3. Verbindung nach Anspruch 1, worin R₂₀ H, eine Alkylgruppe, eine Cycloalkylgruppe, eine Heterocycloalkylgruppe, eine Arylgruppe, eine Heteroarylgruppe, -OR₁₇, -SR₁₇, -NR₁₇R₁₈, -NR₁₉NR₁₇R₁₈ oder NR₁₇OR₁₈ ist, worin R₁₇, R₁₈ und R₁₉ unabhängig H, eine Alkylgruppe, eine Cycloalkylgruppe, eine Heterocycloalkylgruppe, eine Arylgruppe, eine Heteroarylgruppe oder eine Acylgruppe sind, oder ein pharmazeutisch annehmbares Prodrug, Salz, aktiver Metabolit oder Solvat davon.

4. Verbindung nach Anspruch 3, worin R₂₀ die Alkylgruppe -C(R₄₁)(R₄₂)NR₄₃R₄₄ ist, worin:
R₄₁ und R₄₂ unabhängig H, eine Alkylgruppe, eine Cycloalkylgruppe, eine Heterocycloalkylgruppe, eine Arylgruppe oder eine Heteroarylgruppe sind; und R₄₃ und R₄₄ unabhängig H, eine Alkylgruppe, eine Cycloalkylgruppe, eine Heterocycloalkylgruppe, eine Arylgruppe, eine Heteroarylgruppe, -NR₄₅R₄₆, -C(O)R₄₅, -C(S)R₄₅, -C(O)NR₄₅R₄₆, -C(S)NR₄₅R₄₆, -C(O)NR₄₅OR₄₆, -C(S)NR₄₅OR₄₆, -C(O)SR₄₅, -C(O)OR₄₅, -C(S)OR₄₅, -C(S)SR_{45,} -OR₄₅, -SR_{45,} -C(O)NR₄₅NR₄₆R₄₇, -C(S)NR₄₅NR₄₆R₄₇, -SOR₄₅, -SO₂R₄₅, -S(O)NR₄₅R₄₆, -S(O)NR₄₅(OR₄₆), -SO₂NR₄₅R₄₆, -SO₂NR₄₅(OR₄₆) oder -SO₃R₄₅ sind,
worin R₄₅, R₄₆ und R₄₇ unabhängig H, eine Alkylgruppe, eine Cycloalkylgruppe, eine Heterocycloalkylgruppe, eine Arylgruppe oder eine Heteroarylgruppe sind,
oder worin jede geeignete Kombination von R₄₁, R₄₂, R₄₃ und R₄₄ zusammen eine Cycloalkylgruppe oder eine Heterocycloalkylgruppe bilden;
oder ein pharmazeutisch annehmbares Prodrug, Salz, aktiver Metabolit oder Solvat davon.

5. Verbindung nach Anspruch 4, worin wenigstens einer von den Resten R₄₃ oder R₄₄ -C(O)S₄₅ oder -C(O)OR₄₅ ist, oder ein pharmazeutisch annehmbares Prodrug, Salz, aktiver Metabolit oder Solvat davon.

6. Verbindung nach Anspruch 5, worin R₄₅ eine Alkylgruppe, eine Cycloalkylgruppe, eine Arylgruppe, eine Heterocycloalkylgruppe oder eine Heteroarylgruppe ist, oder ein pharmazeutisch annehmbares Prodrug, Salz, aktiver Metabolit oder Solvat davon.

7. Verbindung nach Anspruch 6, worin R₄₅ eine C₁-C₁₀-Alkylgruppe oder eine Cycloalkylgruppe ist, oder ein pharmazeutisch annehmbares Prodrug, Salz, aktiver Metabolit oder Solvat davon.

8. Verbindung nach Anspruch 1, worin wenigstens einer von den Resten R₂ oder R₅ 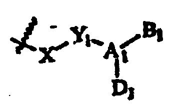 ist;
oder ein pharmazeutisch annehmbares Prodrug, Salz, aktiver Metabolit oder Solvat davon.

9. Verbindung nach Anspruch 8, worin D₁ -OR₂₅, =O, =S, ≡N, =NR₂₅ oder -NR₂₅R₂₆ ist, worin R₂₅ und R₂₆ unabhängig H, eine Alkylgruppe, eine Cycloalkylgruppe, eine Heterocycloalkylgruppe, eine Arylgruppe oder eine Heteroarylgruppe sind oder zusammen mit dem Stickstoffatom, an das sie gebunden sind, eine Heterocycloalkylgruppe bilden; oder ein pharmazeutisch annehmbares Prodrug, Salz, aktiver Metabolit oder Solvat davon.

10. Verbindung nach Anspruch 9, worin D₁ =O ist; oder ein pharmazeutisch annehmbares Prodrug, Salz, aktiver Metabolit oder Solvat davon.

11. Verbindung nach Anspruch 8, worin A₁ C, CH, S oder S(O) ist; oder ein pharmazeutisch annehmbares Prodrug, Salz, aktiver Metabolit oder Solvat davon.

12. Verbindung nach Anspruch 11, worin A₁ C ist; oder ein pharmazeutisch annehmbares Prodrug, Salz, aktiver Metabolit oder Solvat davon.

13. Verbindung nach Anspruch 8, worin B₁ NR₁₇R₁₈ ist, worin R₁₇ und R₁₈ unabhängig H, eine Alkylgruppe, eine Cycloalkylgruppe, eine Heterocydoalkylgruppe, eine Arylgruppe, eine Heteroarylgruppe oder eine Acylgruppe sind; oder ein pharmazeutisch annehmbares Prodrug, Salz, aktiver Metabolit oder Solvat davon.

14. Verbindung nach Anspruch 1, worin wenigstens einer von den Resten R₂ oder R₅ 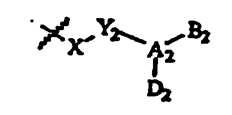 ist;
oder ein pharmazeutisch annehmbares Prodrug, Salz, aktiver Metabolit oder Solvat davon.

15. Verbindung nach Anspruch 14, worin D₂ -OR₂₅, =O, =S, ≡N, =NR₂₅ oder -NR₂₅R₂₆ ist, worin R₂₅ und R₂₆ unabhängig H, eine Alkylgruppe, eine Cycloalkylgruppe, eine Heterocydoalkylgruppe, eine Arylgruppe oder eine Heteroarylgruppe sind oder zusammen mit dem bzw. den Atom(en), an das bzw. die sie gebunden sind, eine Heterocycloalkylgruppe bilden; oder ein pharmazeutisch annehmbares Prodrug, Salz, aktiver Metabolit oder Solvat davon.

16. Verbindung nach Anspruch 15, worin D₂ =O ist; oder ein pharmazeutisch annehmbares Prodrug, Salz, aktiver Metabolit oder Solvat davon.

17. Verbindung nach Anspruch 14, worin A₂ C, CH, S oder S(O) ist; oder ein pharmazeutisch annehmbares Prodrug, Salz, aktiver Metabolit oder Solvat davon.

18. Verbindung nach Anspruch 17, worin A₂ C ist; oder ein pharmazeutisch annehmbares Prodrug, Salz, aktiver Metabolit oder Solvat davon.

19. Verbindung nach Anspruch 14, worin B₂ -NR₁₇R₁₈ ist, worin R₁₇ und R₁₈ unabhängig H, eine Alkylgruppe, eine Cycloalkylgruppe, eine Heterocycloalkylgruppe, eine Arylgruppe, eine Heteroarylgruppe oder eine Acylgruppe sind; oder ein pharmazeutisch annehmbares Prodrug, Salz, aktiver Metabolit oder Solvat davon.

20. Verbindung nach Anspruch 1, worin A₁ C, CH, S oder S(O) ist oder worin A₂ C, CH, S oder S(O) ist; oder ein pharmazeutisch annehmbares Prodrug, Salz, aktiver Metabolit oder Solvat davon.

21. Verbindung nach Anspruch 1, worin Z und Z₁ unabhängig H, eine Arylgruppe oder eine Heteroarylgruppe, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -C(O)NR₂₁NR₂₂R₂₃ oder -C(S)NR₂₁NR₂₂R₂₃ sind;
worin R₂₁, R₂₂ und R₂₃ unabhängig H, eine Alkylgruppe, eine Cycloalkylgruppe, eine Heterocycloalkylgruppe, eine Arylgruppe, eine Heteroarylgruppe, eine Acylgruppe oder eine Thioacylgruppe sind oder worin beliebige zwei von den Resten R₂₁, R₂₂ und R₂₃ zusammen mit dem bzw. den Atom(en), an das bzw. die sie gebunden sind, eine Heterocycloalkylgruppe bilden;
oder ein pharmazeutisch annehmbares Prodrug, Salz, aktiver Metabolit oder Solvat davon.

22. Verbindung nach Anspruch 1, worin M O ist.

23. Verbindung mit der Formel X: 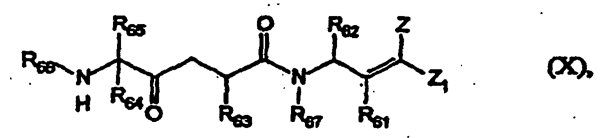 worin:
R₆₁ H, F oder eine Alkyfgruppe ist;
R₆₂ ausgewählt ist aus einer der folgenden Komponenten: 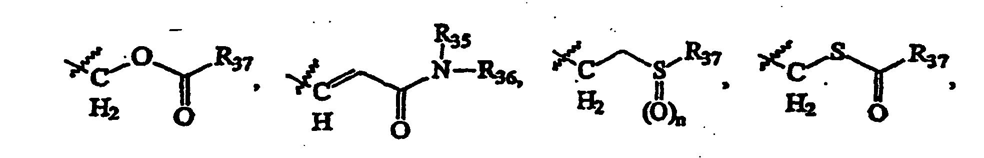 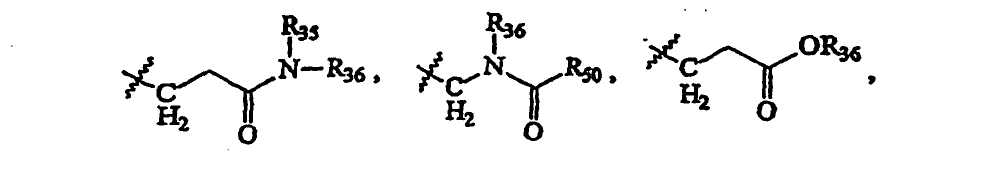 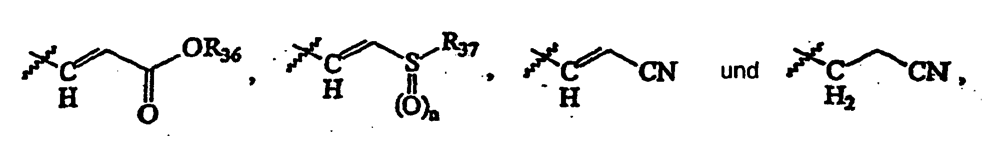 worin:
R₃₅ H, eine Alkylgruppe, eine Arylgruppe, -OR₃₈ oder -NR₃₈R₃₉ ist,
worin R₃₈ und R₃₉ unabhängig H, eine Alkylgruppe, eine Cycloalkylgruppe, eine Heterocycloalkylgruppe, eine Arylgruppe, eine Heteroarylgruppe oder eine Acylgruppe sind; und
R₃₆ H oder eine Alkylgruppe ist,
oder R₃₅ und R₃₆ zusammen mit dem Stickstoffatom, an das sie gebunden sind, eine Heterocycloalkylgruppe oder eine Heteroarylgruppe bilden;
R₃₇ eine Alkylgruppe, eine Arylgruppe oder -NR₃₈R₃₉ ist, worin R₃₈ und R₃₉ wie vorstehend definiert sind;
R₅₀ H, eine Alkylgruppe, eine Arylgruppe, -OR₃₈, -SR₃₉, -NR₃₈R₃₉, -NR₄₀NR₃₈R₃₉ oder -NR₃₈OR₃₉ ist oder R₅₀ und R₃₆ zusammen mit den Atomen, an die sie gebunden sind, eine Heterocycloalkylgruppe bilden;
worin R₃₈ und R₃₉ wie vorstehend definiert sind und R₄₀ H, eine Alkylgruppe, eine Cycloalkylgruppe, eine Heterocycloalkylgruppe, eine Arylgruppe, eine Heteroarylgruppe oder eine Acylgruppe ist; und
n 0, 1 oder 2 ist;
R₆₃ H oder eine Alkylgruppe ist;
R₆₄ H, eine Alkylgruppe, eine Cycloalkylgruppe, eine Heterocycloalkylgruppe, eine Arylgruppe oder eine Heteroarylgruppe ist;
R₆₅ H oder eine Alkylgruppe ist;
R₆₆ H, eine Acylgruppe, eine Alkylgruppe, eine Cycloalkylgruppe, eine Heterocycloalkylgruppe, eine Arylgruppe, eine Sulfonylgruppe oder eine Heteroarylgruppe ist;
R₆₇ H oder eine Alkylgruppe ist;
und
Z und Z₁ unabhängig H, F, eine Alkylgruppe, eine Cycloalkylgruppe, eine Heterocycloalkylgruppe, eine Arylgruppe, eine Heteroarylgruppe -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), -PO(NR₂₁R₂₂)(NR₂₃R₂₄), -C(O)NR₂₁NR₂₂R₂₃ oder -C(S)NR₂₁NR₂₂R₂₃ sind,
worin R₂₁, R₂₂, R₂₃ und R₂₄ unabhängig H, eine Alkylgruppe, eine Cycloalkylgruppe, eine Heterocycloalkylgruppe, eine Arylgruppe, eine Heteroarylgruppe, eine Acylgruppe oder eine Thioacylgruppe sind oder worin beliebige zwei von den Resten R₂₁, R₂₂, R₂₃ und R₂₄ zusammen mit dem bzw. den Atom(en), an das bzw. die sie gebunden sind, eine Heterocycloalkylgruppe bilden,
oder Z und Z₁, beide wie vorstehend definiert, zusammen den Atomen, an welche sie gebunden sind, eine Heterocycloalkylgruppe bilden,
oder ein pharmazeutisch annehmbares Prodrug, Salz, aktiver Metabolit oder Solvat davon.

24. Verbindung nach Anspruch 23, worin R₆₆ die Acylgruppe -C(O)OR₆₈ oder die Acylgruppe -C(O)SR₆₈ ist, worin R₆₈ eine Alkylgruppe, eine Cycloalkylgruppe, eine Arylgruppe, eine Heterocycloalkylgruppe oder eine Heteroarylgruppe ist, oder ein pharmazeutisch annehmbares Prodrug, Salz, aktiver Metabolit oder Solvat davon.

25. Verbindung nach Anspruch 4, mit der Formel II: 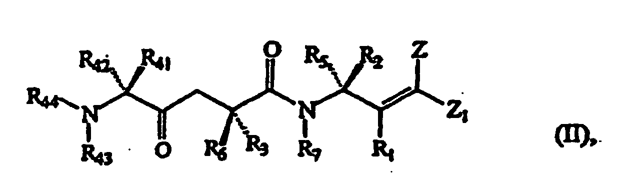 worin R₁, R₅, R₆, R₇, R₄₂, R₄₃ und Z H sind, R₂ CH₂CH₂C(O)NH₂ ist und R₃, R₄₁, Z₁ und R₄₄ aus einer der folgenden Gruppen ausgewählt sind:
R₃ ist CH₂Ph, R₄₁ ist CH₂CH(CH₃)₂, Z₁ ist CO₂CH₂CH₃ und R₄₄ ist 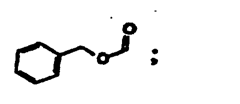
R₃ ist CH₂Ph, R₄₁ ist CH₂CH(CH₃)₂, Z₁ ist CO₂CH₂CH₃ und R₄₄ ist 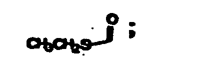
R₃ ist CH₂Ph, R₄₁ ist CH₂CH(CH₃)₂, Z₁ ist 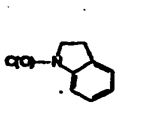 und R₄₄ ist 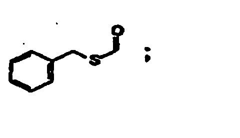
R₃ ist CH₂Ph, R₄₁ ist CH(CH₃)₂, Z₁ ist CO₂CH₂CH₃ und R₄₄ ist 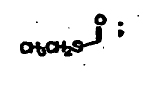
R₃ ist CH₂Ph, R₄₁ ist CH(CH₃)₂, Z₁ ist CO₂CH₂CH₃ und R₄₄ ist 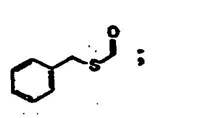
R₃ ist CH₂Ph, R₄₁ ist CH₂CH(CH₃)₂, Z₁ ist CO₂CH₂CH₃ und R₄₄ ist 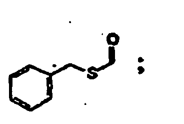
R₃ ist CH₂Ph, R₄₁ ist CH(CH₃)₂, Z₁ ist CO₂CH₂CH₃ und R₄₄ ist: 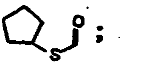
R₃ ist CH₂Ph, R₄₁ ist CH₂CH(CH₃)₂, Z₁ ist CO₂CH₂CH₃ und R₄₄ ist 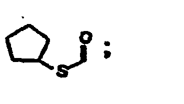
R₃ ist CH₂(p-CH₃)Ph, R₄₁ ist CH(CH₃)₂, Z₁ ist CO₂CH₂CH₃ und R₄₄ ist 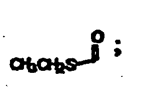 und
R₃ ist CH₂(p-CH₃)Ph, R₄₁ ist CH(CH₃)₂, Z₁ ist CO₂CH₂CH₃ und R₄₄ ist 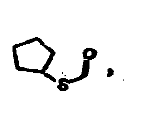
oder ein pharmazeutisch annehmbares Prodrug, Salz, aktiver Metabolit oder Solvat davon.

26. Verbindung nach Anspruch 4, mit der Formel III: 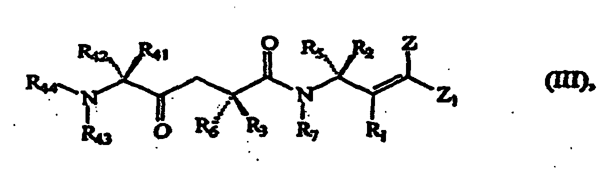 worin R₁, R₅, R₆, R₇, R₄₂, R₄₃ und Z H sind, R₃ CH₂Ph ist, R₂ CH₂CH₂C(O)NH₂ ist,
R₄₁ ist CH₂CH(CH₃)₂ ist, Z₁ CC₂CH₂CH₃ ist und R₄₄ 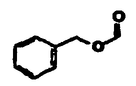 ist,
oder ein pharmazeutisch annehmbares Prodrug, Salz, aktiver Metabolit oder Solvat davon.

27. Verbindung der Formel (IV): 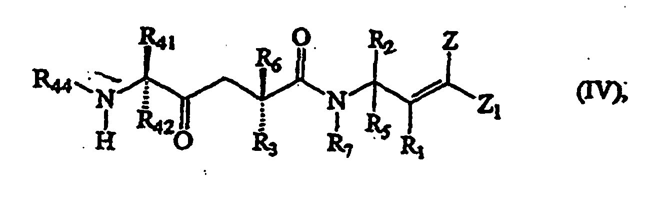 worin:
R₁, R₅, R₆, R₇ und R₄₂ H sind, R₂ CH₂CH₂C(O)NH₂ ist und R₃, Z, Z₁, R₄₁ und R₄₄ ausgewählt sind aus einer der folgenden Gruppen:
R₃ ist CH₂(p-CH₃)Ph, Z ist H, Z₁ ist CO₂CH₂CH₃, R₄₁ ist CH₂Ph und R₄₄ ist 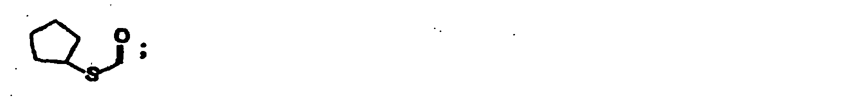
R₃ ist CH₂(p-F)Ph, Z ist H, Z₁ ist CO₂CH₂CH₃, R₄₁ ist CH(CH₃)₂ und R₄₄ ist 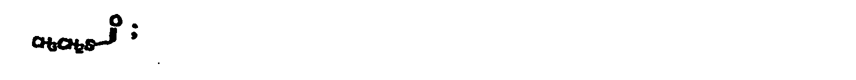
R₃ ist CH₂(p-F)Ph, Z ist H, Z₁ ist CO₂CH₂CH₃, R₄₁ ist CH(CH₃)₂ und R₄₄ ist 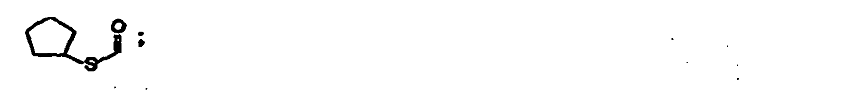
R₃ ist CH₂(p-CF₃)Ph, Z ist H, Z₁ ist CO₂CH₂CH₃, R₄₁ ist CH(CH₃)₂ und R₄₄ ist 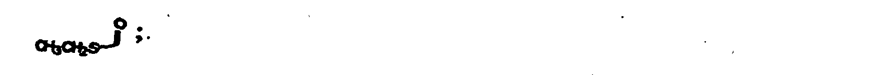
R₃ ist CH₂(p-CF₃)Ph, Z ist H, Z₁ ist CO₂CH₂CH₃, R₄₁ ist CH(CH₃)₂ und R₄₄ ist 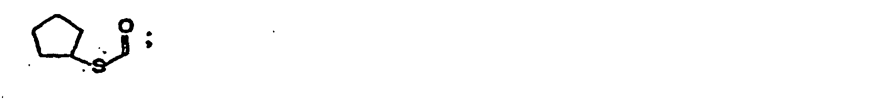
R₃ ist CH₂(p-CH₃)Ph, Z und Z₁ bilden zusammen 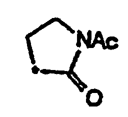 (wobei * die Verknüpfungsstelle angibt und die Carbonylgruppe cis zu der R₁-Gruppe ist),
R₄₁ ist CH(CH₃)₂ und R₄₄ ist 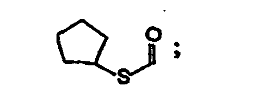
R₃ ist CH₂(p-F)Ph, Z ist H, Z₁ ist CO₂CH₂CH₃, R₄₁ ist CH₂Ph und R₄₄ ist 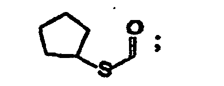
R₃ ist CH₂(p-F)Ph, Z ist H, Z₁ ist CO₂CH₂CH₃, R₄₁ ist CH₂CH(CH₃)₂ und R₄₄ ist 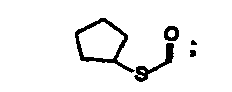
R₃ ist CH₂(p-CH₃)Ph, Z ist H, Z₁ ist CO₂CH₂CH₃, R₄₁ ist CH(CH₃)₂ und R₄₄ ist 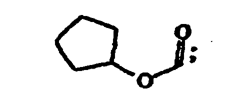
R₃ ist CH₂(p-CH₃)Ph, Z ist H, Z₁ ist CO₂CH₂CH₃, ist CH₂CH(CH₃)₂ und R₄₄ ist 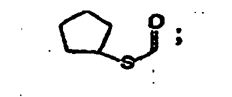
R₃ ist CH₂Ph, Z ist H, Z₁ ist CO₂CH₂CH₃, R₄₁ ist C(CH₃)₃ und R₄₄ ist 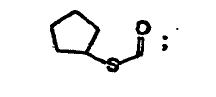
R₃ ist CH₂(p-CH₃)Ph, Z ist H, Z₁ ist CO₂CH₂CH₃, R₄₁ ist CH(CH₃)₂ und R₄₄ ist 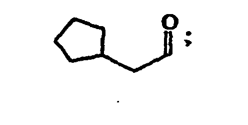
R₃ ist CH₂(p-F)Ph, Z ist H, Z₁ ist CO₂CH₂CH₃, R₄₁ ist Cyclohexyl und R₄₄ ist 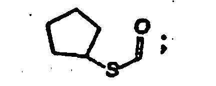
R₃ ist CH₂(p-F)Ph, Z ist H, Z₁ ist CO₂CH₂CH₃, R₄₁ ist CH(CH₃)₂ und R₄₄ ist 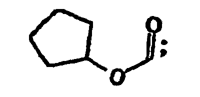
R₃ ist CH₂(p-F)Ph, Z ist H, Z₁ ist CO₂CH₂CH₃, R₄₁ ist CH(CH₃)₂ und R₄₄ ist 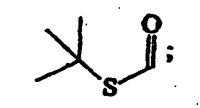
R₃ ist CH₂(p-F)Ph, Z ist H, Z₁ ist CO₂CH₂CH₃, R₄₁ ist CH(CH₃)₂ und R₄₄ ist 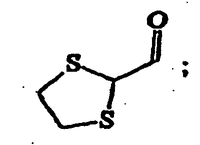
R₃ ist CH₂(p-F)Ph, Z ist H, Z₁ ist 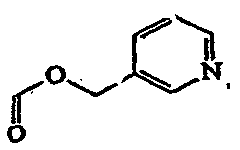 R₄₁ ist CH(CH₃)₂ und R₄₄ ist 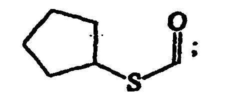
R₃ ist CH₂(p-F)Ph, Z ist H, Z₁ ist 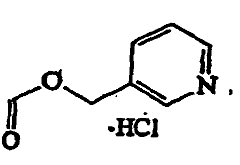 R₄₁ ist CH(CH₃)₂ und R₄₄ ist 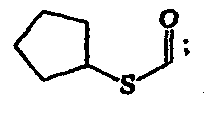
R₃ ist CH₂(p-F)Ph, Z ist H, Z₁ ist 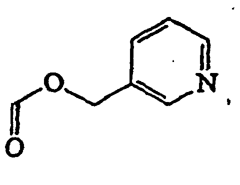 R₄₁ ist CH(CH₃)₂ und R₄₄ ist 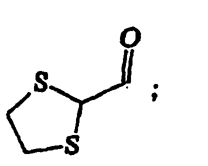
R₃ ist CH₂(p-F)Ph, Z ist H, Z₁ ist CO₂CH₂CH₃, R₄₁ ist CH(CH₃)₂ und R₄₄ ist 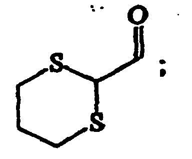
R₃ ist CH₂(p-F)Ph, Z ist H, Z₁ ist 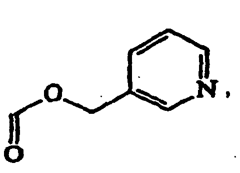 R₄₁ ist CH(CH₃)₂ und R₄₄ ist 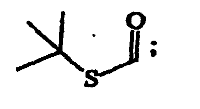
R₃ ist CH₂(p-F)Ph, Z ist H, Z₁ ist CO₂CH₂CH₂OH, R₄₁ ist CH(CH₃)₂ und R₄₄ ist 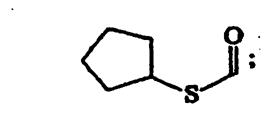 und
R₃ ist CH₂(p-F)Ph, Z ist H, Z₁ ist CO₂CH₂CH₃, R₄₁ ist CH(CH₃)₂ und R₄₄ ist 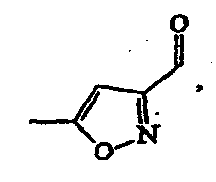
oder ein pharmazeutisch annehmbares Prodrug, Salz, aktiver Metabolit oder Solvat davon.

28. Zusammensetzung, umfassend wenigstens eine Verbindung der Formel II: 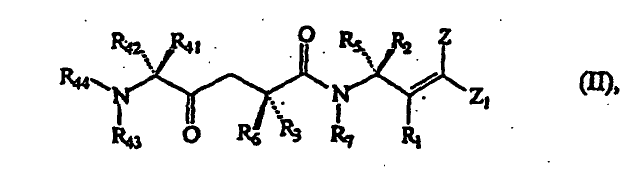 worin R₁, R₅, R₆, R₇, R₄₂, R₄₃ und Z H sind, R₃ CH₂Ph ist, R₂ CH₂CH₂C(O)NH₂ ist,
R₄₁ CH₂CH(CH₃)₂ ist, Z₁ CO₂CH₂CH₃ ist und R₄₄ 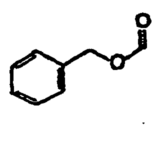 ist,
oder ein pharmazeutisch annehmbares Prodrug, Salz, aktiven Metaboliten oder Solvat davon,
und wenigstens eine Verbindung der Formel III: 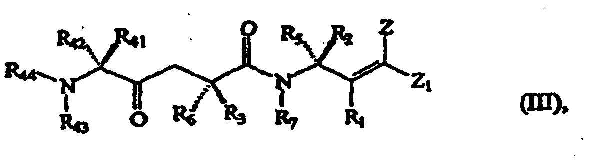 worin R₁, R₅, R₆, R₇, R₄₂, R₄₃ und Z H sind, R₃ CH₂Ph ist, R₂ CH₂CH₂C(O)NH₂ ist,
R₄₁ CH₂CH(CH₃)₂ ist, Z₁ CO₂CH₂CH₃ ist und R₄₄ 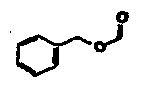 ist, oder ein pharmazeutisch annehmbares Prodrug, Salz, aktiven Metaboliten oder Solvat davon.

29. Pharmazeutische Zusammensetzung umfassend:
(a) eine therapeutisch wirksame Menge von wenigstens einer Verbindung, wie sie in Anspruch 1 definiert ist, oder eines pharmazeutisch annehmbaren Prodrugs, Salzes, aktiven Metaboliten oder Solvats davon; und
(b) einen pharmazeutisch annehmbaren Träger, ein pharmazeutisch annehmbares Verdünnungsmittel, Vehikel oder Excipiens.

30. Verwendung der Verbindung nach Anspruch 1 oder eines pharmazeutisch annehmbaren Prodrugs, Salzes, aktiven Metaboliten oder Solvats davon für die Herstellung eines Arzneimittels für die Behandlung des Erkrankungszustands von Säugern, der durch eine picomavirale Protease-Aktivität vermittelt wird.

31. Verwendung der Verbindung nach Anspruch 1 oder eines pharmazeutisch annehmbaren Prodrugs, Salzes, aktiven Metaboliten oder Solvats davon für die Herstellung eines Arzneimittels zum Hemmen der Aktivität einer picomaviralen 3C-Protease.

32. Verfahren zum Hemmen der Aktivität einer picomaviralen 3C-Protease in vitro, umfassend:
Inkontaktbringen der picornaviralen 3C-Protease mit einer wirksamen Menge von wenigstens einer Verbindung, wie sie in Anspruch 1 definiert ist, oder eines pharmazeutisch annehmbaren Prodrugs, Salzes, aktiven Metaboliten oder Solvats davon.

33. Verwendung der Verbindung nach Anspruch 1 oder eines pharmazeutisch annehmbaren Prodrugs, Salzes, aktiven Metaboliten oder Solvats davon für die Herstellung eines Arzneimittels zum Hemmen der Aktivität einer rhinoviralen Protease.

34. Verfahren zum Hemmen der Aktivität einer rhinoviralen Protease in vitro, umfassend:
Inkontaktbringen der minoviralen Protease mit einer wirksamen Menge von wenigstens einer Verbindung, wie sie in Anspruch 1 definiert ist, oder eines pharmazeutisch annehmbaren Prodrugs, Salzes, aktiven Metaboliten oder Solvats davon.

35. Verbindung nach Anspruch 1 oder ein pharmazeutisch annehmbares Prodrug, Salz, aktiver Metabolit oder Solvat davon, worin die antipicomavirale Aktivität eine antirhinovirale Aktivität ist.

36. Verbindung nach Anspruch 1 oder ein pharmazeutisch annehmbares Prodrug, Salz, aktiver Metabolit oder Solvat davon, worin die antipicomavirale Aktivität eine anticoxsackievirale Aktivität ist.

## Revendications

1. Composé de formule (I): 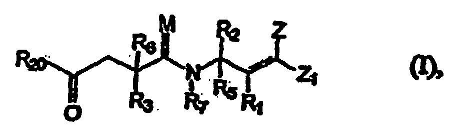 dans laquelle
M est O ou S;
R₁ est H, F, un groupe alkyle, OH, SH ou un groupe O-alkyle;
R₂ et R₅ sont choisis indépendamment parmi H, 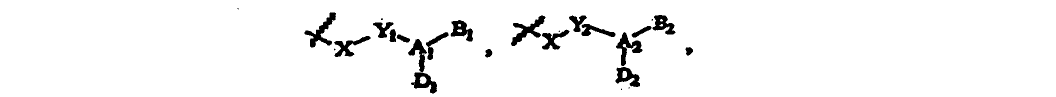 ou un groupe alkyle, où ledit groupe alkyle est différent de 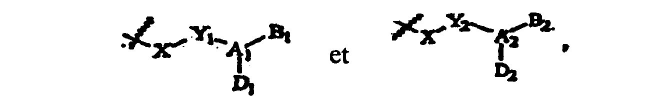 à condition que l'un au moins des radicaux R₂ ou R₅ soit 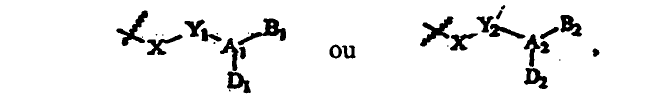 et dans laquelle, lorsque R₂ ou R₅ est 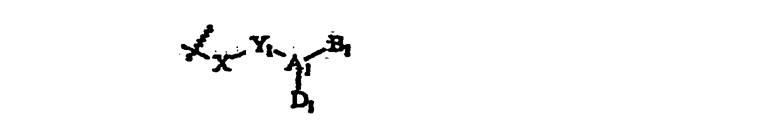
X est =CH ou =CF et Y₁ est =CH ou =CF,
ou X et Y₁, conjointement avec Q', forment un noyau à trois chaînons dans lequel Q' est -C(R₁₀)(R₁₁)- ou -O-, X est -CH- ou -CF-, et Y₁ est -CH-, -CF- ou -C(alkyle)-, où R₁₀ et R₁₁ sont indépendamment H, un halogène ou un groupe alkyle ou bien, conjointement avec l'atome de carbone auquel ils sont fixés, forment un groupe cycloalkyle ou un groupe hétérocycloalkyle,
ou X est -CH₂-, -CF₂-, -CHF- ou -S-, et Y₁ est -O-, -S-, -NR₁₂-, -C(R₁₃)(R₁₄)-, -C(O)-, -C(S)- ou -C(CR₁₃R₁₄)-,
où R₁₂ est H ou un groupe alkyle, et R₁₃ et R₁₄ sont indépendamment H, F ou un groupe alkyle ou, conjointement avec les atomes auxquels ils sont liés, forment un groupe cycloalkyle ou un groupe hétérocycloalkyle;
A₁ est C, CH, CF, S, P, Se, N, NR₁₅, S(O), Se(O), P-OR₁₅ ou P-NR₁₅R₁₆, où R₁₅ et R₁₆ sont indépendamment un groupe alkyle, un groupe cycloalkyle, un groupe hétérocycloalkyle, un groupe aryle ou un groupe hétéroaryle ou bien, conjointement avec l'atome auquel ils sont liés, forment un groupe hétérocycloalkyle;
D₁ est un groupement portant une seule paire d'électrons capable de former une liaison hydrogène; et B₁ est H, F, un groupe alkyle, un groupe cycloalkyle, un groupe hétérocycloalkyle, un groupe aryle, un groupe hétéroaryle, -OR₁₇, -SR₁₇, -NR₁₇R₁₈, -NR₁₉NR₁₇R₁₈ ou -NR₁₇OR₁₈,
où R₁₇, R₁₈ et R₁₉ sont indépendamment H, un groupe alkyle, un groupe cycloalkyle, un groupe hétérocycloalkyle, un groupe aryle, un groupe hétéroaryle ou un groupe acyle;
et à condition que, lorsque D₁ est le groupement ≡N portant une seule paire d'électrons capable de former une liaison hydrogène, B₁ n'existe pas; et que, lorsque A₁ est un carbone sp³, B₁ ne soit pas -NR₁₇R₁₈ lorsque D₁ est le groupement -NR₂₅R₂₆ portant une seule paire d'électrons capable de former une liaison hydrogène, où R₂₅ et R₂₆ sont indépendamment H, un groupe allyle, un groupe cycloalkyle, un groupe hétérocycloalkyle, un groupe aryle ou un groupe hétéroaryle;
et dans laquelle D₁-A₁-B₁ forme éventuellement un groupe nitro où A₁ est N;
et par ailleurs dans laquelle, lorsque R₂ ou R₅ est 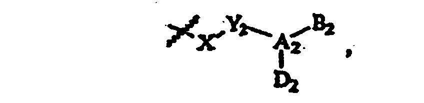
X est =CH ou =CF et Y₂ est =C, =CH ou =CF,
ou X et Y₂, conjointement avec Q', forment un noyau à trois chaînons dans lequel Q' est -C(R₁₀)(R₁₁)- ou -O-, X est -CH- ou -CF-, et Y₂ est -CH-, -CF- ou -C(alkyle)-, où R₁₀ et R₁₁ sont indépendamment H, un halogène ou un groupe alkyle ou, conjointement avec les atomes de carbone auxquels ils sont fixés, forment un groupe cycloalkyle ou un groupe hétérocycloalkyle,
ou X est -CH₂-, -CF₂-, -CHF- ou -S-, et Y₂ est -O-, -S-, -N(R'₁₂)-, -C(O)-, -C(R'₁₃)(R'₁₄)-, -C(S)- ou -C(CR'₁₃R'₁₄)-,
où R'₁₂ est H, un groupe alkyle, un groupe cycloalkyle, un groupe hétérocycloalkyle, un groupe aryle, un groupe hétéroaryle, -OR'₁₃, -NR'₁₃R'₁₄, -C(O)-R'₁₃, -SO₂R'₁₃ ou -C(S)R'₁₃, et R'₁₃ et R'₁₄, sont indépendamment H, F ou un groupe alkyle, un groupe cycloalkyle, un groupe hétérocycloalkyle, un groupe aryle ou un groupe hétéroaryle ou, conjointement avec l'atome auquel ils sont fixés, forment un groupe cycloalkyle ou un groupe hétérocycloalkyle;
A₂ est C, CH, CF, S, P, Se, N, NR₁₅, S(O), Se(O), P-OR₁₅ ou P-NR₁₅R₁₆,
où R₁₅ et R₁₆ sont indépendamment un groupe alkyle, un groupe cycloalkyle, un groupe hétérocycloalkyle, un groupe aryle ou un groupe hétéroaryle ou, conjointement avec l'atome auquel ils sont liés, forment un groupe hétérocycloalkyle;
D₂ est un groupement portant une seule paire d'électrons capable de former une liaison hydrogène; et
B₂ est H, F, un groupe alkyle, un groupe cycloalkyle, un groupe hétérocycloalkyle, un groupe aryle, un groupe hétéroaryle, -OR₁₇, -SR₁₇, -NR₁₇R₁₈, -NR₁₉NR₁₇R₁₈ ou -NR₁₇OR₁₈,
où R₁₇, R₁₈, et R₁₉ sont indépendamment H, un groupe alkyle, un groupe cycloalkyle, un groupe hétérocycloalkyle, un groupe aryle, un groupe hétéroaryle ou un groupe acyle;
et par ailleurs dans laquelle toute combinaison de Y₂, A₂, B₂ et D₂ peut éventuellement former un groupe cycloalkyle, un groupe hétérocycloalkyle, un groupe aryle ou un groupe hétéroaryle,
R₃ et R₆ sont indépendamment H, F, un groupe alkyle, un groupe cycloalkyle, un groupe hétérocycloalkyle, un groupe aryle, un groupe hétéroaryle, -C(O)R₁₇, -OR₁₇, -SR₁₇, -NR₁₉NR₁₇R₁₈ ou -NR₁₇OR₁₈,
où R₁₇, R₁₈ et R₁₉ sont indépendamment H, un groupe alkyle, un groupe cycloalkyle, un groupe hétérocycloalkyle, un groupe aryle, un groupe hétéroaryle ou un groupe acyle;
ou, R₃ et R₆, conjointement avec l'atome de carbone auquel ils sont fixés, forment un groupe cycloalkyle ou un groupe hétérocycloalkyle;
R₇ est H, un groupe alkyle, un groupe cycloalkyle, un groupe hétérocycloalkyle, un groupe aryle, un groupe hétéroaryle, -OR₁₇, -SR₁₇, -NR₁₇R₁₈, -NR₁₉NR₁₇R₁₈ ou -NR₁₇OR₁₈,
où R₁₇, R₁₈, et R₁₉ sont indépendamment H, un groupe alkyle, un groupe cycloalkyle, un groupe hétérocycloalkyle, un groupe aryle, un groupe hétéroaryle ou un groupe acyle;
ou R₇, conjointement avec R₃ ou R₆ et les atomes auxquels ils sont fixés, forme un groupe hétérocycloalkyle;
R₂₀ est H ou un groupe hydroxy, un groupe alkyle, un groupe oxo, un groupe cycloalkyle, un groupe hétérocycloalkyle, un groupe aryle, un groupe hétéroaryle, un groupe acyle, un groupe sulfonyle, un groupe mercapto, un groupe alkylthio, un groupe alcoxy, un groupe carboxy, un groupe amino, un groupe alkylamino, un groupe dialkylamino, un groupe carbamoyle, un groupe arylthio, un groupe hétéroarylthio, -OR₁₇, -SR₁₇, -NR₁₇R₁₈, -NR₁₉NR₁₇R₁₈ ou -NR₁₇OR₁₈, où R₁₇, R₁₈ et R₁₉ sont indépendamment H, un groupe alkyle, un groupe cycloalkyle, un groupe hétérocycloalkyle et un groupe aryle, un groupe hétéroaryle, ou un groupe acyle; et
Z et Z₁ sont indépendamment H, F, un groupe alkyle, un groupe cycloalkyle, un groupe hétérocycloalkyle, un groupe aryle, un groupe hétéroaryle, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), PO(NR₂₁R₂₂)(NR₂₃R₂₄), -C(O)NR₂₁NR₂₂R₂₃ ou -C(S)NR₂₁NR₂₂R₂₃,
où R₂₁, R₂₂, R₂₃ et R₂₄ sont indépendamment H, un groupe alkyle, un groupe cycloalkyle, un groupe hétérocycloalkyle, un groupe aryle, un groupe hétéroaryle, un groupe acyle où un groupe thioacyle, ou où deux quelconques des radicaux R₂₁, R₂₂, R₂₃ et R₂₄, conjointement avec le ou les atome(s) au(x)quel(s) ils sont liés, forment un groupe hétérocycloalkyle;
ou Z₁, tel que défini ci-dessus, conjointement avec R₁ tel que défini ci-dessus, et les atomes auxquels Z₁ et R₁ sont liés, forment un groupe cycloalkyle ou hétérocycloalkyle,
ou Z et Z₁, tous deux tels que définis ci-dessus, conjointement avec les atomes auxquels ils sont liés, forment un groupe cycloalkyle ou hétérocycloalkyle;
ou précurseur de médicament, sel, métabolite actif ou solvate pharmaceutiquement acceptable de ce composé.

2. Composé selon la revendication 1, dans lequel R₁ est H ou F ou précurseur de médicament, sel, métabolite actif ou solvate pharmaceutiquement acceptable de ce composé.

3. Composé selon la revendication 1, dans lequel R₂₀ est H, un groupe alkyle, un groupe cycloalkyle, un groupe hétérocycloalkyle, un groupe aryle, un groupe hétéroaryle, -OR₁₇, -SR₁₇, -NR₁₇R₁₈, -NR₁₉NR₁₇R₁₈ ou -NR₁₇OR₁₈, où R₁₇, R₁₈, et R₁₉ sont indépendamment H, un groupe alkyle, un groupe cycloalkyle, un groupe hétérocycloalkyle, un groupe aryle, un groupe hétéroaryle ou un groupe acyle, ou précurseur de médicament, sel, métabolite actif ou solvate pharmaceutiquement acceptable de ce composé.

4. Composé selon la revendication 3, dans lequel R₂₀ est le groupe alkyle -C(R₄₁)(R₄₂)NR₄₃R₄₄, dans lequel
R₄₁ et R₄₂ sont indépendamment H, un groupe alkyle, un groupe cycloallryle, un groupe hétérocycloalkyle, un groupe aryle ou un groupe hétéroaryle; et
R₄₃ et R₄₄ sont indépendamment H, un groupe alkyle, un groupe cycloalkyle, un groupe hétérocycloalkyle, un groupe aryle, un groupe hétéroaryle, -NR₄₅R₄₆, -C(O)R₄₅, -C(S)R₄₅, -C(O)NR₄₅R₄₆, -C(S)NR₄₅R₄₆, -C(O)NR₄₅OR₄₆, -C(S)NR₄₅OR₄₆, -C(O)SR₄₅, - C(O)OR_{45,} -C(S)OR₄₅, -C(S)SR₄₅, -OR₄₅, -SR₄₅, -C(O)NR₄₅NR₄₆R₄₇, -C(S)NR₄₅NR₄₆R₄₇, -SOR₄₅, -SO₂R₄₅, -S(O)NR₄₅R₄₆, -S(O)NR₄₅(OR₄₆), -SO₂NR₄₅R₄₆, -SO₂NR₄₅(OR₄₆) ou -SO₃R₄₅,
où R₄₅, R₄₆ et R₄₇ sont indépendamment H, un groupe alkyle, un groupe cycloalkyle, un groupe hétérocycloalkyle, un groupe aryle ou un groupe hétéroaryle,
ou dans lequel R₄₁, R₄₂, R₄₃ et R₄₄ associés ensemble d'une manière quelconque, forment un groupe cycloalkyle ou un groupe hétérocycloalkyle;
ou précurseur de médicament, sel, métabolite actif ou solvate pharmaceutiquement acceptable de ce composé.

5. Composé selon la revendication 4, dans lequel l'un au moins des radicaux R₄₃ ou R₄₄ est -C(O)SR₄₅ ou -C(O)OR₄₅, ou précurseur de médicament, sel, métabolite actif ou solvate pharmaceutiquement acceptable de ce composé.

6. Composé selon la revendication 5, dans lequel R₄₅ est un groupe alkyle, un groupe cycloalkyle, un groupe aryle, un groupe hétérocycloalkyle ou un groupe hétéroaryle, ou précurseur de médicament, sel, métabolite actif ou solvate pharmaceutiquement acceptable de ce composé.

7. Composé selon la revendication 6, dans lequel R₄₅ est un groupe alkyle en C₁-C₁₀ ou un groupe cycloalkyle, ou précurseur de médicament, sel, métabolite actif ou solvate pharmaceutiquement acceptable de ce composé.

8. Composé selon la revendication 1, dans lequel l'un au moins des radicaux R₂ ou R₅ est 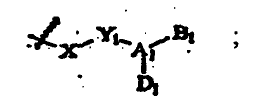 ou précurseur de médicament, sel, métabolite actif ou solvate pharmaceutiquement acceptable de ce composé.

9. Composé selon la revendication 8, dans lequel D₁ est -OR₂₅, =O, =S, ≡N, =NR₂₅ ou -NR₂₅R₂₆, dans lequel R₂₅ et R₂₆ sont indépendamment H, un groupe alkyle, un groupe cycloalkyle, un groupe hétérocycloalkyle, un groupe aryle ou un groupe hétéroaryle ou, conjointement avec l'atome d'azote auquel ils sont liés, forment un groupe hétérocycloalkyle; ou précurseur de médicament, sel, métabolite actif ou solvate pharmaceutiquement acceptable de ce composé.

10. Composé selon la revendication 9, dans lequel D₁ est =O; ou précurseur de médicament, sel, métabolite actif ou solvate pharmaceutiquement acceptable de ce composé.

11. Composé selon la revendication 8, dans lequel A₁ est C, CH, S ou S(O); ou précurseur de médicament, sel, métabolite actif ou solvate pharmaceutiquement acceptable de ce composé.

12. Composé selon la revendication 11, dans lequel A₁ est C; ou précurseur de médicament, sel, métabolite actif ou solvate pharmaceutiquement acceptable de ce composé.

13. Composé selon la revendication 8, dans lequel B₁ est NR₁₇R₁₈ où R₁₇ et R₁₈ sont indépendamment H, un groupe alkyle, un groupe cycloalkyle, un groupe hétérocycloalkyle, un groupe aryle, un groupe hétéroaryle ou un groupe acyle; ou précurseur de médicament, sel, métabolite actif ou solvate pharmaceutiquement acceptable de ce composé.

14. Composé selon la revendication 1, dans lequel l'un au moins des radicaux R₂ ou R₅ est 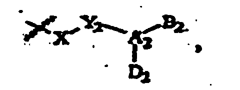 ou précurseur de médicament, sel, métabolite actif ou solvate pharmaceutiquement acceptable de ce composé.

15. Composé selon la revendication 14, dans lequel D₂ est -OR₂₅, =O, =S, ≡N, =NR₂₅ ou -NR₂₅R₂₆ où R₂₅ et R₂₆ sont indépendamment H, un groupe alkyle, un groupe cycloalkyle, un groupe hétérocycloalkyle, un groupe aryle ou un groupe hétéroaryle ou, conjointement avec le ou les atome(s) au(x)quel(s) ils sont liés, forment un groupe hétérocycloalkyle; ou précurseur de médicament, sel, métabolite actif ou solvate pharmaceutiquement acceptable de ce composé.

16. Composé selon la revendication 15, dans lequel D₂ est =O; ou précurseur de médicament, sel, métabolite actif ou solvate pharmaceutiquement acceptable de ce composé.

17. Composé selon la revendication 14, dans lequel A₂ est C, CH, S ou S(O); ou précurseur de médicament, sel, métabolite actif ou solvate pharmaceutiquement acceptable de ce composé.

18. Composé selon la revendication 17, dans lequel A₂ est C; ou précurseur de médicament, sel, métabolite actif ou solvate pharmaceutiquement acceptable de ce composé.

19. Composé selon la revendication 14, dans lequel B₂ est -NR₁₇R₁₈; où R₁₇ et R₁₈ sont indépendamment H, un groupe alkyle, un groupe cycloalkyle, un groupe hétérocycloalkyle, un groupe aryle, un groupe hétéroaryle ou un groupe acyle; ou précurseur de médicament, sel, métabolite actif ou solvate pharmaceutiquement acceptable de ce composé.

20. Composé selon la revendication 1, dans lequel A₁ est C, CH, S ou S(O) ou dans lequel A₂ est C, CH, S ou S(O); ou précurseur de médicament, sel, métabolite actif ou solvate pharmaceutiquement acceptable de ce composé.

21. Composé selon la revendication 1, dans lequel Z et Z₁ sont indépendamment H, un groupe aryle ou un groupe hétéroaryle, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -C(O)NR₂₁NR₂₂R₂₃ ou -C(S)NR₂₁NR₂₂R₂₃;
où R₂₁, R₂₂ et R₂₃ sont indépendamment H, un groupe alkyle, un groupe cycloalkyle, un groupe hétérocycloalkyle, un groupe aryle, un groupe hétéroaryle, un groupe acyle ou un groupe thioacyle, ou où deux quelconques des radicaux R₂₁, R₂₂ et R₂₃, conjointement avec le ou les atome(s) au(x)quel(s) ils sont liés, forment un groupe hétérocycloalkyle;
ou précurseur de médicament, sel, métabolite actif ou solvate pharmaceutiquement acceptable de ce composé.

22. Composé selon la revendication 1, dans lequel M est O.

23. Composé répondant à la formule X: 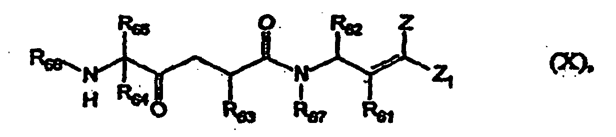 dans laquelle:
R₆₁ est H, F ou un groupe alkyle;
R₆₂ est choisi parmi un des groupements suivants: 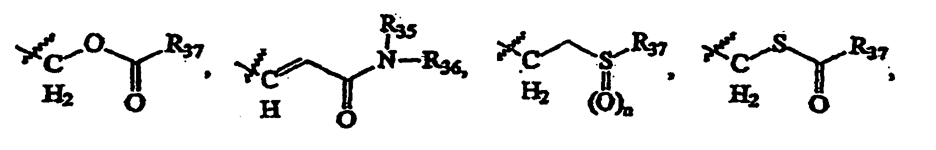 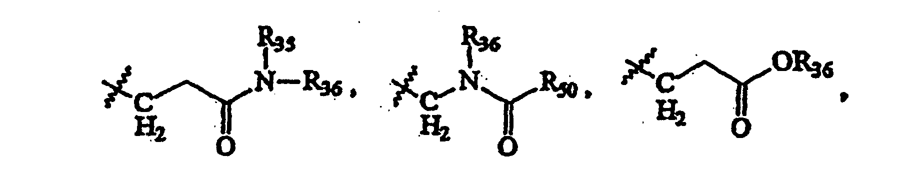 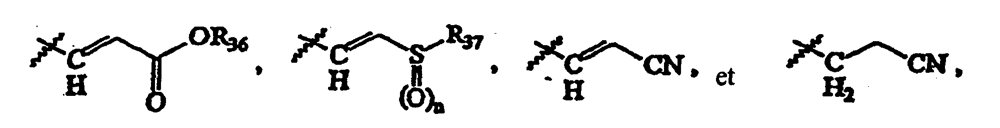
dans lesquels
R₃₅ est H, un groupe alkyle, en groupe aryle, -OR₃₈ ou -NR₃₈R₃₉,
où R₃₈ et R₃₉ sont indépendamment H, un groupe alkyle, un groupe cycloalkyle, un groupe hétérocycloalkyle, un groupe aryle, un groupe hétéroaryle ou un groupe acyle; et
R₃₆ est H ou un groupe alkyle,
ou R₃₅ et R₃₆, conjointement avec l'atome d'azote auquel ils sont fixés, forment un groupe hétérocycloalkyle ou un groupe hétéroaryle;
R₃₇ est un groupe alkyle, un groupe aryle ou -NR₃₈R₃₉, où R₃₈ et R₃₉ sont tels que définis ci-dessus;
R₅₀ est H, un groupe allyle, un groupe aryle, -OR₃₈, -SR₃₉, -NR₃₈R₃₉, -NR₄₀NR₃₈R₃₉ ou -NR₃₈OR₃₉, ou R₅₀ et R₃₆, conjointement avec les atomes auquel ils sont fixés, forment un groupe hétérocycloalkyle;
où R₃₈ et R₃₉ sont tels que définis ci-dessus, et R₄₀ est H, un groupe alkyle, un groupe cycloalkyle, un groupe hétérocycloalkyle, un groupe aryle, un groupe hétéroaryle ou un groupe acyle; et
n vaut 0, 1 ou 2;
R₆₃ est H ou un groupe alkyle;
R₆₄ est H, un groupe alkyle, un groupe cycloalkyle, un groupe hétérocycloalkyle, un groupe aryle ou un groupe hétéroaryle;
R₆₅ est H ou un groupe allyle;
R₆₆ est H, un groupe acyle, un groupe alkyle, un groupe cycloalkyle. un groupe hétérocycloalkyle, un groupe aryle, un groupe sulfonyle ou un groupe hétéroaryle;
R₆₇ est H ou un groupe alkyle;
et
Z et Z₁ sont indépendamment H, F, un groupe alkyle, un groupe cycloalkyle, un groupe hétérocycloalkyle, un groupe aryle, un groupe hétéroaryle, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), -PO(NR₂₁R₂₂)(NR₂₃R₂₄), -C(O)NR₂₁NR₂₂R₂₃ ou -C(S)NR₂₁NR₂₂R₂₃.
où R₂₁, R₂₂, R₂₃ et R₂₄ sont indépendamment H, un groupe alkyle, un groupe cycloalkyle, un groupe hétérocycloalkyle, un groupe aryle, un groupe hétéroaryle, un groupe acyle ou un groupe thioacyle, ou où deux quelconques des radicaux R₂₁, R₂₂, R₂₃ et R₂₄, conjointement avec le ou les atome(s) au(x)quel(s) ils sont liés, forment un groupe hétérocycloalkyle,
ou Z et Z₁, tous deux tels que définis ci-dessus, conjointement avec les atomes auxquels ils sont liés, forment un groupe hétérocycloalkyle;
ou précurseur de médicament, sel, métabolite actif ou solvate pharmaceutiquement acceptable de ce composé.

24. Composé selon la revendication 23, dans lequel R₆₆ est le groupe acyle -C(O)OR₆₈ ou le groupe acyle -C(O)SR₆₈, dans lequel R₆₈ est un groupe alkyle, un groupe cycloalkyle, un groupe aryle, un groupe hétérocycloalkyle ou un groupe hétéroaryle, ou précurseur de médicament, sel, métabolite actif ou solvate pharmaceutiquement acceptable de ce composé.

25. Composé selon la revendication 4, répondant à la formule II: 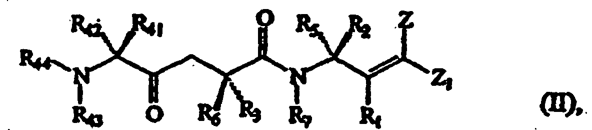 dans laquelle R₁, R₅, R₆, R₇, R₄₂, R₄₃ et Z sont H, R₂ est CH₂CH₂C(O)NH₂, et R₃, R₄₁, Z₁ et R₄₄ sont choisi parmi un des groupes suivants:
R₃ est CH₂Ph, R₄₁ est CH₂CH(CH₃)₂, Z₁ est CO₂CH₂CH₃ et R₄₄ est 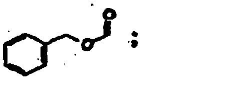
R₃ est CH₂Ph, R₄, est CH₂CH(CH₃)₂, Z₁ est CO₂CH₂CH₃ et R₄₄ est 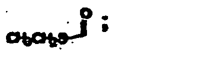
R₃ est CH₂Ph, R₄₁ est CH₂CH(CH₃)₂, Z₁ est 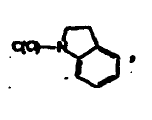 et R₄₄ est 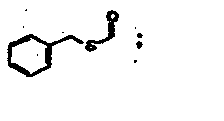
R₃ est CH₂Ph, R₄₁ est CH(CH₃)₂, Z₁ est CO₂CH₂CH₃, et R₄₄ est 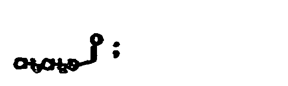
R₃ est CH₂Ph, R₄₁ est CH(CH₃)₂, Z₁ est CO₂CH₂CH₃, et R₄₄ est 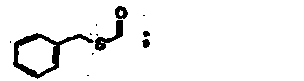
R₃ est CH₂Ph, R₄₁ est CH₂CH(CH₃)₂, Z₁ est CO₂CH₂CH₃, et R₄₄ est 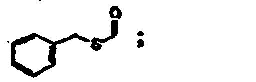
R₃ est CH₂Ph, R₄₁ est CH(CH₃)₂, Z₁ est CO₂CH₂CH₃, et R₄₄ est 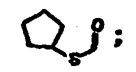
R₃ est CH₂Ph, R₄₁ est CH₂CH(CH₃)₂, Z₁ est CO₂CH₂CH₃, et R₄₄ est 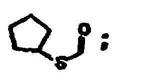
R₃ est CH₂(p-CH₃)Ph, R₄₁ est CH(CH₃)₂, Z₁ est CO₂CH₂CH₃, et R₄₄ est 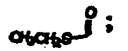 et
R₃ est CH₂(p-CH₃)Ph, R₄₁ est CH(CH₃)₂, Z₁ est CO₂CH₂CH₃, et R₄₄ est 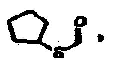
ou précurseur de médicament, sel, métabolite actif ou solvate pharmaceutiquement acceptable de ce composé.

26. Composé selon la revendication 4, répondant à la formule III: 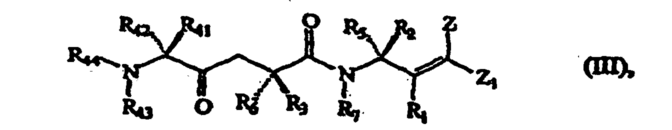 dans laquelle R₁, R₅, R₆, R₇, R₄₂, R₄₃, et Z sont H, R₃ est CH₂Ph, R₂ est CH₂CH₂C(O)NH₂,
R₄₁ est CH₂CH(CH₃)₂, Z₁ est CO₂CH₂CH₃, et R₄₄ est 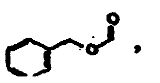 ou précurseur de médicament, sel, métabolite actif ou solvate pharmaceutiquement acceptable de ce composé.

27. Composé de formule (IV): 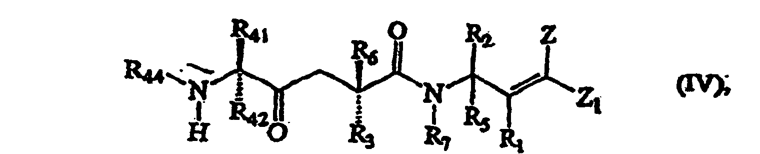 dans laquelle:
R₁, R₅, R₆, R₇ et R₄₂ sont H, R₂ est CH₂CH₂C(O)NH₂, et R₃, Z, Z₁, R₄₁ et R₄₄ sont choisis parmi un des groupes suivants:
R₃ est CH₂(p-CH₃)Ph, Z est H, Z₁ est CO₂CH₂CH₃, R₄₁ est CH₂Ph, et R₄₄ est 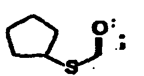
R₃ est CH₂(p-F)Ph, Z est H, Z₁ est CO₂CH₂CH₃, R₄₁ est CH(CH₃)₂, et R₄₄ est 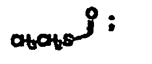
R₃ est CH₂(p-F)Ph, Z est H, Z₁ est CO₂CH₂CH₃, R₄₁ est CH(CH₃)₂, et R₄₄ est 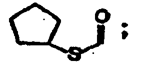
R₃ est CH₂(p-CF₃)Ph, Z est H, Z₁ est CO₂CH₂CH₃, R₄₁ est CH(CH₃)₂, et R₄₄ est 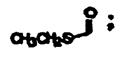
R₃ est CH₂(p-CF₃)Ph, Z est H, Z₁ est CO₂CH₂CH₃, R₄₁ est CH(CH₃)₂, et R₄₄, est 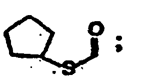
R₃ est CH₂(p-CH₃)Ph, Z et Z₁ forment ensemble 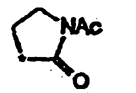 (où * indique le point de fixation et le groupe carbonyle est en cis par rapport au groupe R₁), R₄₁ est CH(CH₃)₂, et R₄₄ est 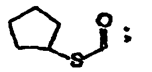
R₃ est CH₂(p-F)Ph, Z est H, Z₁ est CO₂CH₂CH₃, R₄₁ est CH₂Ph, et R₄₄ est 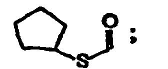
R₃ est CH₂(p-F)Ph, Z est H, Z₁ est CO₂CH₂CH₃, R₄₁ est CH₂CH(CH₃)₂, et R₄₄ est 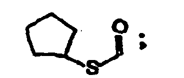
R₃ est CH₂(p-CH₃)Ph, Z est H, Z₁ est CO₂CH₂CH₃, R₄₁ est CH(CH₃)₂, et R₄₄ est 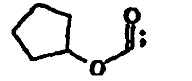
R₃ est CH₂(p-CH₃)Ph, Z est H, Z₁ est CO₂CH₂CH₃, R₄₁ est CH₂CH(CH₃)₂, et R₄₄ est 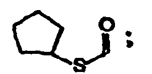
R₃ est CH₂Ph, Z est H, Z₁ est CO₂CH₂CH₃, R₄₁ est C(CH₃)₃, et R₄₄ est 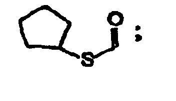
R₃ est CH₂(p-CH₃)Ph, Z est H, Z₁ est CO₂CH₂CH₃, R₄₁ est CH(CH₃)₂, et R₄₄ est 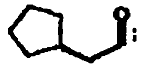
R₃ est CH₂(p-F)Ph, Z est H, Z₁ est CO₂CH₂CH₃, R₄₁ est cyclohexyle, et R₄₄ est 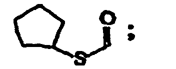
R₃ est CH₂(p-CH₃)Ph, Z est H, Z₁ est CO₂CH₂CH₃, R₄₁ est CH(CH₃)₂, et R₄₄ est 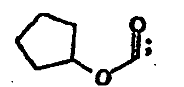
R₃ est CH₂(p-F)Ph, Z est H, Z₁ est CO₂CH₂CH₃, R₄₁ est CH(CH₃)₂, et R₄₄ est 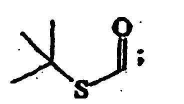
R₃ est CH₂(p-F)Ph, Z est H, Z₁ est CO₂CH₂CH₃, R₄₁ est CH(CH₃)₂, et R₄₄ est 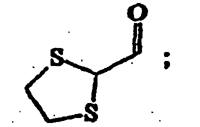
R₃ est CH₂(p-F)Ph, Z est H, Z₁ est 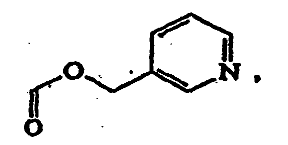
R₄₁ est CH(CH₃)₂, et R₄₄ est 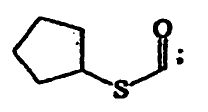
R₃ est CH₂(p-F)Ph, Z est H, Z₁ est 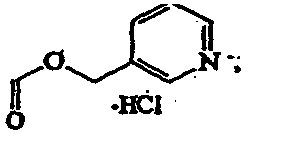
R₄₁ est CH(CH₃)₂, et R₄₄ est 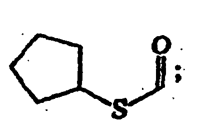
R₃ est CH₂(p-F)Ph, Z est H, Z₁ est 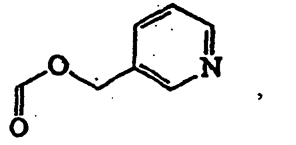
R₄₁ est CH(CH₃)₂, et R₄₄ est 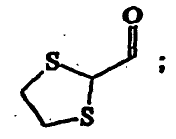
R₃ est CH₂(p-F)Ph, Z est H, Z₁ est CO₂CH₂CH₃, R₄₁ est CH(CH₃)₂, et R₄₄ est 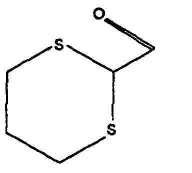
R₃ est CH₂(p-F)Ph, Z est H, Z₁ est 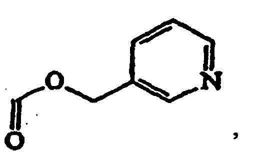
R₄₁ est CH(CH₃)₂, et R₄₄ est 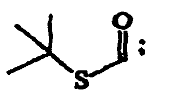
R₃ est CH₂(p-F)Ph, Z est H, Z₁ est CO₂CH₂CH₂OH, R₄₁ est CH(CH₃)₂ et R₄₄ est 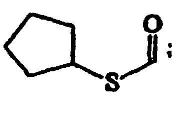 et
R₃ est CH₂(p-F)Ph, Z est H, Z₁ est CO₂CH₂CH₃, R₄₁ est CH(CH₃)₂, et R₄₄ est 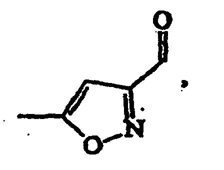
ou précurseur de médicament, sel, métabolite actif ou solvate pharmaceutiquement acceptable de ce composé.

28. Composition comprenant au moins un composé de formule II: 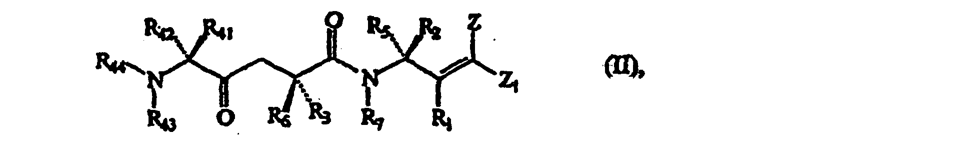 dans laquelle R₁, R₅, R₆, R₇, R₄₂, R₄₃ et Z sont H, R₃ est CH₂Ph, R₂ est CH₂CH₂C(O)NH₂,
R₄₁ est CH₂CH(CH₃)₂, Z₁ est CO₂CH₂CH₃, et R₄₄ est 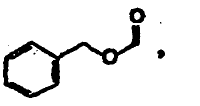 ou précurseur de médicament, sel, métabolite actif ou solvate pharmaceutiquement acceptable de ce composé,
et au moins un composé de formule III: 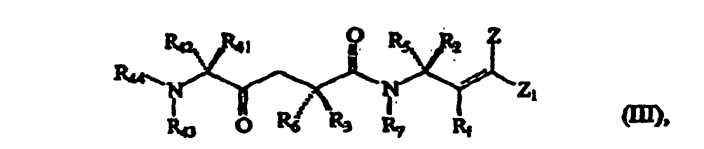 dans laquelle R₁, R₅, R₆, R₇, R₄₂, R₄₃ et Z sont H, R₃ est CH₂Ph, R₂ est CH₂CH₂C(O)NH₂,
R₄₁ est CH₂CH(CH₃)₂, Z₁ est CO₂CH₂CH₃, et R₄₄ est 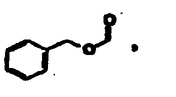 ou précurseur de médicament, sel, métabolite actif ou solvate pharmaceutiquement acceptable de ce composé.

29. Composition pharmaceutique comprenant:
(a) une quantité efficace sur le plan thérapeutique d'au moins un composé tel que défini dans la revendication 1 ou d'un précurseur de médicament, sel, métabolite actif ou solvate pharmaceutiquement acceptable de ce composé, et
(b) un support, diluant, véhicule ou excipient pharmaceutiquement acceptable.

30. Utilisation du composé selon la revendication 1 ou d'un précurseur de médicament, sel, métabolite actif ou solvate pharmaceutiquement acceptable de ce composé, pour la préparation d'un médicament destiné au traitement d'une pathologie chez un mammifère ayant pour médiateur l'activité d'une protéase picornavirale.

31. Utilisation du composé selon la revendication 1 ou d'un précurseur de médicament, sel, métabolite actif ou solvate pharmaceutiquement acceptable de ce composé, pour la préparation d'un médicament destiné à inhiber l'activité d'une protéase 3C picornavirale.

32. Procédé d'inhibition in vitro de l'activité d'une protéase 3C picornavirale, comprenant la mise en contact de la protéase 3C picornavirale avec une quantité efficace d'au moins un composé tel que défini dans la revendication 1 ou d'un précurseur de médicament, sel, métabolite actif ou solvate pharmaceutiquement acceptable de ce composé.

33. Utilisation du composé selon la revendication 1 ou d'un précurseur de médicament, sel, métabolite actif ou solvate pharmaceutiquement acceptable de ce composé pour la préparation d'un médicament destiné à inhiber l'activité d'une protéase rhinovirale.

34. Procédé d'inhibition in vitro de l'activité d'une protéase rhinovirale, comprenant la mise en contact de la protéase rhinovirale avec une quantité efficace d'au moins un composé tel que défini dans la revendication 1 ou un précurseur de médicament, sel, métabolite actif ou solvate pharmaceutiquement acceptable de ce composé.

35. Composé selon la revendication 1 ou précurseur de médicament, sel, métabolite actif ou solvate pharmaceutiquement acceptable de ce composé, dans lequel ladite activité antipicornavirale est une activité antirhinovirale.

36. Composé selon la revendication 1 ou précurseur de médicament, sel, métabolite actif ou solvate pharmaceutiquement acceptable de ce composé, dans lequel ladite activité antipicornavirale est une activité contre le virus Coxsackie.
